# EUROPEAN PATENT APPLICATION

(11) **EP 4 725 946 A1**
(43) Date of publication of application: **15.04.2026**
(21) Application number: 24818572.0
(22) Date of filing: 31.05.2024
(51) Int. Cl.: C07D 487/04, C07D 491/107, C07D 413/14, A61K 31/4545, A61P 35/00, A61P 37/02, A61P 29/00, A61P 19/02, A61P 11/00, A61P 17/00

(54) **IRAK4 DEGRADATION AGENT AND USE THEREOF**

(30) Priority: 09.06.2023 CN 202310688429; 05.01.2024 CN 202410025235; 11.04.2024 CN 202410437935
(71) Applicant: Guangzhou Unirise Pharmaceutical Co., Ltd., Guangzhou, Guangdong 510000 (CN); Guangzhou Apichope Pharmaceutical Co., Ltd., Guangzhou, Guangdong 510000 (CN); Guangzhou Runlin Pharmaceutical Technology Co., Ltd., Guangzhou, Guangdong 510000 (CN); Apichope Bio Pharmaceutical Co., Ltd., Guangzhou, Guangdong 510000 (CN)
(72) Inventor: WU, Xiwei, Guangzhou, Guangdong 510000 (CN); YU, Tianzhu, Guangzhou, Guangdong 510000 (CN); LIU, Xile, Guangzhou, Guangdong 510000 (CN); SITU, Jiajun, Guangzhou, Guangdong 510000 (CN); YANG, Wenqian, Guangzhou, Guangdong 510000 (CN); CHEN, Haijie, Guangzhou, Guangdong 510000 (CN); WANG, Hui, Guangzhou, Guangdong 510000 (CN); CHEN, Zhaomin, Guangzhou, Guangdong 510000 (CN)
(74) Representative: Michalski Hüttermann & Partner Patentanwälte mbB
(86) International application number: PCT/CN2024/096649
(87) International publication number: WO 2024/251040

(57) **Abstract**

The present invention relates to interleukin-1 receptor associated kinase 4 (IRAK4) degraders and applications thereof in the preparation of drugs for treating IRAK4 associated diseases. Specifically, the present invention relates to a compound represented by formula (I), and an isomer, an N-oxide, a hydrate, a solvate, a metabolite, a pharmaceutically acceptable salt or a prodrug thereof.

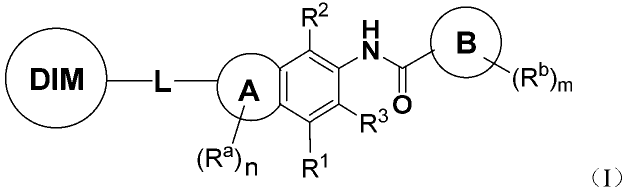

## Description

This application claims the benefit of and priority to CN202310688429.3, filed June 9, 2023; CN202410025235.X, filed January 5, 2024; CN202410437935.X, filed April 11, 2024.

### TECHNICAL FIELD OF THE INVENTION

The present invention relates to the field of medicinal chemistry. Specifically, the invention provides bifunctional compounds for the proteolytic degradation of Interleukin-1 Receptor-Associated Kinase 4 (IRAK4) and methods for treating diseases regulated by IRAK4.

### BACKGROUND OF THE INVENTION

Interleukin-1 Receptor-Associated Kinase 4 (IRAK4) is a threonine/serine protein kinase composed of 460 amino acids, containing a kinase domain and a death domain. Typically, the kinase domain comprises an N-terminal lobe and a C-terminal lobe, which converge to form the ATP-binding site. The death domain functions to bind and interact with MyD88 during protein recruitment. Additionally, the protein possesses three phosphorylation sites involved in trans-phosphorylation. IRAK4 is recognized as a key early-activating protein kinase downstream of the IL-1 receptor and Toll-like receptors (TLRs). It initiates signaling by rapidly activating IRAK1 and IRAK2, leading to innate immune responses. Furthermore, other interleukins, such as IL-18 and IL-33, rely on IRAK4 for signaling. Clinical pathology studies indicate that individuals with IRAK4 mutations exhibit protection against chronic lung diseases and inflammatory bowel disease. IRAK4 deficiency itself is non-lethal, allowing individuals to survive into adulthood with a reduced risk of infection as they age. Substantial evidence suggests that inhibiting IRAK4-mediated signaling represents a promising therapeutic approach. Consequently, IRAK4 has emerged as a significant therapeutic target, attracting extensive research and development interest.

PROTAC (Proteolysis Targeting Chimera) molecules constitute a class of bifunctional compounds capable of simultaneously binding both a target protein and an E3 ubiquitin ligase. Such compounds are recognized by the cellular proteasome, inducing the degradation of the target protein and effectively reducing its intracellular levels. By incorporating ligands that bind different target proteins into PROTAC molecules, the application of PROTAC technology for treating various diseases becomes feasible. This technology has garnered significant attention in recent years.

The specific degradation of IRAK4 can be achieved using heterobifunctional small molecules that recruit IRAK4 to an E3 ubiquitin ligase, thereby promoting its ubiquitination and proteasomal degradation. This strategy offers therapeutic opportunities for IRAK4-associated diseases such as autoimmune disorders, inflammatory diseases, and cancers.

### SUMMARY OF THE INVENTION

The present invention provides a compound, or a pharmaceutical composition thereof, which can serve as an IRAK4 degrader. The present invention further relates to the use of the compound or a pharmaceutical composition thereof for the manufacture of a medicament for treating diseases and/or disorders, wherein the medicament treats diseases and/or disorders by targeted degradation of IRAK4 by said compound.

Specifically:
In one aspect, the present invention relates to a compound, which is a compound as shown in formula (I), or an isomer, an *N*-oxide, a hydrate, a solvate, a metabolite, a pharmaceutically acceptable salt or a prodrug thereof.

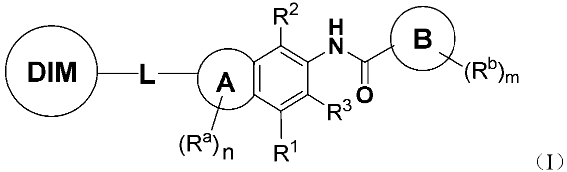

Wherein:
Ring A is an 8-13 membered spirocyclic ring having 0-3 heteroatoms independently selected from nitrogen, oxygen, and sulfur;
Ring B is phenyl, naphthyl, a 5-6 membered monocyclic heteroaryl, or a 9-10 membered bicyclic heteroaryl;
R¹, R², and R³ are each independently hydrogen, deuterium, halogen, CN, OH, NO₂, NH₂, C₁₋₆ alkyl, C₂₋₆ alkenyl, C₁₋₆ alkoxy, -(C₀₋₃ alkylene)-C₃₋₆ cycloalkyl, or -(C₀₋₃ alkylene)-3-8 membered heterocyclyl,wherein C₁₋₆ alkyl, C₂₋₆ alkenyl, C₁₋₆ alkoxy, -(C₀₋₃ alkylene)-C₃₋₆ cycloalkyl, and -(C₀₋₃ alkylene)-3-8 membered heterocyclyl are each independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, C₁₋₃ hydroxyalkyl, and C₁₋₃ haloalkyl;
Each R^{a} and R^{b} is independently hydrogen, deuterium, halogen, oxo, CN, NO₂, -R⁴, -OR^{c}, -SR^{c}, -N(R^{c})₂, -C(R^{c})₃, -S(=O)₂R^{c}, -S(=O)₂N(R^{c})₂, -S(=O)R^{c}, -S(=O)(NR^{c})R^{c}, -P(=O)(OR^{c})₂, -P(=O)(N(R^{c})₂)₂, -CF(R^{c})₂, -CF₂(R^{c}), -CF₃, -C(R^{c})₂-OR^{c}, -C(R^{c})₂-N(R^{c})₂, -C(=O)R^{c}, -C(=O)OR^{c}, or -C(=O)N(R^{c})₂; or two R^{a} groups attached to the same atom optionally join to form a C₃₋₄ cycloalkyl or 3-4 membered heterocyclyl with the atom to which they are attached;
Each R⁴ is independently C₁₋₆ alkyl, C₂₋₆ alkenyl, phenyl, C₃₋₇ cycloalkyl, 3-7 membered heterocyclyl, or 5-6 membered heteroaryl, wherein R⁴ is independently optionally substituted with 1, 2, or 3 substituents selected from deuterium, halogen, CN, OH, NO₂, NH₂, oxo, C₁₋₃ alkyl, C₁₋₃ alkoxy, C₁₋₃ haloalkoxy, and C₁₋₃ haloalkyl;
Each R^{c} is independently hydrogen, deuterium, C₁₋₆ alkyl, phenyl, C₄₋₇ cycloalkyl, 4-7 membered heterocyclyl, or 5-6 membered heteroaryl, wherein C₁₋₆ alkyl, phenyl, C₄₋₇ cycloalkyl, 4-7 membered heterocyclyl, and 5-6 membered heteroaryl are each independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, and C₁₋₃ haloalkyl;
or two R^{c} groups attached to the same atom optionally join to form a C₄₋₇ cycloalkyl, a 4-11 membered bridged bicyclic ring, or a 4-11 membered spirocyclic ring having 0-3 heteroatoms independently selected from nitrogen, oxygen, and sulfur, wherein C₄₋₇ cycloalkyl, 4-11 membered bridged bicyclic ring, and 4-11 membered spirocyclic ring are each independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, and C₁₋₃ haloalkyl;
m and n are each independently 0, 1, 2, 3, 4, 5, 6, 7, or 8;
L is C₁₋₂₀ alkylene, wherein 1, 2, 3, 4, or 5 methylene units are each independently optionally replaced by a unit selected from -CR^{d}=CR^{d}-, -C≡C-, -C(R^{d})₂-, -Cy-, -O-, -C(=O)-, and -N(R^{d})-;wherein each -Cy- is independently C₄₋₇ cycloalkyl, 4-11 membered heterocyclyl, 5-11 membered spirocyclic, 5-11 membered bridged bicyclic, phenyl, or 5-6 membered heteroaryl,and each -Cy- is independently optionally substituted with 1, 2, or 3 substituents selected from deuterium, halogen, CN, OH, NO₂, NH₂, oxo, C₁₋₃ alkyl, C₁₋₃ alkoxy, C₁₋₃ haloalkoxy, and C₁₋₃ haloalkyl;
Each R^{d} is independently hydrogen, deuterium, halogen, CN, OH, NO₂, NH₂, or C₁₋₃ alkyl;
DIM is an E3 ubiquitin ligase binding moiety. The Von Hippel-Lindau (VHL) and cereblon (CRBN) proteins are two universally expressed and biologically significant substrate recognition subunits of Cullin RING E3 ubiquitin ligase complexes. Additionally, inhibitor of apoptosis proteins (IAPs) are a family of proteins involved in suppressing cell death. The human IAP family comprises eight members, and many other organisms contain IAP homologs. IAPs contain E3 ligase-specific domains and baculovirus IAP repeat (BIR) domains, which recognize substrates and facilitate their ubiquitination. The DIM-targeting E3 ligase (VHL, CRBN, or IAP) compounds are utilized by bifunctional compounds to induce ubiquitination of IRAK4 and subsequent proteasomal degradation.

In some embodiments, ring A is: 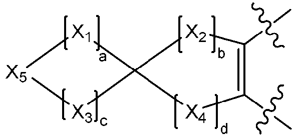 wherein:
X₁, X₂, X₃, X₄, and X₅ are each independently CH₂, -C(=O)-, NH, O, or S;
a and c are each independently 1 or 2;
b and d are each independently 0, 1, or 2, wherein b and d are not both 0, and the sum of b and d is 2, 3, or 4.

In some embodiments, each R^{a} is independently hydrogen, deuterium, halogen, oxo, CN, OH, NO₂, NH₂, CF₃, or C₁₋₆ alkyl; or two R^{a} groups on the same atom together form a C₃₋₄ cycloalkyl group.

In some embodiments, ring B is:

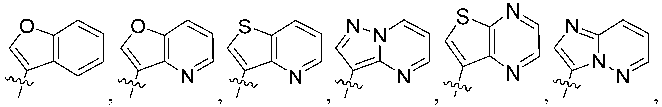

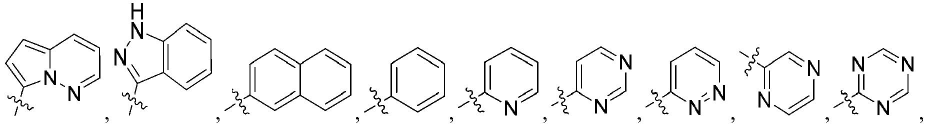

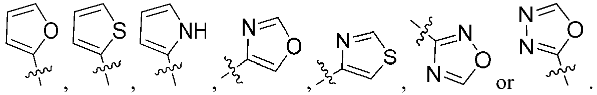

In some embodiments, wherein each R^{b} is independently hydrogen, deuterium, halogen, oxo, CN, OH, NO₂, NH₂, -COOH, C₁₋₆ alkyl, C₃₋₆ cycloalkyl, 4-6 membered heterocyclyl, or 7-8 membered bridged bicyclyl;wherein C₁₋₆ alkyl, C₃₋₆ cycloalkyl, 4-6 membered heterocyclyl, and 7-8 membered bridged bicyclyl are each independently optionally substituted with 1, 2, or 3 substituents independently selected from deuterium, halogen, OH, CN, oxo, C₁₋₃ alkyl, C₁₋₃ alkoxy, C₁₋₃ haloalkoxy, and C₁₋₃ haloalkyl.

In some embodiments, wherein each R^{b} is independently hydrogen, deuterium, halogen, CN, OH, NO₂, NH₂, -COOH, methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, or 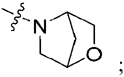 wherein methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, and 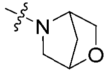 are each independently optionally substituted with 1, 2, or 3 substituents selected from hydrogen, deuterium, halogen, oxo, CN, OH, NO₂, NH₂, methyl, ethyl, methoxy, ethoxy, -CHF₂, -CF₃, -OCHF₂, and -OCF₃.

In some embodiments, wherein 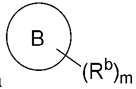 is: 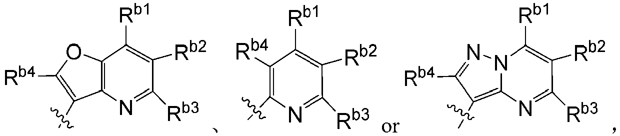 wherein:
R^{b1} and ^{Rb4} are each independently hydrogen, deuterium, halogen, CN,OH,NO₂,NH₂,CH₃,CH₂CH₃,-CF₂ or -CF₃;
R^{b2} and R^{b3} are each independently hydrogen, deuterium, halogen, CN, OH, NO₂, NH₂, -COOH, methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, or 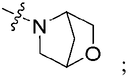 wherein methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, and 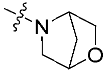 are each independently optionally substituted with 1, 2, or t3 substituents selected from hydrogen, deuterium, halogen, oxo, CN, OH, NO₂, NH₂, methyl, ethyl, methoxy, ethoxy, -CHF₂, -CF₃, -OCHF₂, and -OCF₃.

In some embodiments, wherein 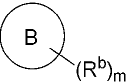 is: 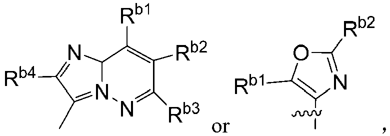 wherein:
R^{b1} and ^{Rb4} are each independently hydrogen, deuterium, halogen, CN,OH,NO₂,NH₂,CH₃,CH₂CH₃,-CF₂ or -CF₃;
R^{b2} and R^{b3} are each independently hydrogen, deuterium, halogen, CN, OH, NO₂, NH₂, -COOH, methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, or 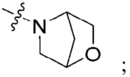 wherein methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, and 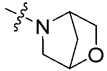 are each independently optionally substituted with 1, 2, or 3 substituents selected from hydrogen, deuterium, halogen, oxo, CN, OH, NO₂, NH₂, methyl, ethyl, methoxy, ethoxy, -CHF₂, -CF₃, -OCHF₂, and -OCF₃.

In some embodiments, wherein R¹ is hydrogen, deuterium, halogen, CN,OH,NO₂,NH₂, C₁₋₆ alkyl, C₁₋₆ alkoxy, or C₃₋₆ cycloalkyl; R² is hydrogen, deuterium, halogen, CN,OH,NO₂,NH₂, C₁₋₆ alkyl, C₁₋₆ alkoxy, or C₃₋₆ cycloalkyl.

In some embodiments, wherein R¹ is hydrogen, deuterium, halogen, CN,OH ,NO₂ ,NH₂, methyl, ethyl, n-propyl, isopropyl, methoxy, cyclopropyl, cyclobutyl, cyclopentyl, or cyclohexyl; R² is hydrogen, deuterium, halogen, CN ,OH, NO₂ ,NH₂, methyl, ethyl, n-propyl, isopropyl, methoxy, cyclopropyl, cyclobutyl, cyclopentyl, or cyclohexyl.

In some embodiments, wherein R³ is hydrogen, deuterium, halogen, CN, OH, NO₂, NH₂, C₁₋₆ alkyl, C₁₋₄ alkoxy, C₃₋₆ cycloalkyl, or a 5-6 membered heterocyclyl group, and wherein the C₁₋₆ alkyl, C₁₋₄ alkoxy, C₃₋₆ cycloalkyl, and 5-6 membered heterocyclyl groups are each independently optionally substituted with 1, 2, or 3 substituents selected from deuterium, halogen, oxo, CN, OH, NO₂, NH₂, C₁₋₃ alkyl, C1-3 alkoxy, C1-3 haloalkoxy, C₁₋₃ hydroxyalkyl, and C₁₋₃ haloalkyl.

In some embodiments, wherein R³ is hydrogen, deuterium, halogen, CN, OH, NO₂, NH₂, methyl, ethyl, isopropyl, methoxy, ethoxy, cyclopropyl, cyclobutyl, cyclopentyl, cyclohexyl, piperidinyl, piperazinyl, or morpholinyl, and wherein the methyl, ethyl, isopropyl, methoxy, ethoxy, cyclopropyl, cyclobutyl, cyclopentyl, cyclohexyl, piperidinyl, piperazinyl, and morpholinyl groups are each independently optionally substituted with 1, 2, or 3 substituents selected from deuterium, halogen, oxo, CN, OH, NO₂, NH₂, methyl, methoxy, -CH₂OH, -CHF₂, -CF₃, -CHFCH₂F, -CF₂CHF₂, -CH₂CF₃, -OCHF₂, -OCF₃, -CH₂CHF₂ and -C(CH₃)₂OH.

In some embodiments, wherein DIM moiety is: 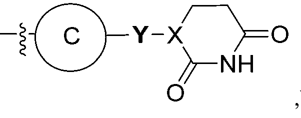 Wherein:X is CH or N; Y is bond, -CH₂-, -NH-, -O-, -C(=O)-, or -C(=O)NH-;Ring C is phenyl, a 5- 6 membered monocyclic heteroaryl, or a 9- 10 membered bicyclic heteroaryl;said Ring C is independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, and C₁₋₃ haloalkyl.

In some embodiments, wherein DIM moiety is:

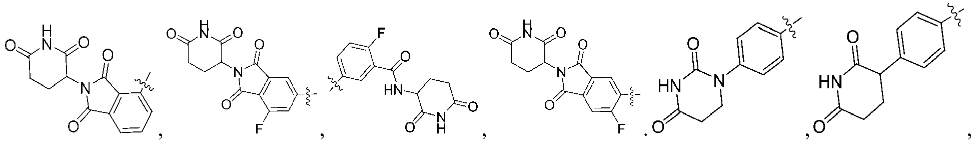

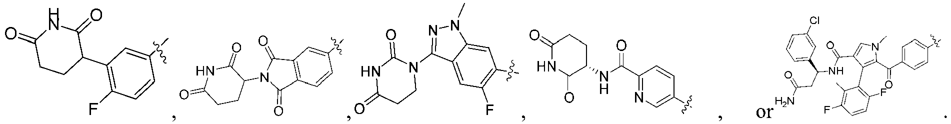

In some embodiments, wherein L is C₃₋₁₂ alkylene, wherein 1, 2 or 3 methylene units are each independently optionally replaced by a unit selected from -CR^{d} =CR^{d}-, -C≡C- ,-C(R^{d})₂-,-O-,-C(=O)-,-NH-, 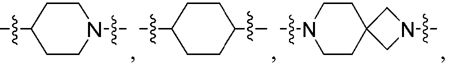 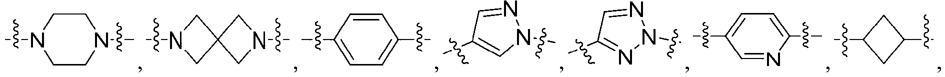 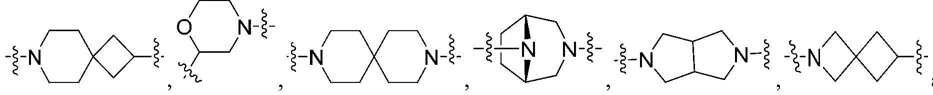 and 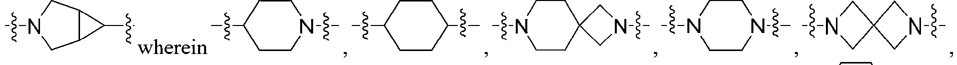 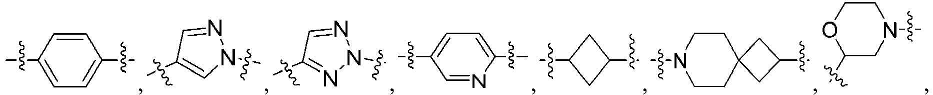 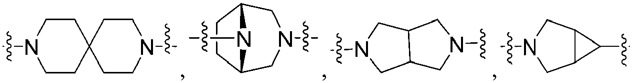 are independently optionally substituted with 1, 2, or 3 substituents selected from deuterium, halogen, CN, OH, NO₂, NH₂, oxo, methyl, ethyl, isopropyl, methoxy, isopropoxy.

In some embodiments, wherein L is C₁-₉ alkylene, wherein 1, 2 or 3 methylene units are each independently optionally replaced by a unit selected from -CR^{d}=CR^{d}, -C≡C-,-C(R^{d})₂-,-O-,-C(=O)-,-NH-, 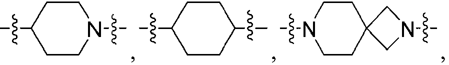 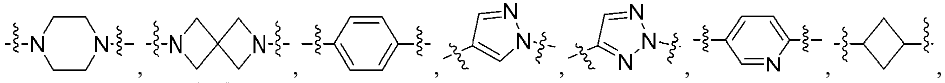 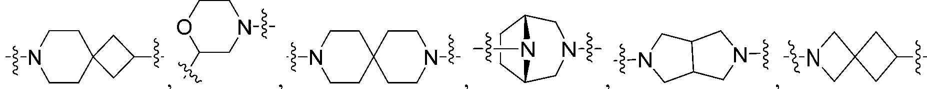 and 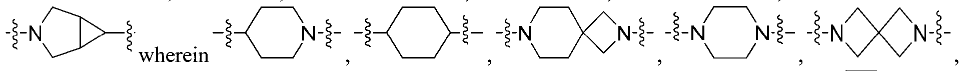 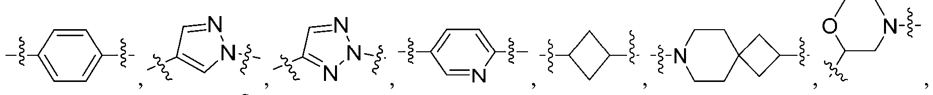 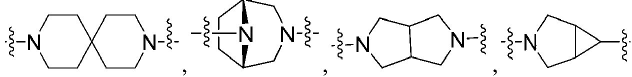 are independently optionally substituted with 1, 2, or 3 substituents selected from deuterium, halogen, CN, OH, NO₂, NH₂, oxo, methyl, ethyl, isopropyl, methoxy, isopropoxy, -CHF₂, -CF ₃, -CHFCH₂F, -CF₂CHF₂, -CH₂CF₃, -OCHF₂, and -OCF₃;

Each R^{d} is independently hydrogen, deuterium, halogen, CN, OH, NO₂, NH₂, methyl, ethyl, n-propyl, or isopropyl.

In some embodiments, wherein L is:

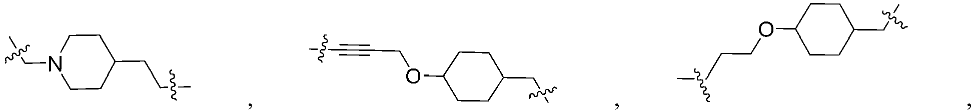

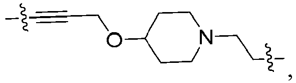

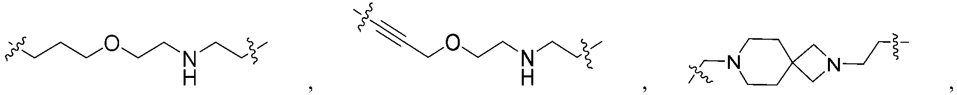

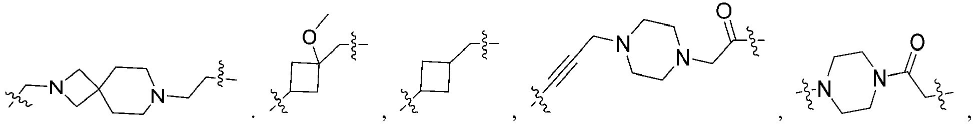

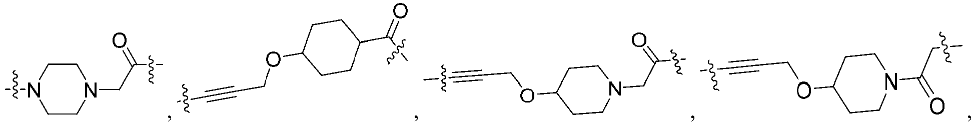

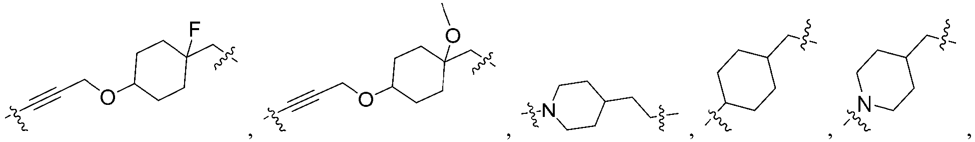

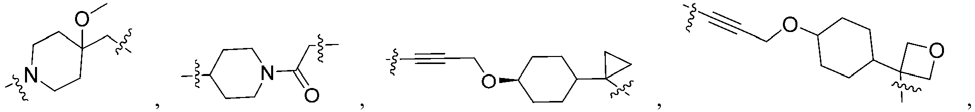

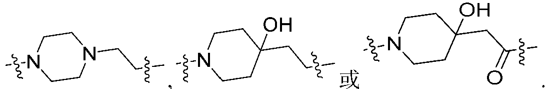

The present invention also includes some embodiments derived from arbitrary combinations of the aforementioned variables.

In some embodiments, wherein a structure represented by Formula (II), (III), (IV), (V),

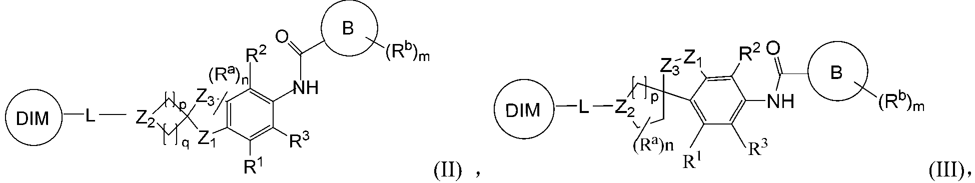

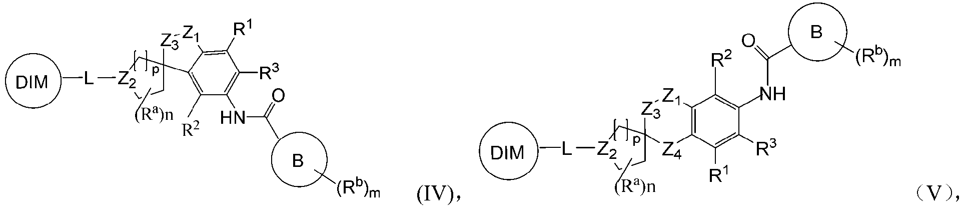

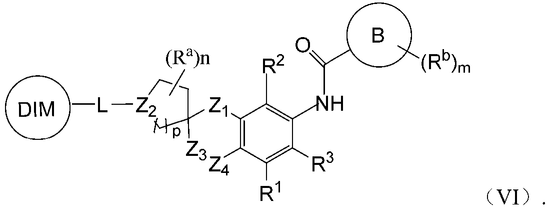

Wherein:Z₁, Z₃, and Z₄ are each independently CH₂, -C(=O)-, NH, O, or S;Z₂ is CH or N;p and q are each independently 1 or 2.

In some embodiments, A compound which is having one of the following structures, and an isomer, an N-oxide, a hydrate, a solvate, a metabolite, a pharmaceutically acceptable salt or a prodrug thereof: 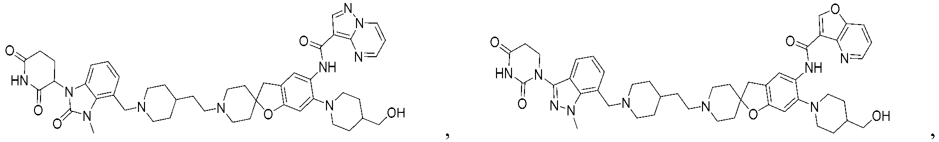 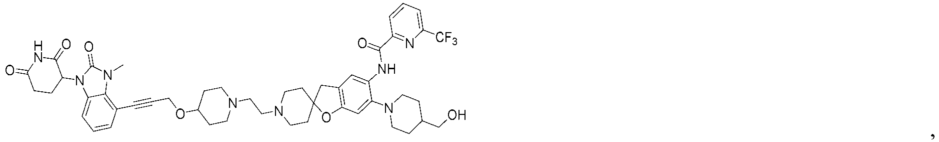 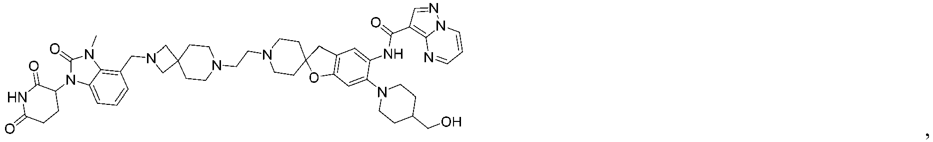 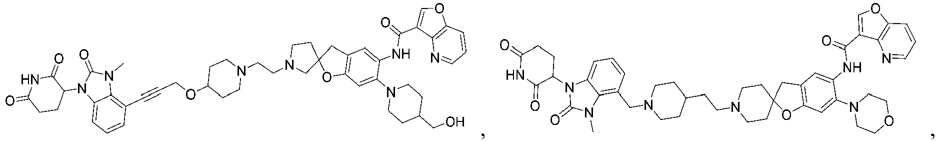 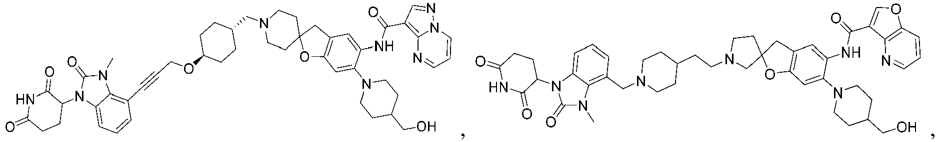 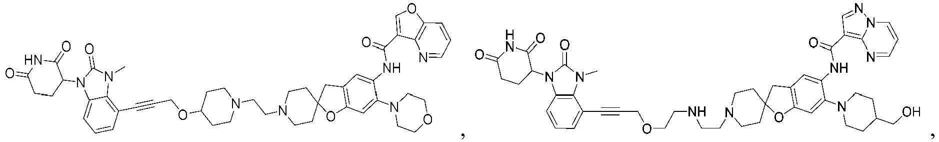 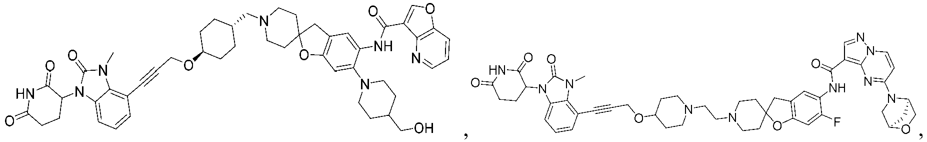 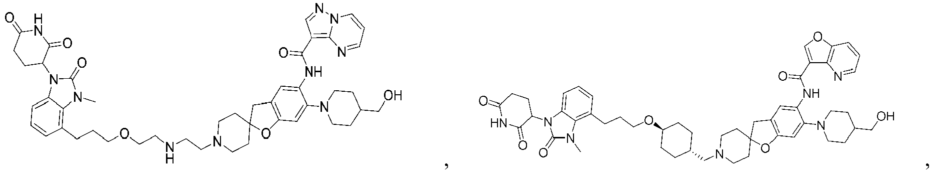 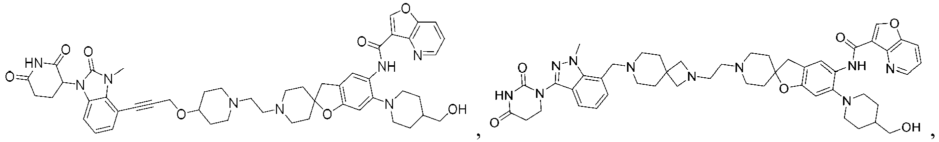 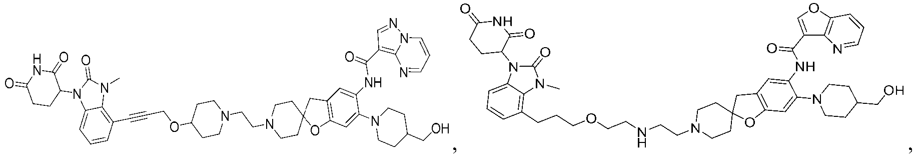 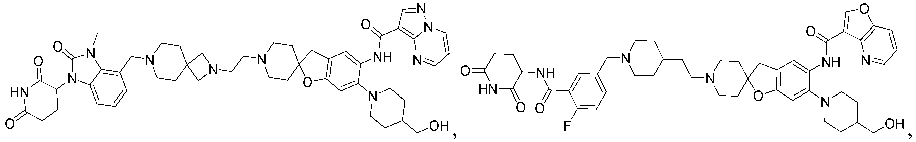 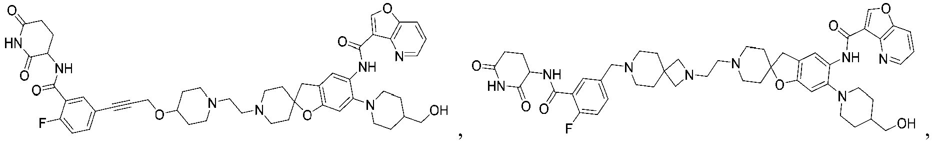 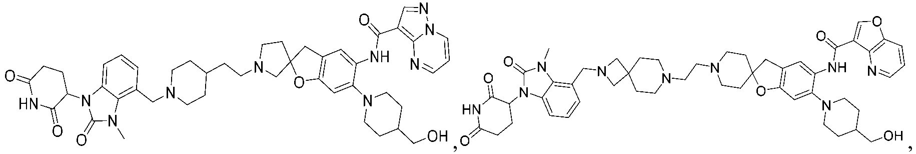 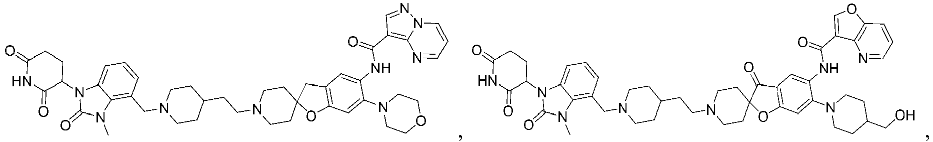 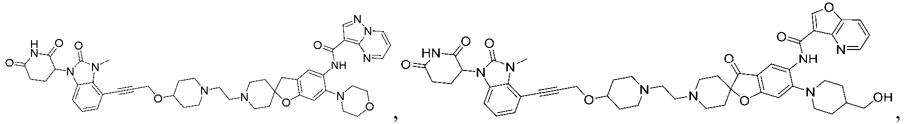 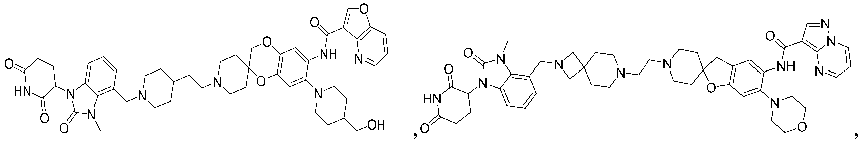 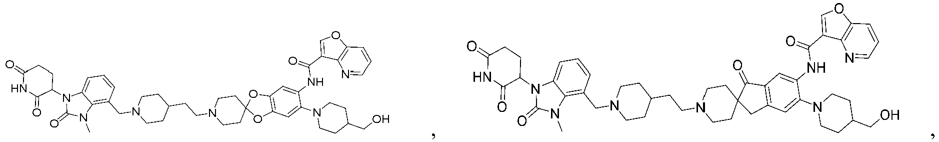 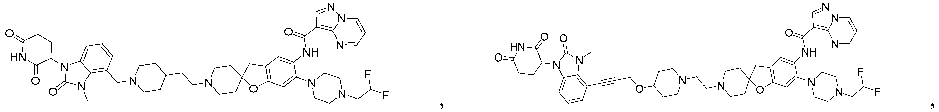 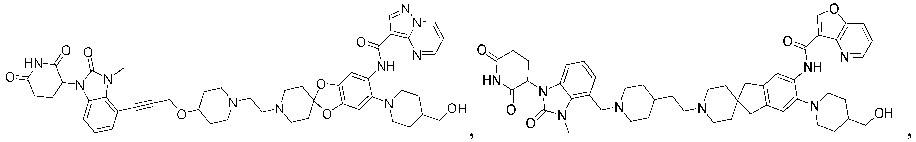 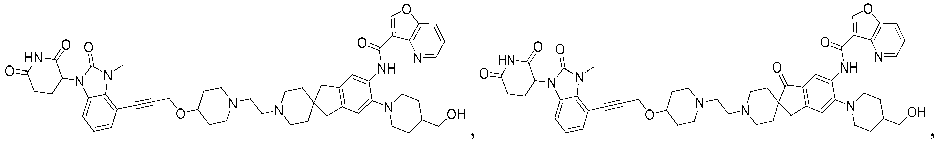 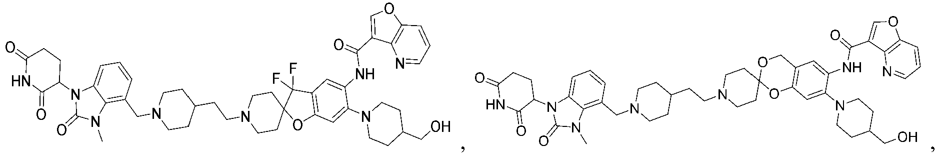 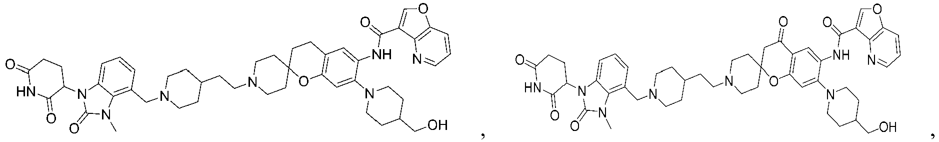 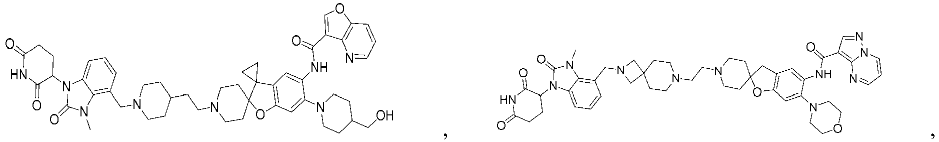 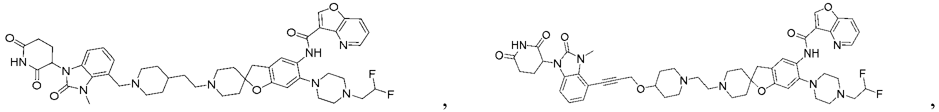 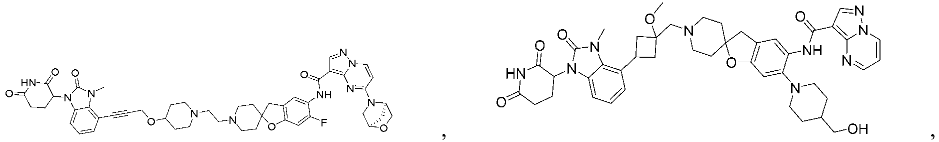 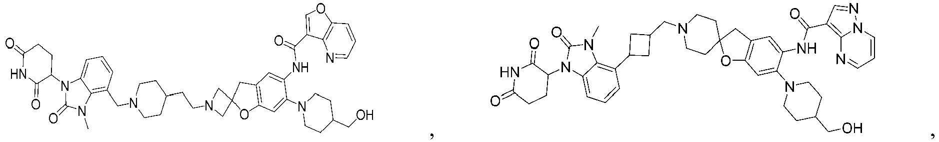 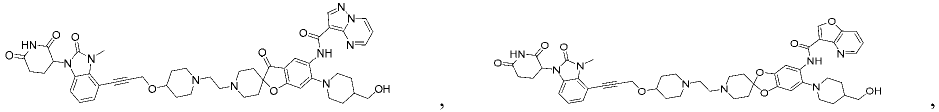 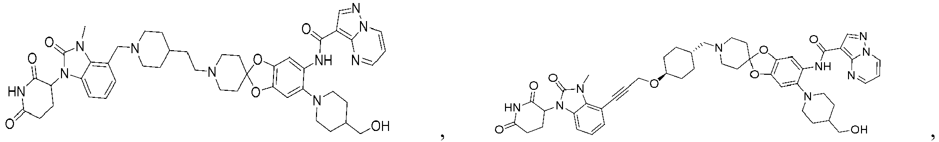 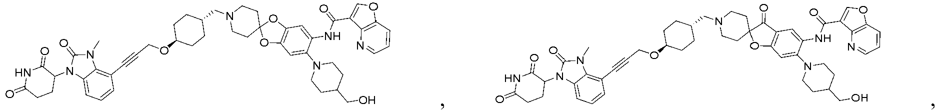 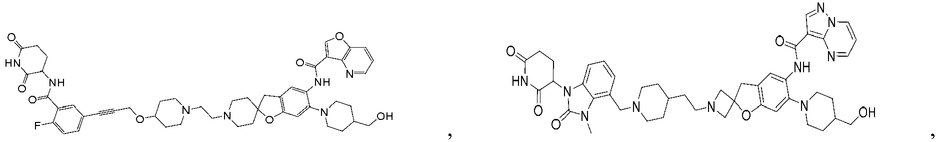 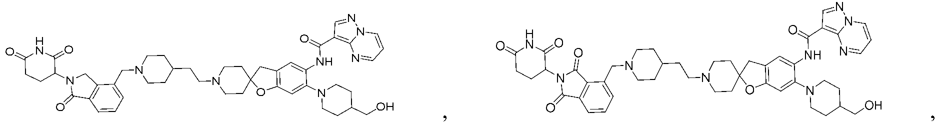 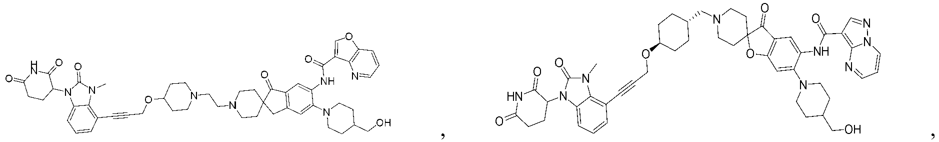 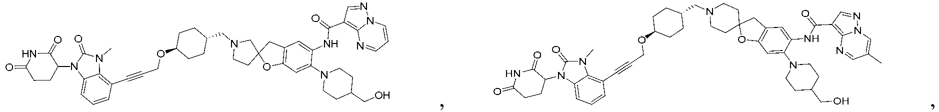 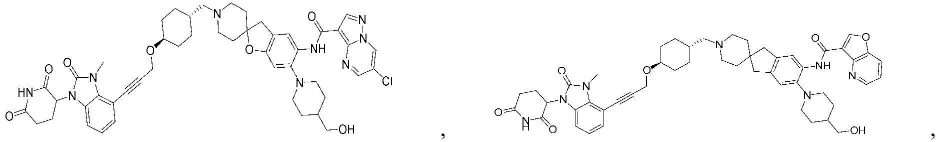 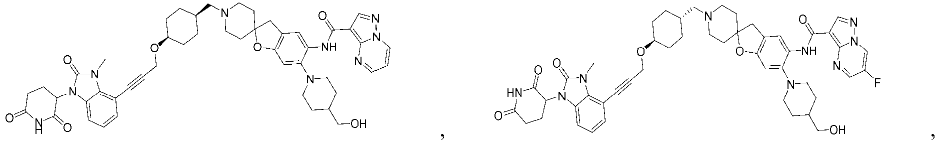 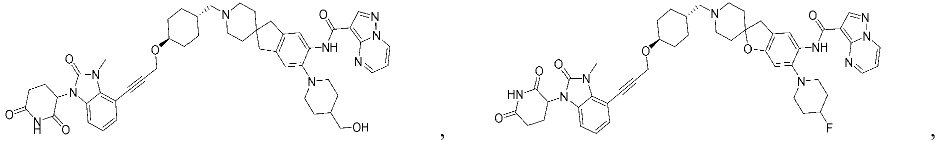 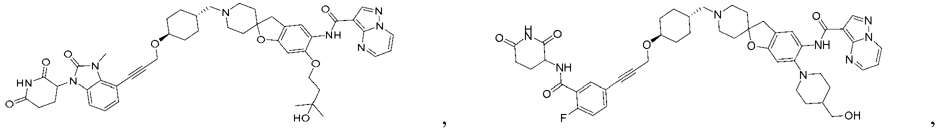 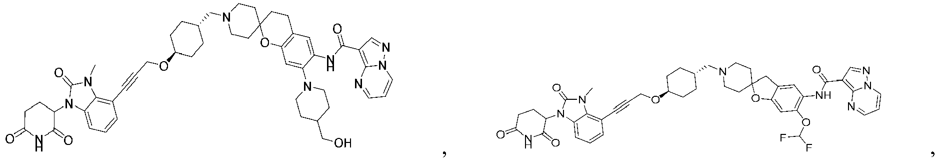 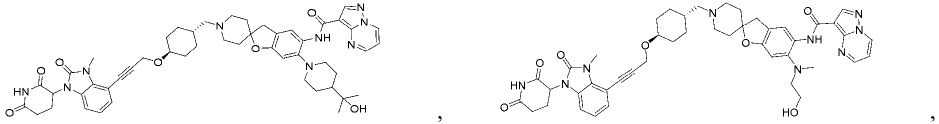 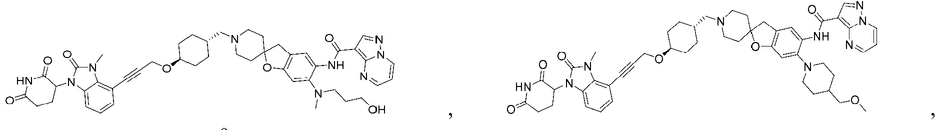 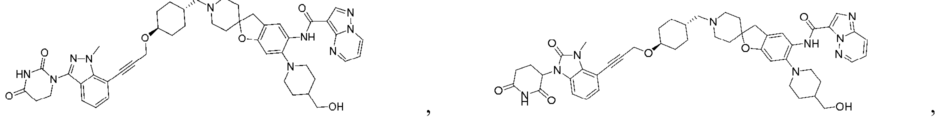 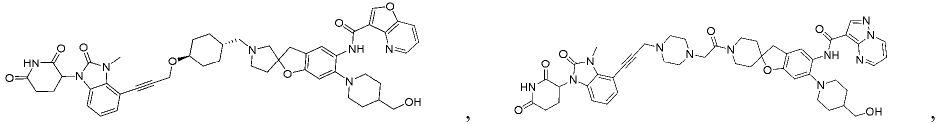 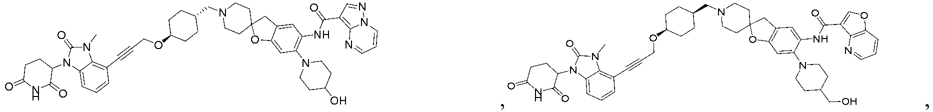 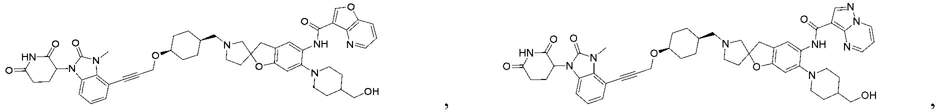 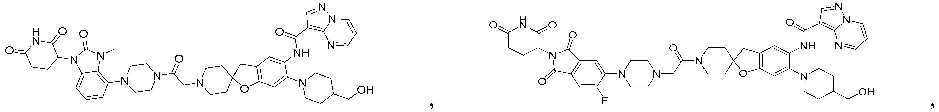 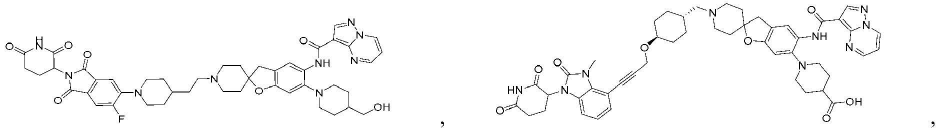 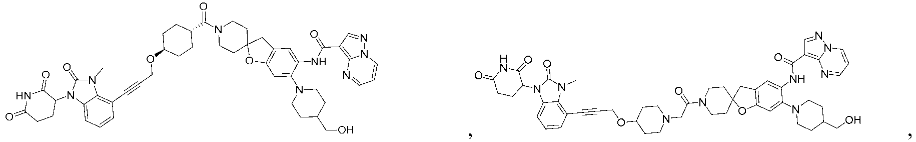 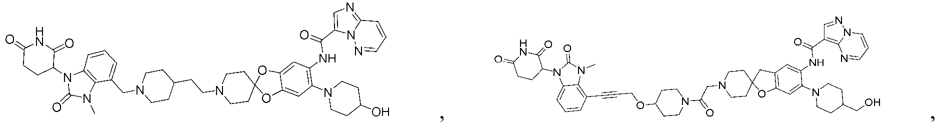 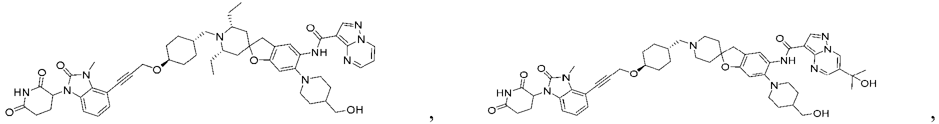 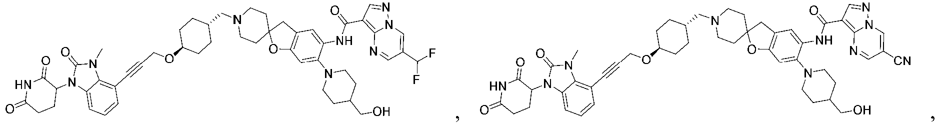 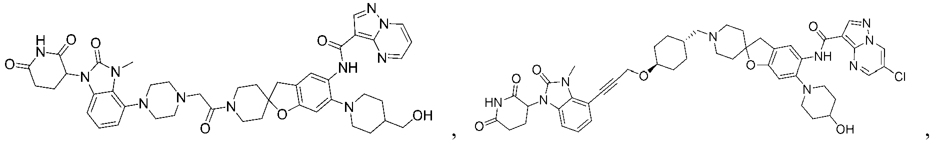 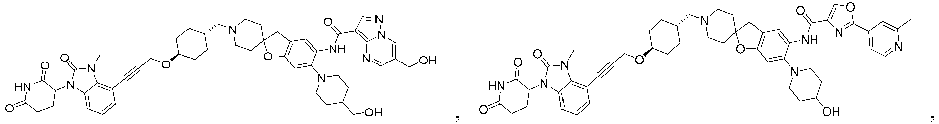 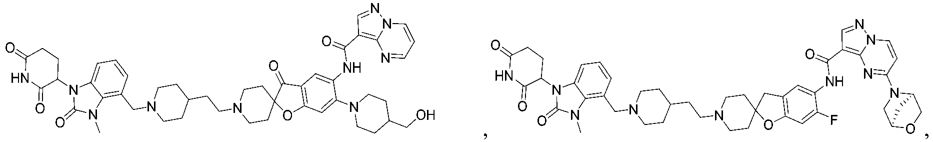 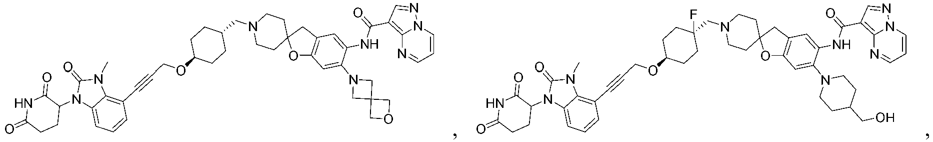 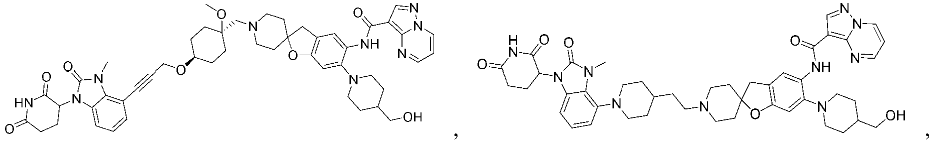 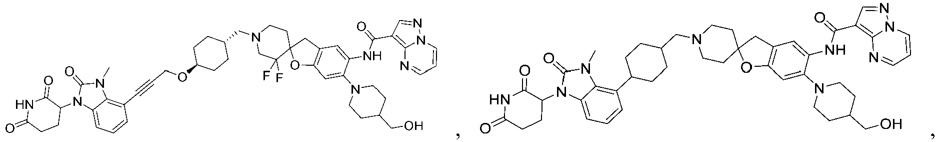 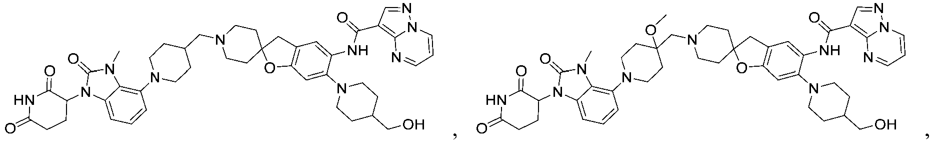 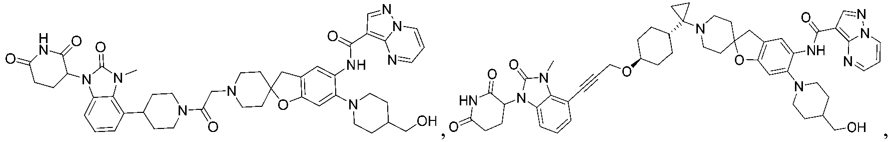 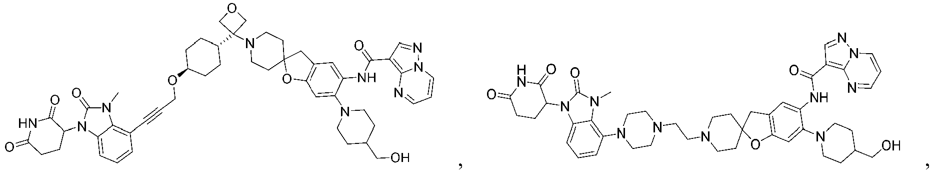 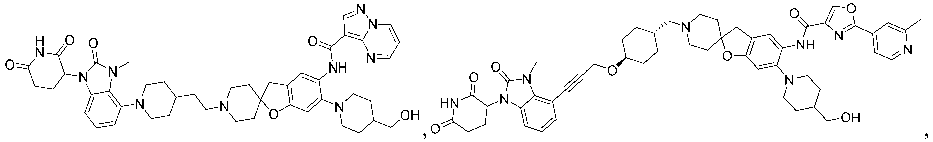 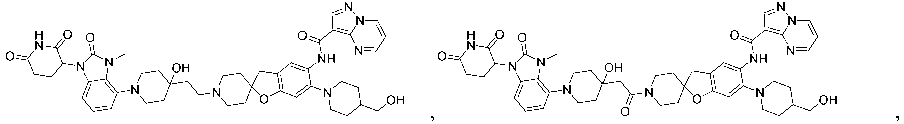 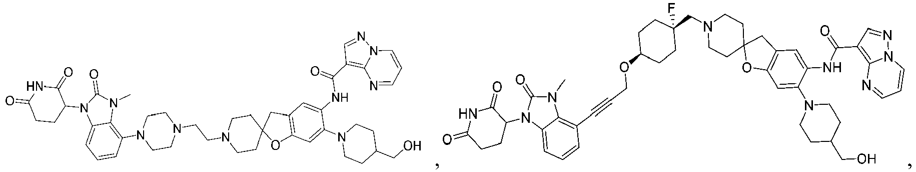 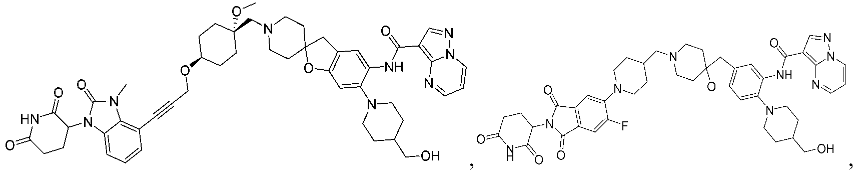 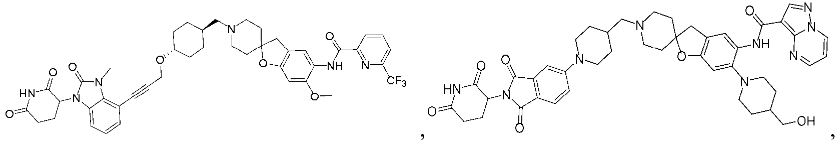 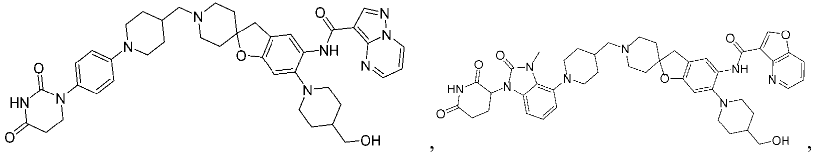 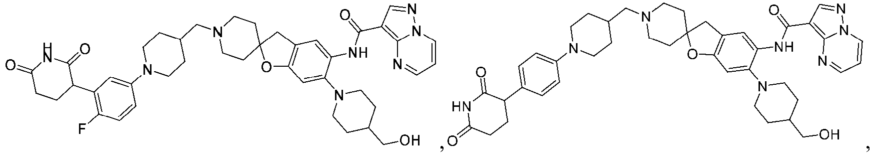 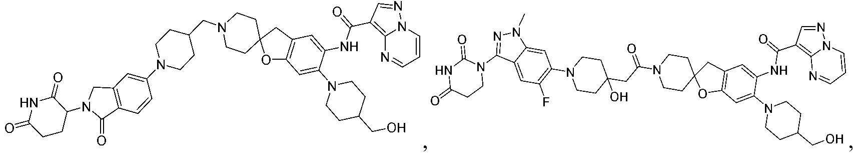 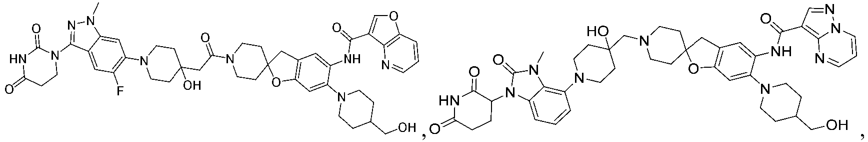 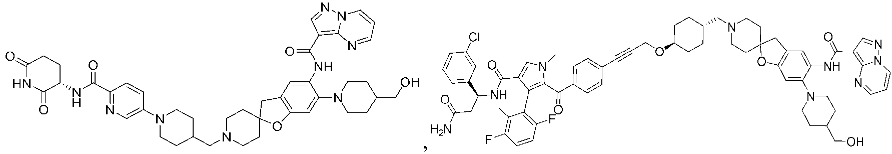 or

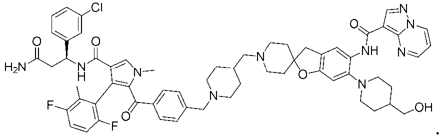

The present invention relates to a pharmaceutical composition comprising a compound of formula (I) as described herein, or an isomer, an N-oxide, a hydrate, a solvate, a metabolite, a pharmaceutically acceptable salt or a prodrug thereof,wherein pharmaceutical composition optionally further comprises a pharmaceutically acceptable excipient, carrier, adjuvant, or any combination thereof.
the present invention also relates to the use of the aforementioned compound or a pharmaceutical composition thereof in the manufacture of a medicament for the prevention, treatment, or alleviation of an IRAK4-associated disease in a patient , Such as inflammatory diseases, infections such as viruses, bacteria, fungi, and parasitic infections HIV-1 infection, sepsis, autoimmune disorders or diseases such as rheumatoid arthritis and multiple sclerosis, gout, juvenile idiopathic arthritis, Muckle Wells disease, familial Mediterranean fever, Behcet's disease, adult Still's disease, proliferative diseases such as cancer, hyperplasia, restenosis, cardiac hypertrophy, leukemia, intravascular coagulation, bone disease, metabolic diseases, neurological and neurodegenerative diseases, cardiovascular diseases, fibrosis and allergic diseases, asthma, atopic dermatitis, suppurative sweat gland inflammation, Alzheimer's disease, hormone related diseases, external injuries, hemodialysis, ischemic diseases, non infectious hepatitis, ultraviolet radiation, closed head injury, pancreatitis, periodontitis, graft-versus-host disease and/or transplant rejection.

This invention provides a series of compounds with high activity, low toxicity, good metabolic stability, and excellent druggable properties.

### Definitions:

Unless otherwise specified, the following terms and phrases used herein are intended to have the meanings set forth below. A particular term or phrase not specifically defined should not be considered indefinite or unclear but should be understood in its ordinary sense. The present invention contemplates covering all alternatives, variations, and equivalents that may be encompassed within the scope of the invention as defined by the claims. Those skilled in the art will recognize many methods and materials similar or equivalent to those described herein, which can be used in the practice of the present invention. The present invention is by no means limited to the methods and materials described.

Unless otherwise indicated, the structural formulas and compounds described in this invention include all isomeric forms, N-oxides, hydrates, solvates, metabolites, pharmaceutically acceptable salts, and prodrugs. Thus, individual stereoisomers, enantiomers, diastereomers, geometric isomers, conformational isomers, N-oxides, hydrates, solvates, metabolites, pharmaceutically acceptable salts, and prodrugs of the compounds of the invention are within the scope of the invention. Additionally, unless otherwise indicated, the structural formulas of the compounds described in this invention include compounds with one or more enriched isotopes of different atoms.

Unless otherwise indicated, the term "isomers" in this invention includes all isomeric forms (e.g., enantiomers, diastereomers, geometric isomers, or conformational isomers), such as R and S configurations at asymmetric centers, (Z) and (E) isomers of double bonds, and conformational isomers of (Z) and (E). Thus, individual stereoisomers or mixtures of enantiomers, diastereomers, geometric isomers, or conformational isomers of the compounds of the invention are within the scope of the invention.

Unless otherwise indicated, the term "N-oxide" in this invention refers to compounds where one or more nitrogen atoms are oxidized when the compound contains multiple amine functional groups, forming N-oxides.

Unless otherwise indicated, the term "hydrate" in this invention refers to an association complex formed when the solvent molecule is water.

Unless otherwise indicated, the term "solvate" in this invention refers to an association complex formed between one or more solvent molecules and the compounds of the invention. Solvents forming solvates include, but are not limited to: water, isopropanol, ethanol, methanol, dimethyl sulfoxide, ethyl acetate, acetic acid, and aminoethanol.

Metabolites refer to products derived from the specific compounds of the invention, or their pharmaceutically acceptable salts, analogs, or derivatives, after metabolic transformation in vivo. These metabolites exhibit activity similar to that of the compound of Formula (I) in vivo or in vitro. Metabolites of a compound may be identified by methods well known in the art and their activity may be characterized by assays described in the invention. Such products may be obtained through oxidation, reduction, hydrolysis, amidation, deamidation, esterification, de-esterification, enzymatic cleavage, etc. Accordingly, the invention includes metabolites of the compounds, including those produced by contacting the compounds of the invention with a mammal for a sufficient period of time.

The term "prodrug" as used in this invention refers to a compound that is converted in vivo to the compound of Formula (I). Such conversion is affected by hydrolysis of the prodrug in the blood or enzymatic transformation of the precursor into the parent structure in blood or tissues. Prodrug compounds of the invention may include esters, such as phenolic esters, aliphatic (C₁₋₂₄) esters, acyloxyalkyl esters, carbonates, carbamates, and amino acid esters, which are known in the art as prodrugs. For example, a compound of the invention containing a hydroxyl group may be acylated to yield a prodrug form. Other prodrug forms include phosphate esters, such as those derived from phosphorylation of a hydroxyl group on the parent compound.

The term "pharmaceutically acceptable" as used herein refers to compounds, materials, compositions, and/or dosage forms that are within the scope of sound medical judgment, suitable for contact with human and animal tissues without excessive toxicity, irritation, allergic response, or other complications, and commensurate with a reasonable benefit/risk ratio.

The term "pharmaceutically acceptable salt" refers to salts of the compounds of the invention prepared from the compounds of the invention with relatively non-toxic acids or bases. When the compounds of the invention contain relatively acidic functional groups, basic addition salts may be obtained by contacting the neutral form of the compound with a sufficient amount of base in pure solution or a suitable inert solvent. Pharmaceutically acceptable basic addition salts include sodium, potassium, calcium, ammonium, organic amine, magnesium, or similar salts. When the compounds of the invention contain relatively basic functional groups, acid addition salts may be obtained by contacting the neutral form of the compound with a sufficient amount of acid in pure solution or a suitable inert solvent. Examples of pharmaceutically acceptable acid addition salts include inorganic acid salts such as hydrochloride, hydrobromide, nitrate, carbonate, bicarbonate, phosphate, hydrogen phosphate, dihydrogen phosphate, sulfate, bisulfate, hydriodic acid, phosphoric acid, etc., as well as organic acid salts such as acetate, propionate, isobutyrate, maleate, malonate, benzoate, succinate, suberate, fumarate, lactate, mandelate, phthalate, benzenesulfonate, p-toluenesulfonate, citrate, tartrate, methanesulfonate, and the like. Salts of amino acids (e.g., arginine) and organic acids such as glucuronic acid are also included. Certain specific compounds of the invention contain both basic and acidic functional groups and may thus be converted to either acid or base addition salts.

The pharmaceutically acceptable salts of the present invention can be synthesized from parent compounds containing acid groups or basic groups via conventional chemical methods. Generally, the preparation of such salts involves reacting the free acid or base form of these compounds with a stoichiometric amount of an appropriate base or acid in water, an organic solvent, or a mixture thereof.

The term "substituted" means that one or more hydrogen atoms on a specific atom are replaced by substituents, which may include deuterium and variants of hydrogen, provided that the valence state of the specific atom is normal and the substituted compound is stable. When the substituent is oxygen (i.e., =O), it indicates that two hydrogen atoms are replaced. Oxygen substitution does not occur on aromatic groups. The term "optionally substituted" means that the group may be substituted or unsubstituted; unless otherwise specified, the type and number of substituents may be arbitrary as long as they are chemically feasible. When any variable (e.g., R) appears more than once in the composition or structure of a compound, its definition in each occurrence is independent. Thus, for example, if a group is described as being substituted with 0-2 R groups, the group may optionally be substituted with up to two R groups, and each R in each case is independently selected. Furthermore, combinations of substituents and/or their variants are only permitted if such combinations result in a stable compound.

"Cycloalkyl" or "cycloalkane" refers to a monovalent or polyvalent saturated monocyclic, bicyclic, or tricyclic carbocyclic system containing 3-12 carbon atoms, which may be a fully saturated ring or a ring containing one or more unsaturated bonds, but never includes aromatic rings. In one embodiment, the cycloalkyl group contains 3-10 carbon atoms; in another embodiment, 3-8 carbon atoms; and in yet another embodiment, 3-6 carbon atoms. Non-limiting examples include cyclopropyl, cyclobutyl, cyclopentyl, and cyclohexyl. The cycloalkyl group may be unsubstituted or substituted with one or more substituents as defined in the invention.

"Heterocyclyl" and "heterocycle" are used interchangeably herein and each refers to a saturated or partially unsaturated monocyclic, bicyclic, or tricyclic ring system containing 3-12 ring atoms, excluding aromatic rings, wherein at least one ring atom is a heteroatom. In one embodiment, "heterocyclyl" or "heterocycle" contains 3-10 ring atoms; in another embodiment, 3-8 ring atoms; in another embodiment, 5-8 ring atoms; in yet another embodiment, 3-6 ring atoms; in a further embodiment, 5-6 ring atoms; and in still another embodiment, 4-6 ring atoms. Unless otherwise specified, the heterocycle may be carbon-based or nitrogen-based, and the heteroatoms have the meanings described in the invention. Non-limiting examples of heterocyclyl groups include: oxiranyl, azetidinyl, oxetanyl, thietanyl, pyrrolidinyl, 2-pyrrolinyl, 3-pyrrolinyl, pyrazolidinyl, pyrazolinyl, imidazolidinyl, imidazolinyl, tetrahydrofuranyl, dihydrofuranyl, tetrahydrothiophenyl, dihydrothiophenyl, 1,3-dioxolanyl, dithiolanyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, oxepanyl, thiepanyl, oxazepinyl, diazepinyl, thiazepinyl, and 2-oxa-5-aza-bicyclo[2.2.1]hept-5-yl. Examples of heterocyclyl groups in which a -CH₂-group is replaced by -C(=O)- include, but are not limited to: 2-oxopyrrolidinyl, 2-oxo-1,3-thiazolidinyl, 2-piperidinonyl, 3,5-dioxopiperidinyl, and pyrimidinedionyl. Examples of heterocyclyl groups in which sulfur atoms are oxidized include, but are not limited to: cyclobutanesulfonyl and 1,1-dioxothiomorpholinyl. The heterocyclyl group may be optionally substituted with one or more substituents as described in the invention.

"Aryl" refers to a monocyclic, bicyclic, or tricyclic carbocyclic system containing 6-14, 6-12, or 6-10 ring atoms, wherein at least one ring is aromatic, and each ring contains 3-7 ring atoms, with one or more points of attachment to the rest of the molecule. The terms "aryl" and "aryl ring" are used interchangeably. Non-limiting examples include phenyl, naphthyl, and anthracenyl. The aryl group may be optionally substituted with one or more substituents as described in the invention.

"Heteroaryl" refers to a monocyclic, bicyclic, or tricyclic system containing 5-12, 5-10, or 5-6 ring atoms, wherein at least one ring is aromatic, and at least one ring contains one or more heteroatoms (selected from O, S, and N), each ring comprising 5-7 atoms and having one or more points of attachment to the rest of the molecule. The terms "heteroaryl", "heteroaromatic ring", and "heteroaromatic compound" are used interchangeably. The heteroaryl group may be optionally substituted with one or more substituents as described in the invention. In one embodiment, the 5-10 membered heteroaryl group contains 1, 2, 3, or 4 heteroatoms independently selected from O, S, and N, wherein the nitrogen atoms may be further oxidized.

Non-limiting examples of heteroaryl groups include: furyl (e.g., 2-furyl, 3-furyl), imidazolyl (e.g., N-imidazolyl, 2-imidazolyl, 4-imidazolyl, 5-imidazolyl), isoxazolyl, oxazolyl (e.g., 2-oxazolyl, 4-oxazolyl, 5-oxazolyl), pyrrolyl (e.g., N-pyrrolyl, 2-pyrrolyl, 3-pyrrolyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl, thiazolyl (e.g., 2-thiazolyl, 4-thiazolyl, 5-thiazolyl), tetrazolyl (e.g., 5-tetrazolyl), triazolyl, thienyl (e.g., 2-thienyl, 3-thienyl), pyrazolyl, isothiazolyl, 1,2,3-oxadiazolyl, 1,2,5-oxadiazolyl, 1,2,4-oxadiazolyl, 1,2,3-triazolyl, 1,2,3-dithiazolyl, 1,3,4-dithiazolyl, 1,2,5-dithiazolyl, pyrazinyl, 1,3,5-triazinyl; and also includes the following bicyclic heteroaryl groups, but are not limited to: benzimidazolyl, benzofuranyl, benzothienyl, indolyl (e.g., 2-indolyl), purinyl, quinolinyl (e.g., 2-quinolinyl, 3-quinolinyl, 4-quinolinyl), 1,2,3,4-tetrahydroisoquinolinyl, 1,3-benzodioxolyl, indolinyl, isoquinolinyl (e.g., 1-isoquinolinyl, 3-isoquinolinyl, or 4-isoquinolinyl), imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl, among others.

"Spirocyclic ring " refers to a 5-20 membered polycyclic group in which two saturated or partially saturated monocyclic rings share a single carbon atom (called a spiro atom), and may contain 0-5 heteroatoms. Preferably, the spirocyclic ring has 6-14 ring atoms, more preferably 8-13 ring atoms, and most preferably 8-10 ring atoms. Unless otherwise specified, the spirocyclic ring group may be optionally substituted with one or more substituents as described in the invention. Non-limiting examples include: 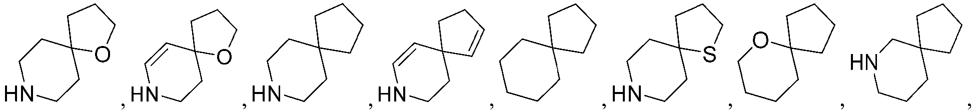 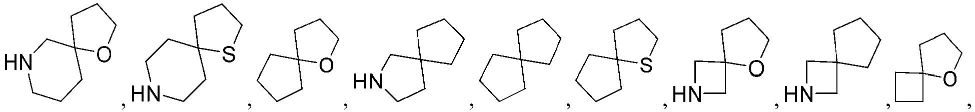 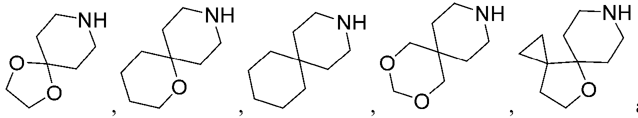 and so on.

"Bridged bicyclic ring" refers to a saturated or partially unsaturated bicyclic system having at least one bridge, which may contain 0-5 heteroatoms. A "bridge" is an unbranched chain of atoms or a bond connecting two bridgehead atoms, where a "bridgehead" is any ring atom (excluding hydrogen) bonded to three or more framework atoms. In some embodiments, the bridged bicyclic group contains 5-12 ring members and 0-4 heteroatoms independently selected from nitrogen, oxygen, or sulfur. These bridged bicyclic groups are well-known in the art and may be attached to the rest of the molecule at any substitutable carbon or nitrogen atom. Unless otherwise specified, the bridged bicyclic group may be optionally substituted with one or more substituents as described in the invention. Alternatively or additionally, any substitutable nitrogen atom in the bridged bicyclic group may be further substituted. Non-limiting examples include: 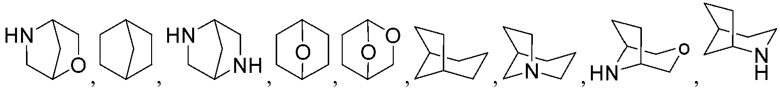 and so on.

"Heteroatom" refers to oxygen, sulfur, nitrogen, phosphorus, or silicon, or any combination thereof (including any oxidized form of nitrogen, sulfur, phosphorus, or silicon; any quaternized form of a basic nitrogen; and any substitutable nitrogen in a heterocycle, such as N (e.g., in 3,4-dihydro-2H-pyrrolyl), NH (e.g., in pyrrolidinyl), or NR⁺ (e.g., in *N*-substituted pyrrolidinyl)).

"Halogen" refers to F, Cl, Br, or I.

"Deuterium" refers to heavy hydrogen, D.

"Alkyl" refers to a straight-chain or branched-chain hydrocarbon group consisting of a certain number of carbon atoms, and is saturated.

"Alkenyl" refers to a straight-chain or branched hydrocarbon group composed of multiple carbon atoms and containing at least one carbon-carbon double bond, where the double bond may be located at any position within the group.

"Alkylene" refers to a saturated divalent hydrocarbon group derived by removing two hydrogen atoms from a straight-chain or branched saturated hydrocarbon.

"Alkoxy" refers to a straight-chain or branched monovalent residue represented by the formula -OR, where R is an alkyl group as defined above.

"Haloalkyl" or "haloalkoxy" refers to an alkyl or alkoxy group substituted with one or more halogen atoms.

"Hydroxyalkyl" refers to an alkyl group substituted with one or more hydroxyl groups.

As used herein, when a substituent is drawn with a bond attached to a ring, it indicates that the substituent may be attached at any substitutable position on the ring, either as a single substitution or multiple substitutions. For example, formula (a) indicates that substituent R may be mono- or poly-substituted at any possible substitutable position on the pyridine ring.

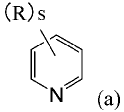

As used herein:A wavy line intersecting a bond in a chemical structure indicates the point where the atom connected by the wavy bond attaches to the rest of the molecule or a fragment of the molecule.A bond attached to a ring system (e.g., as shown in Formula b) signifies that the bond may connect to the rest of the molecule at any possible position on the ring. For example, Formula b indicates that the octahydrocyclopenta[c]pyrrole ring may connect to the rest of the molecule at any available position.

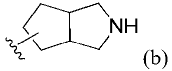

Unless chemically or structurally required, the order in which chemical groups are written or named does not imply or indicate directionality.

If there is any discrepancy between a structure and its name, the structure shall prevail.

### General Synthetic Procedures

The compounds of the present invention can be prepared by various synthetic methods well-known to those skilled in the art, including but not limited to the specific embodiments described below, combinations thereof with other chemical synthetic methods, and equivalent alternatives familiar to those skilled in the field. Preferred embodiments include, but are not limited to, the examples provided in the present invention.

The following abbreviations are used in the present invention:

| | | | |
|---|---|---|---|
| mg | milligramme | mmol | millimole |
| DIEA | *N,N*-diisopropylethylamine | DMF | *N, N*-dimethylformamide |
| HATU | 2-(7-Azabenzotriazol-1-yl)-*N*,*N*,*N*',*N'*-tetramethyluronium hexafluorophosphate | h | hour |
| min | minute | wt% | mass percentage |
| DCM | dichloromethane | ACN, MeCN | acetonitrile |
| PE | petroleum ether | EA | ethyl acetate |
| MeOH | methanol | Pd/C | palladium on carbon |
| THF | tetrahydrofuran | TBSCl | tert-Butyldimethylchlorosilane |
| TFA | Trifluoroacetic Acid | MsCl | Trifluoroacetic Acid |
| ppm | Parts Per Million | TMS | Tetramethylsilane |
| DMSO | Dimethyl Sulfoxide | TPSA | p-Toluenesulfonic Acid |
| LDA | Lithium Diisopropylamide | (Boc)₂O | Di-tert-butyl Dicarbonate |
| TEA | Triethylamine | LAH | Lithium Aluminium Hydride |
| DMAP | 4-Dimethylaminopyridine | TBAF | Tetrabutylammonium Fluoride |
| Pd(PPh₃)₂Cl | bis(triphenylphosphine)palladium(II) chloride | EtOH | Ethanol |
| t-BuOK | potassium tert-butoxide | AcOH | Acetic Acid |
| t-BuONa | Sodium tert-butoxide | | |

The raw materials, reagents, etc., used in the following examples are all sourced from commercial products or known synthetic routes in the literature unless otherwise specified.

¹H NMR spectra were recorded with a Bruker 500 MHz spectrometer using CDCl₃, *d*₆-DMSO, CD₃OD or *d*₆-acetone as solvents (reported in ppm), and using TMS (0 ppm) or chloroform (7.26 ppm) as the reference standard. For multiple peaks, the following abbreviations used: s (singlet), d (doublet), t (triplet), m (multiplet), br (broadened), dd (doublet of doublets), q (quartet), dt (doublet of triplets), tt (triplet of triplets), dddd (doublet of doublet of doublet of doublets), qd (quartet of doublets), ddd (doublet of doublet of doublets), td (triplet of doublets), dq (doublet of quartets), ddt (doublet of doublet of triplets), tdd (triplet of doublet of doublets), dtd (doublet of triplet of doublets). Coupling constants, when given, were reported in Hertz (Hz).

Low-resolution mass spectral (MS) data were determined by an Agilent 6125C Series LC-MS, column model: XBridge BEH C18, 4.6 x 50 mm, 2.5 µm, 6 min, flow rate of 1 mL/min. mobile phase: 0% - 95% (CH₃CN) in (H₂O containing 0.1% formic acid:CH₃CN = 90:10) using electrospray ionization (ESI) at 210 nm/254 nm with DAD detection.

Compounds are named according to conventional nomenclature principles in the field or using naming software. Commercially available compounds adopt the supplier catalog names.

### DESCRIPTION OF THE PREFERRED EMBODIMENTS

The Examples provide preparation methods and structural characterization data for representative compounds represented by Formula (I). it being noted that these examples are merely descriptive and do not limit the invention in any way.

### Synthesis of Intermediates

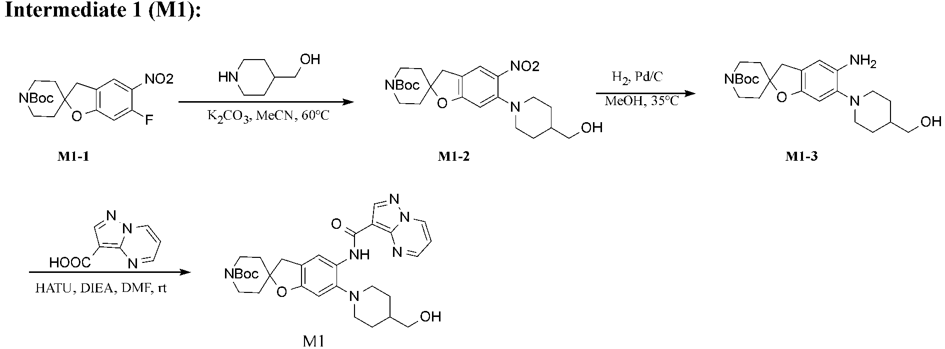

### Step 1:

M1-1 (prepared according to the procedure described in WO2017108723A2, Example 142) (58 mg, 0.16 mmol) was placed in a 50 mL single-necked flask. 4-(Hydroxymethyl)piperidine (37 mg, 0.33 mmol), K₂CO₃,(57 mg, 0.41 mmol), and ACN(1 mL) were added. The mixture was heated to an external temperature of 60 °C and reacted for 1 hour. The reaction mixture was filtered, and the filtrate was collected. The filter cake was dissolved in water (5 mL) and extracted with EA(5 mL × 2). The combined organic phases were dried over anhydrous Na₂SO₄ and purified by column chromatography (eluent: PE:EA = 92:8-60:40) to afford M1-2 as an orange solid (54 mg, yield: 76.0%).

ESI-MS (m/z): 448.4 [M+H]⁺.

¹H NMR (500 MHz, DMSO-*d₆*) δ 7.81 (s, 1H), 6.61 (s, 1H), 4.45 (t, *J* = 5.1 Hz, 1H), 3.57 - 3.48 (m, 2H), 3.41 - 3.32 (m, 2H), 3.14 (d, *J* = 12.0 Hz, 2H), 3.01 (s, 2H), 2.74 (t, *J* = 11.5 Hz, 2H), 1.83 - 1.65 (m, 7H), 1.53 - 1.44 (m, 1H), 1.41 (s, 9H), 1.32 - 1.20 (m, 3H).

### Step 2:

M1-2 (54 mg, 0.12 mmol) and 10 wt% (Pd/C, 15 mg) were placed in a 50 mL single-necked flask. MeOH (10 mL) was added. The atmosphere was replaced with hydrogen three times using a hydrogen balloon and maintained under a hydrogen atmosphere. The reaction mixture was stirred at an external temperature of 35 °C for 2 hours. The reaction mixture was filtered through Celite to remove the Pd/C catalyst. The filter cake was washed with MeOH (20 mL) until colorless. The combined organic filtrates were concentrated under reduced pressure to remove MeOH to afford M1-3 as a gray solid (45 mg, yield: 89.6%).

ESI-MS (m/z): 418.5 [M+H]⁺.

¹H NMR (500 MHz, CDCl₃) *δ* 6.63 (s, 1H), 6.56 (s, 1H), 3.73 (s, 2H), 3.60 (d, *J* = 6.2 Hz, 2H), 3.42 (t, *J* = 10.7 Hz, 2H), 3.21 (d, *J* = 11.3 Hz, 2H), 2.92 (s, 2H), 1.96 - 1.82 (m, 5H), 1.75 - 1.60 (m, 4H), 1.57 - 1.50 (m, 2H), 1.50 (s, 9H).

### Step 3:

M1-3 (320 mg, 0.82 mmol), pyrazolo[1,5-*a*]pyrimidine-3-carboxylic acid (134 mg, 0.82 mmol), HATU (375 mg, 0.99 mmol), and DIEA (531 mg, 4.11 mmol) were placed in a 50 mL single-necked flask. DCM,(18 mL) was added, and the mixture was stirred at room temperature for 2 hours. After reaction completion, DCM (20 mL) and water (15 mL) were added. The mixture was stirred vigorously and then allowed to stand for phase separation. The organic phase was dried over anhydrous Na₂SO₄)and concentrated under reduced pressure. The residue was purified by column chromatography (eluent: DCM:MeOH = 100:0 - 98:2) to afford M1 as a brownish paste (417 mg, yield: 95.0%).

ESI-MS (m/z): 563.3 [M+H]⁺.

¹H NMR (400 MHz, CDCl₃) *δ* 10.51 (s, 1H) 8.75 - 8.87 (m, 3H) 8.33 - 8.43 (m, 1H) 7.03 (dd, *J*=6.97, 4.16 Hz, 1H) 6.71 (s, 1H) 3.68 - 3.82 (m, 2H) 3.64 (d, *J=5.13* Hz, 2H) 3.42 (br t, *J*=10.33 Hz, 2H) 3.16 (br d, *J*=6.60 Hz, 2H) 3.02 (s, 2 H) 2.73 (br s, 2H) 1.92 (br d, *J=13.33* Hz, 2H) 1.70 - 1.86 (m, 7H) 1.49 (s, 9H).

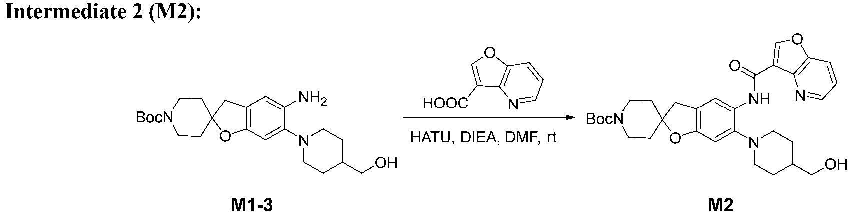

Intermediate M1-3 (1.43 g, 3.43 mmol), furo[3,2-*b*]pyridine-3-carboxylic acid (0.61 g, 3.77 mmol), DIEA (1.7 mL, 10.27 mmol), and HATU (1.56 g, 4.11 mmol) were sequentially added to a 100 mL single-necked flask. Anhydrous DMF (10 mL) was added, and the flask was purged with nitrogen. The reaction mixture was stirred at room temperature for 3 hours. Water (80 mL) was slowly added dropwise to the reaction solution, and the mixture was stirred for another 30 minutes after the addition was complete. The aqueous phase was decanted, and the remaining viscous product in the flask was dissolved by adding the ethyl acetate (EA) phase obtained from extracting the aqueous phase with EA (100 mL). The combined organic phase was washed with saturated brine (20 mL × 3), dried over anhydrous sodium sulfate, filtered, and concentrated under reduced pressure. The residue was purified by column chromatography using DCM: MeOH= 100:0-95:5 as the eluent to afford M2 as a brownish-yellow solid (1.34 g, yield: 69.8%).

ESI-MS (*m*/*z*): 563.2 [M+H]⁺

¹H NMR (400 MHz, CDCl₃) *δ* 10.97 (s, 1H), 8.68 - 8.75 (m, 1H), 8.64 (s, 1H), 8.33 (s, 1H), 7.86 - 7.94 (m, 1H), 7.33 - 7.41 (m, 1H), 6.71 - 6.79 (m, 1H), 3.69 - 3.82 (m, 2H), 3.63 (d, *J* = 5.88 Hz, 2H), 3.36 - 3.49 (m, 2H), 3.14 - 3.26 (m, 2H), 3.02 (s, 2H), 2.76 (t, *J* = 10.07 Hz, 2H), 1.92 (d, *J* = 13.76 Hz, 2H), 1.69 - 1.87 (m, 7H), 1.49 (s, 9H).

The following intermediates were prepared using methods analogous to those described for Intermediate M1 and Intermediate M2, respectively.

| **Intermediate** | **Chemical Structure** | **Characterization Data** |
|---|---|---|
| **Intermediate3 M3** | 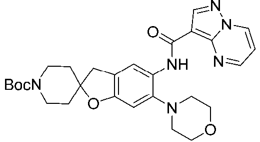 | ESI-MS *(m*/*z):* 535.5 [M+H]⁺. |
| **Intermediate4 M4** | 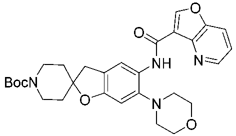 | ESI-MS *(m*/*z):* 535.5 [M+H]⁺. |
| | | ¹H NMR (500 MHz, CDCl₃) *δ* 10.90 (s, 1H), 8.66 (d, *J* = 4.6 Hz, 1H), 8.51 (s, 1H), 8.35 (s, 1H), 7.82 (d, *J* = 8.4 Hz, 1H), 7.32 (dd, *J* = 8.3, 4.8 Hz, 1H), 6.62 (s, 1H), 3.97 - 3.85 (m, 4H), 3.67 (d, *J* = 12.7 Hz, 2H), 3.36 (t, *J* = 10.6 Hz, 2H), 2.95 (s, 2H), 2.90 - 2.81 (m, 4H), 1.85 (d, *J* = 13.4 Hz, 2H), 1.71 - 1.60 (m, 2H), 1.41 (s, 9H). |
| **Intermediate5 M5** | 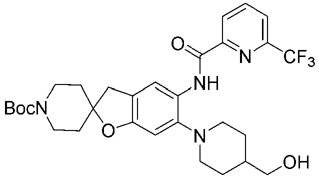 | ESI-MS *(m*/*z):* 591.5 [M+H]⁺ |
| | | ¹H NMR (500 MHz, CDCl₃) *δ* 10.93 (s, 1H), 8.51 (s, 1H), 8.43 (s, 1H), 8.10 (t, *J* = 7.8 Hz, 1H), 7.84 (d, *J* = 7.8 Hz, 1H), 6.69 (s, 1H), 3.74 (d, *J =* 8.1 Hz, 2H), 3.62 (s, 2H), 3.42 (t, *J* = 10.8 Hz, 2H), 3.05 (d, *J* = 11.5 Hz, 2H), 3.01 (s, 2H), 2.69 (t, *J* = 11.2 Hz, 2H), 1.95 - 1.83 (m, 4H), 1.74 - 1.67 (m, 2H), 1.64 - 1.57 (m, 2H), 1.48 (s, 9H), 1.38 (m, 1H). |
| **Intermediate6 M6** | 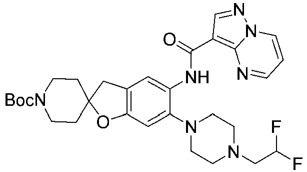 | ESI-MS *(m*/*z):* 598.4 [M+H]⁺. |
| | | ¹H NMR (500 MHz, CDCl₃) *δ* 10.24 (s, 1H), 8.76 (d, *J =* 5.7 Hz, 1H), 8.71 (s, 1H), 8.67 (d, *J* = 1.1 Hz, 1H), 8.33 (s, 1H), 7.00 (dd, *J* = 6.7, 4.2 Hz, 1H), 6.60 (s, 1H), 6.09 - 5.83 (m, 1H), 3.71 - 3.60 (m, 2H), 3.36 (t, *J* = 10.6 Hz, 2H), 3.08 - 2.70 (m, 12H), 1.84 (d, *J* = 13.3 Hz, 2H), 1.68 - 1.60 (m, 2H), 1.42 (s, 9H). |
| **Intermediate7 M7** | 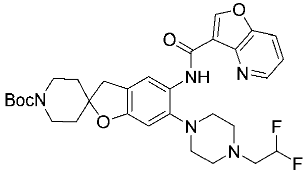 | ESI-MS *(m*/*z):* 598.3 [M+H]⁺. |
| | | ¹H NMR (500 MHz, CDCl₃) *δ* 10.85 (s, 1H), 8.71 (d, *J* = 4.5 Hz, 1H), 8.61 (s, 1H), 8.40 (s, 1H), 7.93 (d, *J* = 8.0 Hz, 1H), 7.46 - 7.39 (m, 1H), 6.71 (s, 1H), 5.43 - 5.27 (m, 1H), 3.87 - 3.67 (m, 3H), 3.53 - 3.35 (m, 3H), 3.14 - 2.79 (m, 10H), 1.99 - 1.86 (m, 2H), 1.79 - 1.67 (m, 2H), 1.51 (s, 9H). |
| **Intermediate8 M8** | 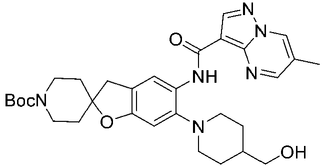 | ESI-MS *(m*/*z):* 577.3 [M+1]⁺. |
| | | ¹H NMR (400 MHz, CD₃OD) *δ* 8.92 (s, 1H), 8.83 (d, *J* = 1.96 Hz, 1H), 8.58 (s, 1H), 8.23 (s, 1H), 6.71 (s, 1H), 3.69 - 3.82 (m, 2H), 3.55 (d, *J* = 5.26 Hz, 2H), 3.36 - 3.49 (m, 2H), 3.00 - 3.08 (m, 4H), 2.65 - 2.76 (m, 2H), 2.48 (s, 3H), 1.84 - 1.94 (m, 2H), 1.68 - 1.82 (m, 6H), 1.55 - 1.67 (m, 1H), 1.48 (s, 9H). |
| **Intermediate9 M9** | 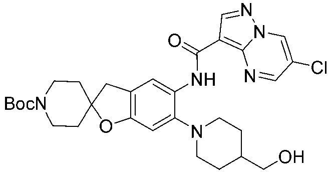 | ESI-MS *(m*/*z):* 597.3 [M+1]⁺. |
| | | ¹H NMR (400 MHz, CD₃OD) *δ* 9.41 (d, *J* = 2.13 Hz, 1H), 8.97 (d, *J* = 2.25 Hz, 1H), 8.68 (s, 1H) 8.27 (s, 1H), 6.75 (s, 1H), 3.74 - 3.82 (m, 2H), 3.58 (d, *J* = 5.13 Hz, 2H), 3.42 - 3.50 (m, 2H), 3.02 - 3.10 (m, 4H), 2.67 - 2.79 (m, 2H), 1.94-1.86 (m, 2H), 1.64 - 1.83 (m, 7H), 1.50 (s, 9H). |
| **Intermediate10 M10** | 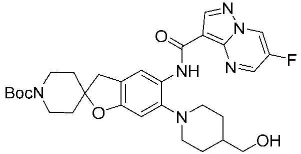 | ESI-MS *(m*/*z):* 581.4 [M+H]⁺ |
| | | ¹H NMR (400 MHz, CDCl₃) *δ* 10.47 (s, 1H), 8.91 (d, *J* = 2.63 Hz, 1H), 8.82 (s, 1H), 8.74 - 8.79 (m, 1H), 8.24 - 8.44 (m, 1H), 6.72 (s, 1H), 3.61 - 3.81 (m, 4H), 3.42 (br t, *J* = 10.51 Hz, 2H), 3.09 - 3.28 (m, 2H), 3.02 (s, 2H), 2.81 (s, 2H), 2.71 - 2.78 (m, 1H), 1.78 - 1.97 (m, 6H), 1.66 - 1.77 (m, 3H), 1.49 (s, 9H). |
| **Intermediate11 M11** | 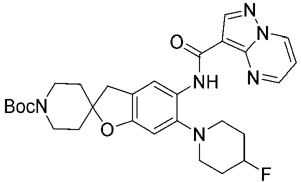 | ESI-MS *(m*/*z):* 551.2 [M+1]⁺. |
| | | ¹H NMR (400 MHz, DMSO-*d₆*) δ 10.47 (s, 1 H), 9.37 (dd, *J* = 7.00, 1.50 Hz, 1H), 8.83 (dd, *J* = 4.13, 1.50 Hz, 1H), 8.68 (s, 1H), 8.34 (s, 1H), 7.34 (dd, *J* = 7.00, 4.13 Hz, 1 H), 6.75 (s, 1H), 3.47 - 3.58 (m, 2H), 3.34 - 3.44 (m, 3H), 3.03 (s, 2H), 2.92 (br d, *J* = 7.00 Hz, 2H), 2.70 - 2.81 (m, 2H), 1.90 - 2.20 (m, 4H), 1.62 - 1.84 (m, 4H), 1.42 (s, 9H). |
| **Intermediate12 M12** | 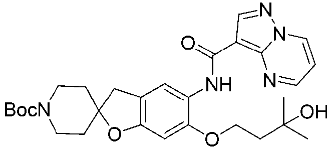 | ESI-MS *(m*/*z):* 552.4 [M+H]⁺ |
| | | ¹H NMR (400 MHz, CDCl₃) *δ* 10.09 (s, 1H), 8.84 (dd, *J* = 7.03, 1.65 Hz, 1H), 8.76 - 8.81 (m, 2H), 8.39 (s, 1H), 7.07 (dd, *J* = 6.97, 4.16 Hz, 1H), 6.54 (s, 1H), 4.28 (t, *J* =6.23 Hz, 2H), 3.72 - 3.83 (m, 2H), 3.40 - 3.52 (m, 2H), 3.04 (s, 2H), 2.20 (t, *J* = 6.30 Hz, 2H), 1.93 - 1.98 (m, 2H), 1.73 - 1.80 (m, 2H), 1.52 (s, 9H), 1.38 (s, 6H). |
| **Intermediate13 M13** | 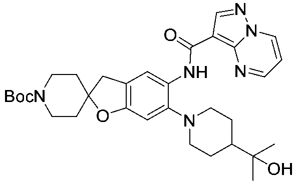 | ESI-MS *(m*/*z):* 591.3 [M+H]⁺. |
| | | ¹H NMR (500 MHz, CDCl₃) *δ* 10.45 (s, 1H), 8.86 (d, *J* = 2.5 Hz, 1H), 8.81 (dd, *J* = 7.5, 6.0 Hz, 2H), 8.43 (s, 1H), 7.02 (dd, *J* = 6.9, 4.1 Hz, 1H), 6.69 (s, 1H), 3.75 (s, 2H), 3.46 (t, *J =* 10.4 Hz, 2H), 3.18 (d, *J* = 11.4 Hz, 2H), 3.03 (s, 2H), 2.66 (t, *J* = 11.1 Hz, 2H), 1.93 (d, *J* = 13.2 Hz, 2H), 1.86 (d, *J* = 11.7 Hz, 2H), 1.82 - 1.72 (m, 4H), 1.50 (s, 9H), 1.46 - 1.40 (m, 1H), 1.28 (s, 6H). |
| **Intermediate14 M14** | 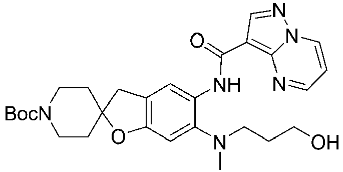 | ESI-MS (*m*/*z*): 537.3 [M+H]⁺. |
| | | ¹H NMR (500 MHz, DMSO-*d₆*) *δ* 10.43 (s, 1H), 8.80 (dd, *J=* 7.0, 1.4 Hz, 1H), 8.76 (s, 1H), 8.74 - 8.70 (m, 1H), 8.36 (s, 1H), 7.01 (dd, *J* = 6.9, 4.1 Hz, 1H), 6.67 (s, 1H), 3.72 (t, *J =* 6.2 Hz, 4H), 3.42 (t, *J* = 10.5 Hz, 2H), 3.12 (t, *J* = 6.2 Hz, 2H), 3.01 (s, 2H), 2.66 (s, 3H), 1.91 (d, *J* = 13.4 Hz, 2H), 1.84 - 1.77 (m, 2H), 1.71 (dd, *J* = 16.5, 6.8 Hz, 2H), 1.48 (s, 9H). |
| **Intermediate15 M15** | 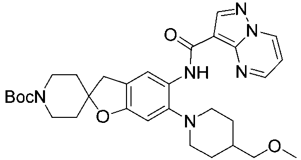 | ESI-MS *(m*/*z):* 577.3 [M+H]⁺. |
| | | ¹H NMR (500 MHz, CDCl₃) *δ* 10.47 (s, 1H), 8.77 - 8.66 (m, 3H), 8.37 (s, 1H), 6.99 - 6.88 (m, 1H), 6.61 (s, 1H), 3.65 (s, 2H), 3.40 - 3.31 (m, 5H), 3.29 (s, 2H), 3.01 (d, *J* = 10.1 Hz, 2H), 2.94 (s, 2H), 2.60 (s, 2H), 1.84 (d, *J* = 13.4 Hz, 2H), 1.74 - 1.60 (m, 7H), 1.41 (s, 9H). |
| **Intermediate16 M16** | 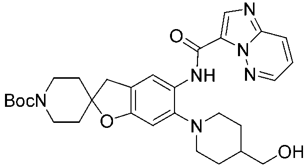 | ESI-MS *(m*/*z):* 563.3 [M+H]⁺. |
| | | ¹H NMR (500 MHz, DMSO-*d₆*) *δ* 11.06 (s, 1H), 8.91 (d, *J* = 3.4 Hz, 1H), 8.45 - 8.35 (m, 3H), 7.52 (dd, *J* = 9.2, 4.5 Hz, 1H), 6.78 (s, 1H), 3.57 - 3.51 (m, 2H), 3.42 (s, 2H), 3.38 (s, 2H), 3.03 (s, 2H), 2.94 (d, *J* = 11.2 Hz, 2H), 2.65 (t, *J* = 10.4 Hz, 2H), 1.80 - 1.68 (m, 6H), 1.58 (d, *J* = 11.4 Hz, 1H), 1.54 (d, *J* = 7.5 Hz, 2H), 1.42 (s, 9H). |
| **Intermediate17 M17** | 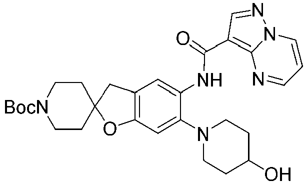 | ESI-MS *(m*/*z):* 549.0 [M+H]⁺. |
| | | ¹H NMR (500 MHz, DMSO-*d₆*) *δ* 10.54 (s, 1H), 9.40 - 9.33 (m, 1H), 8.89 (d, *J* = 2.7 Hz, 1H), 8.35 (s, 1H), 7.53 (s, 1H), 7.45 (s, 1H), 7.36 (dd, *J* = 6.9, 4.2 Hz, 1H), 6.72 (s, 1H), 3.66 (s, 2H), 3.52 (d, *J* = 4.8 Hz, 2H), 3.38 (s, 2H), 3.02 (s, 2H), 2.90 (d, *J* = 11.3 Hz, 2H), 2.68 (t, *J* = 9.7 Hz, 2H), 1.89 (d, *J* = 9.4 Hz, 2H), 1.76 (s, 2H), 1.70 (dd, *J* = 10.9, 6.6 Hz, 2H), 1.42 (s, 9H). |
| **Intermediate18 M18** | 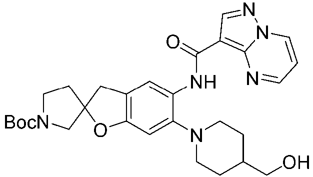 | ESI-MS (*m*/*z*):549.3[M+H]⁺. |
| | | ¹H NMR (500 MHz, DMSO-*d₆*) *δ* 10.49 (s, 1H), 9.35 (d, *J* = 7.0 Hz, 1H), 8.88 (t, *J* = 9.7 Hz, 1H), 8.67 (s, 1H), 8.37 (s, 1H), 7.33 (dd, *J =* 6.9, 4.2 Hz, 1H), 6.77 (d, *J* = 4.0 Hz, 1H), 4.52 (t, *J* = 5.0 Hz, 1H), 3.57 (d, *J* = 11.8 Hz, 1H), 3.50 (t, *J =* 9.2 Hz, 1H), 3.44 - 3.36 (m, 4H), 3.20 (d, *J* = 16.2 Hz, 1H), 2.93 (d, *J* = 11.2 Hz, 2H), 2.84 (s, 1H), 2.63 (t, *J* = 11.0 Hz, 2H), 2.18 - 2.05 (m, 2H), 1.72 (d, *J* = 11.1 Hz, 2H), 1.63 - 1.55 (m, 2H), 1.53 - 1.47 (m, 1H), 1.42 (d, *J= 11.6* Hz, 9H). |
| **Intermediate19 M19** | 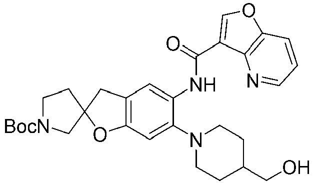 | ESI-MS (*m*/*z*): 549.3 [M+H]⁺. |
| **Intermediate20 M20** | 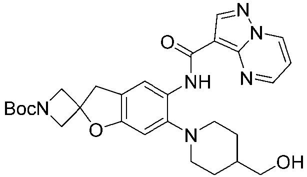 | ESI-MS *(m*/*z):* 535.3 [M+H]⁺. |
| | | ¹H NMR (500 MHz, CDCl₃) *δ* 10.70 (s, 1H), 8.92 - 8.62 (m, 3H), 8.40 (s, 1H), 7.26 (s, 1H), 6.99 (dd, *J* = 6.7, 4.2 Hz, 1H), 5.04 (s, 2H), 4.25 (d, *J* = 9.4 Hz, 2H), 4.07 (d, *J* = 9.4 Hz, 2H), 3.62 (d, *J* = 5.0 Hz, 2H), 3.14 (d, *J* = 9.9 Hz, 2H), 2.80 (d, *J* = 9.2 Hz, 2H), 1.87 - 1.62 (m, 5H), 1.43 (s, 9H). |
| **Intermediate21 M21** | 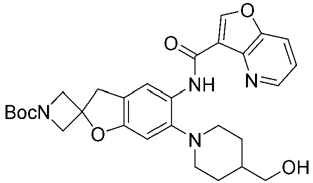 | ESI-MS *(m*/*z):* 535.3 [M+H]⁺. |
| | | ¹H NMR (500 MHz, CDCl₃) *δ* 11.22 (s, 1H), 8.74 (d, *J* = 4.5 Hz, 1H), 8.62 (s, 1H), 8.54 (s, 1H), 7.91 (d, *J* = 8.4 Hz, 1H), 7.40 (dd, *J* = 8.5, 4.9 Hz, 1H), 7.33 (s, 1H), 5.13 (s, 2H), 4.35 (d, *J* = 9.4 Hz, 2H), 3.76 - 3.71 (m, 2H), 3.69 (d, *J* = 5.6 Hz, 2H), 3.21 - 3.15 (m, 2H), 2.01 - 1.69 (m, 10H), 1.53 (s, 9H). |
| **Intermediate22 M22** | 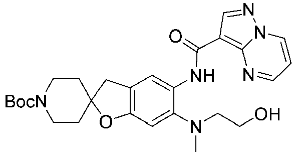 | ESI-MS *(m*/*z):* 523.2 [M+1]⁺. |
| **Intermediate23 M23** | 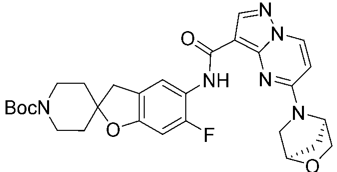 | ESI-MS *(m*/*z):* 565.2 [M+H]⁺. |

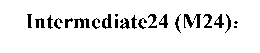

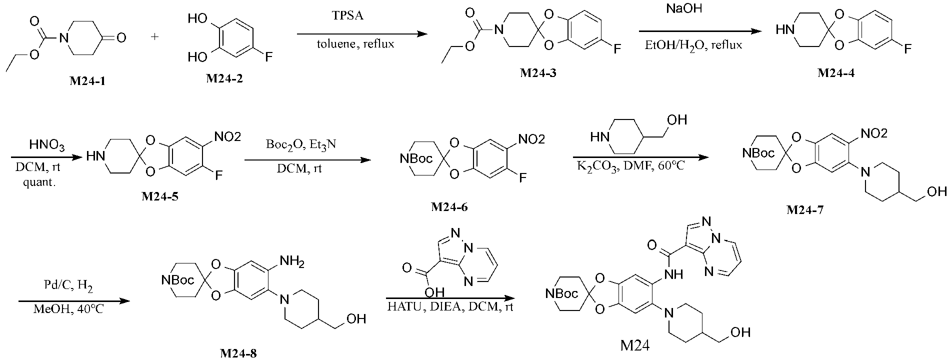

### Step 1:

M24-1 (5.14 g, 30.00 mmol), M24-2 (3.85 g, 30.00 mmol), and p-toluenesulfonic acid (0.86 g, 4.50 mmol) were placed in a 100 mL single-necked flask. Toluene (80 mL) was added, followed by a Dean-Stark trap. The mixture was heated to an external temperature of 135°C and refluxed for 20 hours. After concentration under reduced pressure, water (40 mL) was added to the residue, and the mixture was extracted with EA (80 mL × 2). The combined organic phases were dried over anhydrous sodium sulfate, concentrated under reduced pressure, and purified by column chromatography using PE:EA = 100:0 -92:8 as the eluent to afford compound M24-3 as a white crystalline solid (5.05 g, yield: 59.8%).

ESI-MS (*m*/*z*): 282.1 [M+H]⁺.

¹H NMR (500 MHz, CDCl₃) *δ* 6.67 (dd, *J* = 8.5, 4.5 Hz, 1H), 6.56 (dd, *J* = 8.2, 2.4 Hz, 1H), 6.50 (td, *J* = 9.6, 2.5 Hz, 1H), 4.19 (q, *J* = 7.1 Hz, 2H), 3.77 - 3.61 (m, 4H), 2.00 (t, *J* = 5.4 Hz, 4H), 1.30 (t, *J* = 7.1 Hz, 3H).

### Step 2:

M24-3 (4.57 g, 16.25 mmol) was placed in a 100 mL single-necked flask, and ethanol (64 mL) was added. The mixture was cooled in an ice bath, and a solution of NaOH (2.60 g, 64.99 mmol) in water (8 mL) was slowly added. After the addition was complete, the mixture was heated to an external temperature of 80°C and refluxed overnight. After concentration under reduced pressure, DCM, (100 mL) and water (40 mL) were added to the residue. After thorough stirring, the mixture was allowed to stand for liquid separation. The upper aqueous phase was extracted once with DCM (50 mL). The combined organic phases were dried over anhydrous sodium sulfate, filtered, and the filtrate was concentrated under reduced pressure to afford compound M24-4 as an off-white solid (3.38 g, yield: 99.5%).

ESI-MS (*m*/*z*): 210.0 [M+H]⁺.

¹H NMR (500 MHz, CDCl₃) δ 6.66 (dd, *J* = 8.5, 4.5 Hz, 1H), 6.55 (dd, *J* = 8.3, 2.5 Hz, 1H), 6.48 (td, *J* = 9.6, 2.5 Hz, 1H), 3.12 - 3.04 (m, 4H), 2.07 (s, 2H), 2.04 - 1.98 (m, 4H).

### Step 3:

M24-4 (3.00 g, 14.34 mmol) was placed in a 100 mL single-necked flask, and DCM (30 mL) was added. The mixture was cooled in an ice bath, and fuming nitric acid (3 mL) was slowly added dropwise. After the addition was complete, the reaction was maintained in the ice bath for 2 hours. After the reaction was complete, DCM was removed by concentration under reduced pressure, and then the temperature was raised to a water bath temperature of 60°C to continue concentration to remove excess fuming nitric acid. After concentration was completed, compound M24-5 was obtained as a yellow solid, which was directly used in the next step.

ESI-MS (*m*/*z*): 255.1 [M+H]⁺.

### Step 4:

The yellow solid M24-5 obtained from Step 3 was placed in a 100 mL single-necked flask, mixed with DCM (50 mL), and cooled in an ice bath. Di-tert-butyl dicarbonate (4.69 g, 21.51 mmol) was added, followed by slow dropwise addition of triethylamine (4.35 mg, 43.01 mmol). After the addition was complete, the mixture was allowed to warm to room temperature and reacted overnight. Water (30 mL) was added to the reaction solution, and after thorough extraction, the mixture was allowed to stand for liquid separation. The upper aqueous phase was extracted once more with DCM (30 mL). The combined organic phases were dried over anhydrous sodium sulfate and concentrated, And purified by by column chromatography using PE:EA = 100:0-85:15 as the eluent to afford compound M24-6 as an off-white solid (4.59 g, yield: 85.3%).

ESI-MS (*m*/*z*): 299.0 [M-55]⁺.

¹H NMR (500 MHz, CDCl₃) *δ* 7.50 (d, *J* = 6.4 Hz, 1H), 6.70 (d, *J* = 10.3 Hz, 1H), 3.73 - 3.59 (m, 4H), 2.03 (t, *J =* 5.6 Hz, 4H), 1.51 (s, 9H).

### Step 5:

M24-6 (354 mg, 1.00 mmol) was placed in a 50 mL single-necked flask, and potassium carbonate (414 mg, 3.00 mmol), 4-hydroxymethylpiperidine (230 mg, 2.00 mmol), and acetonitrile (10 mL) were added sequentially. The mixture was heated to an external temperature of 60°C and reacted for 1 hour. Saturated brine (15 mL) was added to the reaction solution, which was then extracted with EA (30 mL). The organic phase was dried over anhydrous sodium sulfate, concentrated, and purified by column chromatography using PE:EA = 100:0- 50:50 as the eluent to afford compound M24-7 as an orange-yellow solid (449 mg, yield: 100%).

ESI-MS (m/z): 450.2 [M+H]⁺.

¹H NMR (500 MHz, CDCl₃) *δ* 7.41 (s, 1H), 6.69 (s, 1H), 3.68-3.62 (m, 4H), 3.59 (d, *J* = 6.3 Hz, 2H), 3.27 (d, *J* = 11.6 Hz, 2H), 2.83 (t, *J* = 11.4 Hz, 2H), 2.00 (t, *J* = 5.3 Hz, 4H), 1.84 (d, *J* = 12.4 Hz, 2H), 1.72-1.64 (m, 2H), 1.55 (td, *J* = 12.2, 2.8 Hz, 2H), 1.51 (s, 9H).

### Step 6:

M24-7 (440 mg, 0.98 mmol) was placed in a 50 mL single-necked flask, and methanol (10 mL) was added, followed by 10% palladium on carbon (100 mg). The atmosphere was replaced with a hydrogen balloon three times, and the mixture was heated to an external temperature of 35°C and reacted for 2 hours until the yellow color completely faded. The reaction solution was filtered through Celite, and the filter residue was washed with methanol until the filtrate was colorless. The combined filtrates were concentrated under reduced pressure to afford compound M24-8 as a grayish foamy solid (400 mg, yield: 97.4%), which was directly used in the next step.

ESI-MS (*m*/*z*): 420.3 [M+H]⁺.

### Step 7:

M24-8 (400 mg, 0.95 mmol), pyrazolo[1,5-*a*]pyrimidine-3-carboxylic acid (156 mg, 0.95 mmol), HATU (435 mg, 1.14 mmol), and DIEA (616 mg, 4.77 mmol) were placed in a 50 mL single-necked flask, and DCM (10 mL) was added. The mixture was stirred at room temperature for 2 hours. DCM (30 mL) and water (15 mL) were added to the reaction solution. After thorough stirring, the mixture was allowed to stand for liquid separation. The aqueous phase was extracted once with DCM (20 mL). The combined organic phases were dried over anhydrous sodium sulfate, concentrated under reduced pressure, and purified by column chromatography using DCM:MeOH = 100:0 - 98:2 as the eluent to afford compound M24 as a yellow solid (471 mg, yield: 87.5%).

ESI-MS (*m*/*z*): 565.3 [M+H]⁺.

¹H NMR (500 MHz, CDCl₃) *δ* 10.67 (s, 1H), 8.81 (d, *J* = 4.5 Hz, 3H), 8.13 (s, 1H), 7.04 (dd, *J* = 6.2, 4.8 Hz, 1H), 6.80 (s, 1H), 3.72 - 3.58 (m, 6H), 3.20-3.05 (m, 2H), 2.85-2.70 (m, 2H), 2.05-1.92 (m, 4H), 1.90-1.76 (m, 4H), 1.75-1.55 (m, 4H), 1.51 (s, 9H).

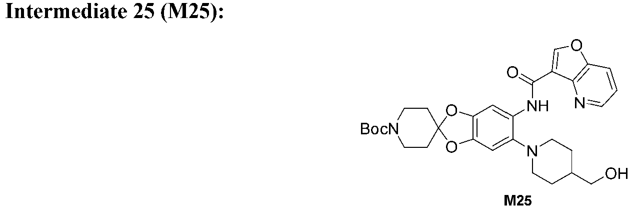

Intermediate 25 (M25) was synthesized using a method analogous to that of Intermediate 24 (M24).

ESI-MS (m/z): 565.3 [M+H]⁺.

¹H NMR (500 MHz, CDCl₃) δ 11.12 (s, 1H), 8.72 (d, *J* = 4.6 Hz, 1H), 8.63 (s, 1H), 8.12 (s, 1H), 7.90 (d, *J* = 8.3 Hz, 1H), 7.38 (dd, *J* = 8.3, 4.8 Hz, 1H), 6.83 (s, 1H), 3.70-3.60 (m, 6H), 3.14 (d, *J* = 10.5 Hz, 2H), 2.84-2.71 (m, 2H), 2.05-1.95 (m, 4H), 1.90 - 1.75 (m, 4H), 1.76 - 1.63 (m, 2H), 1.51 (s, 9H).

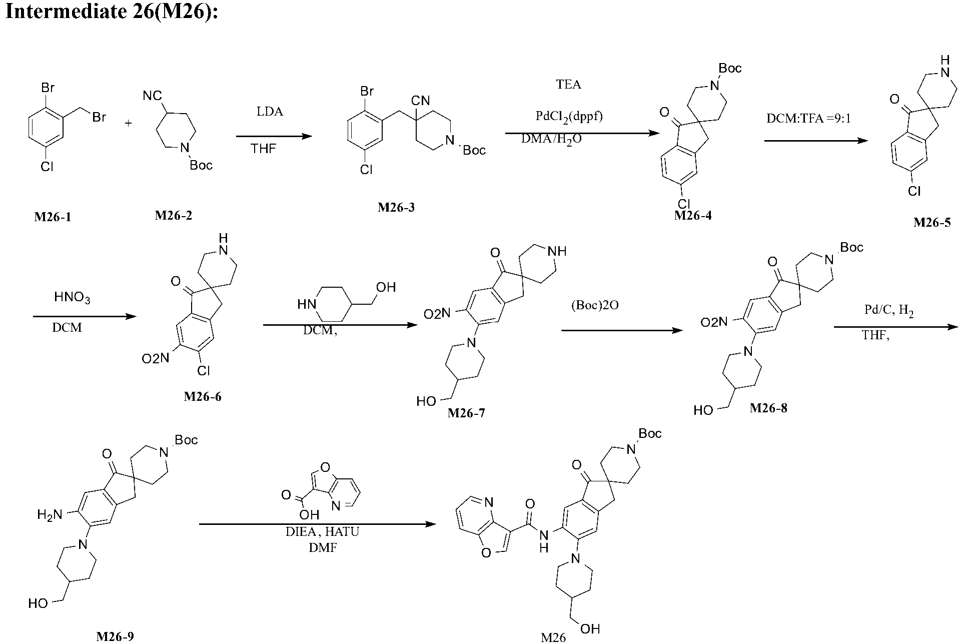

### Step 1

LDA (2.46 mL, 18.6 mmol, 5.29 eq) was added dropwise to a mixture of THF (7 mL) and M26-2 (0.61 g, 2.92 mmol, 0.83 eq) at -72°C. After complete addition, the reaction mixture was stirred at -72°C for 1 hour, followed by dropwise addition of M26-1 (1.00 g, 3.52 mmol, 1.00 eq). The mixture was then stirred at room temperature for 16 hours. The reaction was quenched by slow addition of water (10 mL), and the aqueous layer was extracted with ethyl acetate (10 mL × 3). The combined organic phases were washed with brine (10 mL), dried over anhydrous sodium sulfate, and purified by column chromatography (SiO₂, petroleum ether/ethyl acetate = 100:1 -10:1) to afford M26-3 (920 mg, 2.22 mmol, 63.2% yield) as a pale yellow viscous oil.

ESI-MS (*m*/*z*): 313.0 [M-100+1]⁺.

¹H NMR (400 MHz, CDCl₃) *δ* 7.54 (d, *J* = 8.58 Hz, 1H), 7.48 (d, *J* = 2.50 Hz, 1H), 7.17 (dd, *J* = 8.58, 2.50 Hz, 1H), 4.12 - 4.29 (m, 2H), 3.10 (s, 2H), 3.01 (br s, 2H), 1.89 (br d, *J* = 12.99 Hz, 2H), 1.67 (td, *J* = 13.11, 4.29 Hz, 2H), 1.47 (s, 9H).

### Step 2

M26-3 (20.0 g, 48.34 mmol, 1.00 eq) was dissolved in a mixed solvent of DMA (140 mL) and water (14.0 mL). TEA (26.9 mL, 193 mmol, 4.00 eq) and 1,1-bis(diphenylphosphino)ferrocene palladium dichloride (3.54 g, 4.83 mmol, 0.10 eq) were added, and the mixture was degassed with nitrogen before heating at 130°C for 12 hours. Water (150 mL) was added to precipitate a solid, which was filtered. The filtrate was extracted with ethyl acetate (150 mL × 3), washed with saturated brine, dried over anhydrous sodium sulfate, and concentrated under reduced pressure. and purified by column chromatography (SiO₂, petroleum ether/ethyl acetate = 100:1 to 10:1) to afford M26-4 (13.00 g, 31.97 mmol, 66.2% yield) as a pale yellow solid.

ESI-MS (*m*/*z*): 280.1 [M-55]⁺.

### Step 3

M26-4 (14.0 g, 41.69 mmol, 1.00 eq) was placed in a 100 mL single-neck flask, and DCM (9 mL) followed by 4M HCl in dioxane (20.0 mL) were added. The reaction mixture was stirred at room temperature for 12 hours, then concentrated under reduced pressure. And purified by HPLC to afford M26-5 (2.15 g, 8.97 mmol, 21.5% yield) as a pale yellow solid.

ESI-MS (*m*/*z*): 236.1 [M+1]⁺.

¹H NMR (400 MHz, CDCl₃) *δ* 7.70 (d, *J=* 8.13 Hz, 1H) 7.46 (d, *J* = 0.63 Hz, 1H), 7.36 (dt, *J=* 8.19, 0.78 Hz, 1H), 3.18 (dt, *J* = 13.01, 3.63 Hz, 2H), 3.07 (s, 2H), 2.80 (td, *J* = 12.41, 2.69 Hz, 2H), 1.85 - 1.93 (m, 2H), 1.30 - 1.43 (m, 2H).

### Step 4

M26-5 (1.00 g, 4.24 mmol, 1.00 eq) was placed in a 100 mL single-neck flask and DCM (7.0 mL) was added. Fuming nitric acid (10 mL, 220.61 mmol, 53 eq) was slowly added dropwise at 0 °C. After complete addition, the reaction mixture was stirred at 0 °C for 10 minutes, then warmed to 45 °C and stirred for 12 hours. The reaction mixture was concentrated under reduced pressure to afford M26-6 (1.30 g, 4.63 mmol) as an orange solid.

ESI-MS *(m*/*z):* 281.0 [M+1]⁺.

### Step 5

Hexahydropyridin-4-yl methanol (4.1 mL, 35.09 mmol, 5.00 eq) was dissolved in DCM (14 mL), and M26-6 (1.97 g, 7.02 mmol, 1.00 eq) was added. The reaction mixture was stirred at 25 °C for 2 hours. The resulting reaction mixture containing M26-7 (2.52 g, 7.01 mmol) was used directly in the next step without further purification.

ESI-MS (*m*/*z*): 360.2 [M+1]⁺.

### Step 6

Boc anhydride (4.5 mL, 21.03 mmol, 3.00 eq) was directly added to the above reaction mixture. The mixture was stirred at 25 °C for 12 hours. The reaction was concentrated under reduced pressure and purified by column chromatography (SiO₂, petroleum ether/ethyl acetate = 5:1-0:1) to afford M26-8 (1.58 g, 3.09 mmol) as a yellow solid in 44.1% yield over two steps.

ESI-MS (m/z): 460.2 [M+1]⁺.

### Step 7

In a 250 mL single-neck flask under argon atmosphere, THF (20 mL) was added, followed by wet palladium on carbon (1.1 g, 10.34 mmol, 3.01 eq). M26-8 (1.58 g, 3.44 mmol, 1.00 eq) dissolved in THF (20 mL) was then added. The reaction mixture was stirred at room temperature under hydrogen atmosphere (15 psi) for 3 hours. The reaction was filtered and the filtrate was concentrated under reduced pressure to afford M26-9 (1.50 g, 3.32 mmol, 96.5% yield) as a white solid.

ESI-MS (m/z): 430.3 [M+1]⁺.

### Step 8

M26-9 (1.50 g, 3.32 mmol, 1.0 eq), furano[3,2-b]pyridine-3-carboxylic acid (569 mg, 3.49 mmol, 1.0 eq), DMF (10 mL), DIEA (1.2 mL, 6.98 mmol, 2.0 eq), and HATU (1.46 g, 3.84 mmol, 1.10 eq) were added to a 100 mL single-neck flask. The reaction mixture was stirred at 25 °C for 2 hours. The mixture was concentrated under reduced pressure and purified by column chromatography (petroleum ether/ethyl acetate = 3:1-1:2) to afford compound M26 (1.90 g, 2.24 mmol, 64.2% yield) as a white solid.

ESI-MS (*m*/*z*): 575.3 [M+1]⁺.

¹H NMR (400 MHz, CDCl₃) *δ* 10.92 (s, 1H), 8.84 (s, 1H), 8.68 (dd, *J* = 4.77, 1.19 Hz, 1H), 8.63 (s, 1H), 7.90 (dd, *J* = 8.40, 1.13 Hz, 1H), 7.38 (dd, *J* = 8.40, 4.83 Hz, 1H), 7.21 (s, 1H), 4.14 (br d, *J* = 1.55 Hz, 2H), 3.63 (d, *J=* 5.36 Hz, 2H), 3.30 (br d, *J* = 11.80 Hz, 2H), 3.01 (s, 4H), 2.72 - 2.80 (m, 2H), 1.85 - 1.98 (m, 3H), 1.35 - 1.55 (m, 13H), 0.99 (t, *J* = 7.27 Hz, 2H).

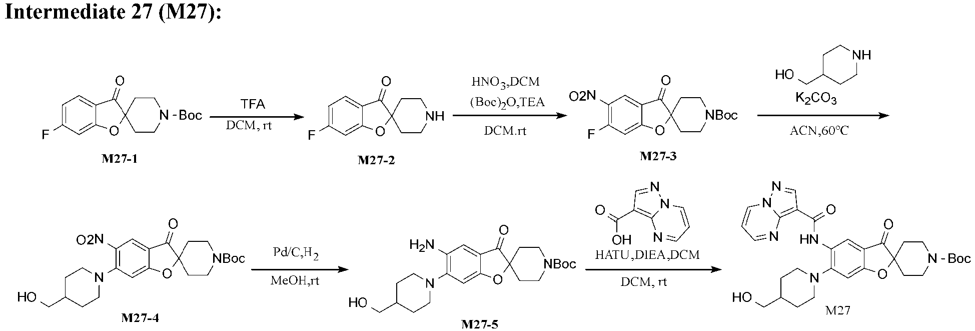

### Step 1:

M27-1 (0.76 g, 2.49 mmol) (prepared according to the procedure described in Patent WO2019183367A1, pp. 309-310) and DCM (6 mL) were added to a 50 mL single-neck flask. Trifluoroacetic acid (TFA, 1 mL) was added dropwise, and the reaction mixture was stirred at 25 °C for 1 h. The mixture was concentrated under reduced pressure to afford M27-2 (1.0 g, 181% yield) as a pale yellow solid.

ESI-MS (*m*/*z*): 222.0[M+H]⁺.

### Step 2:

M27-2 (1.0 g, 2.49 mmol) was dissolved in DCM (12.5 mL) in a 50 mL single-neck flask and cooled in an ice bath. Fuming nitric acid (25 mL) was added dropwise, and the mixture was stirred at 0 °C for 10 min, then at room temperature for 1 h. After completion of nitration, the reaction was concentrated under reduced pressure. The residue was dissolved in DCM (8 mL), cooled to 0 °C, and treated with di-tert-butyl dicarbonate (0.81 g, 3.72 mmol) followed by dropwise addition of triethylamine (0.75 g, 7.44 mmol). The mixture was stirred at 0 °C for 10 min, then at room temperature for 2 h. The reaction was quenched with saturated ammonium chloride solution (10 mL) and saturated sodium chloride solution (10 mL), extracted with ethyl acetate (10 mL × 3), and the combined organic layers were dried over anhydrous sodium sulfate. The solvent was removed under reduced pressure, and the crude product was purified by column chromatography (PE:EA = 100:0 -85:15) to afford M27-3 (0.4 g, 44.0% yield) as a white solid.

ESI-MS (m/z-100): 267.0[M+H]⁺.

¹H NMR (500 MHz, CDCl₃) δ 8.52 (d, *J* = 7.7 Hz, 1H), 7.04 (d, *J* = 10.6 Hz, 1H), 4.15 (d, *J* = 7.2 Hz, 2H), 3.26 (s, 2H), 2.97 (s, 1H), 2.90 (s, 1H), 2.03 - 1.97 (m, 2H), 1.51 (s, 9H).

### Step 3:

M27-3 (400 mg, 1.09 mmol), 4-(hydroxymethyl)piperidine-1-carboxylic acid tert-butyl ester (252 mg, 2.18 mmol), and potassium carbonate (377 mg, 2.73 mmol) were combined in acetonitrile (4 mL) in a 10 mL single-neck flask and stirred at 60 °C for 16 h. The mixture was concentrated under reduced pressure and purified by column chromatography (PE:EA = 100:0-60:40) to afford M27-4 (300 mg, 59.5% yield) as a yellow solid.

ESI-MS (*m*/*z*): 462.2[M+H]⁺.

¹H NMR (500 MHz, CDCl₃) *δ* 8.21 (s, 1H), 6.60 (s, 1H), 4.15 (s, 2H), 3.60 (d, *J* = 6.2 Hz, 2H), 3.47 (d, *J* = 12.8 Hz, 2H), 3.22 (s, 2H), 3.05 (t, *J* = 11.8 Hz, 2H), 2.00 - 1.94 (m, 2H), 1.88 (d, *J* = 12.8 Hz, 2H), 1.84 - 1.76 (m, 1H), 1.64 - 1.58 (m, 4H), 1.51 (s, 9H).

### Step 4:

M27-4 (2700 mg, 5.85 mmol) and Pd/C (600 mg) were suspended in methanol (100 mL) in a 250 mL single-neck flask. The mixture was degassed and stirred under a hydrogen atmosphere at room temperature for 5 h. The catalyst was removed by filtration, and the filtrate was concentrated under reduced pressure to afford M27-5 (2524 mg, 100% yield) as a white solid.

ESI-MS (m/z): 432.3[M+H]⁺.

### Step 5:

M27-5 (1100 mg, 2.55 mmol), pyrazolo[1,5-a]pyrimidine-3-carboxylic acid (830 mg, 5.1 mmol), HATU (1450 mg, 3.82 mmol), DIEA (1290 mg, 12.75 mmol), and DCM (10 mL) were combined in a 50 mL single-neck flask and stirred at room temperature for 16 h. The reaction was quenched with saturated sodium chloride solution (120 mL), extracted with ethyl acetate (120 mL × 3), and concentrated under reduced pressure. The crude product was purified by column chromatography (PE:EA = 100:0 - 50:50) to afford M27 (500 mg, 34.0% yield) as a yellow solid.

ESI-MS (*m*/*z*):577.3[M+H]⁺.

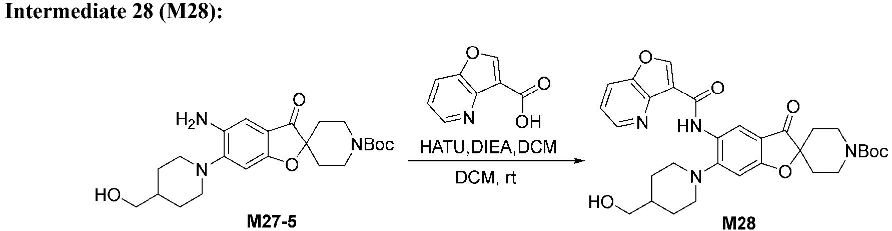

M27-5 (1400 mg, 3.24 mmol), furo[3,2-b]pyridine-3-carboxylic acid (528 mg, 3.24 mmol), HATU (1850 mg, 4.87 mmol), DIEA (1310 mg, 12.98 mmol), and DCM (20 mL) were combined in a 50 mL single-neck flask and stirred at room temperature for 16 h. The reaction was quenched with saturated sodium chloride solution (120 mL), extracted with ethyl acetate (120 mL × 3), and concentrated under reduced pressure. The crude product was purified by column chromatography (PE:EA = 100:0 ~50:50) to afford M28 (900 mg, 95.3% yield) as a yellow solid.

ESI-MS (*m*/*z*): 577.2[M+H]⁺.

¹H NMR (500 MHz, CDCl₃) *δ* 10.59 (s, 1H), 8.59 (s, 1H), 8.55 (d, *J* = 4.7 Hz, 1H), 8.50 (s, 1H), 7.78 (d, *J* = 8.4 Hz, 1H), 7.26 (dd, *J* = 8.3, 4.8 Hz, 1H), 6.74 (s, 1H), 4.10 - 3.99 (m, 2H), 3.48 (d, *J* = 5.0 Hz, 2H), 3.25 (d, *J* = 11.6 Hz, 2H), 3.10 - 3.04 (m, 2H), 2.63 (t, *J* = 10.8 Hz, 2H), 1.89 - 1.86 (m, 1H), 1.83 (dd, *J* = 12.9, 4.1 Hz, 2H), 1.71 (d, *J* = 10.0 Hz, 2H), 1.60 (d, *J* = 7.4 Hz, 2H), 1.48 (s, 2H), 1.38 (s, 9H).

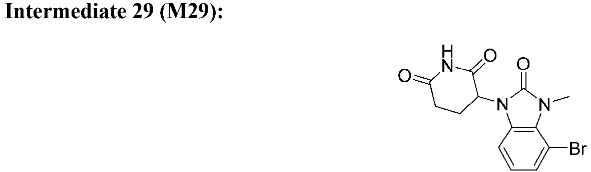

Prepared according to the procedure described in Patent WO2021247897A1, paragraph 443(Intermediate HP).

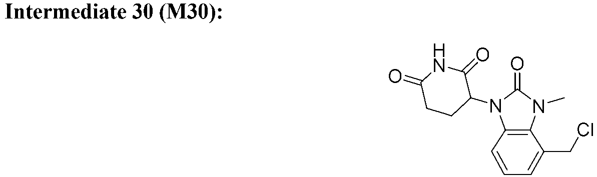

Prepared according to the procedure described in Patent WO2020206424A1, page 573 (synthesis of B32-2). 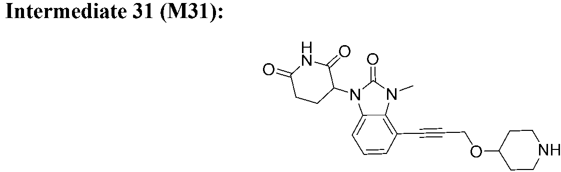 and its salt forms

Prepared according to the procedure described in Patent WO2021247899A1, page 90 (synthesis of intermediate APT).

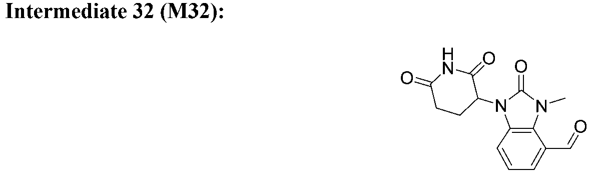

Prepared according to the procedure described in Patent WO2021158634A1, page 195 (synthesis method of intermediate WW).

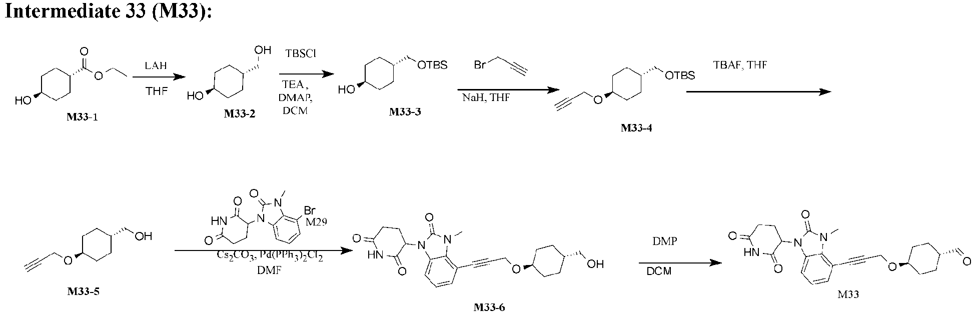

### Step 1:

M33-1 (30.0 g, 174 mmol) was placed in a 1 L three-neck flask and dissolved in tetrahydrofuran (300 mL). The reaction mixture was cooled to 0°C, and a solution of lithium aluminum hydride in THF (105 mL, 261 mmol, 2.5 M) was added dropwise at 0°C. After complete addition, the reaction was stirred at 25°C overnight. The mixture was then cooled back to 0°C, and sodium sulfate decahydrate was slowly added in an ice bath to quench the reaction over 30 minutes. After thorough quenching, the mixture was filtered, and the filtrate was concentrated under reduced pressure to afford M33-2 (22.0 g, 169 mmol) as a white solid. Yield: 97.0%.

ESI-MS (*m*/*z*):No MS response.

¹H NMR (400 MHz, CD₃OD) *δ* 3.64 - 3.42 (m, 1H), 3.36 - 3.34 (m, 2H), 2.03 - 1.90 (m, 2H), 1.87 - 1.76 (m, 2H), 1.45 - 1.33 (m, 1H), 1.30 - 1.16 (m, 2H), 1.06 - 0.92 (m, 2H).

### Step 2:

M33-2 (43.5 g, 334.13 mmol) was dissolved in dichloromethane (400 mL). Chlorodimethyl(2-methylprop-2-yl)silane (57.9 mL, 334.13 mmol, 1.00 eq), triethylamine (35.5 g, 350.83 mmol, 1.05 eq), and DMAP (0.41 g, 3.34 mmol, 0.01 eq) were added to the reaction mixture, which was then stirred at 25°C for 12 h. Water (400 mL) was added, and the aqueous phase was extracted with DCM (400 mL × 3). The combined organic layers were dried over anhydrous sodium sulfate and concentrated under reduced pressure. The crude product was purified by column chromatography (PE/EA = 1:1) to afford the desired intermediate.

ESI-MS (*m*/*z*): No MS response.

### Step 3:

M33-3 (15.25 g, 62.38 mmol) was dissolved in THF (150 mL). The reaction mixture was cooled to 0 °C, and under a nitrogen atmosphere, NaH (3.74 g, 93.58 mmol, 1.5 eq) was added slowly. After the addition was complete, the mixture was stirred at 0 °C for 30 minutes. Propargyl bromide (8.1 mL, 74.86 mmol, 1.2 eq) was then added to the mixture. The reaction mixture was stirred at 25 °C for 12 hours. It was then cooled to 0 °C, and water was added slowly to quench the NaH. After quenching, the mixture was extracted with ethyl acetate (200 mL × 3). The combined organic layers were back-washed once with saturated brine (300 mL), dried over anhydrous sodium sulfate, and concentrated under reduced pressure. And purified by column chromatography (PE/EA = 10:1-5:1) to afford M33-4 (5.33 g, 18.87 mmol, 30.2% yield) as a yellow oil.

ESI-MS (*m*/*z*):No MS response.

### Step 4:

M33-4 (40.6 g, 143.72 mmol) was placed in a 1 L single-neck flask containing THF (400 mL). The mixture was cooled to 0 °C, and TBAF (287.4 mL, 287.43 mmol) was added. The mixture was stirred at room temperature overnight. The reaction mixture was concentrated under reduced pressure and purified by column chromatography (PE/EA = 10:1 - 2:1) to afford M33-5 (25 g, 148.60 mmol) as a brown oil.

ESI-MS (*m*/*z*): No MS response.

¹H NMR (400 MHz, DMSO-*d₆*) *δ* 4.17 (d, *J* = 2.38 Hz, 2H), 3.34 - 3.39 (m, 2H), 3.24 (br t, *J* = 5.57 Hz, 2H), 2.54 (br s, 1H), 1.97 - 2.07 (m, 3H), 1.72 - 1.85 (m, 2H), 1.27 - 1.42 (m, 1H), 1.06 - 1.18 (m, 2H), 0.84 - 1.00 (m, 2H).

### Step 5:

M33-5 (6 g, 35.66 mmol, 2.0 eq) was placed in a 100 mL single-neck flask containing DMF (60 mL) and M29 (6.03 g, 17.83 mmol). Cesium carbonate (11.62 g, 35.66 mmol, 2.0 eq) and bis(triphenylphosphine)palladium(II) chloride (PdCl₂(PPh₃)₂, 1.39 g, 1.78 mmol, 0.1 eq) were added to the reaction mixture. The mixture was stirred at 100 °C under a nitrogen atmosphere for 2 hours. THF (150 mL) was added to the reaction mixture and stirred for 1 hour. The mixture was filtered, and the filter cake was washed with THF (50 mL × 2). The combined filtrates were concentrated under reduced pressure and purified by column chromatography (Petroleum ether : THF = 3:1 - 1:2) to afford M33-6 (2.37 g, 5.57 mmol, 31.2% yield) as a yellow solid.

ESI-MS (*m*/*z*): 426.3 [M+1]⁺.

¹H NMR (400 MHz, DMSO-*d₆*) *δ* 11.15 (s, 1H), 7.18 (d, *J* = 7.8 Hz, 1H), 7.12 (d, *J* = 7.7 Hz, 1H), 7.04 (t, *J* = 7.8 Hz, 1H), 5.41 (dd, *J* = 12.6, 5.3 Hz, 1H), 4.47 (s, 2H), 4.41 (t, *J* = 5.3 Hz, 1H), 3.65 (s, 3H), 3.43 (td, *J* = 10.7, 5.3 Hz, 1H), 3.21 (t, *J =* 5*.*7 Hz, 2H), 2.97 - 2.82 (m, 1H), 2.71 (ddd, *J* = 35.6, 22.0, 10.6 Hz, 2H), 2.05 (d, *J* = 11.3 Hz, 3H), 1.76 (d, *J* = 12.2 Hz, 2H), 1.36 - 1.25 (m, 1H), 1.20 - 1.09 (m, 2H), 0.92 (q, *J* = 11.0 Hz, 2H).

### Step 6:

M33-6 (0.70 g, 1.65 mmol) was dissolved in DCM (10.0 mL). The solution was cooled to 0 °C, and Dess-Martin periodinane (1.05 g, 2.47 mmol, 1.50 eq) was added. After the addition was complete, the reaction mixture was stirred at room temperature for 12 hours. Saturated aqueous sodium bicarbonate solution was added to quench the Dess-Martin oxidant. The mixture was filtered through Celite. The filtrate was extracted with DCM (20.0 mL × 3). The combined organic layers were back-washed with saturated brine (30 mL), dried over anhydrous sodium sulfate, and concentrated under reduced pressure. And purified by column chromatography (PE/EA = 1:1-1:5) to afford M33 (340 mg, 0.80 mmol, 48.8% yield) as a pale yellow solid.

ESI-MS (*m*/*z*): 424.1 [M+1]⁺.

¹H NMR (400 MHz, CDCl₃) δ 9.59 (s, 1H), 8.08 (s, 1H), 7.10 (d, *J=* 7.9 Hz, 1H), 6.92 (t, *J=* 7.9 Hz, 1H), 6.69 (d, *J* = 7.9 Hz, 1H), 5.13 (dd, *J* = 12.5, 5.3 Hz, 1H), 4.39 (s, 2H), 3.71 (s, 3H), 3.51 - 3.41 (m, 1H), 2.91-2.60 (m, 3H), 2.24 - 2.12 (m, 2H), 2.11 - 1.98 (m, 4H), 1.40 - 1.26 (m, 4H).

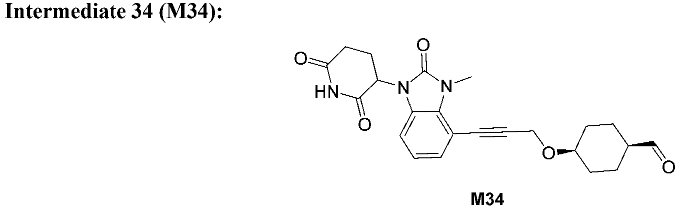

Using (1s,4s)-ethyl 4-hydroxycyclohexanecarboxylate as the starting material, intermediate 34 (M34) was synthesized via the same method as intermediate 33 (M33).

ESI-MS (m/z): 424.2 [M+1]⁺.

¹H NMR (400 MHz, CDCl₃) *δ* 9.65 (s, 1H), 8.12 (br s, 1H), 7.17 (d, *J* = 7.88 Hz, 1H), 6.94 - 7.04 (m, 1H), 6.73 - 6.82 (m, 1H), 5.20 (dd, *J* = 12.57, 5.32 Hz, 1H), 4.39 - 4.45 (m, 2H), 3.75 - 3.82 (m, 4H), 2.67 - 2.96 (m, 3H), 2.15 - 2.35 (m, 2H), 1.87 - 2.09 (m, 4H), 1.62 - 1.76 (m, 4H).

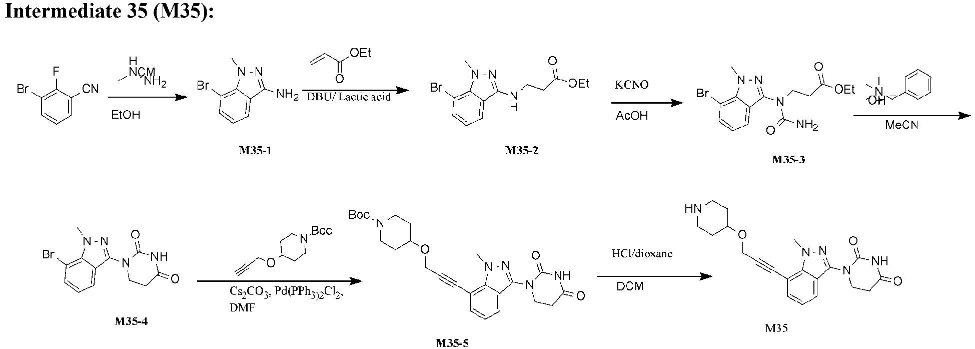

### Step 1:

3-Bromo-2-fluorobenzonitrile (100 g, 500 mmol) was placed in a 2 L three-necked round-bottom flask, and ethanol (700 mL) was added. Methylhydrazine (267.8 mL, 1999.90 mmol, 4 eq, 40% aqueous solution) was slowly added dropwise to the mixture. After completion of the addition, the reaction was heated to 80 °C and stirred overnight. The mixture was vacuum concentrated, and water (500 mL) was added. The resulting mixture was extracted with ethyl acetate (500 mL × 3). The combined organic phases were dried over anhydrous sodium sulfate, filtered, and concentrated under reduced pressure. And purified by column chromatography (eluent: PE/EA = 5:1-1:1) to afford M35-1 (51.0 g, 225.58 mmol) as a yellow solid. Yield: 45.1%.

ESI-MS *(m*/*z):* 226.0 [M+1]⁺.

¹H NMR (400 MHz, DMSO-*d₆*) *δ* 7.72 (dd, *J* = 0.8, 7.9 Hz, 1H), 7.48 (dd, *J* = 0.8, 7.4 Hz, 1H), 6.82 (t, J = 7.7 Hz, 1H), 5.59 (s, 2H), 4.01 (s, 3H).

### Step 2:

DBU (33.7 mL, 225.58 mmol) and lactic acid (16.9 mL, 225.58 mmol) were mixed in equimolar amounts in a 100 mL round-bottom flask and stirred at 25 °C overnight. Ethyl acrylate (184.1 mL, 1691.88 mmol, 7.5 eq) and M35-1 (51.0 g, 225.58 mmol, 1 eq) were placed in a 1 L round-bottom flask, and the equimolar mixture of lactic acid (16.9 mL, 225.58 mmol) and DBU (33.7 mL, 225.58 mmol) was added. The reaction was stirred at 80 °C for 120 h. Water (500 mL) was added to the system, and the mixture was extracted with ethyl acetate (500 mL × 3). The combined organic phases were dried over anhydrous sodium sulfate, filtered, and concentrated under reduced pressure. And purified by column chromatography (eluent: PE/EA = 10:1 - 3:1) to afford M35-2 (30.0 g, 91.97 mmol, yield: 40.8%.) as a brown oil.
ESI-MS (*m*/*z*): 326.0 [M+1]⁺.

### Step 3:

M35-2 (30.0 g, 91.97 mmol, 1 eq) was placed in a 500 mL round-bottom flask, and glacial acetic acid (120 mL) was added. Potassium cyanate (10.9 mL, 275.91 mmol, 3 eq) was then added to the reaction mixture, and the reaction was stirred at 25 °C overnight. The mixture was concentrated under reduced pressure to remove AcOH, and water (100 mL) was poured into the system. The mixture was extracted with ethyl acetate (100 mL × 3). The combined organic phases were dried over anhydrous sodium sulfate, filtered, and concentrated under reduced pressure. And purified by column chromatography (eluent: DCM/MeOH = 30:1 - 20:1) to afford M35-3 (14.0 g, 37.92 mmol) as a brown solid. Yield: 41.2%.

ESI-MS (*m*/*z*): 369.0 [M+1]⁺.

### Step 4:

M35-3 (5.00 g, 13.54 mmol, 1.0 eq) was placed in a 100 mL round-bottom flask, and acetonitrile (25.0 mL) was added. Benzyltrimethylammonium hydroxide (3.7 mL, 20.31 mmol, 1.5 eq, 40 wt.% methanol solution) was added dropwise to the mixture, and the reaction was stirred at 25 °C for 45 minutes. Water (50 mL) was poured into the system, and the mixture was extracted with ethyl acetate (50 mL × 3). The combined organic phases were dried over anhydrous sodium sulfate, filtered, and concentrated under reduced pressure. And purified by column chromatography (eluent: PE/EA = 5:1 to 1:1) to afford M35-4 (2.00 g, 6.19 mmol) as a pale yellow solid. Yield: 45.7%.

ESI-MS *(m*/*z):* 323.0 [M+1]⁺.

### Step 5:

M35-4 (1.00 g, 3.09 mmol, 1.0 eq) was placed in a 100 mL round-bottom flask, and DMF (10 mL) was added. tert-Butyl 4-(prop-2-yn-1-yloxy)piperidine-1-carboxylate (0.89 g, 3.71 mmol, 1.2 eq) and cesium carbonate (2.02 g, 6.19 mmol, 2.0 eq) were added sequentially. After purging with nitrogen three times, bis(triphenylphosphine)palladium(II) dichloride (0.24 g, 0.31 mmol, 0.1 eq) was added to the mixture. The reaction was stirred at 80 °C for 2 hours under nitrogen protection. The mixture was vacuum concentrated to remove DMF, and water (50 mL) was poured into the system. The mixture was extracted with ethyl acetate (50 mL × 3). The combined organic phases were dried over anhydrous sodium sulfate, filtered, and concentrated under reduced pressure. And purified by column chromatography (eluent: PE/EA = 5:1 - 1:1) to afford M35-5 (130 mg, 0.27 mmol) as a brown oil. Yield: 8.7%.

ESI-MS *(m*/*z):* 426.3 [M-55]⁻

### Step 6:

M35-5 (130 mg, 0.27 mmol, 1.0 eq) was placed in a 50 mL round-bottom flask, and DCM (6.00 mL) was added. The reaction mixture was cooled to 0 °C, and hydrochloric acid in dioxane (2.00 mL) was slowly added dropwise. The reaction was stirred at 25 °C for 12 h. The mixture was concentrated under reduced pressure to afford M35 (100 mg, 0.26 mmol) as a brown solid. Yield: 97.1%.

ESI-MS (m/z): 382.2 [M+1]⁺.

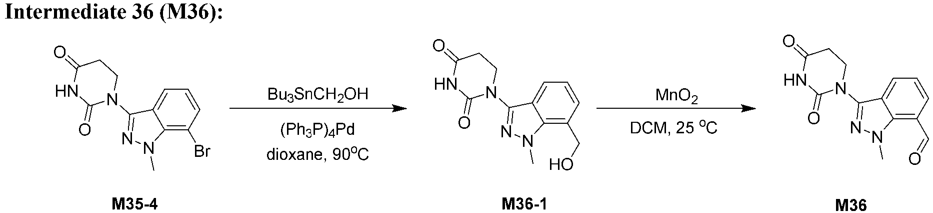

### Step 1:

M35-4 (1.00 g, 3.09 mmol) was placed in a 50 mL single-necked flask. Dioxane (10.0 mL) was added, followed by (tributylstannyl)methanol (1.49 g, 4.64 mmol, 1.5 eq). The reaction mixture was purged with N₂ three times. Tetrakis(triphenylphosphine)palladium(0) (0.18 g, 0.15 mmol) was added to the reaction mixture. The reaction was stirred at 90°C for 12 h. Water (30.0 mL) was poured into the reaction mixture, followed by extraction with ethyl acetate (EA) (30.0 mL × 3). The combined organic phases were dried over anhydrous sodium sulfate, filtered, and concentrated under reduced pressure. Purification by column chromatography (eluent: EA:MeOH = 20:1- 9:1) afforded M36-1 (0.1 g, 0.36 mmol, yield 11.8%) as a pale yellow oil.

ESI-MS *(m*/*z):* 275.2 [M+1]⁺.

### Step 2:

M36-1 (100 mg, 0.36 mmol) was placed in a 50 mL single-necked flask. Dichloromethane (DCM) (10.0 mL) was added, followed by manganese(IV) oxide (MnO₂) (626 mg, 7.20 mmol, 20 eq). After addition, the reaction was stirred at 25°C for 12 h. The reaction mixture was filtered, and the filter cake was washed with DCM (20.0 mL × 5). The filtrate was concentrated under reduced pressure to afford M36 (99 mg, 0.36 mmol, yield 99.7%) as a yellow solid.

ESI-MS *(m*/*z):* 273.2 [M+1]⁺

¹H NMR (400 MHz, DMSO-*d₆*) δ 10.77 - 10.45 (m, 1H), 10.28 (s, 1H), 8.05 (br dd, *J* = 7.8, 11.9 Hz, 2H), 7.35 (t, *J* = 7.6 Hz, 1H), 4.29 (s, 3H), 3.94 (br t*, J=* 6.4 Hz, 2H), 2.78 (br t, *J* = 6.4 Hz, 2H).

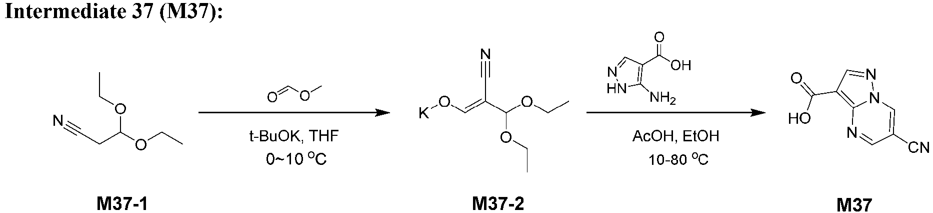

### Step 1:

M37-1 (5.00 g, 34.9 mmol, 1.0 eq) was placed in a 250 mL single-necked flask. THF (50 mL) and methyl formate (2.73 g, 45.3 mmol, 1.3 eq) were added. A solution of t-BuOK in THF (38.4 mL, 38.4 mmol, 1M, 1.1 eq) was added dropwise at 0°C. After stirring for 2 h, n-hexane (40 mL) was added. The mixture was warmed to 10°C and stirred for an additional 0.1 h. White solids precipitated upon standing. The mixture was filtered, and the filter cake was washed with a mixed solvent (THF : Hexane = 1 : 1). The collected solids were concentrated under reduced pressure to afford M37-2 (900 mg, 4.30 mmol, yield 12.3%) as a white solid.

¹H NMR (400 MHz, CD₃OD) *δ* 5.14 - 5.37 (m, 1H), 4.74 (s, 1H), 3.63 (q, *J* = 7.11 Hz, 4H), 1.12 - 1.28 (m, 6H).

### Step 2:

3-Aminopyrazole-4-carboxylic acid (607 mg, 4.78 mmol, 1.0 eq) was placed in a 40 mL single-necked flask. Acetic acid (AcOH) (5.5 mL, 95.56 mmol, 20 eq) was added, and the mixture was stirred at 10°C for 0.1 h. A solution of M37-2 (1000 mg, 4.78 mmol, 1.0 eq) in ethanol (EtOH) (16 mL) was added dropwise to the reaction mixture. The temperature was raised to 80°C and stirred for 2 h. White solids precipitated. The mixture was filtered, and the collected solids were concentrated under reduced pressure to afford M37 (520 mg, 2.76 mmol, yield 57.8%) as a white solid.

¹H NMR (400 MHz, DMSO-*d₆*) *δ* 10.05 (d, J = 2.03 Hz, 1H), 8.96 (d, J = 2.03 Hz, 1H), 8.69 (s, 1H)

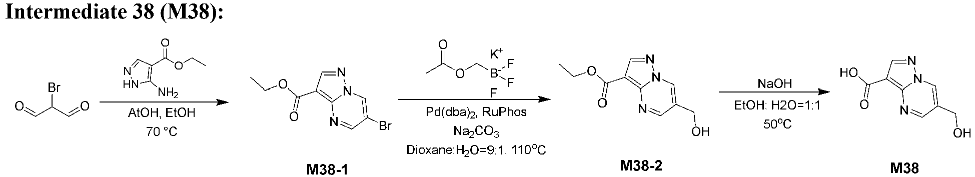

### Step 1:

2-Bromomalondialdehyde (5.86 g, 38.8 mmol, 1.0 eq) was placed in a 100 mL single-necked flask. EtOH (60 mL) was added. After warming to 70°C, ethyl 5-amino-1H-pyrazole-4-carboxylate (6.02 g, 38.8 mmol, 1.0 eq) and AcOH(28.9 mL, 504 mmol, 13 eq) were added. The mixture was stirred at 70°C for 0.5 h. The reaction mixture was placed in an ice bath, and brown solids gradually precipitated. The mixture was filtered, and the filter cake was concentrated under reduced pressure to afford M38-1 (5.80 g, 21.4 mmol, yield: 55.3%) as a brown solid.

ESI-MS *(m*/*z):* 270.0, 272.0 [M+H]⁺

¹H NMR (400 MHz, DMSO-*d₆*) *δ* 9.76 (d, *J* = 2.13 Hz, 1H), 8.91 (d, *J=* 2.13 Hz, 1H), 8.61 (s, 1H), 4.30 (q, *J = 7.05* Hz, 2H), 1.31 (t, *J* 7.07 Hz, 3H)

### Step 2:

M38-1 (1.00 g, 3.70 mmol, 1.0 eq) was placed in a 100 mL single-necked flask. Dioxane (9 mL) and water (1 mL) were added. Potassium methyltrifluoroborate (870 mg, 1.3 eq), Pd(dba)₂ (320 mg, 0.56 mmol, 0.15 eq), RuPhos (0.52 g, 1.11 mmol, 0.3 eq), and Na₂CO₃ (0.59 g, 5.55 mmol, 1.5 eq) were added to the reaction mixture. The reaction was stirred at 110°C for 10 h. The reaction mixture was concentrated under reduced pressure. Water (30 mL) was added, and the mixture was extracted with ethyl acetate (EA) (80 mL × 3). The combined organic phases were dried over anhydrous sodium sulfate. Purification by column chromatography (eluent: petroleum ether : ethyl acetate = 2:1 - 0:1) afforded M38-2 (450 mg, 2.03 mmol, yield: 54.9%) as a pale yellow solid.

ESI-MS *(m*/*z):* 222.1 [M+H]⁺

¹H NMR (400 MHz, DMSO-*d₆*) *δ* 9.12 (s, 1H), 8.83 (d, *J* = 1.91 Hz, 1H), 8.59 (s, 1H), 5.57 (br s, 1H), 4.63 (s, 2H), 4.30 (q, *J* = 7.15 Hz, 2H), 1.31 (t, *J* = 7.09 Hz, 4H).

### Step 3:

M38-2 (370 mg, 1.67 mmol, 1.0 eq) was placed in a 100 mL single-necked flask. Ethanol (EtOH) (5 mL) was added. A solution of sodium hydroxide (NaOH) (100 mg, 2.51 mmol, 1.5 eq) in water (5 mL) was added. The mixture was stirred at 50°C for 2 h. Hydrochloric acid (HCl, 1M) was added dropwise to the reaction mixture until pH = 6. The mixture was concentrated under reduced pressure to afford M38 (323 mg, 1.67 mmol) as a pale yellow solid, which was used directly in the next step.
ESI-MS *(m*/*z):* 194.0 [M+H]⁺.

### Example

### Example 1:

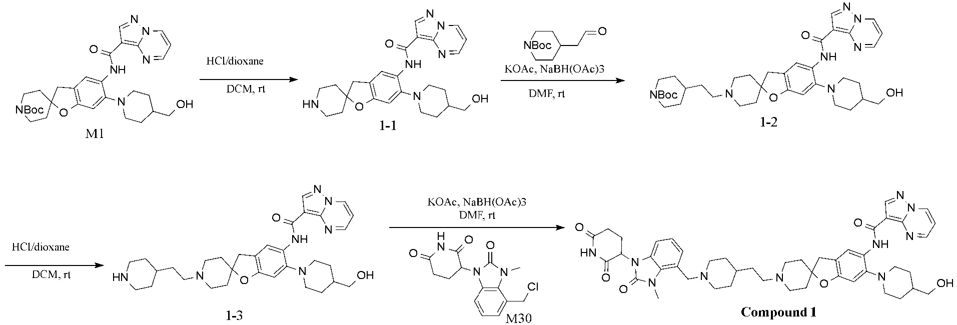

### Step 1:

M1 (3 g, 5.33 mmol) was placed in a 250 mL one-necked flask, and DCM (15 mL) and HCl in dioxane solution (15 mL, 60.00 mmol) were added. The reaction mixture was stirred at room temperature for 2 h. After completion, the reaction solution was concentrated under reduced pressure. Saturated aqueous sodium bicarbonate (30 mL) was then added, and the mixture was stirred for 30 min before filtration. The filter cake was washed with water twice. The collected solid was resuspended in ethyl acetate (15 mL), stirred for 2 h, filtered again, and the resulting filter cake was collected to afford compound 1-1 as a yellow solid (1.8 g, 3.89 mmol, 73.0% yield).

ESI-MS *(m*/*z):* 463.2 [M+H]⁺.

### Step 2:

KOAc (196 mg, 2.00 mmol), 1-1(232 mg, 0.50 mmol), and *tert*-butyl 4-(2-oxoethyl)piperidine-1-carboxylate (114 mg, 0.50 mmol) were charged into a 25 mL one-necked flask and subjected to nitrogen purging. Anhydrous DMF (1.5 mL) was added, and the mixture was stirred at room temperature for 30 min. Sodium triacetoxyborohydride (212 mg, 1.00 mmol) was then added, and the reaction continued at room temperature for another 2 h. The reaction mixture was filtered through a membrane filter, and the filtrate was washed with a small amount of DMF. The combined filtrate was purified by preparative HPLC to afford **1-2** as a gray solid (199 mg, 59.1% yield).

ESI-MS *(m*/*z):* 674.7 [M+H]⁺.

### Step 3:

**1-2** (199 mg, 0.30 mmol) was placed in a 50 mL one-necked flask, dissolved in DCM (10 mL), and 4.0 M HCl in dioxane solution (6 mL) was slowly added. The mixture was stirred at room temperature for 2 h. The reaction solution was directly concentrated under reduced pressure to afford **1-3** as a pale yellow-brown solid, which was used directly in the next step without further purification.

### Step 4:

M30 (92 mg, 0.25 mmol), **1-3** (172 mg, 0.30 mmol), potassium carbonate (103.82 mg, 0.75 mmol), and triethylamine (152 mg, 1.50 mmol) were placed in a 25 mL one-necked flask. Anhydrous acetonitrile (5 mL) and anhydrous DMF (3 mL) were added, and the system was purged with nitrogen. The reaction mixture was heated to 70 °C (external temperature) and stirred overnight. The reaction mixture was filtered, and the filtrate was concentrated under reduced pressure to remove volatile solvents (e.g., acetonitrile). The residual DMF solution was purified by preparative chromatography to afford **Compound 1** as a light yellow solid (50 mg, 23.6% yield).

ESI-MS (*m*/*z*): 845.8 [M+H]⁺.

¹H NMR (500 MHz, CDCl₃) *δ* 10.49 (s, 1H), 8.87 - 8.76 (m, 3H), 8.47 (s, 2H), 8.25 (br s, 1H), 7.04 (dd, *J* = 6.9, 4.2 Hz, 1H), 6.98 (t, *J* = 7.8 Hz, 1H), 6.91 (d, *J* = 7.6 Hz, 1H), 6.77 (d, *J* = 7.7 Hz, 1H), 6.67 (s, 1H), 5.23 (dd, *J* = 12.2, 5.1 Hz, 1H), 3.81 (s, 3H), 3.71 - 3.58 (m, 4H), 3.41 - 3.29 (m, 2H), 3.17 - 3.02 (m, 6H), 3.02 - 2.62 (m, 10H), 3.38 - 2.21 (m, 3H), 2.11 (d, *J* = 14.0 Hz, 2H), 2.01 (t, *J =* 11.3 Hz, 2H), 1.88 - 1.62 (m, 9H), 1.50 - 1.34 (m, 2H).

### Example 2:

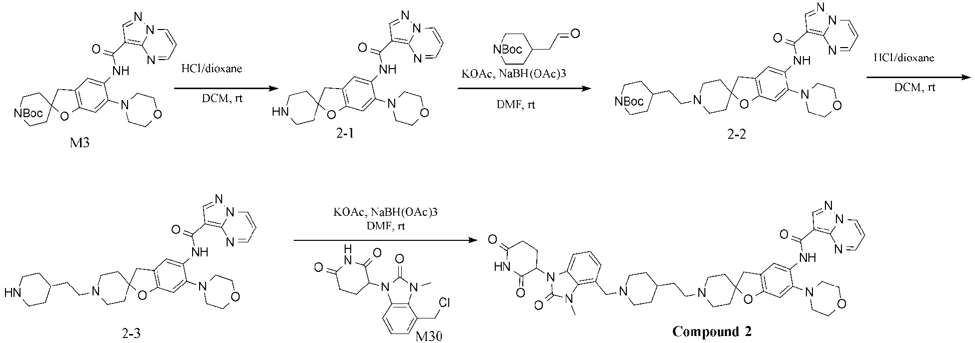

### Step 1:

M3 (1300 mg, 2.43 mmol) was placed in a 100 mL one-necked flask and dissolved in DCM (30 mL). A solution of 4.0 M HCl in dioxane (5 mL) was slowly added. The reaction was stirred at room temperature for 3 h. The mixture was directly concentrated under reduced pressure to afford 2-1 as a pale yellow-brown solid, which was used directly in the next step.

### Step 2:

In a 25 mL one-necked flask, KOAc (197 mg, 2.01 mmol), **2-1** (232 mg, 0.50 mmol), and *tert*-butyl 4-(2-oxoethyl)piperidine-1-carboxylate (114 mg, 0.50 mmol) were dissolved in anhydrous DMF (3 mL). The mixture was stirred at room temperature for 30 min, followed by the addition of sodium triacetoxyborohydride (212 mg, 1.00 mmol). Stirring was continued at room temperature for 2 h. Water (20 mL) was added to the reaction mixture, and the product was extracted with ethyl acetate (EA, 40 mL × 2). The combined organic layers were washed with saturated brine, dried over anhydrous sodium sulfate, and concentrated under reduced pressure. And purified by column chromatography using DCM:MeOH gradient (100:0 -90:10) to afford **2-2** as a light yellow solid (324 mg, 100% yield).

ESI-MS (*m*/*z*): 646.8 [M+H]⁺.

¹H NMR (500 MHz, CDCl₃) *δ* 10.37 (s, 1H), 8.76 (d, *J* = 6.3 Hz, 1H), 8.72-8.67 (m, 2H), 8.38 (s, 1H), 7.00 (dd, *J* = 6.6, 4.2 Hz, 1H), 6.59 (s, 1H), 4.10-3.95 (m, 2H), 3.91-3.85 (m, 4H), 3.35-2.84 (m, 12H), 2.69-2.57 (m, 2H), 2.28-2.02 (m, 4H), 1.75-1.55 (m, 4H), 1.53-1.43 (m, 1H), 1.38 (s, 9H), 1.10 (dd, *J* = 20.9, 11.6 Hz, 2H).

### Step 3:

**2-2** (315 mg, 0.49 mmol) was dissolved in DCM (10 mL) in a 50 mL one-necked flask. 4.0 M HCl in dioxane solution (2 mL) was slowly added, and the mixture was stirred at room temperature for 3 h. The reaction solution was directly concentrated under reduced pressure to afford **2-3** as a pale yellow-brown solid, which was carried forward without purification.

### Step 4:

M30 (73 mg, 0.20 mmol), **2-3** (108 mg, 0.20 mmol), potassium carbonate (82 mg, 0.60 mmol), and triethylamine (121 mg, 1.19 mmol) were sequentially added to a 25 mL one-necked flask. DMF (1 mL) and acetonitrile (5 mL) were added, and the mixture was heated to 50°C (external temperature) and stirred overnight. Water (15 mL) was added to the reaction mixture, and the product was extracted with DCM (40 mL × 2). The combined organic phases were dried over anhydrous sodium sulfate, filtered, and concentrated under reduced pressure. Purification by column chromatography using a DCM:MeOH gradient from 100:0 to 95:5 afforded **Compound 2** as a light yellow solid (79 mg, 84.5% yield).

ESI-MS *(m*/*z):* 817.7 [M+H]⁺.

¹H NMR (500 MHz, CDCl₃) *δ* 10.37 (s, 1H), 8.76 (dd, *J* = 6.9, 1.3 Hz, 1H), 8.73-8.67 (m, 2H), 8.36 (s, 1H), 8.19 (br s, 1H), 6.98 (dd, *J* = 6.9, 4.1 Hz, 1H), 6.88 (t, *J=* 7.7 Hz, 1H), 6.82 (d, *J* = 7.5 Hz, 1H), 6.67 (d, *J* = 7.6 Hz, 1H), 6.59 (s, 1H), 5.14 (dd, *J* = 12.1, 5.2 Hz, 1H), 3.95-3.85 (m, 4H), 3.73 (s, 3H), 3.61 - 3.46 (m, 2H), 2.97 (s, 2H), 2.90 - 2.50 (m, 12H), 2.20 - 1.87 (m, 4H), 1.67 - 1.48 (m, 4H), 1.36 - 1.08 (m, 8H).

The following examples were synthesized using a method analogous to that of Compound 1 and Compound 2:

| **Compound** | **Chemical Structure and Characterization Data** |
|---|---|
| **3** | 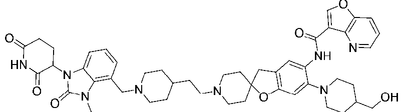 |
| | ESI-MS (*m*/*z*): 845.30 [M+H]⁺ |
| | ¹H NMR (500 MHz, CDCl₃) δ 10.93 (s, 1H), 8.67 (d, *J* = 4.0 Hz, 1H), 8.57 (s, 1H), 8.39 (s, 1H), 8.14 (br s, 1H), 7.87 (dd, *J* = 8.3, 0.6 Hz, 1H), 7.35 (dd, *J* = 8.4, 4.8 Hz, 1H), 6.95 (d, *J* = 7.8 Hz, 1H), 6.89 (d, *J* = 7.6 Hz, 1H), 6.74 (d, *J* = 7.7 Hz, 1H), 6.66 (s, 1H), 5.21 (dd, *J* = 12.2, 5.2 Hz, 1H), 3.79 (s, 4H), 3.63 - 3.59 (m, 4H), 3.15 - 3.02 (m, 6H), 2.98 - 2.91 (m, 2H), 2.88 - 2.63 (m, 10H), 2.28 - 2.18 (m, 2H), 2.10 - 2.04 (m, 2H), 1.99 (t, *J* = 11.5 Hz, 3H), 1.84 - 1.71 (m, 7H), 1.45 - 1.35 (m, 4H). |
| **4** | 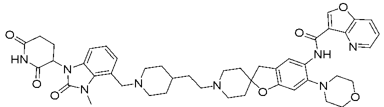 |
| | ESI-MS (*m*/*z*): 817.40 [M+H]⁺. |
| | ¹H NMR (500 MHz, CDCl₃) *δ* 10.89 (s, 1H), 8.65 (d, *J* = 4.0 Hz, 1H), 8.51 (s, 1H), 8.36 (s, 1H), 8.15 - 7.94 (m, 1H), 7.82 (d, *J* = 8.4 Hz, 1H), 7.32 (dd, *J* = 8.3, 4.8 Hz, 1H), 6.89 (t, *J* = 7.7 Hz, 1H), 6.82 (d, *J* = 7.6 Hz, 1H), 6.67 (d, *J* = 7.7 Hz, 1H), 6.60 (s, 1H), 5.14 (dd, *J* = 12.2, 5.2 Hz, 1H), 3.92 (s, 4H), 3.72 (s, 3H), 3.53 (d, *J* = 2.7 Hz, 2H), 3.13 - 3.07 (m, 1H), 2.99 (s, 2H), 2.89 (d, *J* = 2.5 Hz, 1H), 2.85 (d, *J* = 3.8 Hz, 5H), 2.82 - 2.58 (m, 8H), 2.20 - 2.06 (m, 3H), 2.03 - 1.98 (m, 2H), 1.92 (t, *J* = 11.4 Hz, 2H), 1.65 - 1.56 (m, 5H), 1.37 - 1.29 (m, 2H). |
| **5** | 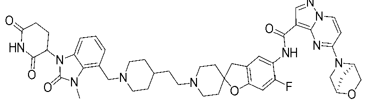 |
| | ESI-MS (m/z): 847.3 [M+H]⁺. |
| | ¹H NMR (500 MHz, DMSO-*d₆*) δ 11.07 (s, 1H), 9.84 (s, 1H), 8.79 (d, *J* = 7.7 Hz, 1H), 8.31-8.22 (m, 2H), 7.07 (d, *J=* 7.4 Hz, 1H), 6.95 (t, *J=* 7.7 Hz, 1H), 6.90 - 6.44 (m, 3H), 5.37 (dd, *J* = 12.6, 5.1 Hz, 1H), 5.13 (d, *J* = 54.5 Hz, 1H), 4.78 (s, 1H), 3.89 - 3.41 (m, 9H), 3.10 - 2.57 (m, 10H), 2.09 - 1.72 (m, 8H), 1.69 - 1.05 (m, 10H). |
| **6** | 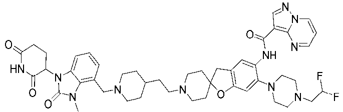 |
| | ESI-MS *(m*/*z):* 880.5 [M+H]⁺. |
| | ¹H NMR (500 MHz, CDCl₃) δ 10.33 (s, 1H), 8.83 (dd, *J* = 7.0, 1.4 Hz, 1H), 8.78 (s, 1H), 8.74 - 8.69 (m, 1H), 8.39 (s, 1H), 8.17 (s, 1H), 7.06 (dd, *J* = 6.9, 4.1 Hz, 1H), 6.96 (t, *J* = 7.8 Hz, 1H), 6.89 (d, *J* = 7.5 Hz, 1H), 6.74 (d, *J* = 7.7 Hz, 1H), 6.65 (s, 1H), 5.94 (tt, *J* = 55.9, 4.1 Hz, 1H), 5.21 (dd, *J* = 12.3, 5.3 Hz, 1H), 3.80 (s, 3H), 3.60 (q, *J* = 12.9 Hz, 2H), 3.03 (s, 2H), 2.97 - 2.74 (m, 16H), 2.23 (dd, *J =* 7.8, 5.2 Hz, 1H), 2.03 - 1.93 (m, 2H), 1.75 - 1.52 (m, 16H). |
| **7** | 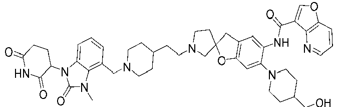 |
| | ESI-MS (*m*/*z*): 831.3[M+H]⁺. |
| | ¹H NMR (500 MHz, DMSO-*d₆*) δ 11.06 (s, 1H), 10.91 (s, 1H), 8.95 (s, 1H), 8.74 (d, *J* = 4.1 Hz, 1H), 8.33 (s, 1H), 8.25 (d, *J =* 8.4 Hz, 1H), 7.56 (dd, *J =* 8.2, 4.7 Hz, 1H), 7.06 (d, *J* = 7.9 Hz, 1H), 6.97 - 6.93 (m, 1H), 6.87 (d, *J* = 7.4 Hz, 1H), 6.73 (s, 1H), 5.36 (d, *J* = 7.1 Hz, 1H), 4.50 (s, 1H), 3.67 (s, 3H), 3.61 (s, 2H), 3.51 (s, 1H), 3.36 (d, *J* = 5.3 Hz, 2H), 3.00 - 2.59 (m, 13H), 2.23 - 1.90 (m, 8H), 1.75 - 1.34 (m, 12H). |
| **8** | 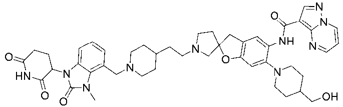 |
| | ESI-MS (*m*/*z*): 831.3 [M+H]⁺. |
| | ¹H NMR (500 MHz, CDCl₃) δ 10.50 (s, 1H), 8.83 - 8.78 (m, 3H), 8.47 (s, 1H), 8.11 (s, 1H), 7.04 (dd, *J* = 6.8, 4.2 Hz, 1H), 6.98 (t, *J* = 7.7 Hz, 1H), 6.92 (d, *J* = 7.5 Hz, 1H), 6.75 (d, *J* = 7.8 Hz, 1H), 6.69 (s, 1H), 5.22 (dd, *J =* 12.2, 5.0 Hz, 1H), 3.82 (s, 3H), 3.66 (d, *J* = 5.2 Hz, 2H), 3.62 (d, *J* = 6.8 Hz, 2H), 3.28 (dd, *J* = 27.5, 15.7 Hz, 3H), 3.15-2.93 (m, 6H), 2.89-2.74 (m, 6H), 2.69 (t, *J* = 11.2 Hz, 3H), 2.41-2.20 (m, 4H), 2.16-1.95 (m, 4H), 1.85 - 1.64 (m, 10H). |
| **9** | 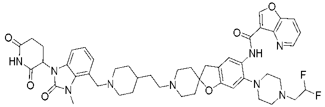 |
| | ESI-MS *(m*/*z):* 880.4 [M+H]⁺. |
| | ¹H NMR (500 MHz, CDCl₃) δ 10.79 (s, 1H), 8.62 (d, *J* = 4.1 Hz, 1H), 8.51 (s, 1H), 8.31 (s, 1H), 8.05 (br s, 1H), 7.82 (d, *J* = 8.4 Hz, 1H), 7.33 (dd, *J* = 8.3, 4.8 Hz, 1H), 6.89 (t, *J* = 7.8 Hz, 1H), 6.82 (d, *J* = 7.7 Hz, 1H), 6.67 (d, *J* = 7.7 Hz, 1H), 6.59 (s, 1H), 6.00 - 5.73 (m, 1H), 5.14 (dd, *J* = 12.0, 5.0 Hz, 1H), 3.73 (s, 3H), 3.53 (q, *J=* 13.0 Hz, 2H), 3.03 - 2.55 (m, 20H), 2.24 - 2.10 (m, 2H), 2.02 - 1.85 (m, 5H), 1.18 (t, *J* = 16.8 Hz, 10H). |
| **10** | 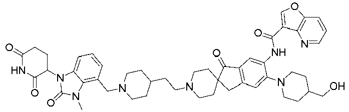 |
| | ESI-MS *(m*/*z):* 857.5 [M+H]⁺ |
| | ¹H NMR (400 MHz, DMSO-*d₆*) *δ* 11.10 (s, 1H), 10.99 (s, 1H), 9.07 (s, 1H), 8.76 (d, *J=* 4.63 Hz, 1H), 8.68 (s, 1H), 8.29 (d, *J* = 8.38 Hz, 1H), 8.20 (br s, 1H), 7.60 (dd, *J* = 8.38, 4.75 Hz, 1H), 7.43 (s, 1H), 7.07 (br d, *J* = 7.88 Hz, 1H), 6.96 (t, *J* = 7.69 Hz, 1H), 6.88 (br d, *J* = 7.63 Hz, 1H), 5.38 (br dd, *J* = 12.51, 5.38 Hz, 1H), 3.68 (s, 3H), 3.61 (br s, 1H), 3.17 (br s, 1H), 3.14 (br s, 1H), 2.98 (br s, 2H), 2.87 - 2.93 (m, 3H), 2.81 (br d, *J* =10.13 Hz, 2H), 2.65 - 2.70 (m, 2H), 2.35 (br d, *J* =7.25 Hz, 2H), 1.93 - 2.14 (m, 6H), 1.71 - 1.86 (m, 5H), 1.51 - 1.69 (m, 6H), 1.25 - 1.45 (m, 6H), 1.00 - 1.20 (m, 3H). |
| **11** | 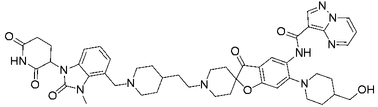 |
| | ESI-MS (*m*/*z*): 859.3[M+H]⁺. |
| | ¹H NMR (500 MHz, DMSO-*d₆*) *δ* 11.07 (s, 1H), 10.29 (s, 1H), 9.39 (d, *J* = 5.9 Hz, 1H), 8.92 (d, *J* = 2.8 Hz, 1H), 8.74 (s, 1H), 8.61 (s, 1H), 7.37 (dd, *J* = 6.8, 4.3 Hz, 1H), 7.07 (s, 1H), 6.95 (d, *J* = 7.6 Hz, 1H), 6.88 (d, *J* = 7.2 Hz, 1H), 5.37 (d, *J* = 8.1 Hz, 1H), 4.54 (t, *J* = 5.0 Hz, 1H), 3.68 (s, 3H), 3.63 (s, 2H), 3.39 (t, *J* = 4.7 Hz, 2H), 3.26 - 3.02 (m, 6H), 2.94 - 2.60 (m, 8H), 2.31 - 1.49 (m, 18H). |
| **12** | 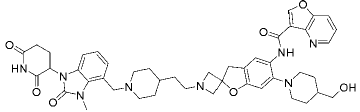 |
| | ESI-MS *(m*/*z):* 817.4[M+H]⁺. |
| | ¹H NMR (500 MHz, CDCl₃) *δ* 11.22 (s, 1H), 8.72 (d, *J* = 4.0 Hz, 1H), 8.59 (s, 1H), 8.49 (s, 1H), 8.36 - 7.98 (m, 2H), 7.88 (d, *J =* 8.3 Hz, 1H), 7.38 (dd, *J* = 8.4, 4.8 Hz, 1H), 7.00 - 6.86 (m, 2H), 6.74 (d, *J* = 7.6 Hz, 1H), 5.21 (dd, *J* = 12.1, 5.1 Hz, 1H), 5.09 (s, 2H), 3.77 (d, *J* = 34.4 Hz, 3H), 3.68 - 3.50 (m, 4H), 3.16 (d, *J* = 11.5 Hz, 2H), 3.02 - 2.61 (m, 7H), 2.27 - 2.16 (m, 1H), 2.00 (t, *J* = 11.2 Hz, 2H), 1.89 - 1.67 (m, 6H), 1.39 (dd, *J* = 43.4, 15.0 Hz, 5H), 1.27 (d, *J =* 14.4 Hz, 8H). |
| **13** | 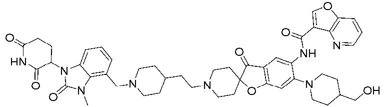 |
| | ESI-MS (*m*/*z*): 859.3[M+H]⁺. |
| | ¹H NMR (500 MHz, DMSO-*d₆*) *δ* 11.07 (s, 1H), 10.79 (s, 1H), 9.06 (s, 1H), 8.75 (d, *J* = 4.3 Hz, 1H), 8.60 (s, 1H), 8.29 (d, *J* = 8.4 Hz, 1H), 7.59 (dd, *J* = 8.4, 4.8 Hz, 1H), 7.12 - 7.02 (m, 2H), 6.96 (t, *J* = 7.5 Hz, 1H), 6.88 (d, *J* = 7.1 Hz, 1H), 5.38 (d, *J* = 7.2 Hz, 1H), 4.53 (s, 1H), 3.70 - 3.59 (m, 5H), 3.53 - 3.34 (m, 3H), 3.25 - 3.13 (m, 5H), 2.95 - 2.59 (m, 8H), 2.42 - 2.12 (m, 4H), 2.07 - 1.84 (m, 6H), 1.78 - 1.50 (m, 10H). |
| **14** | 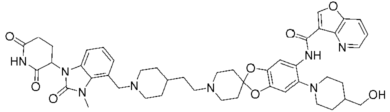 |
| | ESI-MS (*m*/*z*): 847.3 [M+H]⁺. |
| | ¹H NMR (500 MHz, DMSO-*d₆*) *δ* 11.09 (d, *J* = 18.1 Hz, 2H), 8.97 (s, 1H), 8.75 (d, *J* = 4.2 Hz, 1H), 8.25 (d, *J* = 8.3 Hz, 1H), 8.09 (s, 1H), 7.57 (dd, *J* = 8.3, 4.8 Hz, 1H), 7.06 (d, *J =* 7.6 Hz, 1H), 7.01 - 6.92 (m, 2H), 6.87 (d, *J* = 7.5 Hz, 1H), 5.37 (dd, *J* = 12.5, 5.1 Hz, 1H), 4.52 (t, *J =* 5.2 Hz, 1H), 3.67 (s, 3H), 3.61 (s, 2H), 3.40 (t, *J* = 5.5 Hz, 2H), 2.93 - 2.53 (m, 11H), 2.36 (s, 2H), 2.06 - 1.91 (m, 6H), 1.73 (d, *J* = 11.3 Hz, 2H), 1.68 - 1.27 (m, 10H), 1.25 (s, 1H), 1.11 (dd, *J* = 23.0, 12.6 Hz, 2H). |
| **15** | 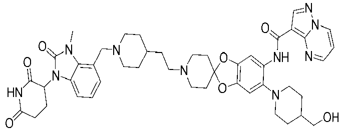 |
| | ESI-MS (*m*/*z*): 847.4 [M+H]⁺. |
| | ¹H NMR (500 MHz, CDCl₃) *δ* 10.60 (s, 1H), 8.80 - 8.60 (m, 3H), 8.15 (s, 1H), 8.02 (s, 1H), 6.94 (dd, *J* = 6.7, 4.3 Hz, 1H), 6.89 (t, *J =* 7.8 Hz, 1H), 6.83 (d, *J* = 7.7 Hz, 1H), 6.66 (d, *J* = 7.0 Hz, 2H), 5.13 (dd, *J* = 12.3, 5.2 Hz, 1H), 3.73 (s, 3H), 3.63 - 3.45 (m, 4H), 3.00 - 2.27 (m, 15H), 2.20 - 1.86 (m, 7H), 1.78 - 1.55 (m, 8H), 1.26 (s, 3H), 1.16 - 1.10 (m, 2H). |
| **16** | 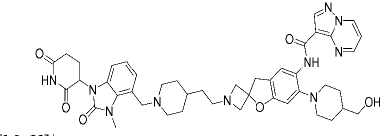 |
| | ESI-MS (*m*/*z*): 817.3[M+H]⁺. |
| | ¹H NMR (500 MHz, CDCl₃) *δ* 10.70 (s, 1H), 8.85 - 8.64 (m, 3H), 8.46 (s, 1H), 8.38 - 8.01 (m, 1H), 7.53 (brs, 1H), 7.02 - 6.92 (m, 1H), 6.88 (t, *J* = 7.7 Hz, 1H), 6.83 (d, *J* = 7.5 Hz, 1H), 6.67 (d, *J =* 7.7 Hz, 1H), 5.14 (dd, *J =* 12.2, 5.1 Hz, 1H), 5.00 (s, 2H), 3.76 - 3.29 (m, 10H), 3.07 (d, *J* = 11.3 Hz, 2H), 2.91 - 2.45 (m, 9H), 2.20 - 2.10 (m, 1H), 1.92 (t, *J* = 11.6 Hz, 2H), 1.85 - 1.67 (m, 6H), 1.36 - 1.10 (m, 8H). |
| **17** | 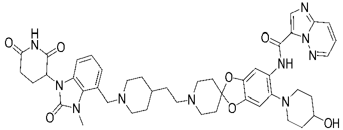 |
| | ESI-MS (*m*/*z*): 833.6 [M+H]⁺. |
| | ¹H NMR (500 MHz, DMSO-*d₆*) *δ* 11.27 (s, 1H), 11.08 (s, 1H), 8.88 (d, *J* = 3.4 Hz, 1H), 8.47 (s, 1H), 8.42 (d, *J* = 9.2 Hz, 1H), 8.12 (s, 1H), 7.58 (dd, *J* = 9.2, 4.4 Hz, 1H), 7.07 (d, *J* = 7.7 Hz, 1H), 7.01 - 6.93 (m, 2H), 6.88 (d, *J=* 7.6 Hz, 1H), 5.38 (dd, *J* = 12.5, 4.8 Hz, 1H), 4.79 (s, 1H), 3.68 (s, 3H), 3.62 (s, 2H), 2.95-2.77 (m, 5H), 2.75-2.60 (m, 4H), 2.60-2.49 (m, 4H), 2.37 (t, *J* = 6.1 Hz, 2H), 2.08 - 1.85 (m, 9H), 1.79 - 1.59 (m, 4H), 1.43-1.22 (m, 4H), 1.12 (dd, *J* = 21.1, 10.3 Hz, 2H). |

### Example 18:

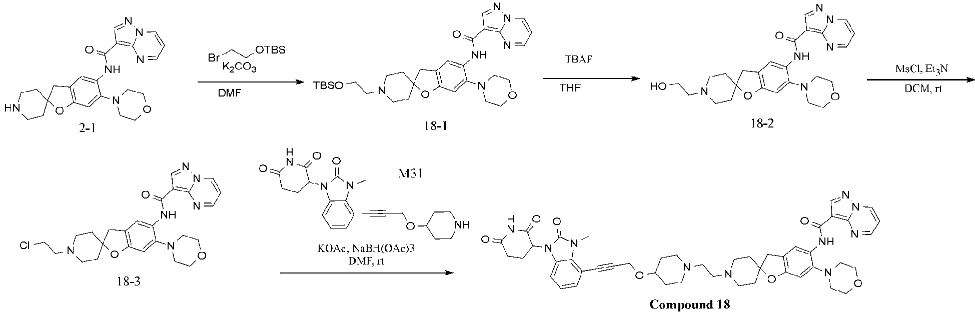

### Step 1:

A mixture of compound 2-1 (218 mg, 0.50 mmol) and potassium carbonate (208 mg, 1.51 mmol) in anhydrous DMF (6 mL) was stirred at room temperature until dissolved. To this solution was added (2-bromoethoxy)-tert-butyldimethylsilyl (480 mg, 2.01 mmol). The reaction mixture was heated to 60 °C and stirred overnight. After completion, the reaction was diluted with DCM (50 mL), washed with water (15 mL), and the organic layer was separated. The aqueous phase was extracted twice more with DCM (20 mL each). The combined organic phases were dried over anhydrous sodium sulfate, filtered, and concentrated under reduced pressure. And purified by silica gel column chromatography (DCM:MeOH =100:0 -90:10) to afford compound 18-1 as a pale yellow solid (244 mg, 82.0% yield).

ESI-MS (m/z): 593.6 [M+H]⁺.

¹H NMR (500 MHz, CDCl₃) *δ* 10.36 (s, 1H), 8.74 (dd, *J =* 7.0, 1.4 Hz, 1H), 8.70 (s, 1H), 8.68 (dd, *J =* 3.9, 1.4 Hz, 1H), 8.35 (s, 1H), 6.97 (dd, *J =* 6.9, 4.1 Hz, 1H), 6.60 (s, 1H), 3.95 - 3.81 (m, 4H), 3.78-3.71 (m, 2H), 2.93 (s, 2H), 2.82 (dd, *J =* 12.3, 8.0 Hz, 4H), 2.75-2.50 (m, 6H), 1.95-1.85 (m, 4H), 0.83 (s, 9H), -0.00 (s, 6H).

### Step 2:

To a solution of 18-1 (235 mg, 0.40 mmol) in THF (8 mL) at 0 °C (ice bath) was slowly added 1.0 M TBAF in THF (0.5 mL, 0.48 mmol). After addition, the ice bath was removed and the reaction was warmed to 30 °C and stirred for 4 hours. Upon completion, the solvent was removed under reduced pressure without aqueous workup. The residue was purified by flash column chromatography on silica gel (DCM:MeOH = 100:0-90:10) to afford compound 18-2 as a yellow solid (165 mg, 87.0% yield).

ESI-MS (m/z): 479.5 [M+H]⁺.

¹H NMR (500 MHz, CDCl₃) *δ* 10.38 (s, 1H), 8.76 (dd, *J =* 7.0, 1.4 Hz, 1H), 8.73-8.67 (m, 2H), 8.37 (s, 1H), 6.99 (dd, *J =* 6.9, 4.1 Hz, 1H), 6.61 (s, 1H), 3.94 - 3.81 (m, 4H), 3.63 (t, *J =* 4.9 Hz, 2H), 2.96 (s, 2H), 2.89 - 2.79 (m, 4H), 2.76-2.58 (m, 6H), 2.00 - 1.80 (m, 4H).

### Step 3:

Compound 18-2 (161 mg, 0.34 mmol) was dissolved in DCM (8 mL) with triethylamine (170 mg, 1.68 mmol) under nitrogen at 0 °C. MsCl (58 mg, 0.50 mmol) was added dropwise. After complete addition, the cooling bath was removed, and the reaction was allowed to warm to room temperature and stirred for 3 hours. The mixture was quenched with water (15 mL), and the aqueous layer was extracted with DCM (30 mL × 2). The combined organic layers were dried over anhydrous Na₂SO₄, filtered, and concentrated under reduced pressure to afford compound 18-3 as a pale yellow solid (164 mg, 87.6%), which was used directly in the next step without further purification.

ESI-MS (*m*/*z*): 497.4 [M+H]⁺.

### Step 4:

In a 25 mL round-bottom flask, M31 (67 mg, 0.17 mmol) and 18-3 (82 mg, 0.15 mmol) were suspended in acetonitrile (3 mL). Potassium carbonate (61 mg, 0.44 mmol) and DMF (1 mL) were added. The mixture was heated to 60 °C and stirred overnight. The reaction was filtered through a membrane filter, and the filtrate was purified by preparative HPLC. The collected fractions were lyophilized to afford Compound 18 as a off-white solid (74 mg, 58.6% yield).

ESI-MS (*m*/*z*)*:* 857.7 [M+H]⁺.

### Example 19:

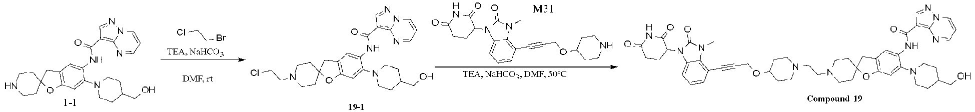

### Step 1:

To a solution of compound 1-1 (600 mg, 1.30 mmol) and triethylamine (0.4 mL, 2.59 mmol) in DMF (8 mL) was added 1-bromo-2-chloroethane (923 mg, 6.50 mmol) and sodium bicarbonate (218 mg, 2.59 mmol). The mixture was stirred at room temperature overnight. The solvent was removed under reduced pressure. Water (20 mL) was added, and the aqueous layer was extracted with DCM (20 mL × 3). The combined organic layers were dried over anhydrous Na₂SO₄, filtered, concentrated. And purified by silica gel column chromatography (DCM/MeOH = 20:1) to afford compound 19-1 as a yellow solid (450 mg, 66.1% yield).

ESI-MS (m/z): 525.2 [M+H]⁺.

### Step 2:

M31 (40 mg, 0.10 mmol) and triethylamine (20 mg, 0.20 mmol) were dissolved in DMF (5 mL) and stirred for 10 minutes. Then 19-1 (50 mg, 0.10 mmol) and sodium bicarbonate (17 mg, 0.20 mmol) were added. The reaction mixture was heated to 50 °C and stirred for 5 hours. After cooling to room temperature, the solvent was removed under reduced pressure. The crude product was purified by preparative liquid chromatography, followed by lyophilization to afford **Compound 19** as a pale yellow solid (6.5 mg, 7.7% yield).

ESI-MS (m/z): 885.4 [M+H]⁺.

¹H NMR (400 MHz, DMSO-*d*₆) *δ* 11.12 (s, 1H), 10.48 (s, 1H), 9.36 (dd, *J =* 7.0, 1.6 Hz, 1H), 8.91 - 8.87 (m, 1H), 8.67 (s, 1H), 8.33 (s, 1H), 7.36 - 7.30 (m, 1H), 7.18 (d, *J =* 7.8 Hz, 1H), 7.14 - 7.10 (m, 1H), 7.03 (t, *J =* 8.0 Hz, 1H), 6.72 (s, 1H), 5.40 (dd, *J =* 12.6, 5.4 Hz, 1H), 4.47 (s, 2H), 3.64 (s, 3H), 3.61 - 3.52(m, 2H), 3.44 - 3.37 (m, 4H), 3.01 - 2.88 (m, 5H), 2.82 - 2.56 (m, 8H), 2.46 (s, 4H), 2.22 - 2.11 (m, 2H), 2.08 - 1.99 (m, 1H), 1.93 - 1.68 (m, 8H), 1.64 - 1.44 (m, 5H).
The following examples were synthesized using a method analogous to that of Compound 18 and Compound 19.

| Compound | **Chemical Structure and Characterization Data** |
|---|---|
| | 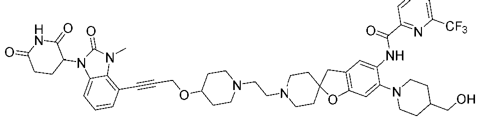 |
| **20** | ESI-MS *(m*/*z):* 913.80[M+H]⁺. |
| | ¹H NMR (500 MHz, DMSO-*d*₆) *δ* 11. 10 (s, 1H), 10.82 (s, 1H), 8.46 (d, *J =* 7.9 Hz, 1H), 8.38 (dd, *J =* 15.5, 7.5 Hz, 2H), 8.19 (d, *J =* 7.8 Hz, 1H), 7.19 (d, *J =* 7.5 Hz, 1H), 7.13 (d, *J =* 7.5 Hz, 1H), 7.04 (t, *J =* 7.9 Hz, 1H), 6.78 (s, 1H), 5.75 (s, 1H), 5.40 (dd, *J =* 12.8, 5.5 Hz, 1H), 4.51 (s, 2H), 3.66 (s, 2H), 3.52 (s, 3H), 3.18 (s, 1H), 3.04 (brs, 2H), 2.95 - 2.85 (m, 5H), 2.78 - 2.58 (m, 9H), 2.07 - 2.01 (m, 2H), 1.98 - 1.92 (m, 2H), 1.92 - 1.76 (m, 7H), 1.58 - 1.44 (m, 4H), 1.43 - 1.32 (m, 4H). |
| **21** | 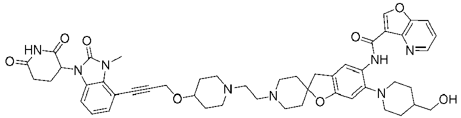 |
| | ESI-MS *(m*/*z):* 885.70 [M+H]⁺. |
| | ¹H NMR (500 MHz, DMSO-*d*₆) *δ* 11.10 (s, 1H), 10.92 (s, 1H), 8.95 (s, 1H), 8.75 (d, *J* = 4.6 Hz, 1H), 8.33 (s, 1H), 8.26 (d, *J =* 8.4 Hz, 1H), 7.57 (dd, *J =* 8.2, 4.7 Hz, 1H), 7.18 (d, *J =* 7.5 Hz, 1H), 7.13 (d, *J =* 7.8 Hz, 1H), 7.04 (t, *J* = 7.9 Hz, 1H), 6.74 (s, 1H), 5.40 (dd, *J =* 12.8, 5.2 Hz, 1H), 4.49 (s, 2H), 3.65 (s, 4H), 3.52 (s, 3H), 3.04 - 2.85 (m, 7H), 2.76 - 2.60 (m, 9H), 2.08 - 1.69 (m, 10H), 1.56 (m, 7H). |
| **22** | 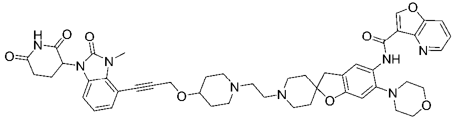 |
| | ESI-MS *(m*/*z):* 857.30[M+H]⁺. |
| | ¹H NMR (500 MHz, CDCl₃) *δ* 10.90 (s, 1H), 8.66 (d, *J =* 3.5 Hz, 1H), 8.53 (s, 1H), 8.37 (s, 1H), 7.82 (d, *J =* 8.7 Hz, 1H), 7.32 (dd, *J =* 8.3, 4.6 Hz, 1H), 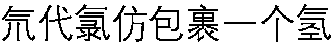 ,7.08 (d, *J =* 7.8 Hz, 1H), 6.93 (t, *J =* 7.9 Hz, 1H), 6.72 (d, *J =* 7.8 Hz, 1H), 6.60 (s, 1H), 5.14 - 5.10 (m, 1H), 4.38 (s, 1H), 3.92 (s, 3H), 3.70 (s, 2H), 3.57 (s, 1H), 3.31 - 2.58 (m, 21H), 2.36 - 2.07 (m, 12H). |
| **23** | 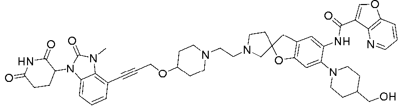 |
| | ESI-MS *(m*/*z):* 871.3[M+H]⁺. |
| | ¹H NMR (500 MHz, CDCl₃) *δ* 10.97 (s, 1H), 8.70 (d, *J =* 4.4 Hz, 1H), 8.61 (s, 1H), 8.44 (s, 1H), 7.89 (d, *J =* 8.3 Hz, 1H), 7.38 (dd, *J =* 8.1, 4.7 Hz, 1H), 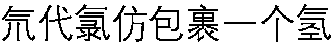 ,7.18 (d, *J =* 7.7 Hz, 1H), 7.01 (d, *J =* 7.6 Hz, 1H), 6.80 (d, *J =* 7.4 Hz, 1H), 6.68 (s, 1H), 5.21 (d, *J =* 7.8 Hz, 1H), 4.47 (s, 1H), 3.79 (s, 3H), 3.64 (d, *J =* 5.6 Hz, 2H), 3.27-2.99 (m, 12H), 2.89 - 2.65 (m, 6H), 2.43 - 2.10 (m, 8H), 1.86 - 1.62 (m, 10H). |
| **24** | 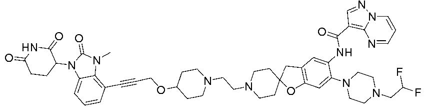 |
| | ESI-MS *(m*/*z):* 920.3[M+H]⁺. |
| | ¹H NMR (500 MHz, CDCl₃) *δ* 10.34 (s, 1H), 8.83 (d, *J =* 6.9 Hz, 1H), 8.78 (s, 1H), 8.72 (d, *J =* 2.4 Hz, 1H), 8.39 (s, 1H), 8.35 - 8.14 (m, 1H), 7.16 (d, *J =* 7.8 Hz, 1H), 7.06 (dd, *J* = 6.9, 4.2 Hz, 1H), 6.99 (t, *J =* 7.9 Hz, 1H), 6.77 (d, *J =* 7.7 Hz, 1H), 6.66 (s, 1H), 5.94 (tt, *J =* 56.1, 4.0 Hz, 1H), 5.19 (dd, *J =* 12.5, 5.3 Hz, 1H), 4.44 (s, 2H), 3.78 (s, 3H), 3.68 (s, 1H), 3.02 - 2.64 (m, 23H), 2.44 (s, 2H), 2.26 - 2.20 (m, 1H), 1.85 (s, 10H). |
| **25** | 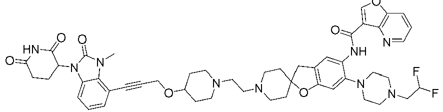 |
| | ESI-MS *(m*/*z):* 920.3 [M+H]⁺ |
| | ¹H NMR (500 MHz, CDCl₃) δ 10.87 (s, 1H), 8.69 (d, *J =* 4.5 Hz, 1H), 8.60 (br s, 1H), 8.49 (s, 1H), 8.39 (s, 1H), 7.90 (d, *J =* 8.2 Hz, 1H), 7.40 (dd, *J =* 8.3, 4.8 Hz, 1H), 7.15 (d, *J =* 7.9 Hz, 1H), 6.99 (t, *J =* 7.9 Hz, 1H), 6.79 *(d, J =* 7.7 Hz, 1H), 6.65 (s, 1H), 6.08 - 5.80 (m, 1H), 5.20 (dd, *J =* 12.6, 5.2 Hz, 1H), 4.44 (s, 2H), 3.76 (s, 2H), 3.26 - 2.78 (m, 20H), 2.29 - 2.01 (m, 10H), 1.51 - 1.41 (m, 8H). |
| **26** | 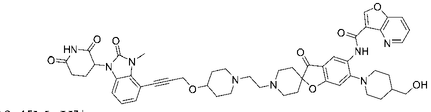 |
| | ESI-MS (m/z): 899.4[M+H]⁺. |
| | 1H NMR (500 MHz, DMSO-*d*₆) *δ* 11.09 (s, 1H), 10.78 (s, 1H), 9.05 (s, 1H), 8.75 (d, *J =* 4.3 Hz, 1H), 8.57 (s, 1H), 8.28 (d, *J =* 8.3 Hz, 1H), 7.59 (dd, *J =* 8.2, 4.9 Hz, 1H), 7.17 (d, *J =* 7.9 Hz, 1H), 7.12 (d, *J =* 6.9 Hz, 2H), 7.03 (t, *J=* 7.9 Hz, 1H), 5.39 (dd, *J=* 12.7, 5.3 Hz, 1H), 4.49 (d, *J =* 14.1 Hz, 2H), 3.65 (s, 3H), 3.35 (d, *J =* 4.9 Hz, 2H), 3.21 (d, *J =* 11.6 Hz, 2H), 2.90-2.73 (m, 9H), 2.64-2.32 (m, 4H), 2.21 - 1.95 (m, 6H), 1.91-1.82 (m, 4H), 1.73 (d, *J =* 10.9 Hz, 2H), 1.60-1.40 (m, 8H). |
| **27** | 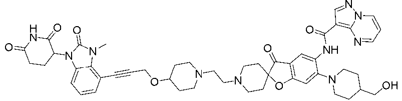 |
| | ESI-MS *(m*/*z):* 899.4[M+H]⁺. |
| | 1H NMR (500 MHz, DMSO-*d*₆) *δ* 11.09 (s, 1H), 10.29 (s, 1H), 9.38 (*d, J =* 6.9 Hz, 1H), 8.92 (d, J = 3.0 Hz, 1H), 8.74 (s, 1H), 8.58 (s, 1H), 7.37 (d, *J =* 4.2 Hz, 1H), 7.17 (d, *J =* 7.8 Hz, 1H), 7.12 (d, *J =* 5.0 Hz, 2H), 7.04 (d, *J =* 7.8 Hz, 1H), 5.41 - 5.37 (m, 1H), 5.32 (s, 1H), 4.53 (s, 1H), 4.48 (s, 2H), 3.65 (s, 3H), 3.57 (s, 2H), 3.38 (s, 2H), 3.19 (d, *J* = 11.5 Hz, 2H), 2.94-2.87 (m, 2H), 2.77-2.70 (m, 3H), 2.66-2.60 (m, 2H), 2.37 - 2.27 (m, 4H), 2.20-2.16 (m, 2H), 2.05 - 1.96 (m, 4H), 1.91 - 1.82 (m, 4H), 1.74 (d, *J =* 11.6 Hz, 2H), 1.54 (d, *J =* 11.2 Hz, 6H). |
| **28** | 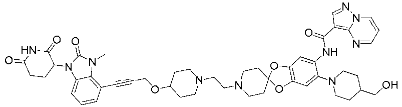 |
| | ESI-MS *(m*/*z):* 887.4 [M+H]⁺. |
| | ¹H NMR (500 MHz, DMSO-*d*₆) *δ* 11.10 (s, 1H), 10.70 (s, 1H), 9.35 (d, *J* = 6.3 Hz, 1H), 8.91 (d, *J* = 2.8 Hz, 1H), 8.69 (s, 1H), 8.11 (s, 1H), 7.34 (dd, *J =* 6.8, 4.2 Hz, 1H), 7.18 (d, *J =* 7.8 Hz, 1H), 7.12 (d, *J =* 7.7 Hz, 1H), 7.04 (t, *J =* 7.9 Hz, 1H), 6.95 (s, 1H), 5.40 (dd, *J =* 12.7, 5.3 Hz, 1H), 4.60-4.55 (m, 1H), 4.47 (s, 2H), 3.65 (s, 3H), 3.60-3.55 (m, 1H), 3.45-3.38 (m, 3H), 2.96 - 2.81 (m, 3H), 2.77 - 2.55 (m, 10H), 2.46-2.39 (m, 2H), 2.12 (t, *J =* 10.0 Hz, 2H), 2.07 - 1.82 (m, 8H), 1.78-1.68 (m, 2H), 1.65-1.55 (m, 2H), 1.55 - 1.40 (m, 4H). |
| **29** | 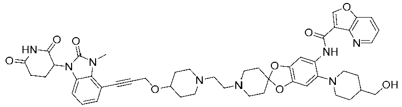 |
| | ESI-MS *(m*/*z):* 887.4 [M+H]⁺. |
| | ¹H NMR (500 MHz, DMSO-*d*₆) *δ* 11.10 (s, 1H), 10.70 (s, 1H), 9.35 (d, *J =* 6.3 Hz, 1H), 8.91 (d, *J* = 2.8 Hz, 1H), 8.69 (s, 1H), 8.11 (s, 1H), 7.34 (dd, *J =* 6.8, 4.2 Hz, 1H), 7.18 (d, *J =* 7.8 Hz, 1H), 7.12 (d, *J =* 7.7 Hz, 1H), 7.04 (t, *J =* 7.9 Hz, 1H), 6.95 (s, 1H), 5.40 (dd, *J =* 12.7, 5.3 Hz, 1H), 4.60-4.55 (m, 1H), 4.47 (s, 2H), 3.65 (s, 3H), 3.60-3.55 (m, 1H), 3.45-3.38 (m, 3H), 2.96 - 2.81 (m, 3H), 2.77 - 2.55 (m, 10H), 2.46-2.39 (m, 2H), 2.12 (t, *J =* 10.0 Hz, 2H), 2.07 - 1.82 (m, 8H), 1.78-1.68 (m, 2H), 1.65-1.55 (m, 2H), 1.55 - 1.40 (m, 4H). |
| **30** | 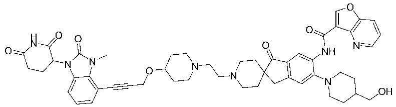 |
| | ESI-MS (m/z): 897.4 [M+H]⁺ |
| | ¹H NMR (400 MHz, DMSO-*d*₆) *δ* 10.90 - 11.23 (m, 2H), 9.07 (s, 1H), 8.76 (d, *J =* 4.75 Hz, 1H), 8.66 - 8.71 (m, 1H), 8.29 (d, *J =* 8.38 Hz, 1H), 7.60 (dd, *J* =8.44, 4.82 Hz, 1H), 7.44 (s, 1H), 7.11 - 7.21 (m, 2H), 7.00 - 7.09 (m, 1H), 5.40 (br dd, *J =* 12.63, 5.50 Hz, 1H), 4.60 (br s, 1H), 4.47 (s, 2H), 3.65 (s, 3H), 3.13 - 3.18 (m, 2H), 2.95 - 3.00 (m, 2H), 2.85 - 2.93 (m, 3H), 2.64 - 2.78 (m, 8H), 2.43 (s, 3H), 2.00 - 2.17 (m, 5H), 1.72 - 1.93 (m, 7H), 1.44 - 1.67 (m, 5H), 1.30 (br d, *J =* 12.13 Hz, 2H). |

### Example 31:

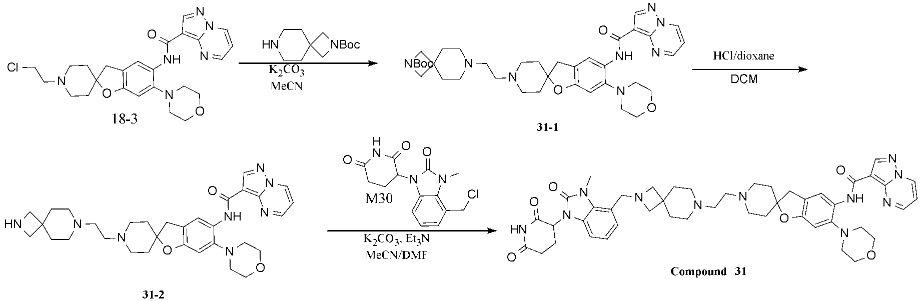

### Step 1:

18-3 (82 mg, 0.15 mmol), tert-butyl 2,7-diazaspiro[3.5]nonane-2-carboxylate (50 mg, 0.22 mmol), and potassium carbonate (61 mg, 0.44 mmol) were placed sequentially into a 25 mL single-neck flask. Acetonitrile (2 mL) was added, and the mixture was heated to an external temperature of 60°C and stirred overnight. The reaction mixture was diluted with DCM (50 mL) and washed once with water (20 mL). The layers were separated, and the organic phase was dried over anhydrous sodium sulfate. After concentration under reduced pressure, the residue was purified by column chromatography (eluent: DCM:MeOH = 100:0 - 90:10) to afford Compound 31-1 as a yellow solid (85 mg, yield: 84.0%).

ESI-MS *(m*/*z):* 687.6 [M+H]⁺.

¹H NMR (500 MHz, CDCl₃) *δ* 10.37 (s, 1H), 8.75 (dd, *J =* 7.0, 1.5 Hz, 1H), 8.73 - 8.64 (m, 2H), 8.36 (s, 1H), 6.98 (dd, *J =* 6.9, 4.1 Hz, 1H), 6.60 (s, 1H), 3.92 - 3.82 (m, 4H), 3.54 (s, 4H), 2.94 (s, 2H), 2.87 - 2.80 (m, 4H), 2.71 - 2.47 (m, 6H), 2.43 - 2.11 (m, 6H), 1.98-1.78 (m, 4H), 1.75-1.65 (m, 4H), 1.37 (s, 9H).

### Step 2:

31-1 (80 mg, 0.12 mmol) was placed into a 25 mL single-neck flask and dissolved in DCM (5 mL). A solution of HCl in 1,4-dioxane (4 M, 1.5 mL) was added dropwise slowly at room temperature. After addition, the mixture was stirred at room temperature for 2 h. The reaction mixture was concentrated directly under reduced pressure to afford Compound 31-2 as a pale pink-yellow solid, which was used directly in the next step.

ESI-MS (m/z): 587.6 [M+H]⁺.

### Step 3:

M30 (46.84 mg, 0.13 mmol), 31-2 (68 mg, 0.12 mmol), triethylamine (70 mg, 0.70 mmol), and potassium carbonate (64 mg, 0.46 mmol) were placed sequentially into a 25 mL single-neck flask. Anhydrous acetonitrile (3 mL) and anhydrous DMF (1 mL) were added. The mixture was heated to an external temperature of 60°C and stirred overnight. The reaction mixture was diluted with DCM (50 mL) and washed once with water (15 mL). The layers were separated after standing, and the organic phase was dried over anhydrous sodium sulfate. After concentration under reduced pressure, the residue was purified by preparative thin-layer chromatography. The resulting pale yellow solid was further purified by preparative HPLC to afford **Compound 31** as a pale yellow solid (15 mg, yield: 14.6%).

ESI-MS (m/z): 858.7 [M+H]⁺.

¹H NMR (500 MHz, CDCl₃) *δ* 10.47 (s, 1H), 8.85 (dd, *J=* 7.0, 1.4 Hz, 1H), 8.81 (s, 1H), 8.79 - 8.76 (m, 1H), 8.45 (s, 1H), 8.28 (br s, 1H), 7.08 (dd, *J =* 6.9, 4.2 Hz, 1H), 6.96 (dt, *J =* 15.2, 7.5 Hz, 2H), 6.75 (d, *J =* 7.5 Hz, 1H), 6.69 (s, 1H), 5.22 (dd, *J =* 12.3, 5.2 Hz, 1H), 4.03 - 3.90 (m, 4H), 3.87 - 3.72 (m, 5H), 3.04-2.96 (m, 6H), 2.96-2.91 (m, 4H), 2.89 - 2.38 (m, 12H), 2.27 - 1.96 (m, 4H), 1.92-1.75 (m, 8H).

### Example 32:

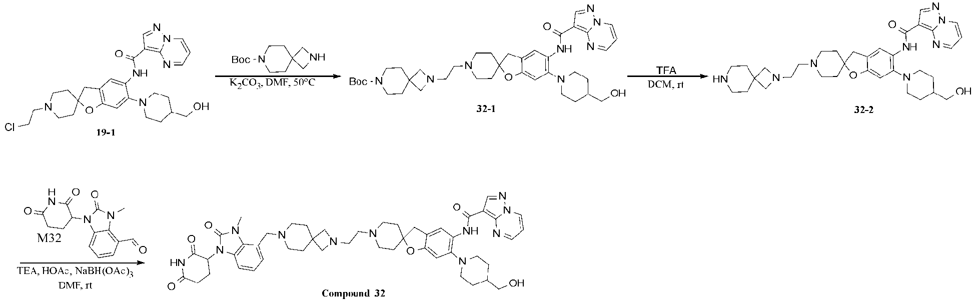

### Step 1:

Compound 19-1 (60 mg, 0.11 mmol), tert-butyl 2,7-diazaspiro[3.5]nonane-7-carboxylate (26 mg, 0.11 mmol), triethylamine (23 mg, 0.23 mmol), and potassium carbonate (24 mg, 0.17 mmol) were added to DMF (6 mL). The solution was stirred at 50°C overnight, then cooled to room temperature and concentrated under reduced pressure. And purified by preparative TLC (DCM/MeOH = 20:1) to afford Compound 32-1 as a yellow solid (50 mg, yield: 61.2%).

ESI-MS (m/z): 715.4 [M+H]⁺.

### Step 2:

Compound 32-1 (50 mg, 0.07 mmol) was dissolved in dichloromethane (5 mL). TFA,(1 mL, 13.46 mmol) was added to the solution. The mixture was stirred at room temperature for 2 hours, then concentrated under reduced pressure to afford Compound 32-2 as an oily crude product (45 mg), which was used directly in the next step.

ESI-MS (m/z): 615.4 [M+H]⁺.

### Step 3:

32-2 (45 mg, 0.07 mmol) was dissolved in anhydrous DMF (6 mL). Triethylamine (15 mg, 0.15 mmol) was added, and the solution was stirred for 5 minutes. Acetic acid (41 mg, 0.22 mmol) and M32 (21 mg, 0.07 mmol) were then added sequentially. After stirring at room temperature for 30 minutes, sodium triacetoxyborohydride (6 mg, 0.16 mmol) was added. The solution was stirred at room temperature overnight. The mixture was concentrated under reduced pressure and purified by preparative HPLC (ACN/H₂O = 1:1). Lyophilization afforded **Compound 32** as a pale yellow solid (8.0 mg, yield: 12.3%).

ESI-MS (m/z): 886.5 [M+H]⁺.

¹H NMR (400 MHz, DMSO-*d*₆) *δ* 11.10 (s, 1H), 10.48 (s, 1H), 9.38 - 9.33 (m, 1H), 8.92 - 8.87 (m, 1H), 8.67 (s, 1H), 8.33 (s, 1H), 7.36 - 7.31 (m, 1H), 7.06 (d, *J =* 8.0 Hz, 1H), 6.95 (t, *J =* 7.6 Hz, 1H), 6.86 (d, *J =* 7.6 Hz, 1H), 6.72 (s, 1H), 5.37 (dd, *J =* 12.6, 5.4 Hz, 1H), 4.55 (t, *J =* 5.4 Hz, 1H), 3.66 (s, 3H), 3.58 (s, 2H), 3.46 - 3.37 (m, 2H), 3.04 - 2.83 (m, 9H), 2.78 - 2.54 (m, 6H), 2.48 - 2.36 (m, 4H), 2.36 - 2.21 (m, 5H), 2.07 - 2.07-1.97 (m, 1H), 1.86 - 1.64 (m, 7H), 1.65 - 1.45 (m, 7H).

### Example 33:

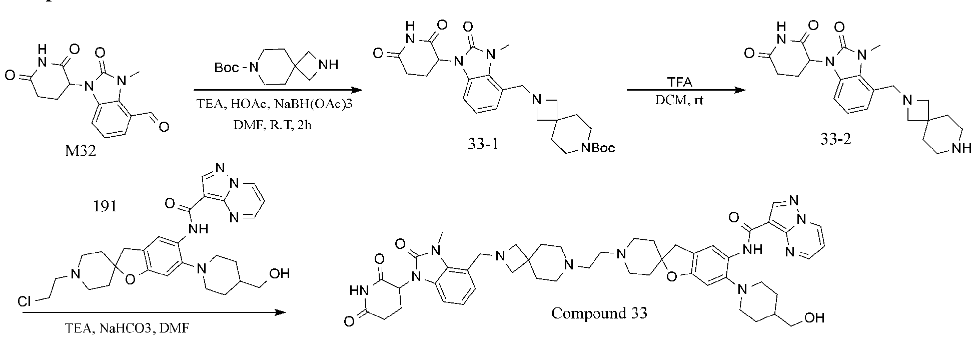

### Step 1:

2-(tert-Butoxycarbonyl)-2,7-diazaspiro[3.5]nonane (24 mg, 0.11 mmol) and triethylamine (21 mg, 0.21 mmol) were dissolved in DMF (5 mL). The solution was stirred at room temperature for 5 minutes, followed by addition of acetic acid (0.1 mL, 0.31 mmol) and compound M32 (30 mg, 0.10 mmol). After stirring for an additional 30 minutes, sodium triacetoxyborohydride (8 mg, 0.21 mmol) was added. The reaction mixture was stirred overnight at room temperature. The solvent was removed under reduced pressure, and the residue was purified by reversed-phase preparative HPLC (ACN/H₂O = 1:1) to afford compound 33-1 as a white solid (40 mg, 77.0% yield).

ESI-MS (*m*/*z*): 498.3 [M+H]⁺.

### Step 2:

Compound 33-1 (40 mg, 0.08 mmol) was dissolved in dichloromethane (5 mL), and trifluoroacetic acid (1 mL, 13.46 mmol) was added. The solution was stirred at room temperature for 2 hours. After completion, the solvent was removed under reduced pressure to afford compound 33-2 as a colorless oil (45 mg), which was used directly in the next step.

ESI-MS (m/z): 398.2 [M+H]⁺.

### Step 3:

To a solution of 33-2 (45 mg, 0.11 mmol) and triethylamine (23 mg, 0.23 mmol) in DMF (5 mL) was added sodium bicarbonate (29 mg, 0.35 mmol) and 19-1 (60 mg, 0.11 mmol). The mixture was heated to 50 °C and stirred overnight. After reaction completion, the solvent was removed under reduced pressure. The crude product was purified by preparative HPLC (ACN/H₂O = 1:1), followed by lyophilization to afford **Compound 33** as a pale yellow solid (3 mg).

ESI-MS (m/z): 886.3 [M+H]⁺.

¹H NMR (400 MHz, DMSO-*d*₆) *δ* 11.09 (s, 1H), 10.48 (s, 1H), 9.38 - 9.33 (m, 1H), 8.91 - 8.87 (m, 1H), 8.67 (s, 1H), 8.32 (s, 1H), 7.67 - 7.49 (m, 1H), 7.35 - 7.31 (m, 1H), 7.04 (d, *J =* 6.8 Hz, 1H), 6.94 - 6.91 (m, 1H), 6.72 (s, 1H), 5.37 (dd, *J =* 12.4, 5.4 Hz, 1H), 4.55 (t, *J =* 5.4 Hz, 1H), 3.81 - 3.77 (m, 2H), 3.65 (s, 3H), 3.39 (t, *J =* 5.4 Hz, 2H), 3.02 - 2.83 (m, 9H), 2.78 - 2.54 (m, 6H), 2.47 - 2.38 (s, 4H), 2.36 - 2.25 (m, 5H), 2.07 - 1.96 (m, 1H), 1.84 - 1.65 (m, 7H), 1.65 - 1.43 (m, 7H).

### Example 34:

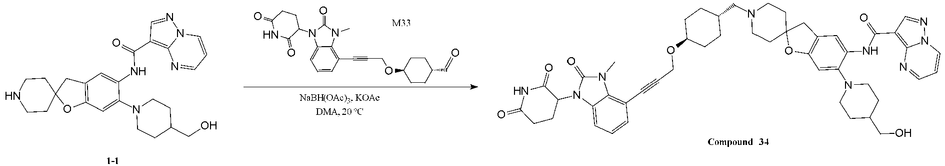

M33 (1.3 g, 3.07 mmol, 1.0 equiv) was placed in a 100 mL round-bottom flask, and DMA (15 mL) was added. Then 1-1 (1.42 g, 3.07 mmol, 1.0 equiv), potassium acetate (0.60 g, 6.14 mmol, 2.0 equiv), and sodium triacetoxyborohydride (0.78 g, 3.68 mmol, 1.2 equiv) were added sequentially. The reaction mixture was stirred at room temperature for 2 hours. After filtration, the filtrate was purified by HPLC to afford **Compound 34** as a yellow solid (1.34 g, 1.53 mmol, 49.7% yield).

ESI-MS *(m*/*z):* 870.5 [M+1]⁺.

¹H NMR (400 MHz, DMSO-*d*₆) *δ* 11.13 (s, 1H), 10.49 (s, 1H), 9.36 (dd, *J =* 7.00, 1.50 Hz, 1H), 8.90 (dd, *J =* 4.13, 1.50 Hz, 1H), 8.68 (s, 1H), 8.34 (s, 1H), 7.31 - 7.38 (m, 1H), 7.18 (d, *J =* 7.75 Hz, 1H), 7.10 - 7.15 (m, 1H), 6.99 - 7.09 (m, 1H), 6.73 (s, 1H), 5.41 (dd, *J =* 12.57, 5.32 Hz, 1H), 4.55 (t, *J =* 5.32 Hz, 1H), 4.47 (s, 2H), 3.65 (s, 3H), 3.37 - 3.51 (m, 3H), 2.84 - 3.03 (m, 5H), 2.53 - 2.78 (m, 5H), 2.32 - 2.48 (m, 3H), 2.01 - 2.15 (m, 5H), 1.68 - 1.87 (m, 8H), 1.42 - 1.65 (m, 4H), 1.12 - 1.27 (m, 2H), 0.84 - 0.98 (m, 2H).
The following examples were synthesized using a method analogous to that of Compound 34:

| **Example** | **Chemical Structure and Structural Characterization Data** |
|---|---|
| **35** | 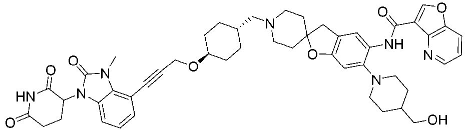 |
| | ESI-MS *(m*/*z):* 870.5 [M+1]⁺. |
| | 1H NMR (400 MHz, DMSO-*d*₆) *δ* 11.13 (s, 1H), 10.92 (s, 1H), 8.96 (s, 1H), 8.74 (d, *J* = 4.38 Hz, 1H), 8.32 (s, 1H), 8.25 (d, *J =* 8.50 Hz, 1H), 7.57 (dd, *J =* 8.50, 4.75 Hz, 1H), 7.18 (d, *J =* 7.75 Hz, 1H), 7.09 - 7.15 (m, 1H), 7.01 - 7.07 (m, 1H), 6.74 (s, 1H), 5.41 (dd, *J =* 12.57, 5.32 Hz, 1H), 4.53 *(t, J =* 5.25 Hz, 1H), 4.47 (s, 2H), 3.65 (s, 3H), 3.42 - 3.49 (m, 1H), 3.37 (t, *J =* 5.38 Hz, 2H), 2.86 - 3.01 (m, 5H), 2.61 - 2.74 (m, 4H), 2.32 - 2.48 (m, 3H), 1.99 - 2.18 (m, 5H), 1.65 - 1.92 (m, 9H), 1.46 - 1.64 (m, 4H), 1.11 - 1.25 (m, 2H), 0.82 - 0.97 (m, 2H). |
| **36** | 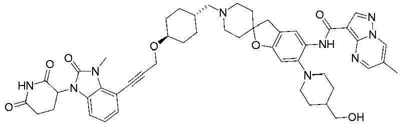 |
| | ESI-MS *(m*/*z):* 884.5 [M+1]⁺. |
| | ¹H NMR (400 MHz, DMSO-*d*₆) *δ* 11.13 (br s, 1H), 10.47 (s, 1H), 9.22 (s, 1H), 8.83 (d, *J =* 1.88 Hz, 1H), 8.59 (s, 1H), 8.32 (s, 1H), 7.18 *(d, J =* 8.00 Hz, 1H), 7.09 - 7.15 (m, 1H), 7.01 - 7.07 (m, 1H), 6.72 (s, 1H), 5.41 (dd, *J =* 12.69, 5.32 Hz, 1H), 4.59 (t, *J =* 5.19 Hz, 1H), 4.48 (s, 2H), 3.65 (s, 3H), 3.39 - 3.51 (m, 3H), 2.85 - 3.00 (m, 5H), 2.60 - 2.75 (m, 4H), 2.32 - 2.49 (m, 7H), 2.00 - 2.18 (m, 5H), 1.59 - 1.86 (m, 10H), 1.42 - 1.55 (m, 2H), 1.12 - 1.24 (m, 2H), 0.84 - 0.97 (m, 2H). |
| 37 | 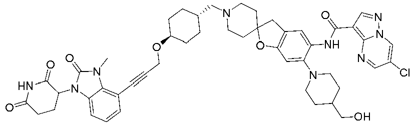 |
| | ESI-MS *(m*/*z):* 904.1 [M+1]⁺. |
| | ¹H NMR (400 MHz, DMSO-*d*₆) *δ* 11.12 (s, 1H), 10.39 (s, 1H), 9.80 (d, *J* = 2.13 Hz, 1H), 8.97 (d, *J =* 2.13 Hz, 1H), 8.69 (s, 1H), 8.32 (s, 1H), 7.17 (d, *J =* 7.75 Hz, 1H), 7.09 - 7.14 (m, 1H), 6.98 - 7.07 (m, 1H), 6.73 (s, 1H), 5.40 (dd, *J =* 12.63, 5.38 Hz, 1H), 4.65 (t, *J* = 5.13 Hz, 1H), 4.47 (s, 2H), 3.64 (s, 3H), 3.40 - 3.47 (m, 3H), 2.84 - 3.02 (m, 5H), 2.60 - 2.76 (m, 4H), 2.30 - 2.43 (m, 2H), 1.98 - 2.18 (m, 5H), 1.61 - 1.89 (m, 11H), 1.41 - 1.56 (m, 2H), 1.12 - 1.25 (m, 2H), 0.83 - 0.96 (m, 2H). |
| **38** | 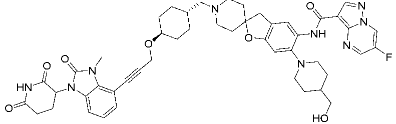 |
| | ESI-MS *(m*/*z):* 888.4 [M+H]⁺ |
| | ¹H NMR (400 MHz, DMSO-*d*₆) *δ* 11.00 - 11.25 (m, 1H), 10.43 (s, 1H), 9.81 (br s, 1H), 9.15 (br s, 1H), 8.71 (s, 1H), 8.34 (s, 1H), 7.18 (br d, *J =* 7.58 Hz, 1H), 7.12 (br d, *J =* 7.70 Hz, 1H), 7.00 - 7.08 (m, 1H), 6.74 (s, 1H), 5.37 - 5.46 (m, 1H), 4.65 (br s, 1H), 4.48 (s, 2H), 3.65 (s, 3H), 3.44 (br s, 3H), 2.85 - 3.02 (m, 5H), 2.60 - 2.74 (m, 4H), 2.32 - 2.47 (m, 3H), 1.99 - 2.16 (m, 5H), 1.61 - 1.89 (m, 10H), 1.49 (br d, *J =* 3.30 Hz, 2H), 1.12 - 1.26 (m, 2H), 0.84 - 0.98 (m, 2H). |
| **39** | 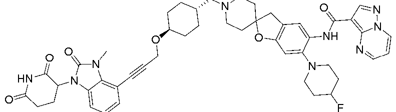 |
| | ESI-MS *(m*/*z):* 858.4 [M+1]⁺. |
| | ¹H NMR (400 MHz, DMSO-*d*₆) *δ* 11.00 - 11.28 (m, 1H), 10.47 (s, 1H), 9.37 (d, *J* = 6.88 Hz, 1H), 8.84 (br d, *J =* 2.88 Hz, 1H), 8.69 (s, 1H), 8.33 (s, 1H), 7.32 - 7.38 (m, 1H), 7.18 (br d, *J =* 7.88 Hz, 1H), 7.12 (d, *J =* 7.63 Hz, 1H), 7.00 - 7.08 (m, 1H), 6.73 (s, 1H), 5.41 (br dd, *J =* 12.76, 5.25 Hz, 1H), 4.80 - 5.01 (m, 1H), 4.41 - 4.50 (m, 2H), 3.65 (s, 3H), 2.84 - 3.02 (m, 6H), 2.61 - 2.82 (m, 6H), 2.37 (br s, 2H), 1.95 - 2.22 (m, 10H), 1.70 - 1.90 (m, 7H), 1.41 - 1.55 (m, 2H), 1.13 - 1.32 (m, 3H), 0.82 - 0.97 (m, 2H). |
| **40** | 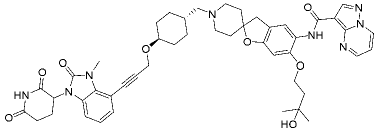 |
| | ESI-MS *(m*/*z):* 859.5 [M+H]⁺ |
| | ¹H NMR (400 MHz, DMSO-*d*₆) *δ* 10.92 - 11.22 (m, 1H), 10.21 (s, 1H), 9.37 (d, *J* = 6.88 Hz, 1H), 8.83 (br d, *J* = 4.13 Hz, 1H), 8.67 (s, 1H), 8.26 (s, 1H), 7.36 (dd, *J =* 6.94, 4.31 Hz, 1H), 7.18 (br d, *J* = 7.88 Hz, 1H), 7.10 - 7.15 (m, 1H), 7.01 - 7.08 (m, 1H), 6.64 (s, 1H), 5.40 (br dd, *J =* 12.51, 5.25 Hz, 1H), 4.41 - 4.51 (m, 3H), 4.16 (br t, *J* = 7.00 Hz, 2H), 3.65 (s, 3H), 2.85 - 2.99 (m, 4H), 2.66 - 2.77 (m, 3H), 2.36 - 2.43 (m, 2H), 2.01 - 2.16 (m, 7H), 1.73 - 1.87 (m, 6H), 1.42 - 1.57 (m, 2H), 1.19 (s, 8H), 0.85 - 0.97 (m, 2H). |
| **41** | 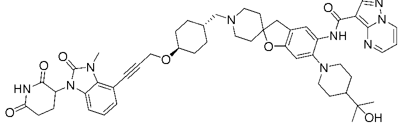 |
| | ESI-MS *(m*/*z):* 898.3 [M+H]⁺. |
| | ¹H NMR (500 MHz, DMSO-*d*₆) *δ* 11.09 (s, 1H), 10.37 (s, 1H), 9.33 (d, *J =* 7.0 Hz, 1H), 8.93 (d, *J* = 2.7 Hz, 1H), 8.67 (s, 1H), 8.26 (s, 1H), 7.31 (dd, *J* = 6.9, 4.3 Hz, 1H), 7.17 (d, *J =* 7.9 Hz, 1H), 7.12 (d, *J =* 7.6 Hz, 1H), 7.03 (t, *J =* 7.9 Hz, 1H), 6.69 (s, 1H), 5.39 (dd, *J =* 12.8, 5.3 Hz, 1H), 4.47 (s, 2H), 4.16 (s, 1H), 3.65 (s, 3H), 3.46 (t, *J =* 10.6 Hz, 1H), 2.97 (s, 4H), 2.93 - 2.84 (m, 1H), 2.78 - 2.54 (m, 4H), 2.36 (s, 2H), 2.13 (d, *J =* 5.0 Hz, 2H), 2.04 (d, *J =* 10.1 Hz, 3H), 1.82 (d, *J =* 13.1 Hz, 4H), 1.74 (d, *J =* 11.1 Hz, 4H), 1.63 (dd, *J =* 21.5, 11.6 Hz, 2H), 1.47 (s, 1H), 1.32 - 1.13 (m, 5H), 1.10 (s, 6H), 0.95 - 0.84 (m, 2H). |
| **42** | 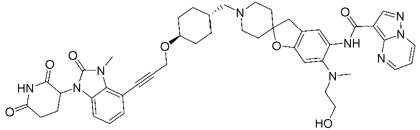 |
| | ESI-MS (m/z): 830.3 [M+1]⁺. |
| | ¹H NMR (400 MHz, DMSO-*d*₆) *δ* 11.23 - 10.96 (m, 1H), 10.56 (s, 1H), 9.35 (dd, *J =* 1.5, 7.0 Hz, 1H), 8.86 (dd, *J =* 1.5, 4.1 Hz, 1H), 8.66 (s, 1H), 8.26 (s, 1H), 7.32 (dd, *J =* 4.1, 7.0 Hz, 1H), 7.19 - 7.09 (m, 2H), 7.07 - 6.99 (m, 1H), 6.74 (s, 1H), 5.39 (br dd, *J =* 5.3, 12.6 Hz, 1H), 4.51 - 4.48 (m, 1H), 4.49 - 4.34 (m, 2H), 3.64 (s, 3H), 3.58 (br t, *J =* 6.6 Hz, 2H), 3.02 - 2.82 (m, 6H), 2.73 - 2.59 (m, 6H), 2.40 - 2.32 (m, 2H), 2.13 - 2.01 (m, 5H), 1.85 - 1.71 (m, 6H), 1.47 (br d, *J* = 3.3 Hz, 1H), 1.23 - 1.12 (m, 2H), 1.05 (t, *J =* 7.0 Hz, 1H), 0.96 - 0.83 (m, 2H). |
| **43** | 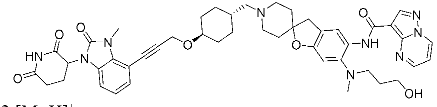 |
| | ESI-MS *(m*/*z):* 844.5 [M+H]⁺. |
| | ¹H NMR (500 MHz, DMSO-*d*₆) *δ* 11.11 (s, 1H), 10.62 (s, 1H), 9.34 (d, *J* = 7.0 Hz, 1H), 8.87 (d, *J* = 2.7 Hz, 1H), 8.66 (s, 1H), 8.30 (s, 1H), 7.32 (dd, *J =* 6.9, 4.2 Hz, 1H), 7.18 (d, *J =* 7.8 Hz, 1H), 7.12 (d, *J =* 7.8 Hz, 1H), 7.04 (t, *J =* 7.9 Hz, 1H), 6.72 (s, 1H), 5.41 (dd, *J =* 12.7, 5.3 Hz, 1H), 4.48 (s, 2H), 4.30 (t, *J =* 4.9 Hz, 1H), 3.65 (s, 3H), 3.46 (dd, *J =* 11.5, 6.0 Hz, 4H), 3.01 (s, 2H), 2.98 - 2.85 (m, 3H), 2.78 - 2.60 (m, 4H), 2.59 (s, 3H), 2.45 - 2.21 (m, 2H), 1.95 (dd, *J =* 100.9, 11.7 Hz, 9H), 1.68 - 1.60 (m, 2H), 1.55 (s, 1H), 1.20 (dd, *J =* 25.6, 13.1 Hz, 3H), 1.00 - 0.89 (m, 2H). |
| **44** | 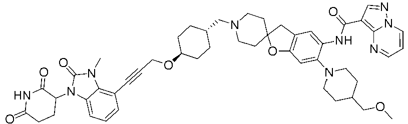 |
| | ESI-MS *(m*/*z):* 884.5 [M+H]⁺. |
| | ¹H NMR (500 MHz, DMSO-*d*₆) *δ* 11.06 (s, 1H), 10.47 (s, 1H), 9.33 (d, *J =* 6.9 Hz, 1H), 8.86 (d, *J* = 2.4 Hz, 1H), 8.66 (s, 1H), 8.33 (s, 1H), 7.39 - 7.31 (m, 1H), 7.18 (d, *J =* 7.8 Hz, 1H), 7.11 (d, *J* = 7.8 Hz, 1H), 7.03 (t, *J* = 7.8 Hz, 1H), 6.70 (s, 1H), 5.41 (dd, *J* = 12.7, 5.1 Hz, 1H), 4.45 (s, 2H), 3.65 (s, 3H), 3.42 (d, *J=* 10.5 Hz, 1H), 3.30 (s, 5H), 2.91 (dd, *J =* 25.4, 16.0 Hz, 5H), 2.71 (m, *J =* 37.3, 23.0, 8.2 Hz, 4H), 2.46 (s, 2H), 2.34 (s, 2H), 2.06 (dd, *J =* 29.2, 8.4 Hz, 5H), 1.80 (d, *J =* 10.7 Hz, 4H), 1.68 (dd, *J =* 23.6, 11.9 Hz, 7H), 1.44 (s, 1H), 1.16 (dd, *J =* 24.8, 12.9 Hz, 2H), 0.87 (dd, *J* = 23.7, 11.4 Hz, 2H). |
| **45** | 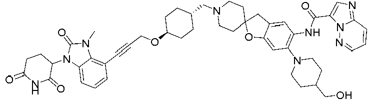 |
| | ESI-MS *(m*/*z):* 870.4 [M+H]⁺. |
| | ¹H NMR (500 MHz, DMSO-*d*₆) *δ* 11.05 (s, 2H), 8.91 (d, *J =* 3.5 Hz, 1H), 8.43 (s, 1H), 8.39 (d, *J* = 8.5 Hz, 1H), 8.35 (s, 1H), 7.52 (dd, *J =* 9.2, 4.5 Hz, 1H), 7.17 (d, *J =* 7.8 Hz, 1H), 7.12 (d, *J =* 7.7 Hz, 1H), 7.03 (t, *J =* 7.9 Hz, 1H), 6.75 (s, 1H), 5.39 (dd, *J =* 12.7, 5.2 Hz, 1H), 4.57 (t, *J = 5.1* Hz, 1H), 4.45 (d, *J =* 13.8 Hz, 2H), 3.65 (s, 3H), 3.49 - 3.36 (m, 4H), 2.98 (s, 2H), 2.94 (d, *J =* 10.7 Hz, 2H), 2.91 - 2.84 (m, 1H), 2.69 (dd, *J =* 33.5, 17.3, 10.5 Hz, 4H), 2.37 (s, 2H), 2.12 (d, *J =* 7.0 Hz, 2H), 2.04 (d, *J =* 8.2 Hz, 2H), 1.82 (d, *J =* 12.7 Hz, 4H), 1.73 (d, *J =* 12.1 Hz, 4H), 1.52 (dd, *J* = 25.1, 18.6 Hz, 4H), 1.23 (s, 1H), 1.18 (dd, J= 23.1, 10.7 Hz, 2H), 0.90 (dd, *J =* 24.1, 11.4 Hz, 2H). |
| **46** | 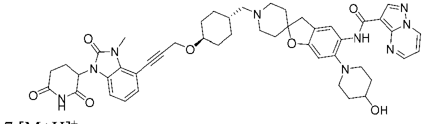 |
| | ESI-MS *(m*/*z):* 856. / [M+H]⁺ . |
| | ¹H NMR (400 MHz, DMSO-*d*₆) *δ* 11.14 (s, 1H), 10.55 (s, 1H), 9.38 (dd, *J =* 7.00, 1.50 Hz, 1H), 8.89 (dd, *J =* 4.19, 1.56 Hz, 1H), 8.69 (s, 1H), 8.34 (s, 1H), 7.34 - 7.40 (m, 1 H), 7.18 (d, *J =* 7.88 Hz, 1H), 7.11 - 7.14 (m, 1H), 7.01 - 7.07 (m, 1H), 6.72 (s, 1H), 5.41 (dd, *J* = 12.57, 5.19 Hz, 1H), 4.78 (d, *J* = 4.00 Hz, 1H), 4.48 (s, 2H), 3.65 (s, 3H), 3.41 - 3.51 (m, 1H), 3.27 - 3.31 (m, 1H), 2.98 (s, 2H), 2.84 - 2.93 (m, 3H), 2.58 - 2.78 (m, 5H), 2.31 - 2.43 (m, 3H), 2.12 (d, *J =* 6.88 Hz, 2H), 2.00 - 2.08 (m, 3H), 1.67 - 1.93 (m, 10H), 1.43 - 1.53 (m, 1H), 1.12 - 1.24 (m, 2H), 0.83 - 0.97 (m, 2H). |
| **47** | 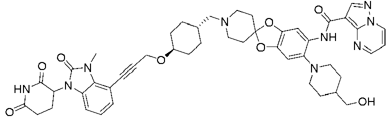 |
| | ESI-MS *(m*/*z):* 872.5 [M+1]⁺. |
| | ¹H NMR (400 MHz, DMSO-*d*₆) *δ* 11.16 - 11.06 (m, 1H), 10.70 (s, 1H), 9.36 (dd, *J* = 1.5, 6.9 Hz, 1H), 8.90 (dd, *J =* 1.6, 4.2 Hz, 1H), 8.69 (s, 1H), 8.11 (s, 1H), 7.34 (dd, *J =* 4.2, 7.0 Hz, 1H), 7.17 *(d, J =* 7.4 Hz, 1H), 7.14 - 7.08 (m, 1H), 7.07 - 6.99 (m, 1H), 6.96 (s, 1H), 5.40 (dd, *J =* 5.4, 12.4 Hz, 1H), 4.57 (t, *J =* 5.3 Hz, 1H), 4.50 - 4.42 (m, 2H), 3.64 (s, 3H), 3.41 (br t, *J =* 5.5 Hz, 3H), 2.97 - 2.76 (m, 4H), 2.24 - 2.11 (m, 3H), 2.11 - 1.87 (m, 8H), 1.76 - 1.67 (m, 2H), 1.86 - 1.65 (m, 3H), 1.64 - 1.40 (m, 6H), 1.31 - 1.02 (m, 4H), 0.99 - 0.82 (m, 2H). |
| **48** | 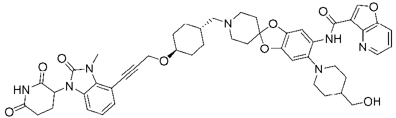 |
| | ESI-MS *(m*/*z):* 872.5 [M+1]⁺. |
| | 1H NMR (400 MHz, DMSO-*d*₆) *δ* 11.12 (s, 2H), 8.99 (s, 1H), 8.75 (d, *J =* 3.9 Hz, 1H), 8.31 (s, 1H), 8.26 (d, *J =* 8.6 Hz, 1H), 8.09 (s, 1H), 7.57 (dd, *J =* 4.9, 8.2 Hz, 1H), 7.17 (d, *J =* 7.4 Hz, 1H), 7.14 - 7.08 (m, 1H), 7.07 - 7.00 (m, 1H), 7.00 - 6.95 (m, 1H), 5.44 - 5.36 (m, 1H), 4.55 (t, *J =* 5.4 Hz, 1H), 4.47 (s, 2H), 3.64 (s, 3H), 3.39 (br d, *J =* 5.7 Hz, 4H), 2.97 - 2.79 (m, 4H), 2.21 - 2.13 (m, 2H), 2.10 - 1.91 (m, 8H), 1.87 - 1.78 (m, 2H), 1.91 - 1.67 (m, 2H), 1.67 - 1.41 (m, 5H), 1.28 - 1.03 (m, 4H), 1.02 - 0.81 (m, 3H). |
| **49** | 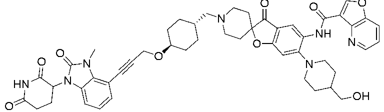 |
| | ESI-MS *(m*/*z):* 884.4 [M+1]⁺. |
| | 1H NMR (400 MHz, DMSO-*d*₆) *δ* 11.26 - 10.95 (m, 1H), 10.80 (s, 1H), 9.07 (s, 1H), 8.75 (dd, *J =* 0.9, 4.8 Hz, 1H), 8.58 (s, 1H), 8.33 - 8.27 (m, 1H), 7.63 - 7.56 (m, 1H), 7.18 (d, *J =* 7.4 Hz, 1H), 7.15 - 7.10 (m, 2H), 7.08 - 7.00 (m, 1H), 5.45 - 5.37 (m, 1H), 4.57 - 4.51 (m, 1H), 4.50 - 4.43 (m, 2H), 3.65 (s, 3H), 3.25 - 3.15 (m, 4H), 2.91 - 2.81 (m, 3H), 2.73 (br s, 2H), 2.24 - 2.16 (m, 3H), 2.09 - 1.99 (m, 3H), 1.89 - 1.81 (m, 4H), 1.76 - 1.71 (m, 2H), 1.61 - 1.55 (m, 5H), 1.31 (br d, *J =* 7.6 Hz, 1H), 1.25 - 1.07 (m, 5H), 0.96 - 0.84 (m, 3H). |
| **50** | 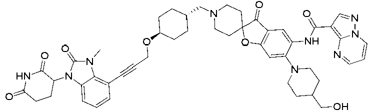 |
| | ESI-MS *(m*/*z):* 884.1 [M+1]⁺. |
| | 1H NMR (400 MHz, DMSO-*d*₆) *δ* 11.25 - 10.95 (m, 1H), 10.30 (s, 1H), 9.39 (dd, *J =* 1.5, 7.0 Hz, 1H), 8.92 (dd, *J =* 1.5, 4.2 Hz, 1H), 8.74 (s, 1H), 8.58 (s, 1H), 7.39 - 7.34 (m, 1H), 7.17 (d, *J =* 8.0 Hz, 1H), 7.14 - 7.10 (m, 2H), 7.06 - 7.01 (m, 1H), 5.40 (dd, *J* = 5.2, 12.7 Hz, 1H), 4.55 (t, *J =* 5.4 Hz, 1H), 4.47 (s, 2H), 3.65 (s, 3H), 3.51 - 3.43 (m, 1H), 3.38 (br t, *J =* 4.9 Hz, 2H), 3.18 (br d, *J =* 11.9 Hz, 2H), 2.94 - 2.79 (m, 3H), 2.78 - 2.63 (m, 4H), 2.29 - 2.13 (m, 4H), 2.11 - 1.98 (m, 3H), 1.90 - 1.80 (m, 1H), 1.65 - 1.46 (m, 6H), 1.25 - 1.12 (m, 2H), 0.99 - 0.84 (m, 2H). |
| **51** | 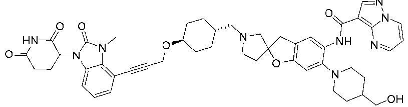 |
| | ESI-MS *(m*/*z):* 856.5[M+H]⁺ESI-MS *(m*/*z):* 856.5[M+H]⁺ |
| | ¹H NMR (400 MHz, DMSO-*d*₆) *δ* 11.14 (br s, 1H), 10.50 (s, 1H), 9.36 (d, *J =* 7.18 Hz, 1H), 8.91 (d, *J* = 3.09 Hz, 1H), 8.69 (s, 1H), 8.34 (s, 1H), 7.34 (dd, *J =* 6.93, 4.21 Hz, 1H), 7.10 - 7.22 (m, 2H), 7.01 - 7.07 (m, 1H), 6.73 (s, 1H), 5.41 (br dd, *J =* 12.50, 5.20 Hz, 1H), 4.57 (t, *J* =5.26 Hz, 1H), 4.48 (s, 2H), 3.66 (s, 3H), 3.40 - 3.51 (m, 3H), 3.17 - 3.25 (m, 2H), 2.81 - 2.97 (m, 4H), 2.58 - 2.78 (m, 6H), 2.12 - 2.31 (m, 3H) 1.96 - 2.10 (m, 4H), 1.69 - 1.93 (m, 4H), 1.36 - 1.65 (m, 4H), 1.11 - 1.26 (m, 2H), 0.84 - 0.99 (m, 2H). |
| **52** | 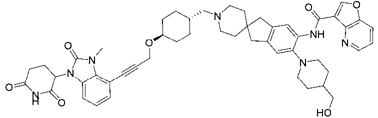 |
| | ESI-MS *(m*/*z):* 868.3 [M+1]⁺. |
| | ¹H NMR (400 MHz, DMSO-*d*₆) *δ* 11.02 - 11.24 (m, 2H), 8.98 (s, 1H), 8.76 (d, *J =* 4.63 Hz, 1H), 8.33 (s, 1H), 8.26 (d, *J =* 8.38 Hz, 1H), 7.57 (dd, *J =* 8.50, 4.75 Hz, 1H), 7.09 - 7.22 (m, 3H), 7.00 - 7.07 (m, 1H), 5.40 (br dd, *J =* 12.51, 5.25 Hz, 1H), 4.56 (br t, *J =* 5.25 Hz, 1H), 4.43 - 4.51 (m, 2H), 3.65 (s, 3H), 2.85 - 2.98 (m, 3H), 2.57 - 2.77 (m, 9H), 2.33 (br s, 3H), 1.99 - 2.14 (m, 5H), 1.70 - 1.85 (m, 4H), 1.42 - 1.67 (m, 9H), 1.11 - 1.30 (m, 3H), 0.82 - 0.96 (m, 2H). |
| **53** | 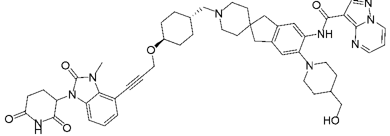 |
| | ESI-MS *(m*/*z):* 868.4 [M+H]⁺ |
| | ¹H NMR (400 MHz, DMSO-*d*₆) *δ* 10.97 - 11.25 (m, 1H), 10.66 (s, 1H), 9.36 (dd, *J =* 6.85, 1.22 Hz, 1H), 8.91 (dd, *J =* 4.10, 1.28 Hz, 1H), 8.69 (s, 1H), 8.35 (s, 1H), 7.35 (dd, *J =* 6.91, 4.22 Hz, 1H), 7.18 (d, *J =* 7.95 Hz, 1H), 7.12 (d, *J =* 5.75 Hz, 2H), 6.99 - 7.08 (m, 1H), 5.40 (br dd, *J =* 12.59, 5.38 Hz, 1H), 4.53 - 4.68 (m, 1H), 4.40 - 4.51 (m, 2H), 3.65 (s, 3H), 3.42 (br d, *J =* 5.14 Hz, 2H), 2.84 - 2.98 (m, 3H), 2.60 - 2.79 (m, 8H), 2.33 (br d, *J =* 1.59 Hz, 4H), 1.98 - 2.15 (m, 5H), 1.71 - 1.87 (m, 4H), 1.40 - 1.67 (m, 9H), 1.10 - 1.28 (m, 2H), 0.73 - 1.02 (m, 2H). |
| **54** | 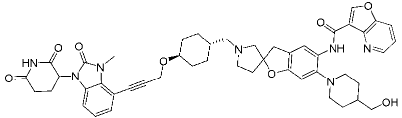 |
| | ESI-MS (m/z): *856.4* [M+H]⁺ |
| | ¹H NMR (400 MHz, DMSO-*d*₆) *δ* 11.133 (s, 1H), 10.85 - 10.92 (m, 1H), 8.96 (s, 1H), 8.77 (d, *J =* 4.65 Hz, 1H), 8.755 (s, 1H), 8.743 (d, *J =* 8.19 Hz, 1H), 7.57 (dd, *J =* 8.38, 4.83 Hz, 1H), 7.15 - 7.20 (m, 1H), 7.12 (d, *J =* 7.46 Hz, 1H), 7.00 - 7.07 (m, 1H), 6.74 (s, 1H), 5.32 - 5.47 (m, 1H), 4.47 (s, 2H), 3.65 (s, 3H), 3.17 - 3.27 (m, 5H), 2.87 - 3.02 (m, 4H), 2.66 (br s, 6H), 2.10 - 2.28 (m, 4H), 2.00 - 2.07 (m, 4H), 1.80 - 1.89 (m, 2H), 1.72 (br d, *J =* 11.13 Hz, 2H), 1.45 - 1.65, (m, 4H), 1.10 - 1.22 (m, 2H), 0.84 - 0.99 (m, 2H). |
| **55** | 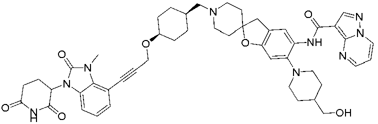 |
| | ESI-MS *(m*/*z):* 870.4[M+H]⁺ |
| | ¹H NMR (400 MHz, DMSO-*d*₆) *δ* 11.03 - 11.23 (m, 1H), 10.48 (s, 1H), 9.35 (d, *J=* 6.93 Hz, 1H), 8.89 (d, *J* =3.96 Hz, 1H), 8.67 (s, 1H), 8.33 (s, 1H), 7.33 (dd, *J =* 6.93, 4.21 Hz, 1H), 7.17 (br d, *J* = 7.55 Hz, 1H), 7.09 - 7.14 (m, 1H), 7.00 - 7.06 (m, 1H), 6.72 (s, 1H), 5.39 (br dd, *J=* 12.56, 5.01 Hz, 1H), 4.56 (br s, 1H), 4.44 (s, 2H), 3.78 (br s, 1H), 3.64 (s, 3H), 3.38 (br d, *J =* 3.34 Hz, 3H), 2.82 - 3.01 (m, 5H), 2.58 - 2.75 (m, 4H), 2.29 - 2.43 (m, 2H), 2.14 (br d, *J =* 6.93 Hz, 2H), 1.99 - 2.08 (m, 1H), 1.66 - 1.86 (m, 8H), 1.42 - 1.64 (m, 8H), 1.18 - 1.30 (m, 2H). |
| **56** | 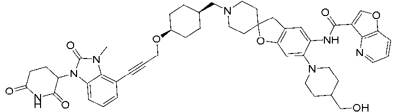 |
| | ESI-MS *(m*/*z):* 870.4[M+H]⁺ |
| | ¹H NMR (400 MHz, DMSO-*d*₆) *δ* 11.15 (br s, 1H), 10.93 (s, 1H), 8.98 (s, 1H), 8.75 (d, *J =* 4.33 Hz, 1H), 8.33 (s, 1H), 8.26 (d, *J =* 8.41 Hz, 1H), 7.58 (dd, *J =* 8.41, 4.82 Hz, 1H), 7.19 (d, *J =* 7.79 Hz, 1H), 7.11 - 7.16 (m, 1H), 7.02 - 7.08 (m, 1H), 6.76 (s, 1H), 5.42 (br dd, *J =* 12.62, 5.32 Hz, 1H), 4.56 (t, *J=* 5.20 Hz, 1H), 4.45 (s, 2H), 3.79 (br s, 1H), 3.66 (s, 3H), 3.38 - 3.41 (m, 3H), 2.85 - 3.02 (m, 5H), 2.61 - 2.78 (m, 4H), 2.31 - 2.45 (m, 2H), 2.16 (br d, *J =* 6.80 Hz, 2H), 2.00 - 2.10 (m, 1H), 1.68 - 1.90 (m, 8H), 1.43 - 1.66 (m, 8H), 1.19 - 1.32 (m, 2H). |
| **57** | 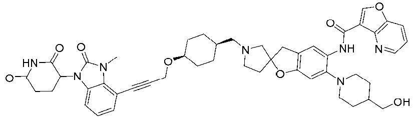 |
| | ESI-MS *(m*/*z):* 856.4 [M+H]⁺. |
| | ¹H NMR (400 MHz, DMSO-*d*₆) δ ppm 10.97 - 11.25 (m, 1H), 10.49 (s, 1H), 9.36 (br d, *J =* 6.91 Hz, 1H), 8.90 (br d, *J =* 3.10 Hz, 1H), 8.68 (s, 1H), 8.34 (s, 1H), 7.34 (dd, *J =* 6.79, 4.17 Hz, 1H), 7.17 (br d, *J =* 7.75 Hz, 1H), 7.12 (br d, *J =* 7.87 Hz, 1H), 6.99 - 7.07 (m, 1H), 6.73 (s, 1H), 5.39 (br dd, *J =* 12.46, 5.19 Hz, 1H), 4.52 - 4.66 (m, 1H), 4.44 (s, 2H), 3.78 (br s, 1H), 3.64 (s, 3H), 3.40 (br d, *J =* 4.65 Hz, 2H), 3.21 (br d, *J =* 6.32 Hz, 2H), 2.82 - 2.96 (m, 4H), 2.58 - 2.77 (m, 6H), 2.22 - 2.35 (m, 2H), 1.97 - 2.19 (m, 3H), 1.68 - 1.87 (m, 4H), 1.43 - 1.65 (m, 8H), 1.20 - 1.33 (m, 2H). |
| **58** | 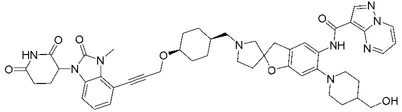 |
| | ESI-MS *(m*/*z):* 856.5[M+H]⁺ |
| | ¹H NMR (400 MHz, DMSO-*d*₆) *δ* 10.97 - 11.25 (m, 1H), 10.49 (s, 1H), 9.36 (br d, *J =* 6.91 Hz, 1H), 8.90 (br d, *J =* 3.10 Hz, 1H), 8.68 (s, 1H), 8.34 (s, 1H), 7.34 (dd, *J =* 6.79, 4.17 Hz, 1H), 7.17 (br d, *J =* 7.75 Hz, 1H), 7.12 (br d, *J =* 7.87 Hz, 1H), 6.99 - 7.07 (m, 1H), 6.73 (s, 1H), 5.39 (br dd, *J =* 12.46, 5.19 Hz, 1H), 4.52 - 4.66 (m, 1H), 4.44 (s, 2H), 3.78 (br s, 1H), 3.64 (s, 3H), 3.40 (br d, *J =* 4.65 Hz, 2H), 3.21 (br d, *J =* 6.32 Hz, 2H), 2.82 - 2.96 (m, 4H), 2.58 - 2.77 (m, 6H), 2.22 - 2.35 (m, 2 H), 1.97 - 2.19 (m, 3H), 1.68 - 1.87 (m, 4H), 1.43 - 1.65 (m, 8H), 1.20 - 1.33 (m, 2H). |

### Example 59:

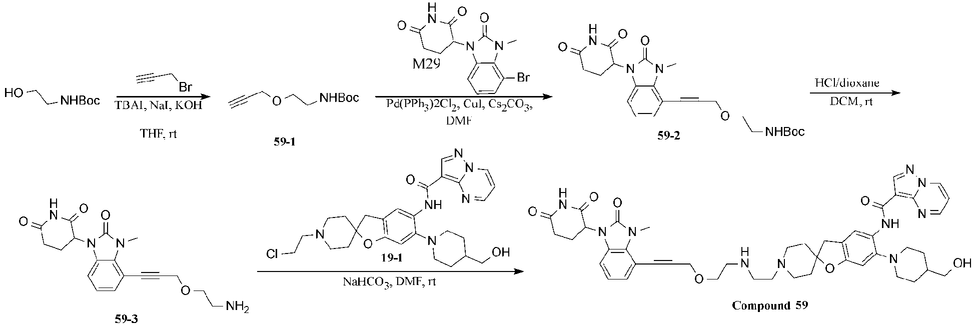

### Step 1:

(2-Hydroxyethyl)carbamic acid tert-butyl ester (5 g, 31.11 mmol) and 3-bromoprop-1-yne (11 g, 93.16 mmol) were dissolved in tetrahydrofuran (75 mL). Tetrabutylammonium iodide (1.14 g, 3.11 mmol), potassium iodide (465 mg, 3.11 mmol), and potassium hydroxide (5.22 g, 93.33 mmol) were added. The mixture was stirred at room temperature overnight. After completion, the reaction was quenched with water and extracted with ethyl acetate (50 mL × 3). The organic phases were combined and concentrated under reduced pressure. The crude product was purified by column chromatography (eluent: DCM/MeOH = 15: 1) to afford compound 59-1 (4.6 g, 74.19% yield) as a colorless oil.

ESI-MS (m/z): 200.2 [M+H]⁺.

### Step 2:

Compound **59-1** (710 mg, 3.56 mmol), copper(I) iodide (2.68 g, 0.356 mmol), cesium carbonate (3.471 g, 10.68 mmol), and bis(triphenylphosphine)palladium(II) dichloride (125 mg, 0.18 mmol) were dissolved in DMF (30 mL). Under nitrogen protection, the mixture was stirred at 85°C overnight. After completion, the reaction was quenched with a small amount of water and extracted with ethyl acetate (50 mL × 3). The organic phases were combined and concentrated under reduced pressure. The residue was loaded onto a column for purification (eluent: DCM/MeOH, = 15: 1) to afford compound 59-2 (400 mg, 24.69% yield) as a pale yellow oil.

ESI-MS (m/z): 457.1 [M+H]⁺.

### Step 3:

Compound 59-2 (200 mg, 0.44 mmol) was dissolved in dichloromethane (20 mL). A solution of HCl in 1,4-dioxane (4 mL) was added. The mixture was stirred at room temperature overnight. After completion, the reaction mixture was concentrated under reduced pressure. Dichloromethane (20 mL × 3) was added for extraction. The organic phases were combined and concentrated to dryness to afford compound 59-3 (120 mg, 76.69% yield) as a yellow oil.

ESI-MS (m/z): 357.1 [M+H]⁺.

### Step 4:

Compound **59-3** (50 mg, 0.14 mmol) and 19-1 (73 mg, 0.14 mmol) were dissolved in DMF (20 mL). Sodium bicarbonate (17 mg, 1.4 mmol) was added. Under nitrogen protection, the mixture was stirred at room temperature overnight. After completion, the reaction mixture was concentrated under reduced pressure. The crude product was purified by preparative chromatography (ACN:H₂O *=* 1:1). Lyophilization afforded **Compound 59** (2.4 mg, 2% yield) as a white solid.

ESI-MS (*m*/*z*): 845.3 [M+H]⁺.

¹H NMR (400 MHz, DMSO-*d*₆) *δ* 11.12 (s, 1H), 10.48 (s, 1H), 9.35 (d, *J =* 7.2 Hz, 1H), 8.90 (d, *J =* 4.2Hz, 1H), 8.67 (s, 1H), 8.31 (s, 1H), 7.33 - 7.31 (m, 4.2 Hz, 1H), 7.16 -7.14 (m, 2H), 7.04 - 6.97(m, 1H), 6.72 (s, 1H), 5.40 - 5.38 (m, 1H), 4.57 - 4.55(m, 1H), 4.47 (s, 2H), 3.66 (s, 3H), 3.64 - 3.60 (m, 3H), 2.91 (d, *J* = 10.0 Hz, 6H), 2.76 (s, 2H), 2.69 - 2.60 (m, 6H), 2.48 - 2.37 (m, 6H), 2.10 - 1.87 (m, 2H), 1.81 - 1.66 (m, 6H), 1.62 - 1.48 (m, 3H).

### Example 60:

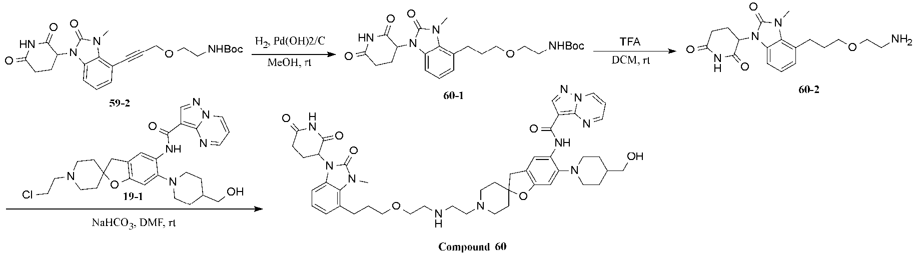

### Step 1:

Compound 59-2 (200 mg, 0.43 mmol) was dissolved in methanol (40 mL). Pd/C (60 mg) and Pd(OH)₂ (60 mg) were added. The mixture was stirred under a hydrogen balloon overnight. After completion, the mixture was filtered and the filtrate was concentrated under reduced pressure to afford compound **60-1** (200 mg, 99.0% yield) as a colorless oil.

ESI-MS (m/z): 461.1 [M+H]⁺.

### Step 2:

Compound 60-1 (200 mg, 0.44 mmol) was dissolved in dichloromethane (20 mL). Trifluoroacetic acid (4 mL) was added. The mixture was stirred at room temperature for 2 hours. After completion, the solvent was evaporated. The residue was extracted and separated to afford compound 60-2 (120 mg, 76.9% yield) as a colorless oil.

ESI-MS (m/z): 361.1 [M+H]⁺.

### Step 3:

Compound 19-1 (50 mg, 0.14 mmol) and compound 60-2 (73 mg, 0.14 mmol) were dissolved in DMF (20 mL). Sodium bicarbonate (17 mg, 1.4 mmol) was added. Under nitrogen protection, the mixture was stirred at room temperature overnight. After completion, the reaction mixture was concentrated under reduced pressure to give a crude product (60 mg). Purification by preparative chromatography (eluent: ACN:H₂O = 1:1) followed by lyophilization afforded **Compound 60** (2.0 mg, 1.79% yield) as a white solid.

ESI-MS (m/z): 849.0 [M+H]⁺.

1H NMR (400 MHz, DMSO-*d*₆) *δ* 11.08 (s, 1H), 10.48 (s, 1H), 9.36 (d, J = 11.0 Hz, 1H), 8.89 (d, J = 4.2 Hz, 1H), 8.67 (d, J = 5.2 Hz, 1H), 8.31 (s, 1H), 7.33 (d, J = 11.4 Hz, 1H), 7.06 - 6.82 (m, 3H), 6.71 (s, 1H), 5.47 - 5.30 (m, 1H), 4.56 (d, J = 13.2Hz, 1H), 3.58 (s, 3H), 3.49 - 3.45 (m, 4H), 3.01 - 2.85 (m, 9H), 2.76 - 2.58 (m, 10H), 2.33 (s, 2H), 2.03 - 1.42 (m, 15H).

### Example 61:

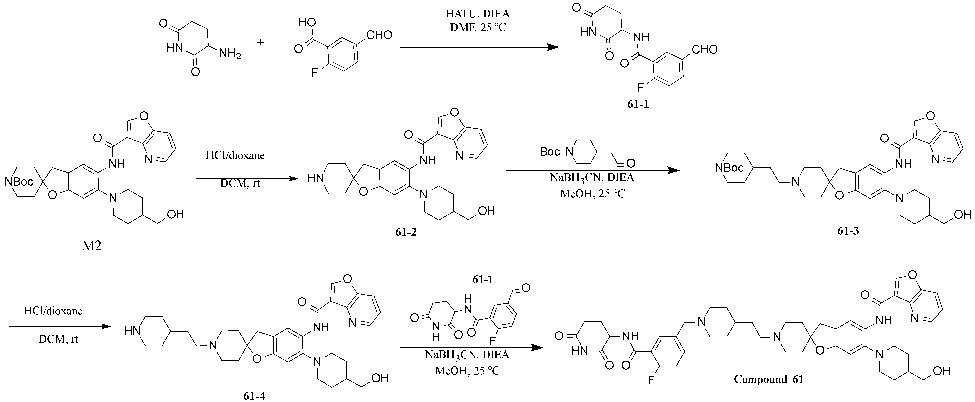

### Step 1:

2-Fluoro-5-formylbenzoic acid (6.00 g, 35.69 mmol, 1.0 eq) was dissolved in N,N-dimethylformamide (60 mL). HATU (14.93 g, 39.26 mmol, 1.1 eq) and DIEA (14.7 mL, 89.22 mmol, 2.5 eq) were added and the mixture was activated for 1 hour. 3-Aminopiperidine-2,6-dione (4.57 g, 35.69 mmol, 1.0 eq) was added. The mixture was stirred at room temperature for 4 hours. After completion, the reaction mixture was slowly poured into ice water with stirring. The mixture was extracted with ethyl acetate (twice). The combined organic phases were back-washed with brine (once), dried over magnesium sulfate, filtered, and concentrated. Solids precipitated upon concentration and were slurry washed with ethyl acetate. The filtrate was purified by column chromatography. The combined product afforded compound **61-1** (3.68 g, 37.1% yield) as a green solid.

ESI-MS (m/z): 279.1 [M+H]⁺

¹H NMR (400 MHz, DMSO-*d*₆) *δ* 10.90 (s, 1H), 10.04 (s, 1H), 8.83 (br d, *J =* 8.3 Hz, 1H), 8.21 (dd, *J*= 2.0, 6.9 Hz, 1H), 8.11 (ddd, *J =* 2.1, 5.0, 8.3 Hz, 1H), 7.57 (dd, *J =* 8.7, 9.9 Hz, 1H), 4.94 - 4.69 (m, 1H), 2.92 - 2.56 (m, 2H), 2.21 - 1.94 (m, 2H).

### Step 2:

To M2 (1.00 g, 1.78 mmol) dissolved in dichloromethane (3 mL), a 4.0 M solution of HCl in dioxane (7 mL, 28.00 mmol) was slowly added dropwise. The reaction mixture was stirred at 25°C for 2 hours. The mixture was concentrated under reduced pressure to afford compound 61-2 (900 mg, 1.80 mmol) as a pale yellow solid.

ESI-MS (m/z): 463.3 [M+H]⁺

### Step 3:

To a solution of 61-2 (200 mg, 0.43 mmol) in methanol (3 mL), DIEA (0.1 mL, 0.86 mmol, 2.0 eq) was added, followed by tert-butyl 4-(2-oxoethyl)piperidine-1-carboxylate (98 mg, 0.43 mmol, 1.0 eq). Acetic acid was added to adjust the pH to 5-6. Sodium cyanoborohydride (40 mg, 0.64 mmol, 1.5 eq) was added. The reaction mixture was stirred at 25°C for 2 hours. After concentration under reduced pressure, water (15 mL) was added to the residue. The mixture was extracted with ethyl acetate (20 mL × 2). The combined organic phases were washed with saturated brine (10 mL), dried, and concentrated under reduced pressure. And purified by thin-layer chromatography (ethyl acetate:methanol = 10:1) to afford compound 61-3 (197 mg, 0.26 mmol, 60.3% yield) as a white solid.

ESI-MS (m/z): 674.4 [M+H]⁺

### Step 4:

To a solution of 61-3 (176 mg, 0.26 mmol) in dichloromethane (4 mL), a solution of HCl in dioxane (4 mL, 16.0 mmol) was added. The reaction mixture was stirred at 25°C for 1 hour. The mixture was concentrated under reduced pressure to afford compound 61-4 (200 mg, 0.35 mmol) as a yellow solid.

ESI-MS (m/z): 574.4 [M+H]⁺

### Step 5:

Compound 61-4 (103 mg, 0.18 mmol, 1.0 eq) was dissolved in methanol (2 mL). DIEA (0.1 mL, 0.36 mmol, 2.0 eq) was added, followed by compound 61-1 (50 mg, 0.18 mmol, 1.0 eq). Glacial acetic acid was added to adjust the pH to 5-6. Sodium cyanoborohydride (17 mg, 0.27 mmol, 1.5 eq) was added. The reaction mixture was stirred at 50°C for 2 hours. After concentration under reduced pressure to remove methanol, And purified by preparative HPLC (formic acid conditions) to afford Compound 61 (35 mg, 0.04 mmol, 21.9% yield) as a pale yellow solid.

ESI-MS (m/z): 836.5 [M+H]⁺

¹H NMR (400 MHz, CD₃OD) *δ* 8.78 (dd, *J =* 4.75, 1.13 Hz, 1H), 8.73 (s, 1H), 8.43 (br d, *J =* 5.50 Hz, 2H), 8.30 (s, 1H), 8.10 (dd, *J* = 8.44, 1.06 Hz, 1H), 7.89 (dd, *J =* 6.88, 1.75 Hz, 1H), 7.60 - 7.67 (m, 1H), 7.53 (dd, *J =* 8.44, 4.82 Hz, 1H), 7.31 (dd, *J =* 10.44, 8.57 Hz, 1H), 6.78 (s, 1 H), 3.93 (s, 2H), 3.52 (d, *J=* 5.88 Hz, 2H), 3.37 - 3.46 (m, 2H), 3.27 - 3.30 (m, 1H), 3.06 - 3.22 (m, 8H), 2.68 - 2.91 (m, 4H), 2.46 - 2.57 (m, 2H), 2.01 - 2.38 (m, 7H), 1.41 - 1.96 (m, 13H).

### Example 62:

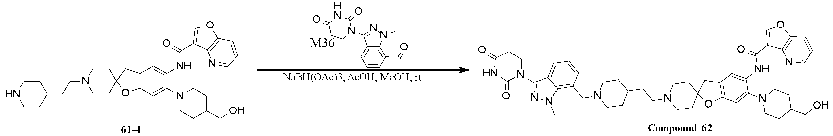

Compound 61-4 (84.29 mg, 0.15 mmol, 1.0 eq) was placed in a 40 mL one-necked flask, followed by addition of MeOH (2 mL), DIEA (38 mg, 0.29 mmol, 2.0 eq), and M36 (40 mg, 0.15 mmol, 1.0 eq). The pH of the reaction solution was adjusted to 5-6 with glacial acetic acid, then sodium triacetoxyborohydride (11.08 mg, 0.18 mmol, 1.2 eq) was added. The reaction mixture was stirred at 45 °C for 16 hours. After completion, the reaction solution was concentrated under reduced pressure and purified by preparative HPLC to afford **Compound 62** (5.78 mg, 0.01 mmol) as a white solid in 4.7% yield.

ESI-MS (*m*/*z*): 830.5 [M+1]⁺.

¹H NMR (400 MHz, DMSO-*d*₆) *δ* 10.92 (s, 1H), 10.41 - 10.63 (m, 1H), 8.96 (s, 1H), 8.74 (d, *J =* 4.89 Hz, 1H), 8.31 (s, 1H), 8.25 (d, *J =* 8.53 Hz, 1H), 7.52 - 7.60 (m, 2H), 7.17 (d, *J =* 6.40 Hz, 1H), 7.02 (t, *J =* 7.40 Hz, 1H), 6.73 (s, 1H), 4.30 (s, 3H), 3.89 (br t, *J =* 6.65 Hz, 2H), 3.74 (s, 2H) 2.98 (br s, 2H), 2.94 (br d, *J =* 11.54 Hz, 3H), 2.81 - 2.84 (m, 2H), 2.77 (br t, *J =* 6.59 Hz, 2H), 2.64 (br t, *J =* 11.17 Hz, 3H), 2.29 - 2.38 (m, 4H), 1.99 (br t, *J =* 10.16 Hz, 2H), 1.49 - 1.87 (m, 14H), 1.30 - 1.40 (m, 3H), 1.10 (br d, *J* = 9.66 Hz, 1H).

### Example 63:

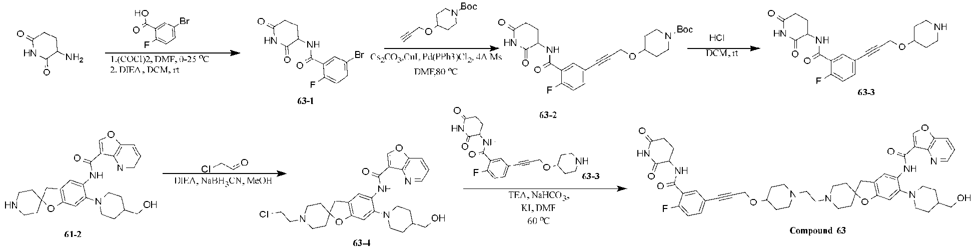

### Step 1:

5-Bromo-2-fluorobenzoic acid (7.00 g, 31.9 mmol) was placed in a 100 mL three-necked flask, followed by DCM (70 mL) and DMF (0.2 mL, 3.20 mmol). The mixture was cooled to 0 °C and oxalyl chloride (2.70 mL, 31.9 mmol, 1.0 eq) was added dropwise. After addition, the reaction was stirred at 0 °C for 10 min, then warmed to room temperature and stirred for an additional 2 h. The solvent was removed under reduced pressure to afford 5-bromo-2-fluorobenzoyl chloride (7.00 g, 29.5 mmol) as a yellow oil. 3-aminopiperidine-2,6-dione (3.02 g, 23.6 mmol) was dissolved in DCM (30 mL), and DIEA (12.2 mL, 73.7 mmol, 3.0 eq) was added. The solution was cooled to 0 °C, and a solution of the above-prepared 5-bromo-2-fluorobenzoyl chloride (7.00 g, 29.48 mmol, 1.25 eq) in DCM (40 mL) was added dropwise. The reaction was allowed to warm to room temperature and stirred for 4 h. Upon completion, the mixture was filtered, and the filter cake was washed with DCM. The collected solid was dried to afford compound 63-1 (5.02 g, 15.2 mmol) as a yellow solid in 51.7% yield.

ESI-MS (m/z): 329.9, 331.9 [M+1]⁺.

¹H NMR (400 MHz, DMSO-*d*₆) *δ* 10.90 (s, 1H), 8.75 (br d, *J =* 8.2 Hz, 1H), 7.79 - 7.70 (m, 2H), 7.37 - 7.28 (m, 1H), 4.85 - 4.65 (m, 1H), 2.86 - 2.68 (m, 1H), 2.59 - 2.52 (m, 1H), 2.18 - 1.95 (m, 2H).

### Step 2:

Compound 63-1 (1.00 g, 3.04 mmol) was dissolved in DMF (10 mL), followed by addition of 2-methylpropan-2-yl 4-(prop-2-ynyloxy)hexahydropyridine-1-carboxylate, (1.09 g, 4.56 mmol, 1.5 eq), Cs₂CO₃ (3.96 g, 12.15 mmol, 4.0 eq), CuI (60 mg, 0.30 mmol, 0.1 eq), and molecular sieves (4 Å). The flask was purged with nitrogen, then Pd(PPh₃)₂Cl₂ (60 mg, 0.085 mmol) was added. After three nitrogen flushes, the reaction was heated to 80 °C and stirred for 2 h. Upon completion, the mixture was poured into water (50 mL) and extracted with ethyl acetate (100 mL × 2). The combined organic layers were washed once with brine, dried over Na₂SO₄, filtered, and concentrated under reduced pressure. And Purified by column chromatography (PE:EA = 20:1-0:1) to afford compound 63-2 (902 mg, 1.85 mmol) as a brown solid in 60.9% yield.

ESI-MS (m/z): 488.3 [M+1]⁺.

### Step 3:

Compound 63-2 (900 mg, 1.64 mmol) was dissolved in DCM (8 mL), and HCl/dioxane (4 M in dioxane, 2 mL) was added. The reaction was stirred at room temperature for 4 h. The resulting precipitate was filtered, washed with DCM, and the collected solid was dried to afford compound 63-3 (455 mg, 1.17 mmol) in 71.6% yield.

ESI-MS (m/z): 388.2 [M+1]⁺.

### Step 4:

Compound 61-2 (300 mg, 0.65 mmol) was placed in a 40 mL single-neck flask, and MeOH (3 mL) was added. DIEA (0.3 mL, 1.95 mmol, 3.0 eq) and chloroacetaldehyde (20 wt% in water, 0.2 mL, 1.30 mmol, 2.0 eq) were then added. The pH was adjusted to 5-6 with acetic acid, followed by addition of sodium cyanoborohydride (81.5 mg, 1.30 mmol, 2.0 eq). The reaction was stirred at room temperature for 2 h. The mixture was filtered, and the filter cake was washed with ethyl acetate (5 mL). The solid was collected to afford compound 63-4 (80 mg, 0.15 mmol) as a white solid in 23.5% yield.

ESI-MS (m/z): 525.3 [M+1]⁺.

¹H NMR (400 MHz, CD₃OD) *δ* 8.78 (dd, *J =* 4.75, 1.13 Hz, 1H), 8.73 (s, 1H), 8.29 (s, 1H), 8.09 (dd, *J=* 8.44, 1.06 Hz, 1H), 7.52 (dd, *J =* 8.38, 4.75 Hz, 1H), 6.78 (s, 1H), 3.97 (br t, *J =* 6.25 Hz, 2H), 3.37 - 3.57 (m, 6H), 3.15 (s, 2H), 3.08 (br d, *J =* 11.01 Hz, 2H), 2.66 - 2.78 (m, 2H), 1.99 - 2.28 (m, 4H), 1.55 - 1.88 (m, 5H).

### Step 5:

Compound 63-3 (35 mg, 0.09 mmol) was placed in a 40 mL single-neck flask and dissolved in DMF (2 mL). Then compound 63-4 (40 mg, 0.08 mmol), NaHCO₃ (13 mg, 0.15 mmol), TEA (15 mg, 0.15 mmol), and KI (4 mg, 0.02 mmol) were added. The reaction mixture was stirred at 60 °C for 10 h. After completion, the solution was concentrated under reduced pressure and purified by preparative HPLC to afford **Compound 63** (2.2 mg) as a white solid in 3.3% yield.

ESI-MS *(m*/*z):* 875.7 [M+1]⁺.

¹H NMR (400 MHz, DMSO-*d*₆) *δ* 10.81 - 10.95 (m, 2H), 8.95 (s, 1H), 8.66 - 8.80 (m, 2H), 8.22 - 8.34 (m, 3H), 7.50 - 7.71 (m, 3H), 7.29 - 7.39 (m, 1H), 6.73 (s, 1H), 4.70 - 4.83 (m, 1H), 4.46 - 4.58 (m, 1H), 4.40 (s, 2H), 2.89 **-** 3.01 (m, 6H), 2.53 - 2.77 (m, 11H), 1.97 - 2.17 (m, 5H), 1.68 - 1.92 (m, 9H), 1.41 - 1.64 (m, 6H).

### Example 64:

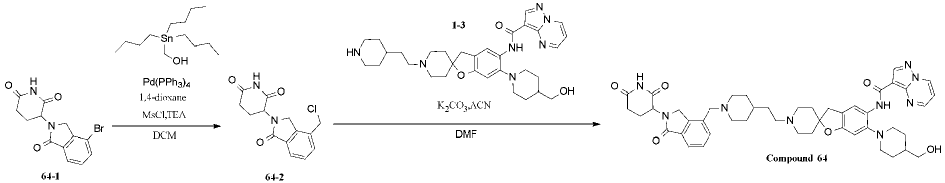

### Step 1:

Compound 64-1 (1 g, 3.09 mmol) and Pd(PPh₃)₄ (0.36 g, 0.31 mmol) were dissolved in ACN,(20 mL). The atmosphere was replaced with nitrogen, and (tributylstannyl)methanol (1.99 g, 6.19 mmol) was added dropwise. The mixture was heated to an external temperature of 90°C and reacted for 48 h. After completion, the reaction mixture was concentrated under reduced pressure. Ethyl acetate (20 mL) was added, and the mixture was stirred and filtered. The filter cake was concentrated under reduced pressure to afford 3-(4-(hydroxymethyl)-1-oxoisoindolin-2-yl)piperidine-2,6-dione (400 mg, 1.46 mmol). This compound (400 mg, 1.46 mmol) was placed in a 100 mL round-bottom flask, dissolved in DCM (10 mL) and TEA, (0.4 mL). MsCl (0.3 mL, 3.65 mmol) was added dropwise slowly at room temperature, and the mixture was stirred at room temperature for 2 h. After completion, DCM (50 mL) and water (50 mL) were added. The mixture was stirred vigorously, allowed to stand, and separated. The organic phase was dried over anhydrous sodium sulfate, concentrated under reduced pressure, and purified by column chromatography (DCM:MeOH = 1:0 - 10:1) to afford compound **64-2** as a white solid (180 mg, yield: 21%).

ESI-MS (*m*/*z*): 293.1 [M+H]⁺.

¹H NMR (500 MHz, DMSO-*d*₆) *δ* 11.13 (d, *J =* 10.5 Hz, 1H), 7.96 (d, *J =* 4.0 Hz, 2H), 5.69 (s, 1H), 5.16 (dd, *J =* 12.9, 5.4 Hz, 1H), 3.32 (s, 2H), 2.92 (d, *J =* 17.6 Hz, 1H), 2.66 - 2.53 (m, 2H), 2.29 (s, 1H), 2.11 - 2.04 (m, 1H), 1.24 (s, 1H).

### Step 2:

Compound 64-2 (105 mg, 0.36 mmol), 1-3 (190 mg, 0.28 mmol), K₂CO₃ (114.37 mg, 0.83 mmol), and TEA (0.2 mL, 1.66 mmol) were added to a mixture of acetonitrile (5 mL) and DMF (1 mL). The mixture was stirred at 50°C for 12 h. After completion, the reaction mixture was filtered to remove inorganic salts and purified by preparative HPLC to afford **Compound 64** as a pale yellow solid (26 mg, yield: 11.4%).

ESI-MS (m/z): 830.3 [M+H]⁺.

¹H NMR (500 MHz, DMSO-*d*₆) *δ* 10.96 (s, 1H), 10.47 (s, 1H), 9.34 (d, *J =* 7.0 Hz, 1H), 8.89 (d, *J =* 2.7 Hz, 1H), 8.66 (s, 1H), 8.32 (s, 1H), 7.62 (d, *J =* 7.3 Hz, 1H), 7.53 (d, *J =* 7.4 Hz, 1H), 7.47 (t, *J =* 7.5 Hz, 1H), 7.32 (dd, *J* = 6.9*,* 4.2 Hz, 1H), 6.71 (s, 1H), 5.12 (dd, *J =* 13.2, 5.1 Hz, 1H), 4.53 - 4.47 (m, 2H), 4.39 (d, *J =* 17.5 Hz, 1H), 3.55 (t, *J =* 9.0 Hz, 2H), 3.39 (t, *J =* 5.2 Hz, 2H), 3.25 (s, 1H), 2.97 (s, 2H), 2.92 (d, *J =* 10.5 Hz, 2H), 2.75 (s, 2H), 2.64 (d, *J =* 11.1 Hz, 2H), 2.45 (d, *J =* 4.5 Hz, 1H), 2.43 - 2.37 (m, 2H), 2.32 (d, *J =* 7.2 Hz, 2H), 2.05 - 2.00 (m, 1H), 1.94 (t, *J =* 11.9 Hz, 2H), 1.79 (s, 2H), 1.72 (d, *J =* 11.3 Hz, 4H), 1.63 (d, *J =* 12.4 Hz, 2H), 1.61 - 1.54 (m, 2H), 1.51 (s, 1H), 1.37 (d, *J =* 6.6 Hz, 2H), 1.30 (s, 1H), 1.25 (d, *J =* 11.3 Hz, 2H), 1.14 (d, *J* = 11.9 Hz, 2H).

### Example 65:

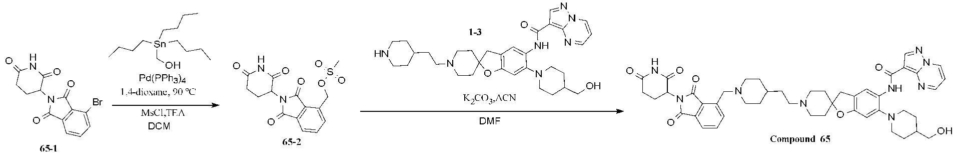

### Step 1:

Compound **65-1** (1.0 g, 3.09 mmol) and Pd(PPh₃)₄ (0.36 g, 0.31 mmol) were dissolved in 1,4-dioxane (20 mL). The atmosphere was replaced with nitrogen, and (tributylstannyl)methanol (1.99 g, 6.19 mmol) was added dropwise. The mixture was heated to an external temperature of 90°C and reacted for 48 h. After completion, the reaction mixture was concentrated under reduced pressure. Ethyl acetate (20 mL) was added, and the mixture was stirred and filtered. The filter cake was concentrated under reduced pressure to afford 2-(2,6-dioxopiperidin-3-yl)-4-(hydroxylmethyl)isoindoline-1,3-dione (400 mg, 1.46 mmol). This compound (400 mg, 1.46 mmol) was placed in a 100 mL round-bottom flask, dissolved in DCM (10 mL) and TEA (0.4 mL). MsCl (0.3 mL, 3.65 mmol) was added dropwise at room temperature, and the mixture was stirred at room temperature for 2 h. After completion, DCM (50 mL) and water (50 mL) were added. The mixture was stirred vigorously, allowed to stand, and separated. The organic phase was dried over anhydrous sodium sulfate, concentrated under reduced pressure, and purified by column chromatography (DCM:MeOH = 1:0- 10:1) to afford compound **65-2** as a white solid (180 mg, yield: 21%).

ESI-MS (*m*/*z*):384.1[M+18].

### Step 2:

Compound 65-2 (50 mg, 0.14 mmol), 1-3 (72.31 mg, 0.10 mmol), K₂CO₃ (43.53 mg, 0.31 mmol), and TEA (63 mg, 0.63 mmol) were added to a mixture of acetonitrile (5 mL) and DMF (1 mL) in a 50 mL round-bottom flask. The mixture was stirred at 50°C for 16 h. After completion, the mixture was filtered and purified by preparative HPLC to afford **Compound 65** as a pale yellow solid (20 mg, yield: 22%).

ESI-MS (m/z): 844.3 [M+H]⁺.

¹H NMR (500 MHz, DMSO-*d*₆) *δ* 11.10 (s, 1H), 10.49 (s, 1H), 9.35 (d, *J =* 7.0 Hz, 1H), 8.90 (d, *J =* 2.7 Hz, 1H), 8.67 (s, 1H), 8.37 (s, 1H), 7.88 (d, *J =* 29.9 Hz, 3H), 7.34 (dd, *J =* 6.9, 4.2 Hz, 1H), 6.72 (s, 1H), 5.13 (d, *J =* 7.3 Hz, 1H), 4.55 (d, *J =* 5.1 Hz, 1H), 3.94 (s, 1H), 3.46 (d, *J =* 20.4 Hz, 2H), 3.40 (t, *J =* 5.1 Hz, 2H), 3.13 (s, 2H), 3.08 (d, *J =* 7.0 Hz, 4H), 2.93 (d, *J=* 8.4 Hz, 2H), 2.87 (d, *J =* 4.8 Hz, 2H), 2.63 (s, 2H), 2.31 (s, 4H), 2.07 (s, 4H), 1.68 (dd, *J* = 38.1, 13.3 Hz, 6H), 1.53 (s, 2H), 1.19 (t, *J* = 7.3 Hz, 6H).

### Example 66:

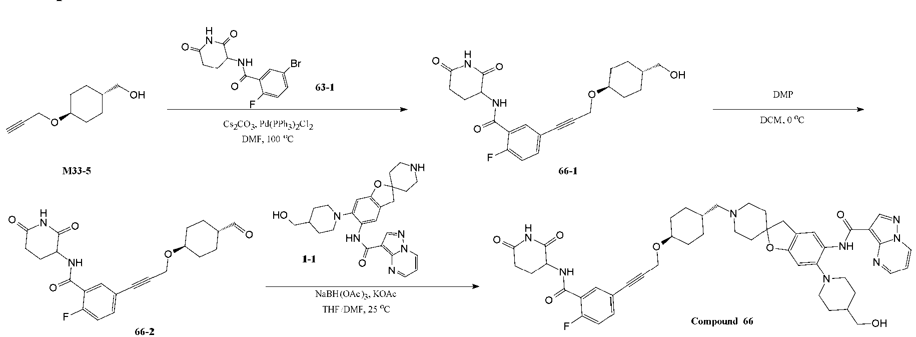

### Step 1:

Compound M33-5 (511 mg, 3.04 mmol, 2.0 eq) was placed in a 40 mL round-bottom flask. DMF (5 mL), 63-1 (500 mg, 1.52 mmol), cesium carbonate (Cs₂CO₃, 990 mg, 3.04 mmol), and bis(triphenylphosphine)palladium(II) chloride (Pd(PPh₃)₂Cl₂, 118 mg, 0.15 mmol, 0.1 eq) were added. The reaction mixture was stirred at 100°C under nitrogen protection for 2 h. After completion, the mixture was concentrated under reduced pressure. THF (10 mL) was added, stirred for 10 min, and filtered. The filtrate was concentrated under reduced pressure and purified by thin-layer chromatography (PE : THF = 1 : 2) to afford 66-1 as a colorless oil (220 mg, 0.53 mmol, yield: 34.8%).

ESI-MS (m/z): 417.3 [M+1]⁺.

### Step 2:

Compound 66-1 (220 mg, 0.53 mmol) was placed in a 100 mL round-bottom flask and dissolved in DCM (5 mL). Dess-Martin periodinane (DMP, 336 mg, 0.79 mmol, 1.5 eq) was added at 0°C. The reaction mixture was stirred at room temperature overnight. After completion, saturated aqueous sodium bicarbonate solution (10 mL) was added and stirred for 10 min. The mixture was extracted with DCM (15 mL × 2). The combined organic phases were dried over anhydrous sodium sulfate, concentrated under reduced pressure, and purified by TLC (PE : THF = 1 : 1) to afford 66-2 as a white solid (115 mg, 0.25 mmol, yield: 46.5%).

ESI-MS (m/z): 415.2 [M+1]⁺.

¹H NMR (400 MHz, CDCl₃) *δ* 9.66 (d, *J =* 0.86 Hz, 1H), 8.17 (dd, *J =* 7.34, 2.20 Hz, 1H), 8.09 (br s, 1H), 7.13 (dd, *J =* 11.49, 8.56 Hz, 1H), 4.75 - 4.85 (m, 1H), 4.42 (s, 2H), 3.49 - 3.57 (m, 1H), 2.70 - 2.94 (m, 3H), 2.00 - 2.26 (m, 6H), 1.33 - 1.43 (m, 4H).

### Step 3:

Compound 66-2 (40 mg, 0.10 mmol) was placed in a 40 mL round-bottom flask and dissolved in DMF (1 mL) and THF (1 mL). Compound 1-1 (89 mg, 0.19 mmol, 2.0 eq) was added, followed by potassium acetate (KOAc, 19 mg, 0.19 mmol, 2.0 eq) at 0°C. The mixture was stirred at room temperature for 0.5 h. Sodium triacetoxyborohydride (NaBH(OAc)₃, 24 mg, 0.12 mmol, 1.2 eq) was added at 0°C, and the reaction mixture was stirred at 0°C for 2 h. After completion, the mixture was concentrated under reduced pressure and purified by preparative HPLC to afford **Compound 66** as a yellow solid (28.03 mg, 0.03 mmol, yield: 30.5%).

ESI-MS *(m*/*z):* 861.3 [M+1]⁺.

¹H NMR (400 MHz, DMSO-*d*₆) *δ* 10.64 - 11.06 (m, 1H), 10.49 (s, 1H), 9.35 (dd, *J =* 7.00, 1.50 Hz, 1H), 8.87 - 8.93 (m, 1H), 8.73 (br d, *J =* 8.38 Hz, 1H), 8.66 - 8.70 (m, 1H), 8.33 (s, 1H), 7.56 - 7.79 (m, 2H), 7.29 - 7.41 (m, 2H), 6.73 (s, 1H), 4.73 - 4.81 (m, 1H), 4.54 - 4.63 (m, 1H), 4.38 - 4.44 (m, 2H), 2.87 - 3.05 (m, 5H), 2.75 - 2.86 (m, 2H), 2.64 (br t, *J =* 11.07 Hz, 3H), 2.37 (br s, 2H), 1.98 - 2.22 (m, 7H), 1.69 - 1.85 (m, 8H), 1.40 - 1.64 (m, 5H), 1.10 - 1.23 (m, 2H), 0.83 - 0.97 (m, 2H).

### Example 67:

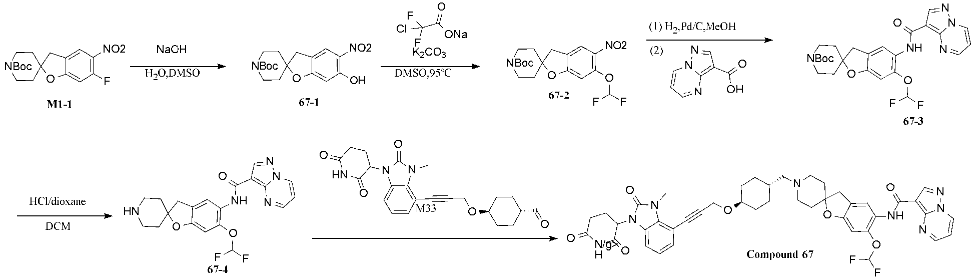

### Step 1:

Compound M1-1 (1000 mg, 2.84 mmol), NaOH (283.80 mg, 7.10 mmol), water (5 mL), and DMSO (10 mL) were added to a 50 mL round-bottom flask. The mixture was stirred at 70°C for 2 h. After completion, ethyl acetate (100 mL) and water (100 mL) were added. The mixture was extracted, and the combined organic phases were concentrated under reduced pressure. And purified by column chromatography (PE:EA = 1:0-1:1) to afford compound 67-1 as a yellow solid (700 mg, yield: 70.49%).

ESI-MS (m/z):295.1 [M-55]⁺.

¹H NMR (500 MHz, DMSO-*d*₆) δ 7.90 (s, 1H), 6.48 (s, 1H), 3.50 (dd, *J =* 11.8, 6.7 Hz, 2H), 3.37 (s, 2H), 3.02 (s, 2H), 1.82 - 1.72 (m, 4H), 1.44 (s, 1H), 1.41 (s, 9H).

### Step 2:

Compound 67-1 (650 mg, 1.86 mmol), sodium 2-chloro-2,2-difluoroacetate (850 mg, 5.58 mmol), K₂CO₃ (897 mg, 6.49 mmol), and DMSO (6.5 mL) were added to a 50 mL round-bottom flask. The mixture was stirred at 95°C for 2 h. After completion, water (65 mL) was added, and the mixture was extracted with ethyl acetate (65 mL × 2). The combined organic phases were concentrated under reduced pressure and purified by column chromatography (PE:EA = 1:0-1:1) to afford compound 67-2 as a yellow solid (650 mg, yield: 87.60%).

ESI-MS (m/z): No response;

¹H NMR (500 MHz, DMSO-*d*₆) *δ* 8.01 (s, 1H), 7.43 -7.14 (m, 1H), 6.90 (s, 1H), 3.57 - 3.53 (m, 2H), 3.36 (s, 2H), 3.12 (s, 2H), 1.84 - 1.76 (m, 4H), 1.42 (s, 9H).

### Step 3:

Compound 67-2 (650 mg, 1.62 mmol) was dissolved in methanol (10 mL). Palladium on carbon (Pd/C, 130 mg, 10% w/w, 0.162 mmol Pd) was added. The mixture was stirred at room temperature for 1 h. After completion, the mixture was filtered, and the filtrate was concentrated under reduced pressure to afford a black solid (601 mg, 1.62 mmol). This black solid (601 mg, 1.62 mmol), pyrazolo[1,5-a]pyrimidine-3-carboxylic acid (264 mg, 1.62 mmol), N-methylimidazole (465 mg, 5.67 mmol), TCFH (2-Chloro-1,3-dimethylimidazolinium hexafluorophosphate, 545 mg, 1.94 mmol), and acetonitrile (10 mL) were sequentially placed in a 100 mL round-bottom flask. The mixture was stirred at room temperature for 2 h. After completion, the mixture was concentrated under reduced pressure and purified by column chromatography (PE:EA = 1:0-1:1) to afford compound 67-3 as a yellow solid (650 mg, yield: 59.88%).

ESI-MS (m/z):516.2 [M+H]⁺.

¹H NMR (500 MHz, DMSO-*d*₆) *δ* 10.22 (s, 1H), 9.37 (dd, *J =* 7.0, 1.4 Hz, 1H), 8.85 (dd, *J =* 4.1*,* 1.4 Hz, 1H), 8.69 (s, 1H), 8.29 (s, 1H), 7.33 (dd, *J =* 6.9, 4.2 Hz, 1H), 7.43- 7.14 (t,J, 1H), 6.78 (s, 1H), 3.54 (dd, *=* 11.7, 6.5 Hz, 2H), 3.38 (s, 2H), 3.08 (s, 2H), 1.80 (d, *J =* 13.8 Hz, 2H), 1.76 - 1.70 (m, 2H), 1.42 (s, 9H).

### Step 4:

Compound 67-3 (500 mg, 0.97 mmol) was dissolved in DCM (10 mL). A solution of HCl in 1,4-dioxane (4 M, 2 mL) was added dropwise. The mixture was stirred at room temperature for 1 h. The mixture was concentrated under reduced pressure and used directly in the next step to afford compound 67-4 as a white solid (650 mg, yield: 124.1%).

ESI-MS (m/z):416.0 [M+H]⁺.

### Step 5:

Compound 67-4 (166.16 mg, 0.24 mmol), compound M33 (100 mg, 0.24 mmol), potassium acetate (KOAc, 70 mg, 0.72 mmol), sodium triacetoxyborohydride (NaBH(OAc)₃, 99 mg, 0.47 mmol), and DMF (1 mL) were added to a 50 mL round-bottom flask. The mixture was stirred at room temperature for 2 h. After completion, the mixture was concentrated under reduced pressure and purified by preparative HPLC to afford **Compound 67** as a yellow solid (10 mg, yield: 5.1%).

ESI-MS (m/z):823.3 [M+H]⁺.

¹H NMR (500 MHz, DMSO-*d*₆) *δ* 11.09 (s, 1H), 10.20 (s, 1H), 9.37 (d, *J =* 6.9 Hz, 1H), 8.84 (d, *J* = 2.5 Hz, 1H), 8.68 (s, 1H), 8.26 (s, 1H), 7.42-7.28 (m, 2H), 7.17 (d, *J =* 7.5 Hz, 1H), 7.12 (d, *J =* 7.4 Hz, 1H), 7.03 (t, *J =* 7.8 Hz, 1H), 6.76 (s, 1H), 5.39 (dd, *J =* 12.3, 5.0 Hz, 1H), 4.47 (s, 2H), 3.65 (s, 3H), 3.03 (s, 2H), 2.93 - 2.87 (m, 1H), 2.70 (d, *J =* 12.5 Hz, 1H), 2.64 (d, *J =* 17.3 Hz, 2H), 2.12 (d, *J =* 6.9 Hz, 2H), 2.05 (d, *J =* 17.6 Hz, 4H), 1.87 - 1.75 (m, 6H), 1.47 (s, 2H), 1.24 (s, 2H), 1.18 (d, *J =* 11.1 Hz, 2H), 0.90 (dd, *J =* 24.0, 11.6 Hz, 2H).

### Example 68:

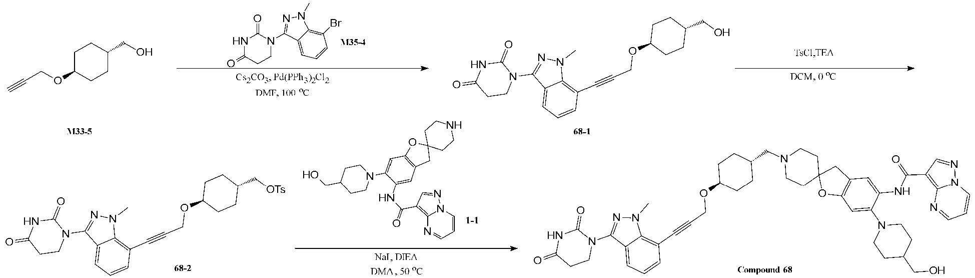

### Step 1:

M33-5 (520 mg, 3.09 mmol, 2.0 eq) was placed in a 40 mL single-neck flask and DMF (5.0 mL) was added. M35-4 (500 mg, 1.55 mmol, 1.0 eq) and Cs₂CO₃ (1.08 g, 3.09 mmol, 2.0 eq), along with dichlorobis(triphenylphosphine)palladium(II) (120 mg, 0.15 mmol, 0.10 eq), were introduced into the reaction mixture. The solution was stirred under nitrogen atmosphere at 100 °C for 2 hours. After completion, the reaction mixture was concentrated . THF (300 mL) was added and the resulting suspension was stirred for 1 hour, then filtered. The filtrate was concentrated under reduced pressure, and the residue was purified by column chromatography (petroleum ether/ethyl acetate = 2:1-1:2) to afford 68-1 (200 mg, 0.49 mmol, 31.5% yield) as a pale yellow solid.

ESI-MS *(m*/*z):* 411.1 [M+1]⁺.

¹H NMR (400 MHz, CDCl₃) *δ* 7.56 - 7.62 (m, 2H), 7.41 - 7.46 (m, 1H), 7.02 (dd, *J =* 8.11, 7.27 Hz, 1H), 4.43 (s, 2H), 4.28 (s, 3H), 3.99 - 4.06 (m, 2H), 3.38 - 3.43 (m, 2H), 2.82 (t, *J =* 6.74 Hz, 2H), 2.05 - 2.14 (m, 2H), 1.73 - 1.88 (m, 3H), 1.36 (s, 1H), 1.20 - 1.30 (m, 2H), 0.91 - 1.02 (m, 2H).

### Step 2:

Compound 68-1 (200 mg, 0.49 mmol, 1.0 eq) was dissolved in DCM (5.0 mL) in a 40 mL single-neck flask, and TEA (148 mg, 1.46 mmol, 3.0 eq) was added. The reaction mixture was cooled to 0 °C before TsCl (186 mg, 0.97 mmol, 2.0 eq) was added. The mixture was allowed to warm to room temperature and stirred overnight. Water (10.0 mL) was added, and the product was extracted twice with DCM (10.0 mL × 2). The combined organic phases were dried over anhydrous Na₂SO₄, concentrated under reduced pressure, and purified by thin-layer chromatography (petroleum ether/THF = 1:1) to afford 68-2 (150 mg, 0.19 mmol, 38.9% yield) as a grayish-white foam-like solid.

ESI-MS (m/z):565.2 [M+1]⁺.

### Step 3:

To a 40 mL single-neck flask containing 68-2 (40.0 mg, 0.07 mmol, 1.0 eq) was added DMA (2.0 mL), followed by 1-1 (32.7 mg, 0.07 mmol, 1.0 eq), NaI (31.8 mg, 0.21 mmol, 3.0 eq), and DIEA (54.9 mg, 0.43 mmol, 6.0 eq). The reaction mixture was stirred at 50 °C for 16 hours, concentrated under reduced pressure, and purified by preparative HPLC to afford **Compound 68** (32.9 mg, 0.04 mmol, 54.3% yield) as a yellow solid.

ESI-MS (m/z): 855.5 [M+1]⁺.

¹H NMR (400 MHz, DMSO-*d*₆) *δ* 10.59 (br s, 1H), 10.48 (br s, 1H), 9.34 (br d, *J =* 6.38 Hz, 1H), 8.88 (br s, 1H), 8.67 (s, 1H), 8.32 (br s, 1H), 7.69 (br d, *J =* 7.88 Hz, 1H), 7.52 (br d, *J =* 6.75 Hz, 1H), 7.32 (br s, 1H), 7.11 (br *t, J =* 7.32 Hz, 1H), 6.71 (br s, 1H), 4.56 (br s, 1H), 4.52 (br s, 2H), 4.28 (br s, 3H), 3.91 (br d, *J =* 5.50 Hz, 3H), 2.86 - 3.02 (m, 5H), 2.59 - 2.79 (m, 5H), 2.37 (br d, *J =* 1.63 Hz, 3H), 1.99 - 2.20 (m, 5H), 1.67 - 1.86 (m, 8H), 1.42 - 1.61 (m, 4H), 1.13 - 1.24 (m, 2H), 0.83 - 0.97 (m, 2H).

### Example 69:

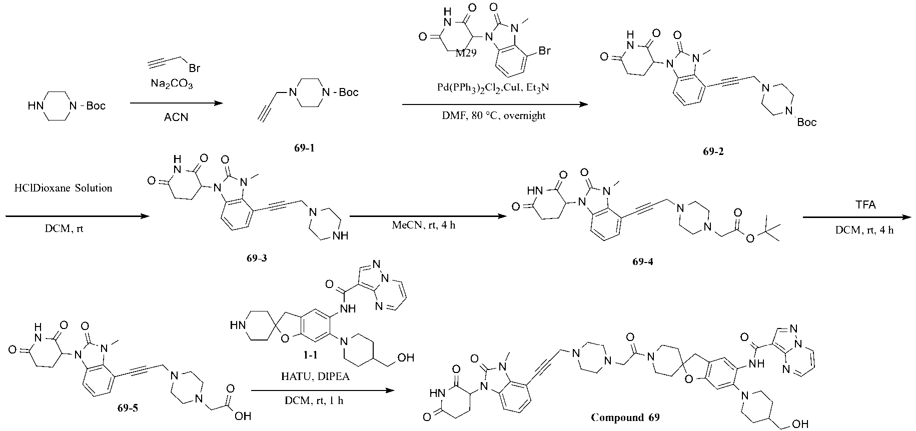

### Step 1:

1-tert-Butoxycarbonylpiperazine (1.00 g, 5.37 mmol) was placed in a 50 mL single-neck flask, and acetonitrile (10 mL) was added. Sodium carbonate (1.71 g, 16.11 mmol) was added and the mixture was stirred at room temperature for 20 minutes. 3-Bromopropyne (0.7 mL, 8.05 mmol) was then added, and the mixture was transferred to a 50 °C oil bath and stirred for 4 hours. The reaction was quenched with ethyl acetate (10 mL), washed twice with saturated NH₄Cl solution (10 mL × 2), dried over anhydrous Na₂SO₄, and purified by column chromatography (PE/EA = 95:5-75:25) to afford 69-1 (672 mg, 56.0% yield) as a pale yellow oil.

ESI-MS (m/z): 225.0 [M+H]⁺;

¹H NMR (500 MHz, DMSO-*d*₆) *δ* 3.34 - 3.31 (m, 4H), 3.28 (d, *J =* 2.2 Hz, 2H), 3.14 (s, 1H), 2.41 - 2.35 (m, 4H), 1.42 - 1.37 (m, 9H).

### Step 2:

M29 (452 mg, 1.34 mmol) was dissolved in DMF (4 mL) in a 50 mL single-neck flask. To this solution was added 69-1 (300 mg, 1.34 mmol), triethylamine (406 mg, 4.01 mmol), bis(triphenylphosphine)palladium(II) dichloride (104 mg, 0.13 mmol), and CuI (50 mg, 0.27 mmol). The flask was purged with nitrogen three times, and the reaction was stirred at 80 °C overnight. The mixture was diluted with DCM (15 mL), washed twice with saturated NH₄Cl solution (15 mL × 2), dried over Na₂SO₄, and purified by column chromatography (DCM/MeOH = 100:0 - 95:5) to afford 69-2 (176 mg, 25.9% yield) as a brown foamy solid.

ESI-MS (*m*/*z*): 482.4 [M+H]⁺.

¹H NMR (500 MHz, CDCl₃) *δ* 8.38 (s, 1H), 7.16 (d, *J =* 7.8 Hz, 1H), 6.98 (t, *J =* 7.9 Hz, 1H), 6.76 (d, *J =* 7.8 Hz, 1H), 5.19 (dd, *J =* 12.6, 5.3 Hz, 1H), 3.76 (s, 3H), 3.66 (s, 2H), 3.56 (s, 4H), 2.93 (*d, J =* 17.2 Hz, 1H), 2.86 - 2.63 (m, 6H), 2.26 - 2.17 (m, 1H), 1.46 (s, 9H).

### Step 3:

69-2 (65 mg, 0.13 mmol) was dissolved in DCM (3 mL) in a 50 mL flask. A solution of 4.0 M HCl in dioxane (0.7 mL) was slowly added dropwise at room temperature. The reaction was stirred for 1 hour, then concentrated under reduced pressure to afford 69-3 (72 mg, 142.1% yield) as a brown solid.

ESI-MS (m/z): 382.3 [M+H]⁺.

### Step 4:

69-3 (49 mg, 0.13 mmol), tert-butyl bromoacetate (38 mg, 0.20 mmol), and DIEA (168 mg, 1.30 mmol) were mixed in acetonitrile (2 mL) in a 50 mL flask and stirred at room temperature for 5 hours. The mixture was diluted with DCM (15 mL), washed with saturated NH₄Cl solution (15 mL), dried over Na₂SO₄, and purified by column chromatography (DCM/MeOH = 100:0 -95:5) to afford 69-4 (38 mg, 59.0% yield) as a brown solid.

ESI-MS (m/z): 496.5 [M+H]⁺.

¹H NMR (500 MHz, CDCl₃) *δ* 8.02 (s, 1H), 7.08 (t, *J =* 10.3 Hz, 1H), 6.94 - 6.84 (m, 1H), 6.66 (d, *J =* 7.8 Hz, 1H), 5.11 (dd, *J =* 12.5, 5.2 Hz, 1H), 3.70 (s, 3H), 3.49 (s, 2H), 3.06 (s, 2H), 2.87 (d, *J =* 17.4 Hz, 1H), 2.79 - 2.54 (m, 7H), 2.15 (dd, *J =* 13.7, 6.2 Hz, 1H), 1.39 (s, 9H), 1.22 (s, 2H).

### Step 5:

69-4 (35 mg, 0.07 mmol) was dissolved in DCM (2 mL), and TFA (196 µL, 2.55 mmol) was slowly added. The reaction was stirred at room temperature for 2 hours, then concentrated under reduced pressure to afford 69-5 (42 mg, 135.3% yield) as a black solid.

ESI-MS (m/z): 440.4 [M+H]⁺.

### Step 6:

69-5 (30 mg, 0.07 mmol), 1-1 (32 mg, 0.04 mmol), HATU (39 mg, 0.10 mmol), and DIEA (54 mg, 0.42 mmol) were placed in a 25 mL single-neck flask, and DCM (5 mL) was added. The mixture was stirred at 25 °C overnight. After dilution with DCM (10 mL), it was washed with saturated NH₄Cl solution (10 mL), dried over Na₂SO₄, and purified by column chromatography to afford **Compound 69** (29 mg, 46.9% yield, purity: 97.23%) as a brown solid.

ESI-MS (m/z): 884.6 [M+H]⁺.

¹H NMR (500 MHz, DMSO-*d*₆) *δ* 11.09 (s, 1H), 10.49 (s, 1H), 9.34 (d, *J =* 6.9 Hz, 1H), 8.90 (d, *J =* 2.7 Hz, 1H), 8.66 (s, 1H), 8.36 (s, 1H), 7.33 (dd, *J =* 6.9*,* 4.2 Hz, 1H), 7.15 (dd, *J=* 15.8, 7.7 Hz, 2H), 7.02 (t, *J =* 7.9 Hz, 1H), 6.73 (s, 1H), 5.40 (dd, *J =* 12.8, 5.2 Hz, 1H), 4.53 (s, 1H), 4.07 (s, 6H), 3.84 (s, 1H), 3.68 (s, 5H), 3.52 (s, 2H), 3.41 (s, 3H), 3.04 (s, 3H), 2.94 (d, *J =* 10.6 Hz, 3H), 2.63 (d, *J =* 11.4 Hz, 4H), 2.08 - 1.79 (m, 5H), 1.78 - 1.41 (m, 8H).

### Example 70:

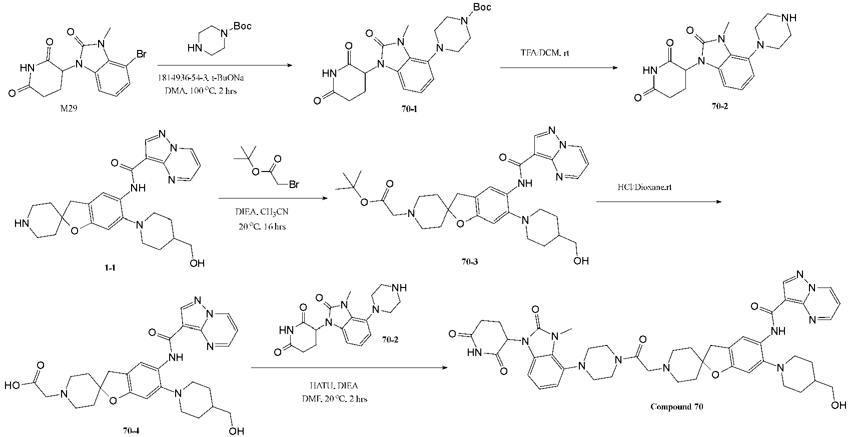

### Step 1:

M29 (1.00 g, 2.96 mmol, 1.0 eq) was placed in a 100 mL single-necked flask, and DMF (10 mL), N-Boc piperazine (0.83 g, 4.44 mmol, 1.5 eq), sodium tert-butoxide (0.85 g, 8.87 mmol, 3.0 eq) and palladium catalyst (CAS: 1814936-54-3) (29 mg, 0.03 mmol, 0.1 eq) were added to the reaction solution. The reaction solution was stirred at 100°C for 2 h under nitrogen protection. Water (100 mL) was added to the reaction solution, and the mixture was extracted with dichloromethane (80 mL × 2). The combined organic phases were back-washed once with anhydrous sodium chloride (50 mL). The organic phase was separated, dried over anhydrous sodium sulfate, and concentrated under reduced pressure. Column chromatography separation (petroleum ether: ethyl acetate = 2:1 - 0:1) was performed, and the obtained sample was stirred with ethyl acetate (10 mL) for 1 h, then filtered. The filter cake was collected to afford 70-1 (220 mg, 0.50 mmol, yield 16.8%) as a white solid.

ESI-MS (m/z): 444.2 [M+H]⁺;

¹H NMR (400 MHz, CDCl₃) *δ* 8.23 (s, 1H), 6.98 - 7.07 (m, 1H), 6.93 (d, *J =* 8.03 Hz, 1H), 6.63 (d, *J* = 7.78 Hz, 1H), 5.24 (br dd, *J =* 11.61, 4.83 Hz, 1H), 4.03 - 4.25 (m, 2H), 3.79 (s, 3H), 2.72 - 3.17 (m, 9H), 2.18 - 2.29 (m, 1H), 1.51 (s, 9H).

### Step 2:

70-1 (235 mg, 0.53 mmol) was placed in a 40 mL single-necked flask, and a mixed solution of TFA (1 mL) and DCM (9 mL) was added. The reaction solution was stirred at room temperature for 1 h. The reaction solution was concentrated under reduced pressure to afford 70-2 (180 mg, 0.52 mmol, yield 98.9%) as a brown oil.

ESI-MS (m/z): 344.1 [M+H]⁺.

### Step 3:

1-1 (500 mg, 1.08 mmol, 1.0 eq) was placed in a 100 mL single-necked flask, acetonitrile (5 mL) was added, followed by tert-butyl bromoacetate (316 mg, 1.62 mmol, 1.5 eq) and DIEA (1.39 g, 10.8 mmol, 10 eq). The reaction solution was stirred at room temperature overnight. The reaction solution was filtered, and the filter cake was washed once with acetonitrile (5 mL). The filter cake was collected to afford 70-3 (466 mg, 0.81 mmol, yield 74.8%) as a yellow solid.

ESI-MS (m/z):577.5 [M+H]⁺.

### Step 4:

70-3 (466 mg, 0.81 mmol) was placed in a 40 mL single-necked flask, and DCM (1 mL) and a dioxane solution of HCl (5 mL) were added. The reaction solution was stirred at room temperature for 2 h. The reaction solution was concentrated under reduced pressure to afford 70-4 (400 mg, 0.77 mmol, yield 95.1%) as a yellow solid.

ESI-MS (m/z):521.4 [M+H]⁺.

### Step 5:

70-4 (341 mg, 0.52 mmol, 1.0 eq) was placed in a 40 mL single-necked flask, DMF (4 mL) was added, followed by 70-2 (180 mg, 0.52 mmol, 1.0 eq) and DIEA (203 mg, 1.57 mmol, 3.0 eq). HATU (219 mg, 0.58 mmol, 1.1 eq) was added to the reaction solution, which was then stirred at room temperature for 1 h. After the reaction solution was filtered, the filtrate was separated and purified by HPLC to afford **Compound** 70 (173 mg, 0.19 mmol, yield 36.6%) as a yellow solid.

ESI-MS (m/z): 846.4 [M+H]⁺.

¹H NMR (400 MHz, DMSO-*d*₆) *δ* 11.10 (br s, 1H), 10.50 (s, 1H), 9.36 (dd, *J =* 6.94, 1.56 Hz, 1H), 8.90 (dd, *J =* 4.06, 1.56 Hz, 1H), 8.68 (s, 1H), 8.35 (s, 1H), 7.34 (dd, *J =* 6.94, 4.19 Hz, 1H), 6.97 - 7.04 (m, 1H), 6.90 - 6.97 (m, 2H), 6.74 (s, 1H), 5.37 (br dd, *J =* 12.51, 5.25 Hz, 1H), 4.56 (br s, 1H), 4.37 - 4.50 (m, 1H), 4.16 - 4.33 (m, 1H), 3.67 (s, 3H), 3.40 (br s, 3H), 3.06 - 3.21 (m, 3H), 3.01 (br s, 2H), 2.79 - 2.96 (m, 5H), 2.55 - 2.75 (m, 9H), 1.97 - 2.06 (m, 1H), 1.66 - 1.96 (m, 7H), 1.46 - 1.65 (m, 3H).

### Example 71:

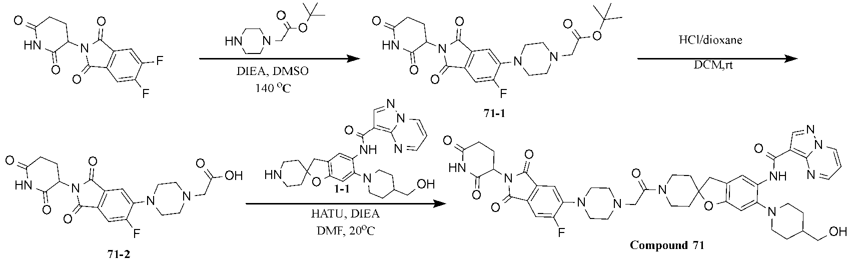

### Step 1:

2-(2,6-Dioxopiperidin-3-yl)-5,6-difluoroisoindoline-1,3-dione (200 mg, 0.68 mmol, 1.0 eq) was placed in a 40 mL single-necked flask, DMSO (2 mL) was added, followed by tert-butyl 2-(piperazin-1-yl)acetate (136 mg, 0.68 mmol, 1.0 eq) and DIEA (263 mg, 2.04 mmol, 3.0 eq). The reaction solution was stirred at 140°C for 1 h. Water (10 mL) was added to the reaction solution, and the mixture was filtered. The filter cake was washed twice with water (10 mL × 2). The filter cake was collected to afford 71-1 (200 mg, 0.40 mmol, yield 59.4%) as an earthy yellow solid.

ESI-MS (m/z): 475.2[M+H]⁺

¹H NMR (400 MHz, DMSO-*d*₆) *δ* 11.12 (s, 1H), 7.74 (d, *J =* 11.38 Hz, 1H), 7.47 (d, *J =* 7.38 Hz, 1H), 5.11 (dd, *J =* 12.82, 5.44 Hz, 1H), 3.22 - 3.29 (m, 4H), 3.20 (s, 2H), 2.84 - 2.95 (m, 1H), 2.66 - 2.72 (m, 4H), 2.55 (s, 2H), 1.98 - 2.12 (m, 1H), 1.43 (s, 9H).

### Step 2:

71-1 (160 mg, 0.34 mmol) was placed in a 40 mL single-necked flask, DCM (1 mL) was added, followed by a dioxane solution of HCl (4 mL, 16.00 mmol). The reaction solution was stirred at room temperature for 6 h. The reaction solution was concentrated under reduced pressure to afford 71-2 (160 mg, 0.38 mmol) as a gray solid.

ESI-MS (m/z): 419.2[M+H]⁺

### Step 3:

71-2 (70 mg, 0.17 mmol, 1.0 eq) was placed in a 40 mL single-necked flask, DMF (2 mL) was added, followed by DIEA (65 mg, 0.50 mmol, 3.0 eq) and 1-1 (77 mg, 0.17 mmol, 1.0 eq). Finally, HATU (70 mg, 0.18 mmol, 1.1 eq) was added. The reaction solution was stirred at room temperature overnight. After the reaction solution was concentrated under reduced pressure, it was separated and purified by HPLC to afford **Compound** 71 (53.45 mg, 0.06 mmol, yield 35.3%) as a yellow solid.

ESI-MS (m/z): 863.4[M+H]⁺

¹H NMR (400 MHz, DMSO-*d*₆) *δ* 11.11 (s, 1H), 10.50 (s, 1H), 9.37 (dd, *J =* 7.00, 1.50 Hz, 1H), 8.90 (dd, *J =* 4.19*,* 1.56 Hz, 1H), 8.68 (s, 1H), 8.37 (s, 1H), 7.74 (d, *J =* 11.38 Hz, 1H), 7.49 (d, *J =* 7.38 Hz, 1H), 7.34 (dd, *J =* 6.94, 4.19 Hz, 1H), 6.76 (s, 1H), 5.12 (dd, *J =* 12.88, 5.38 Hz, 1H), 4.56 (t, *J =* 5.25 Hz, 1H), 3.56 - 3.85 (m, 3H), 3.36 - 3.49 (m, 4H), 3.21 - 3.30 (m, 5H), 3.07 (s, 2H), 2.83 - 2.98 (m, 3H), 2.55 - 2.73 (m, 8H), 1.99 - 2.11 (m, 1H), 1.67 - 1.94 (m, 6H), 1.45 - 1.65 (m, 3H).

### Example 72:

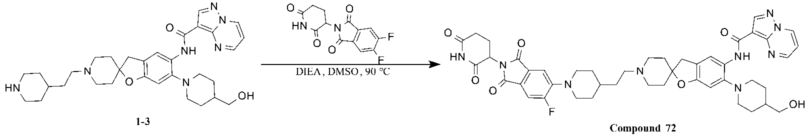

Compound 1-3 (70 mg, 0.12 mmol) was placed in a 40 mL flask. DMSO (2 mL), 2-(2,6-dioxopiperidin-3-yl)-5,6-difluoroisoindoline-1,3-dione (36 mg, 0.12 mmol, 1.0 eq), and DIEA (32 mg, 0.24 mmol, 2.0 eq) were added to the reaction mixture. The mixture was stirred at 90°C for 1 h. The reaction mixture was filtered, and the filtrate was purified by preparative HPLC to afford **Compound 72** (38 mg, 0.04 mmol, 36.4%) as a yellow solid.

ESI-MS (*m*/*z*): 848.5[M+H]⁺

¹H NMR (400 MHz, DMSO-*d*₆) *δ* 11.11 (s, 1H), 10.49 (s, 1H), 9.36 (dd, *J =* 7.03, 1.55 Hz, 1H), 8.90 (dd, *J =* 4.17, 1.55 Hz, 1H), 8.68 (s, 1H), 8.34 (s, 1H), 7.71 (d, *J =* 11.44 Hz, 1H), 7.45 (d, *J =* 7.63 Hz, 1H), 7.34 (dd, *J* = 6.97, 4.23 Hz, 1H), 6.73 (s, 1H), 5.11 (dd, *J =* 12.81, 5.42 Hz, 1H), 4.55 (t, *J =* 5.25 Hz, 1H), 3.60 (br d, *J =* 11.32 Hz, 2H), 3.40 (t, *J =* 5.25 Hz, 2H), 2.82 - 3.04 (m, 8H), 2.60 - 2.69 (m, 4H), 2.36 - 2.42 (m, 2H), 1.67 - 2.10 (m, 11H), 1.41 - 1.65 (m, 7H), 1.26 - 1.39 (m, 2H).

### Example 73:

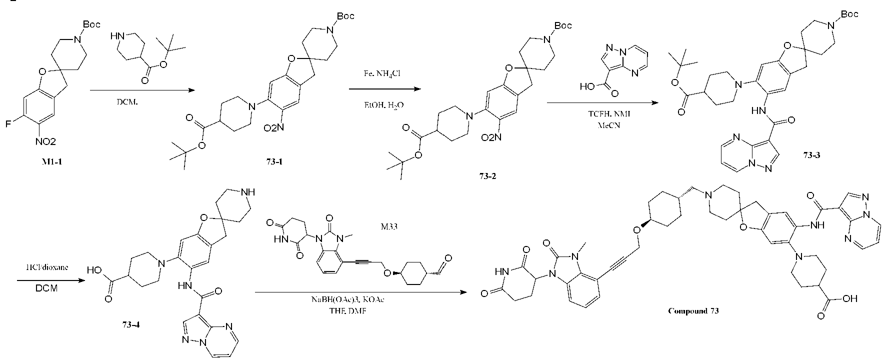

### Step 1:

tert-Butyl hexahydropyridine-4-carboxylate (208 mg, 1.12 mmol) was placed in a 40 mL flask. Dichloromethane (3 mL) was added, followed by M1-1 (330 mg, 0.94 mmol, 1.0 eq). The mixture was stirred at 25°C for 12 h. The reaction mixture was concentrated under reduced pressure and purified by TLC (petroleum ether/ethyl acetate = 3:1) to afford 73-1 (400 mg, 0.77 mmol, yield 82.5%) as a colorless oil.

ESI-MS (m/z): 518.2 [M+H]⁺

¹H NMR (400 MHz, DMSO-*d*₆) *δ* 7.90 (s, 1H), 6.68 (s, 1H), 3.54 - 3.66 (m, 2H), 3.13 - 3.24 (m, 2H), 3.07 (s, 2H), 2.85 (t, *J* = 10.6 Hz, 2H), 2.34 - 2.45 (m, 1H), 2.05 (s, 1H), 1.59 - 1.94 (m, 9H), 1.47 (s, 18H).

### Step 2:

73-1 (250 mg, 0.48 mmol) was placed in a 40 mL flask. Ethanol (2 mL) was added, followed by ammonium chloride (129 mg, 2.41 mmol, 5.0 eq) and water (0.40 mL). The mixture was warmed to 60°C, then iron powder (134 mg, 2.41 mmol, 5.0 eq) was added. The reaction mixture was stirred at 60°C for 4 h. The mixture was filtered, and the filtrate was concentrated under reduced pressure to afford 73-2 (180 mg, 0.37 mmol, yield: 76.4%) as a yellow solid.

ESI-MS (m/z): 488.2 [M+H]⁺

### Step 3:

Pyrazolo[1,5-*a*]pyrimidine-3-carboxylic acid (50 mg, 0.31 mmol) was placed in a 40 mL flask. Acetonitrile (2 mL) was added, followed by 73-2 (150 mg, 0.31 mmol, 1.0 eq), 1-methylimidazole (75 mg, 0.06 mmol, 3.0 eq), and *N,N,N',N'*-tetramethylchloroformamidinium hexafluorophosphate (8 mg, 0.02 mmol, 1.2 eq). The mixture was stirred at 25°C for 12 h. The reaction mixture was concentrated under reduced pressure and purified by TLC (petroleum ether/ethyl acetate = 3:1) to afford 73-3 (100 mg, 0.16 mmol, yield 51.4%) as a yellow solid.

ESI-MS (m/z): 633.4 [M+H]⁺

### Step 4:

73-3 (100 mg, 0.16 mmol) was placed in a 40 mL flask. Dichloromethane (1 mL) was added, followed by a dioxane solution of HCl (1 mL, 4 M). The mixture was stirred at 25°C for 2 h. The reaction mixture was concentrated under reduced pressure. The pH was adjusted to 8 with saturated sodium bicarbonate solution, precipitating a white solid. The solid was collected by filtration to afford 73-4 (50 mg, 0.10 mmol, yield: 66.4%) as a yellow solid.

ESI-MS (*m*/*z*): 477.3 [M+H]⁺

### Step 5:

73-4 (20 mg, 0.04 mmol) was placed in a 40 mL flask. Tetrahydrofuran (1 mL) and N,N-dimethylformamide (1 mL) were added, followed by M33 (17 mg, 0.04 mmol, 1 eq), sodium triacetoxyborohydride (10 mg, 0.05 mmol, 1.2 eq), and potassium acetate (8 mg, 0.08 mmol, 2 eq). The mixture was stirred at 25°C for 12 h. The reaction mixture was filtered, and the filtrate was purified by preparative HPLC to afford **Compound 73** (19 mg, 0.02 mmol, yield 52.6%) as a white solid.

ESI-MS (*m*/*z*): 884.5 [M+H]⁺

¹H NMR (400 MHz, DMSO-*d*₆) *δ* ppm 11.12 (s, 1H), 10.54 (s, 1H), 9.36 (d, *J* = 7.00 Hz, 1H), 8.89 (br d, *J* = 3.00 Hz, 1H), 8.68 (s, 1H), 8.37 (s, 1H), 8.17 (s, 1H), 7.34 (dd, *J* = 6.94, 4.19 Hz, 1H), 7.17 (d, *J* = 7.75 Hz, 1H), 7.09 - 7.13 (m, 1H), 6.99 - 7.06 (m, 1H), 6.72 (s, 1H), 5.40 (br dd, *J* = 12.51, 5.25 Hz, 1H), 4.46 (s, 2H), 3.64 (s, 3H), 3.42 - 3.46 (m, 2H), 2.98 (s, 2H), 2.84 - 2.92 (m, 3H), 2.59 - 2.77 (m, 5H), 2.32 - 2.43 (m, 3H), 2.13 (br d, *J=* 6.88 Hz, 2H), 1.98 - 2.08 (m, 5H), 1.73 - 1.92 (m, 8H), 1.47 (br s, 1H), 1.12 - 1.23 (m, 2H), 0.84 - 0.96 (m, 2H).

### Example 74:

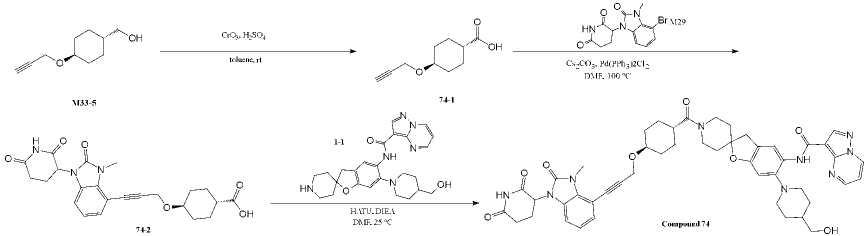

### Step 1:

Chromium trioxide (4.75 g, 47.55 mmol, 4.00 eq) was placed in a 500 mL flask. Concentrated sulfuric acid (10.49 g, 106.99 mmol, 9.00 eq) was added at 0°C. The mixture was cooled to 0°C, then a toluene (60 mL) solution of M33-5 (2.00 g, 11.89 mmol, 1.00 eq) was added. The mixture was then stirred at 25°C for 2 h. The reaction mixture was slowly poured into ice water. Ethyl acetate (100 mL × 3) was added for extraction. The organic phase was washed with saturated sodium chloride solution, dried over anhydrous sodium sulfate, and concentrated under reduced pressure to afford 74-1 (1.20 g, 6.59 mmol, yield: 55.4%) as a green solid.

### Step 2:

M29 (100 mg, 0.30 mmol, 1.00 eq) was placed in a 40 mL flask. DMF (1 mL) was added, followed by 74-1 (107 mg, 0.59 mmol, 2.00 eq), cesium carbonate (192 mg, 0.59 mmol, 2.00 eq), and bis(triphenylphosphine)palladium(II) dichloride (46 mg, 0.06 mmol, 0.2 eq). The mixture was stirred at 100°C under nitrogen protection for 2 h. The reaction mixture was filtered, and the filtrate was purified by preparative HPLC to afford 74-2 (40 mg, 0.09 mmol, yield: 30.8%) as a yellow solid.

ESI-MS (m/z): 440.1 [M+H]⁺

### Step 3:

74-2 (45 mg, 0.10 mmol, 1.0 eq) was placed in a 40 mL flask. DMF (2 mL) was added, followed by 1-1 (47 mg, 0.10 mmol, 1.1 eq), DIEA (39 mg, 0.31 mmol, 3.0 eq), and HATU (42 mg, 0.11 mmol, 1.1 eq). The mixture was stirred at 25°C for 2 h. The reaction mixture was filtered, and the filtrate was purified by preparative HPLC to afford **Compound 74** (20 mg, 0.02 mmol, yield: 22.8%) as a yellow solid.

ESI-MS (m/z):884.4 [M+H]⁺

¹H NMR (400 MHz, DMSO-*d*₆) *δ* 11.10 (br s, 1H), 10.50 (s, 1H), 9.36 (dd, *J* = 7.00, 1.38 Hz, 1H), 8.90 (dd, *J =* 4.00, 1.25 Hz, 1H), 8.68 (s, 1H), 8.36 (s, 1H), 7.34 (dd, *J =* 7.00, 4.25 Hz, 1H), 7.18 (d, *J =* 7.75 Hz, 1H), 7.13 (d, *J* = 7.75 Hz, 1H), 7.01 - 7.06 (m, 1H), 6.76 (s, 1H), 5.41 (dd, *J =* 12.63, 5.25 Hz, 1H), 4.55 (br t, *J* = 5.13 Hz, 1H), 4.48 (s, 2H), 3.75 - 3.85 (m, 1H), 3.66 (s, 4H), 3.46 - 3.58 (m, 2H), 3.37 - 3.43 (m, 3H), 3.01 - 3.08 (m, 2H), 2.83 - 2.97 (m, 3H), 2.60 - 2.77 (m, 5H), 2.00 - 2.13 (m, 3H), 1.51 - 1.92 (m, 11H), 1.37 - 1.49 (m, 2H), 1.21 - 1.34 (m, 2H).

### Example 75:

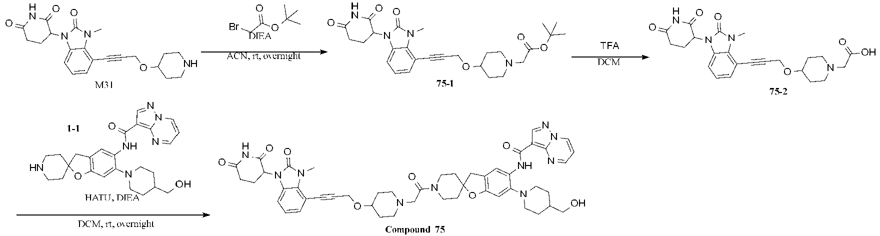

### Step 1:

M31 (158 mg, 0.4 mmol), tert-butyl bromoacetate (117 mg, 0.60 mmol), and DIEA (516 mg, 4.00 mmol) were mixed in acetonitrile (4 mL) in a 50 mL flask and stirred at room temperature overnight. Dichloromethane (15 mL) was added to the reaction mixture, followed by washing with saturated ammonium chloride solution (15 mL). The organic phase was dried over anhydrous sodium sulfate and purified by column chromatography (DCM/MeOH = 100:0-95:5) to afford 75-1 (127 mg, yield: 62.2%) as a brown solid.

ESI-MS (*m*/*z*): 511.6 [M+H]⁺.

¹H NMR (500 MHz, CDCl₃) *δ* 8.72 (s, 1H), 7.08 (d, *J =* 7.8 Hz, 1H), 6.89 (t, *J =* 7.9 Hz, 1H), 6.70 (d, *J =* 7.8 Hz, 1H), 5.14 (dd, *J* = 12.7, 5.3 Hz, 1H), 4.36 (s, 2H), 3.69 (s, 3H), 3.63 - 3.53 (m, 1H), 3.08 (s, 2H), 2.86 - 2.58 (m, 5H), 2.38 (t, *J =* 8.9 Hz, 2H), 2.17 - 2.08 (m, 1H), 1.91 (d, *J* = 10.1 Hz, 2H), 1.68 (dt, *J* = 16.7, 6.1 Hz, 2H), 1.39 (s, 9H).

### Step 2:

75-1 (127 mg, 0.25 mmol) was placed in a 50 mL flask and dissolved in DCM (3 mL). Trifluoroacetic acid (TFA, 1020 mg, 8.95 mmol) was added slowly. The mixture was stirred at room temperature for 1.5 h. The reaction mixture was concentrated under reduced pressure to afford 75-2 (148.3 mg, yield: 131.2%) as a brown viscous solid.

ESI-MS (m/z): 455.4 [M+H]+.

### Step 3:

75-2 (113 mg, 0.25 mmol), 1-1 (191 mg, 0.25 mmol), HATU (142 mg, 0.38 mmol), and DIEA (193 mg, 1.50 mmol) were placed in a 25 mL flask. DCM (7 mL) was added, and the mixture was stirred at 25°C overnight. Dichloromethane (20 mL) was added to the reaction mixture, followed by washing with saturated ammonium chloride solution (20 mL). The organic phase was dried over anhydrous sodium sulfate and purified by column chromatography (DCM/MeOH = 100:0 -93:7) to afford **Compound 75** (170 mg, yield: 75.6%) as a pale yellow solid.

ESI-MS *(m*/*z):* 899.7 [M+H]⁺.

¹H NMR (500 MHz, DMSO-*d₆*) *δ* 11.10 (s, 1H), 10.50 (s, 1H), 9.35 (d, *J =* 6.0 Hz, 1H), 8.90 (d, *J =* 2.7 Hz, 1H), 8.68 (s, 1H), 8.37 (s, 1H), 7.34 (dd, *J =* 6.8, 4.2 Hz, 1H), 7.19 (d, *J =* 7.7 Hz, 1H), 7.13 (d, *J* = 7.6 Hz, 1H), 7.05 (t, *J* = 7.9 Hz, 1H), 6.75 (s, 1H), 5.40 (dd, *J* = 12.8, 5.2 Hz, 1H), 4.59 - 4.44 (m, 3H), 3.83 (s, 1H), 3.66 (s, 3H), 3.61 - 3.35 (m, 5H), 3.26 (d, *J =* 14.8 Hz, 3H), 3.14 - 2.84 (m, 7H), 2.77 - 2.58 (m, 4H), 2.11 - 1.42 (m, 15H).

### Example 76:

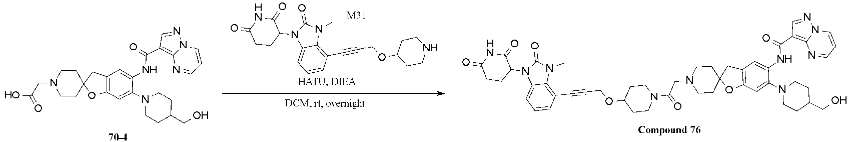

A mixture of 70-4 (203 mg, 0.39 mmol), M31 (154 mg, 0.39 mmol), HATU (222 mg, 0.58 mmol), and DIEA (302 mg, 2.34 mmol) was added to a 25 mL single-neck flask, followed by the addition of DCM (7 mL). The reaction mixture was stirred at 25°C overnight. After completion, DCM (15 mL) was added to the reaction mixture, which was then washed with saturated aqueous ammonium chloride solution (15 mL) and dried over anhydrous sodium sulfate. And purified by column chromatography ( DCM:MeOH = 100:0-95:5) to afford **Compound** 76 as a pale yellow solid (212 mg, yield: 60.5%).

ESI-MS *(m*/*z):* 899.7 [M+H]⁺.

¹H NMR (500 MHz, DMSO-*d₆*) *δ* 11.10 (s, 1H), 10.48 (s, 1H), 9.34 (d, *J =* 6.6 Hz, 1H), 8.89 (d, *J* = 2.7 Hz, 1H), 8.67 (s, 1H), 8.33 (s, 1H), 7.33 (dd, *J* = 6.7, 4.3 Hz, 1H), 7.18 (d, *J =* 7.7 Hz, 1H), 7.14 (d, *J* = 7.7 Hz, 1H), 7.04 (t, *J =* 7.8 Hz, 1H), 6.72 (s, 1H), 5.40 (dd, *J =* 12.7, 5.2 Hz, 1H), 4.57 - 4.49 (m, 3H), 3.92 - 3.78 (m, 3H), 3.66 (s, 3H), 3.43 - 3.39 (m, 2H), 3.18 - 3.03 (m, 2H), 3.02 - 2.84 (m, 5H), 2.77 - 2.44 (m, 11H), 2.08 - 2.00 (m, 1H), 1.98 - 1.92 (m, 1H), 1.84 (d, *J =* 13.1 Hz, 3H), 1.74 (dd, *J =* 19.6, 10.9 Hz, 4H), 1.64 - 1.46 (m, 4H).

### Example 77:

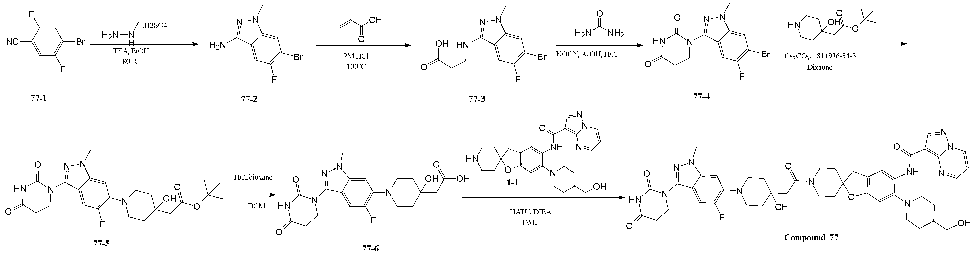

### Step 1:

4-Bromo-2,5-difluorobenzonitrile (10.0 g, 45.87 mmol, 1.0 eq) was placed in a 250 mL single-necked flask, followed by the addition of ethanol (EtOH, 70 mL), methylhydrazine sulfate (19.8 g, 137.61 mmol, 3.0 eq), and triethylamine (25.5 mL, 183.49 mmol, 4.0 eq). The reaction mixture was stirred at 80 °C for 6 hours. After completion, the reaction was concentrated under reduced pressure to remove ethanol. Water (150 mL) was added, resulting in the precipitation of a solid product. The solid was collected by filtration. The filter cake was further washed with DCM (20 mL) by stirring for 30 minutes, followed by filtration to afford compound 77-2 (4 g, 16.39 mmol, 35.7%) as a white solid.

ESI-MS (*m*/*z*): Br, 244.0, 246.0 [M+1, M+3]⁺.

¹H NMR (400 MHz, CDCl₃) *δ* 7.44 (d, *J* = 5.25 Hz, 1H), 7.24 (d, *J* = 8.13 Hz, 1H), 3.98 (s, 2H), 3.82 (s, 3H).

### Step 2:

Compound 77-2 (4 g, 16.39 mmol) was placed in a 100 mL single-necked flask, and aqueous HCl (30 mL, 2M) was added. Acrylic acid (1.54 g, 21.31 mmol, 1.3 eq) was then introduced into the reaction mixture. The solution was stirred at 100 °C overnight. After cooling, the reaction was neutralized to pH 7-8 with saturated sodium bicarbonate solution, followed by adjustment to pH 5-6 with acetic acid. A solid precipitate formed, which was filtered and collected to afford compound 77-3 (4.68 g, 14.80 mmol, 90.3%) as a gray solid.

ESI-MS (*m*/*z*): Br, 316.0, 318.0 [M+1, M+3]⁺.

¹H NMR (400 MHz, DMSO-*d*₆) *δ* 7.84 (d, *J* = 5.50 Hz, 1H), 7.67 (d, *J* = 9.01 Hz, 1H), 3.77 (s, 3H), 3.45 (br t, *J =* 6.75 Hz, 2H), 2.59 (br t, *J* = 6.82 Hz, 2H).

### Step 3:

To a 100 mL single-necked flask was added compound 77-3 (4.58 g, 14.49 mmol), acetic acid (45 mL), and potassium cyanate (2.35 g, 28.98 mmol, 2.0 eq). The mixture was stirred at 60 °C for 3 hours. Then, HCl (45 mL, 2N) was added, and the reaction was stirred at 60 °C overnight. After cooling, the solid product was filtered, washed with water twice, and collected to afford compound 77-4 (3 g, 8.79 mmol, 60.7%) as a white solid.

ESI-MS (*m*/*z*): Br, 340.9, 342.9 [M+1, M+3]⁺.

¹H NMR (400 MHz, DMSO-*d*₆) *δ* 10.61 (s, 1H), 8.17 (d, *J =* 5.63 Hz, 1H), 7.63 (d, *J =* 9.13 Hz, 1H), 4.01 (s, 3H), 3.93 (t, *J =* 6.69 Hz, 2H), 2.76 (t, *J =* 6.69 Hz, 2H).

### Step 4:

Compound 77-4 (300 mg, 0.88 mmol, 1.0 eq) was placed in a 40 mL single-necked flask, and 1,4-dioxane (5 mL), 2-(4-hydroxypiperidin-4-yl)acetic acid tert-butyl ester (378 mg, 1.76 mmol, 2.0 eq), cesium carbonate (859 mg, 2.64 mmol, 3.0 eq), and a palladium catalyst (CAS No.: 1814936-54-3, 85 mg, 0.09 mmol, 0.1 eq) were added. The reaction was stirred at 100 °C for 2 hours. Upon completion, the reaction was diluted with DCM (30 mL), filtered, and concentrated under reduced pressure. The crude product was purified by column chromatography (PE:EA = 5:1 -0:1) to afford compound 77-5 (300 mg, 0.63 mmol, 71.7%) as a light yellow solid.

ESI-MS (*m*/*z*): 476.2 [M+H]⁺.

¹H NMR (400 MHz, DMSO-*d*₆) *δ* ppm 10.53 (s, 1H), 7.33 (d, *J* = 12.87 Hz, 1H), 7.12 (d, *J* = 7.15 Hz, 1H), 4.58 (s, 1H), 3.94 (s, 3H), 3.90 (t, *J =* 6.68 Hz, 2H), 3.12 - 3.22 (m, 2H), 3.01 - 3.10 (m, 2H), 2.74 (t, *J =* 6.68 Hz, 2H), 2.40 (s, 2H), 1.81 - 1.91 (m, 2H), 1.70 - 1.79 (m, 2H), 1.43 (s, 9H).

### Step 5:

Compound 77-5 (270 mg, 0.57 mmol, 1.0 eq) was placed in a 50 mL single-necked flask. DCM (6 mL) and a dioxane solution of HCl (4 mL) were added, and the mixture was stirred at room temperature for 12 hours. The reaction was concentrated under reduced pressure to afford compound 77-6 (238 mg, 0.57 mmol) as a white solid.

ESI-MS (*m*/*z*): 420.1 [M+H]⁺.

### Step 6:

Compound 77-6 (238 mg, 0.57 mmol, 1.0 eq) was placed in a 50 mL single-necked flask, and DMF (6 mL), compound 1-1 (262.48 mg, 0.57 mmol, 1.0 eq), DIEA (0.3 mL, 1.70 mmol, 3.0 eq), and HATU (237 mg, 0.62 mmol, 3.0 eq) were added. The reaction was stirred at room temperature for 1 hour. After completion, the reaction was filtered and purified by preparative HPLC to afford **Compound** 77 (176 mg, 0.20 mmol, 36.0%) as a yellow solid.

ESI-MS (*m*/*z*): 864.6 [M+H]⁺

¹H NMR (400 MHz, DMSO-*d₆) δ* 10.47 - 10.54 (m, 2H), 9.36 (d, *J =* 7.00 Hz, 1H), 8.90 (d, *J =* 2.88 Hz, 1H), 8.68 (s, 1H), 8.36 (s, 1H), 7.30 - 7.38 (m, 2H), 7.13 (d, J *=* 7.00 Hz, 1H), 6.76 (s, 1H), 5.01 (s, 1H), 4.55 (t, J = 5.07 Hz, 1H), 3.95 (s, 3H), 3.89 (t, J = 6.63 Hz, 3H), 3.71 - 3.79 (m, 1H), 3.55 - 3.65 (m, 1H), 3.38 - 3.48 (m, 3H), 3.18 (m, 2H), 3.03 - 3.12 (m, 4H), 2.93 (m, 2H), 2.74 (t, *J =* 6.63 Hz, 2H), 2.59 - 2.68 (m, 4H), 1.78 - 1.90 **(m, 5H),** 1.68 - 1.77 (m, 5H), 1.46 - 1.65 (m, 3H).

### Example 78:

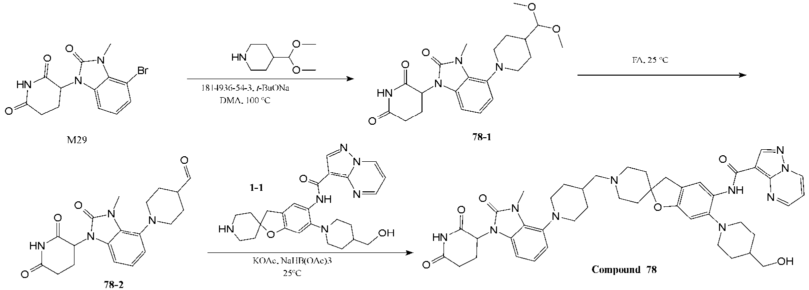

### Step 1:

M29 (500 mg, 1.48 mmol, 1.0 eq) was placed in a 100 mL single-necked flask, and DMA (5 mL), 4-(dimethoxymethyl)piperidine (941 mg, 5.91 mmol, 4.0 eq), sodium tert-butoxide (426 mg, 4.44 mmol, 3.0 eq), and palladium catalyst (CAS No.: 1814936-54-3) (143 mg, 0.15 mmol, 0.1 eq) were added to the reaction mixture. The reaction solution was stirred at 100°C for 2 hours. The reaction mixture was concentrated under reduced pressure and purified by column chromatography (petroleum ether : ethyl acetate = 20:1 -1:1). The obtained product was stirred with ethyl acetate (10 mL) for 1 hour, then filtered. The filter cake was collected to afford 78-1 (100 mg, 0.24 mmol, yield 16.2%) as a white solid.

ESI-MS (*m*/*z*): 417.2 [M+H]⁺.

¹H NMR (400 MHz, DMSO-*d*₆) *δ* 11.12 (s, 1H), 6.94 - 7.04 (m, 1H), 6.86 - 6.93 (m, 2H), 5.37 (br dd, *J* = 12.41, 5.32 Hz, 1H), 4.15 (br d, *J*=5.75 Hz, 1H), 3.63 (s, 3H), 3.31 (s, 6H), 3.13 (br d, *J =* 11.49 Hz, 2H), 2.86 - 2.95 (m, 1H), 2.61 - 2.77 (m, 4H), 1.97 - 2.06 (m, 1H), 1.67 - 1.81 (m, 3H), 1.41 - 1.57 (m, 2H).

### Step 2:

78-1 (100 mg, 0.24 mmol, 1.0 eq) was placed in a 10 mL single-necked flask, and formic acid (2 mL) was added. The system was stirred at 25°C for 1 hour. The reaction mixture was concentrated under reduced pressure to afford 78-2 (80 mg, 0.22 mmol, yield 89.9%) as a yellow oil.

ESI-MS (*m*/*z*): 371.2 [M+H]⁺.

### Step 3:

78-2 (70 mg, 0.19 mmol, 1.0 eq) was placed in a 10 mL single-necked flask, and DMA (2 mL) was added, followed by 1-1 (87 mg, 0.19 mmol, 1.0 eq), potassium acetate (47 mg, 0.23 mmol, 3.0 eq), and sodium cyanoborohydride (55 mg, 0.57 mmol, 1.2 eq). The mixture was stirred at 25°C for 12 hours. The reaction mixture was concentrated under reduced pressure and purified by HPLC to afford **Compound 78** (53 mg, 0.07 mmol, yield 34.5%) as a yellow solid.

ESI-MS (*m*/*z*): 817.5 [M+H]⁺.

¹H NMR (400 MHz, DMSO-*d₆*) *δ* 10.49 (s, 1H), 9.36 (dd, *J =* 7.00, 1.38 Hz, 1H), 8.90 (dd, *J =* 4.06, 1.31 Hz, 1H), 8.68 (s, 1H), 8.34 (s, 1H), 7.34 (dd, *J* = 6.94, 4.19 Hz, 1H), 6.95 - 7.01 (m, 1H), 6.88 (dd, *J* = 12.07, 8.07 Hz, 2H), 6.74 (s, 1H), 5.36 (dd, *J* = 12.63, 5.38 Hz, 1H), 4.57 (t, *J* = 5.25 Hz, 1H), 3.64 (s, 3H), 3.39 - 3.42 (m, 3H), 3.12 (m, 2H), 3.00 (s, 2H), 2.93 (m, 3H), 2.60 - 2.76 (m, 8H), 2.44 (m, 2H), 2.26 (m, 2H), 1.97 - 2.04 (m, 1H), 1.68 - 1.88 (m, 9H), 1.51 - 1.63 (m, 3H), 1.27 - 1.41 (m, 2H).

### Example 79:

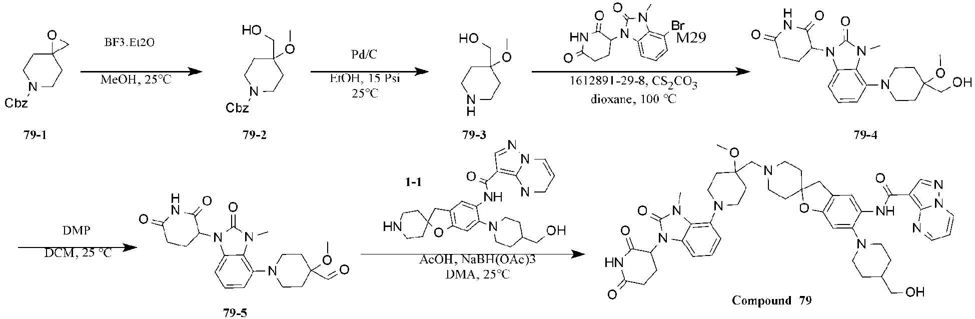

### Step 1:

Compound **79-1** (6.00 g, 24.2 mmol, 1.0 eq) was placed in a 100 mL single-necked flask. Methanol (60 mL) and boron trifluoride etherate (2.06 g, 0.6 eq) were added. The reaction mixture was stirred at 25°C for 2 h. The reaction solution was concentrated under reduced pressure and purified by column chromatography (petroleum ether:ethyl acetate = 20:1 - 5:1) to afford 79-2 (3.40 g, 12.1 mmol, yield: 50.2%) as a yellow oil.

ESI-MS (*m*/*z*): 280.3 [M+H]⁺.

¹H NMR (400 MHz, CDCl₃) *δ* 7.30 - 7.42 (m, 5H), 5.14 (s, 2H), 3.87 (d, *J =* 12.96 Hz, 2H), 3.53 (s, 2H), 3.17 - 3.28 (m, 5H), 1.83 (d, *J =* 13.57 Hz, 2H), 1.37 - 1.51 (m, 2H).

### Step 2:

10% wet palladium on carbon (500 mg, 4.70 mmol, 1.4 eq) was placed in a 100 mL single-necked flask. Ethanol (20 mL) was added, followed by 79-2 (900 mg, 3.22 mmol, 1.0 eq). The mixture was purged with H₂ three times and then stirred under H₂ (15 Psi) at 25°C for 12 h. The reaction mixture was filtered, and the filtrate was concentrated under reduced pressure to afford **79-3** (500 mg, 3.44 mmol, yield: 96.9%) as a yellow solid.

ESI-MS (m/z): 146.2 [M+H]⁺.

¹H NMR (400 MHz, CDCl₃) *δ* 3.69 (q, *J =* 7.00 Hz, 1H), 3.50 (s, 2H), 3.16 - 3.28 (s, 3H), 2.72 - 2.99 (m, 4H), 1.76 (d, *J* = 13.63 Hz, 2H), 1.45 (ddd, *J =* 13.70, 9.94, 4.25 Hz, 2H).

### Step 3:

Compound M29 (200 mg, 0.59 mmol, 1.0 eq) was placed in a 100 mL single-necked flask. Dioxane (2 mL) was added, followed by 79-3 (128 mg, 0.89 mmol, 1.5 eq) and cesium carbonate (578 mg, 1.77 mmol, 3.0 eq). Catalyst (CAS number: 1612891-29-8) (49 mg, 0.06 mmol, 0.1 eq) was added. The reaction mixture was stirred at 100 °C under a nitrogen atmosphere for 2 h. The reaction solution was concentrated under reduced pressure. Water (10 mL) was added, and the mixture was extracted with ethyl acetate (10 mL × 3). The combined organic phases were washed with saturated brine, dried over anhydrous sodium sulfate, and concentrated under reduced pressure. And Purified by thin-layer chromatography (dichloromethane:methanol = 15:1) to afford 79-4 (50 mg, 0.12 mmol, yield: 21.0%) as a yellow solid.

ESI-MS (*m*/*z*): 403.4 [M+H]⁺.

¹H NMR (400 MHz, CDCl₃) *δ* 9.69 (s, 1H), 8.28 (dd, *J* = 7.70, 1.22 Hz, 1H), 7.90 - 7.98 (m, 1H), 7.73 (t, *J =* 7.27 Hz, 1H), 5.22 (dd, *J* = 12.29, 5.20 Hz, 1H), 3.76 (s, 3H), 3.39 (s, 2H), 3.02 - 3.11 (s, 3H), 2.70 - 2.94 (m, 3H), 2.18-2.27 (m, 1H), 1.85 - 2.05 (m, 4H), 1.43 - 1.48 (m, 2H), 1.22 - 1.28 (m, 2H).

### Step 4:

Compound 79-4 (20 mg, 0.05 mmol, 1.0 eq) was placed in a 40 mL single-necked flask. DCM,(1 mL) and DMP,(31 mg, 0.07 mmol, 1.5 eq) were added. The reaction mixture was stirred at 25°C for 2 h. The reaction was quenched by adding saturated sodium bicarbonate solution. The mixture was extracted with ethyl acetate (10 mL × 3). The combined organic phases were washed with saturated brine, dried over anhydrous sodium sulfate, and concentrated under reduced pressure to afford the crude product 79-5 (20 mg, 0.05 mmol), which was used directly in the next step.

ESI-MS (*m*/*z*): 401.4 [M+H]⁺.

### Step 5:

Compound 79-5 (25 mg, 0.06 mmol, 1.0 eq) was placed in a 40 mL single-necked flask. DMA (1 mL) was added, followed by the addition of 1-1 (28 mg, 0.06 mmol, 1.0 eq), acetic acid (37 mg, 0.01 mmol, 0.1 eq), and sodium triacetoxyborohydride (13 mg, 0.06 mmol, 1.0 eq). The reaction mixture was stirred at room temperature for 2 h. The reaction solution was filtered and then purified by HPLC to afford **Compound 79** (4.93 mg, 0.01 mmol, 9.3% yield) as a yellow solid.

ESI-MS (*m*/*z*): 847.9 [M+H]⁺.

¹H NMR (400 MHz, DMSO-*d₆*) *δ* 11.09 (s, 1H), 10.49 (s, 1H), 9.36 (dd, *J =* 7.07, 1.56 Hz, 1H), 8.90 (dd, *J =* 4.19, 1.56 Hz, 1H), 8.68 (s, 1H), 8.34 (s, 1H), 7.29 - 7.39 (m, 1H), 6.91 - 7.01 (m, 2H), 6.87 (d, *J =* 8.00 Hz, 1H), 6.73 (s, 1H), 5.36 (dd, *J* = 12.69, 5.32 Hz, 1H), 4.55 (t, *J* = 5.32 Hz, 1H), 3.65 (s, 3H), 3.40 (t, *J* = 5.13 Hz, 3H), 3.19 (s, 3H), 3.00 (s, 2H), 2.85 - 2.96 (m, 7H), 2.61 - 2.75 (m, 8H), 1.91 - 2.03 (m, 3H), 1.69 - 1.85 (m, 8H), 1.47 - 1.65 (m, 4H).

### Example 80:

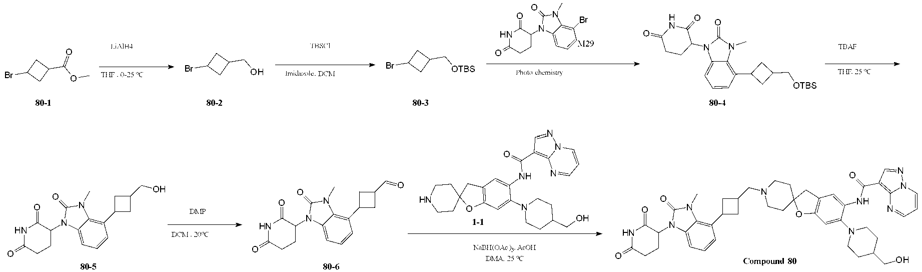

### Step 1:

Compound 80-1 (3.00 g, 15.5 mmol, 1.0 eq) was placed in a 100 mL three-necked flask and dissolved in THF (30 mL). At 0 °C, a THF solution of lithium aluminum hydride (6.50 mL, 16.3 mmol, 2.5 M, 1.05 eq) was added dropwise. After the addition, the reaction mixture was stirred at room temperature overnight. The reaction was cooled to 0 °C, and 0.62 mL of H₂O, 0.62 mL of 15% NaOH solution, and 1.24 mL of H₂O were added sequentially. Additional THF (30 mL) was added, and the mixture was stirred for 10 min. The reaction was filtered, and the filtrate was concentrated under reduced pressure to afford 80-2 (2.58 g, 15.63 mmol) as a colorless liquid.

¹H NMR (400 MHz, CDCl₃) *δ* 4.57 (quin, *J =* 7.05 Hz, 1H), 3.67 (d, *J* = 6.43 Hz, 2H), 2.71 - 2.83 (m, 1H), 2.50 - 2.65 (m, 4H).

### Step 2:

To a 100 mL single-necked flask, 80-2 (2.50 g, 15.1 mmol, 1.0 eq) was dissolved in dichloromethane (25 mL), and imidazole (2.06 g, 30.3 mmol, 2.0 eq) was added. TBSCl (3.42 g, 22.7 mmol, 1.5 eq) was then added to the reaction mixture, which was stirred at room temperature overnight. After completion, water (80 mL) and dichloromethane (100 mL) were added. The organic layer was separated, washed with brine (80 mL), dried over anhydrous sodium sulfate, and concentrated under reduced pressure. And purified by column chromatography (petroleum ether: ethyl acetate = 10:1 - 1:1) to afford 80-3 (4.00 g, 14.3 mmol, 94.5%) as a colorless oil.

¹H NMR (400 MHz, CDCl₃) *δ* 4.57 (qd, *J =* 7.16 Hz, 1H), 3.60 (d, *J* = 5.25 Hz, 2H), 2.66 - 2.78 (m, 1H), 2.52 - 2.62 (m, 4H), 0.91 (s, 9H), 0.06 (s, 6H).

### Step 3:

M29 (1.00 g, 2.96 mmol, 1.0 eq) was dissolved in DME (25 mL), and 80-3 (1.24 g, 4.44 mmol, 1.5 eq), TTMSS (1.10 g, 4.44 mmol, 1.5 eq), and sodium carbonate (0.63 g, 5.91 mmol, 2.0 eq) were added. The flask was purged with nitrogen for 10 min. NiCl₂·dtbbpy (0.06 g, 0.15 mmol, 0.05 eq) and [Ir(df(CF₃)ppy)₂(dtbbpy)]PF₆ (CAS: 870987-63-6) (0.03 g, 0.03 mmol, 0.01 eq) were added under nitrogen atmosphere. The system was purged again for 10 min. The flow rate was calibrated to S1 = 0.17 mL/min, and the reaction was carried out at 50 °C for 60 min. After flushing the tubing, the light source (λ = 395 nm, 24 W) and pump were activated. After 60 min, the reaction mixture was collected. Water (100 mL) was added, and the product was extracted with ethyl acetate (100 mL × 2). The combined organic layers were washed with brine (80 mL), dried over anhydrous sodium sulfate, and concentrated under reduced pressure. And Purified by column chromatography (petroleum ether: ethyl acetate = 5:1 to 1:2) to afford 80-4 (560 mg, 1.22 mmol, 41.4%) as a yellow solid.

ESI-MS (*m*/*z*): 458.2 [M+H]⁺.

¹H NMR (400 MHz, CDCl₃) *δ* 8.02 (br s, 1H), 7.15 - 7.24 (m, 1H), 7.08 (q, *J =* 7.96 Hz, 1H), 6.65 - 6.70 (m, 1H), 5.18 - 5.24 (m, 1H), 3.74 - 3.76 (m, 2H), 3.64 - 3.72 (m, 3H), 3.61 (d, *J =* 5.13 Hz, 1H), 2.92 - 3.02 (m, 2H), 2.73 - 2.88 (m, 2H), 2.12 - 2.38 (m, 5H), 0.93 (d, *J =* 8.38 Hz, 9H), 0.09 (d, *J =* 12.01 Hz, 6H).

### Step 4:

Compound 80-4 (560 mg, 1.22 mmol, 1.0 eq) was placed in a 40 mL single-necked flask, and THF (5 mL) was added. TBAF in THF (2.40 mL, 2.45 mmol, 1 M, 2.0 eq) was added, and the mixture was stirred at room temperature overnight. Water (30 mL) was added, and the product was extracted with ethyl acetate (50 mL × 2). The combined organic layers were dried over anhydrous sodium sulfate and concentrated under reduced pressure. The residue was purified by column chromatography (petroleum ether: ethyl acetate = 2:1 to 0:1). The resulting oil was treated with ethyl acetate (10 mL), stirred for 30 min, and filtered to afford 80-5 (110 mg, 0.32 mmol, 26.2%) as a white solid.

ESI-MS (*m*/*z*): 344.1 [M+H]⁺.

¹H NMR (400 MHz, CDCl₃) *δ* 8.10 (br s, 1H), 7.12 - 7.25 (m, 1H), 7.08 (q, *J =* 8.13 Hz, 1H), 6.69 (dd, *J* = 7.44, 5.44 Hz, 1H), 5.22 (dd, *J =* 12.01, 5.13 Hz, 1H), 4.09 - 4.19 (m, 1H), 3.82 (d, *J* = 6.75 Hz, 1H), 3.62 - 3.72 (m, 4H), 2.92 - 2.99 (m, 1H), 2.70 - 2.89 (m, 2H), 2.59 (m, 1H), 2.40 - 2.50 (m, 2H), 2.19 - 2.32 (m, 2H), 2.06 - 2.15 (m, 1H).

### Step 5:

To a 40 mL single-necked flask, 80-5 (80 mg, 0.23 mmol, 1.0 eq) was dissolved in DCM (3 mL), and Dess-Martin periodinane (DMP) (148 mg, 0.35 mmol, 1.5 eq) was added. The mixture was stirred at room temperature for 5 h. Saturated sodium bicarbonate solution (10 mL) was added, and the product was extracted with dichloromethane (10 mL × 2). The combined organic layers were dried over anhydrous sodium sulfate and concentrated under reduced pressure to afford 80-6 (50 mg, 0.15 mmol, 62.9%) as a light yellow solid.

ESI-MS (*m*/*z*): 342.1 [M+H]⁺.

¹H NMR (400 MHz, CDCl₃) *δ* 9.75 - 10.10 (m, 1H), 8.10 (br s, 1H), 7.11 - 7.25 (m, 2H), 6.75 (d, *J* = 7.70 Hz, 1H), 5.20 - 5.30 (m, 1H), 4.17 - 4.21 (m, 1H), 3.66 - 3.77 (m, 3H), 3.26 - 3.36 (m, 1H), 2.95 - 3.04 (m, 1H), 2.74 - 2.92 (m, 3H), 2.45 - 2.66 (m, 3H), 2.24 - 2.30 (m, 1H).

### Step 6:

Compound 80-6 (50 mg, 0.15 mmol, 1.0 eq) was placed in a 40 mL single-necked flask, and DMA (2 mL) was added. Compound 1-1 (68 mg, 0.15 mmol, 1.0 eq), acetic acid (AcOH) (18 mg, 0.29 mmol, 2.0 eq), and sodium triacetoxyborohydride (37 mg, 0.18 mmol, 1.2 eq) were added. The mixture was stirred at room temperature for 2 h and then filtered. The reaction mixture was purified by preparative HPLC to yield **Compound 80** (22.29 mg, 0.03 mmol, 19.3%) as a light yellow solid.

ESI-MS (*m*/*z*): 788.5 [M+H]⁺.

¹H NMR (400 MHz, DMSO-*d*₆) *δ* 11.09 (s, 1H), 10.49 (s, 1H), 9.36 (dd, *J =* 7.00, 1.50 Hz, 1H), 8.90 (dd, *J =* 4.25, 1.50 Hz, 1H), 8.68 (s, 1H), 8.34 (s, 1H), 7.34 (dd, *J =* 7.07, 4.19 Hz, 1H), 6.97 - 7.22 (m, 3H), 6.74 (s, 1H), 5.33 - 5.40 (m, 1H), 4.55 (t, *J =* 5.44 Hz, 1H), 3.93 - 4.21 (m, 1H), 3.51 - 3.62 (m, 3H), 3.40 (br t, *J* = 5.00 Hz, 2H), 2.84 - 3.03 (m, 5H), 2.56 - 2.74 (m, 6H), 2.32 - 2.46 (m, 6H), 2.12 - 2.23 (m, 1H), 1.69 - 2.05 (m, 9H), 1.48 - 1.65 (m, 3H).

### Example 81:

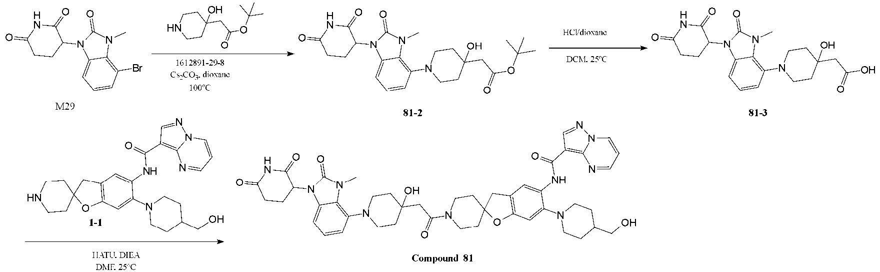

### Step 1:

M29 (300 mg, 0.89 mmol, 1.0 eq) was placed in a 40 mL single-necked flask. After adding 1,4-dioxane (3 mL), tert-butyl 2-(4-hydroxypiperidin-4-yl)acetate (287 mg, 1.33 mmol, 1.5 eq) and cesium carbonate (869 mg, 2.67 mmol, 3.0 eq) were added, followed by the palladium catalyst (CAS No.: 1612891-29-8) (74 mg, 0.09 mmol, 0.1 eq). The reaction mixture was stirred at 100°C for 2 h. The reaction mixture was concentrated under reduced pressure, water (30 mL) was added, and the mixture was extracted with ethyl acetate (30 mL × 3). The organic phase was washed with saturated sodium chloride solution and then purified by thin-layer chromatography (dichloromethane:methanol = 15:1) to afford 81-2 (120 mg, 0.25 mmol, yield: 28.6%) as a yellow solid.

ESI-MS (*m*/*z*): 473.5 [M+H]⁺.

¹H NMR (400 MHz, CDCl₃) *δ* 8.21 (s, 1H), 7.01 (br d, *J =* 4.82 Hz, 2H), 6.53 - 6.62 (m, 1H), 5.21 (dd, *J* = 12.37, 5.20 Hz, 1H), 3.77 (s, 3H), 3.16 - 3.28 (m, 2H), 2.89 - 2.99 (m, 3H), 2.67 - 2.87 (m, 3H), 2.46 (s, 2H), 2.17 - 2.27 (m, 1H), 1.73 - 1.91 (m, 4H), 1.50 (s, 9H).

### Step 2:

81-2 (110 mg, 0.23 mmol, 1.0 eq) was placed in a 40 mL single-necked flask. After adding DCM (1 mL), a dioxane hydrochloride solution (1 mL) was added, and the reaction was stirred at 25°C for 2 h. The reaction mixture was concentrated under reduced pressure to afford 81-3 (90 mg, 0.22 mmol, yield: 92.8%) as a yellow solid.

ESI-MS (*m*/*z*): 417.4 [M+H]⁺.

### Step 3:

1-1 (99 mg, 0.22 mmol, 1.0 eq) was placed in a 40 mL single-necked flask. After adding 81-3 (90 mg, 0.22 mmol, 1.0 eq), DIEA (83 mg, 0.65 mmol, 3.0 eq) was added, followed by HATU (90 mg, 0.24 mmol, 1.1 eq). The reaction was stirred at 25°C for 2 h. The reaction mixture was purified by HPLC to afford **Compound 81** (89.26 mg, 0.10 mmol, yield: 48.0%) as a yellow solid.

ESI-MS (*m*/*z*): 861.5 [M+H]⁺.

¹H NMR (400 MHz, DMSO-*d₆*) *δ* 11.08 (s, 1H), 10.50 (s, 1H), 9.36 (dd, *J =* 7.03, 1.53 Hz, 1H), 8.90 (dd, *J* = 4.16, 1.47 Hz, 1H), 8.68 (s, 1H), 8.36 (s, 1H), 7.33 (dd, *J =* 6.97, 4.16 Hz, 1H), 6.90 - 7.01 (m, 2H), 6.86 (d, *J* = 7.95 Hz, 1H), 6.76 (s, 1H), 5.35 (dd, *J* = 12.59, 5.26 Hz, 1H), 4.96 (br s, 1H), 4.56 (t, *J =* 4.83 Hz, 1H), 3.70 - 3.93 (m, 2H), 3.55 - 3.67 (m, 4H), 3.37 - 3.49 (m, 3H), 3.00 - 3.11 (m, 4H), 2.82 - 2.97 (m, 5H), 2.58 - 2.76 (m, 6H), 1.95 - 2.04 (m, 1H), 1.68 - 1.90 (m, 10H), 1.46 - 1.65 (m, 3H).

### Example 82:

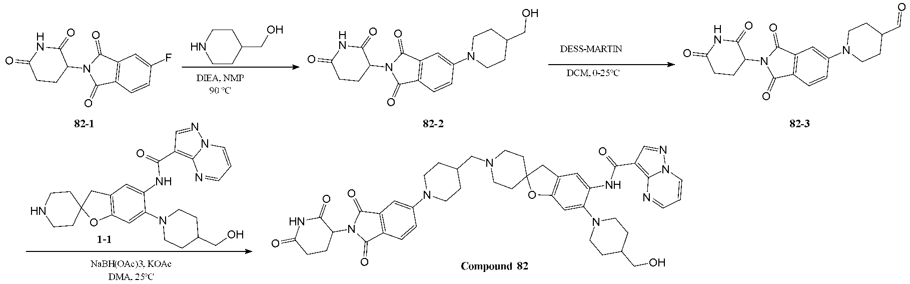

### Step 1:

Compound 82-1 (2.00 g, 7.24 mmol, 1.0 eq) was placed in a 100 mL single-necked flask, and NMP (40 mL) was added, followed by piperidin-4-ylmethanol (830 mg, 7.24 mmol, 1.0 eq) and DIEA (2.81 g, 21.72 mmol, 3.0 eq). The mixture was stirred at 90°C for 12 h. After completion, H₂O (50 mL) was added to the reaction solution, and the mixture was extracted with ethyl acetate (80 mL × 2). The combined organic phases were dried over anhydrous sodium sulfate, concentrated under reduced pressure, and purified by column chromatography (petroleum ether:ethyl acetate = 0:1) to afford 82-2 (1.60 g, 4.31 mmol, yield: 59.5%) as a yellow oil.

ESI-MS (m/z): 372.1 [M+H]⁺.

¹H NMR (400 MHz, CDCl₃) *δ* 8.11 (br s, 1H), 7.62 - 7.71 (m, 1H), 7.29 (d, *J=* 2.38 Hz, 1H), 7.06 (dd, *J* = 8.58, 2.38 Hz, 1H), 4.94 (dd, *J=* 12.28, 5.25 Hz, 1H), 3.99 (d, *J=* 12.99 Hz, 2H), 3.51 - 3.58 (m, 2H), 3.36 - 3.42 (m, 2H), 3.00 (m, 2H), 2.34 - 2.41 (m, 2H), 2.08 - 2.17 (m, 1H), 1.88 (m, 2H), 1.31 - 1.43 (m, 2H).

### Step 2:

Compound 82-2 (500 mg, 1.35 mmol, 1.0 eq) was placed in a 100 mL single-necked flask, and DCM (5 mL) was added. The mixture was cooled to 0°C, and DMP (599 mg, 1.41 mmol, 1.05 eq) was added. The reaction was stirred at 25°C for 2 h. Saturated sodium bicarbonate solution was added to quench the reaction and adjust the pH to 7-8, followed by extraction with dichloromethane (10 mL × 3). The organic phase was washed with saturated sodium chloride solution, dried over anhydrous sodium sulfate, concentrated under reduced pressure, and purified by column chromatography (petroleum ether:ethyl acetate = 1:1) to afford 82-3 (140 mg, 0.38 mmol, yield: 28.2%) as a white solid.

ESI-MS (*m*/*z*): 370.1 [M+H]⁺.

¹H NMR (400 MHz, DMSO-*d*₆) *δ* 11.14 (s, 1H), 9.68 (s, 1H), 7.72 (d, *J =* 8.50 Hz, 1H), 7.40 (s, 1H), 7.32 (dd, *J* = 8.57, 1.94 Hz, 1H), 5.13 (dd, *J* = 12.88, 5.25 Hz, 1H), 4.00 (br d, *J* = 13.26 Hz, 2H), 3.18 - 3.29 (m, 2H), 2.87 - 3.03 (m, 1H), 2.58 - 2.80 (m, 4H), 1.95 - 2.03 (m, 2H), 1.57 - 1.69 (m, 2H).

### Step 3:

Compound 82-3 (100 mg, 0.27 mmol, 1.0 eq) was placed in a 40 mL single-necked flask, and DMA (2 mL) and compound 1-1 (124 mg, 0.27 mmol, 1.0 eq) were added. Potassium acetate (79 mg, 0.81 mmol, 3.0 eq) and sodium triacetoxyborohydride (57 mg, 0.27 mmol, 1.0 eq) were then added to the reaction mixture, which was stirred at 25°C for 1 h. The reaction solution was separated and purified by HPLC to afford **Compound 82** (83.64 mg, 0.10 mmol, yield: 37.9%) as a yellow solid.

ESI-MS (*m*/*z*): 816.4 [M+H]⁺.

¹H NMR (400 MHz, DMSO-*d₆*) *δ* 11.11 (s, 1H), 10.51 (s, 1H), 9.37 (dd, *J* = 6.97, 1.47 Hz, 1H), 8.91 (dd, *J =* 4.03, 1.34 Hz, 1H), 8.69 (s, 1H), 8.35 (s, 1H), 7.67 (d, *J =* 8.56 Hz, 1H), 7.30 - 7.38 (m, 2H), 7.20 - 7.28 (m, 1H), 6.75 (s, 1H), 5.08 (dd, *J* = 12.96, 5.26 Hz, 1H), 4.60 (t, *J* = 5.13 Hz, 1H), 4.07 (br d, *J* = 12.71 Hz, 2H), 3.41 - 3.43 (m, 3H), 2.91 - 3.03 (m, 6H), 2.55 - 2.72 (m, 5H), 2.34 - 2.48 (m, 2H), 2.20 (br d, *J* = 6.97 Hz, 2H), 1.98 - 2.08 (m, 1H), 1.71 - 1.89 (m, 9H), 1.50 - 1.66 (m, 3H), 1.08 - 1.24 (m, 2H).

### Example 83:

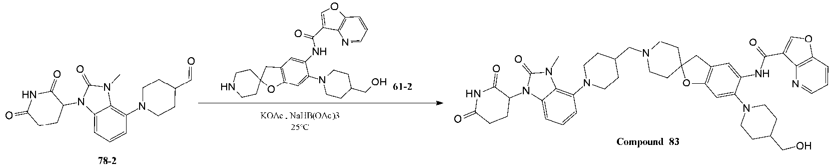

78-2 (53 mg, 0.14 mmol) was placed in a 40 mL single-necked flask. DMA (2 mL), 61-2 (66 mg, 0.14 mmol, 1.0 eq), potassium acetate (28 mg, 0.29 mmol, 2.0 eq), and sodium triacetoxyborohydride (36 mg, 0.17 mmol, 1.2 eq) were added. The reaction mixture was stirred at room temperature for 2 h. The reaction mixture was filtered, and the filtrate was collected and purified by HPLC to afford **Compound 83** (34.8 mg, 0.04 mmol, 29.8% yield) as a white solid.

ESI-MS (*m*/*z*): 817.5 [M+H]⁺.

1H NMR (400 MHz, DMSO-*d*₆) *δ* 11.09 (br s, 1H), 10.93 (s, 1H), 8.97 (s, 1H), 8.75 (br d, *J* = 4.41 Hz, 1H), 8.33 (s, 1H), 8.25 (br d, *J* = 8.58 Hz, 1H), 7.57 (dd, *J* = 8.34, 4.77 Hz, 1H), 6.94 - 7.01 (m, 1H), 6.85 - 6.92 (m, 2H), 6.75 (s, 1H), 5.36 (dd, *J =* 12.40, 5.01 Hz, 1H), 4.54 (t, *J =* 5.13 Hz, 1H), 3.63 (s, 3H), 3.38 (t, *J* = 5.13 Hz, 2H), 3.11 (m, 2H), 2.84 - 3.04 (m, 5H), 2.56 - 2.76 (m, 7H), 2.42 (m, 2H), 2.25 (m, 2H), 1.96 - 2.05 (m, 1H), 1.67 - 1.91 (m, 9H), 1.45 - 1.67 (m, 4H), 1.32 (m, 2H).

### Example 84 and Example 85:

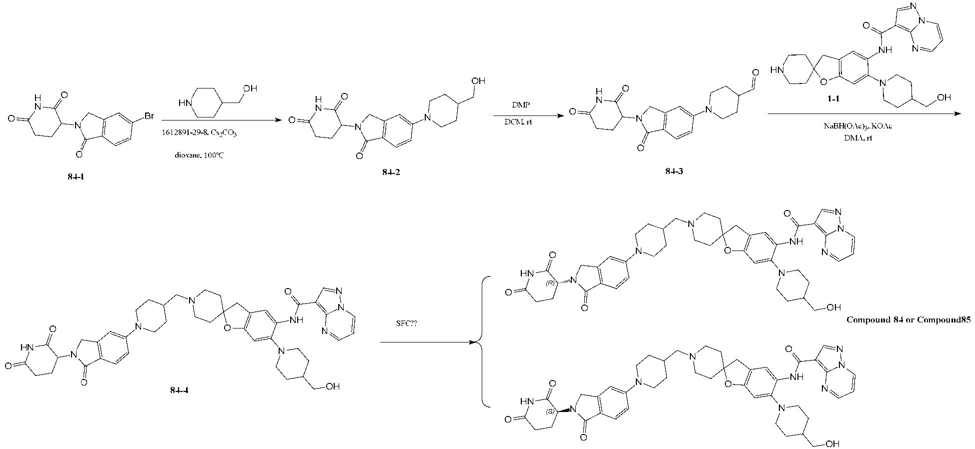

### Step 1:

84-1 (200 mg, 0.62 mmol) was placed in a 40 mL single-necked flask. Dioxane (4 mL), (piperidin-4-yl)methanol (107 mg, 0.93 mmol, 1.5 eq), cesium carbonate (403 mg, 1.24 mmol, 2.0 eq), and catalyst (CAS number: 1612891-29-8) (60 mg, 0.06 mmol, 0.1 eq) were added to the reaction mixture. The reaction mixture was stirred at 100°C for 2 h. Dichloromethane (20 mL) was added to the reaction mixture, which was then filtered. The filtrate was concentrated under reduced pressure and purified by column chromatography (DCM:MeOH = 15:1 -10:1) to afford 84-2 (120 mg, 0.34 mmol, 54.2% yield) as a white solid.

ESI-MS (*m*/*z*): 358.1 [M+H]⁺.

¹H NMR (400 MHz, DMSO-*d₆*) *δ* 10.95 (s, 1H), 7.50 (br d, *J* = 8.50 Hz, 1H), 6.98 - 7.20 (m, 2H), 5.05 (dd, *J =* 13.32, 5.07 Hz, 1H), 4.49 (t, *J* = 5.25 Hz, 1H), 4.27 - 4.37 (m, 1H), 4.16 - 4.25 (m, 1H), 3.90 (br d, *J =* 12.76 Hz, 2H), 3.28 (br t, *J* = 5.69 Hz, 2H), 2.77 - 2.97 (m, 3H), 2.37 (br dd, *J* = 13.01, 4.38 Hz, 1H), 1.92 - 2.02 (m, 1H), 1.58 - 1.80 (m, 4H), 1.15 - 1.30 (m, 2H).

### Step 2:

84-2 (100 mg, 0.28 mmol, 1.0 eq) was placed in a 40 mL single-necked flask. DCM (3 mL) and DMP (125 mg, 0.29 mmol, 0.1 eq) were added to the reaction mixture. The reaction mixture was stirred at room temperature for 1 h. Saturated aqueous sodium bicarbonate solution (10 mL) was added to the reaction mixture, followed by extraction with dichloromethane (10 mL × 2). The combined organic phases were dried over anhydrous sodium sulfate and concentrated under reduced pressure. Purification by preparative TLC(DCM:MeOH = 10:1) afforded 84-3 (80 mg, 0.23 mmol, 80.5% yield) as a yellow oil.

ESI-MS (*m*/*z*): 356.1 [M+H]⁺.

### Step 3:

84-3 (80 mg, 0.23 mmol, 1.0 eq) was placed in a 40 mL single-necked flask. DMA (2 mL), 1-1 (104 mg, 0.23 mmol, 1.0 eq), potassium acetate (44 mg, 0.45 mmol, 2.0 eq), and sodium triacetoxyborohydride (47 mg, 0.23 mmol, 1.0 eq) were added to the reaction mixture. The reaction mixture was stirred at room temperature for 2 h. The reaction mixture was filtered, and the filtrate was purified by preparative HPLC to afford 84-4 (40 mg, 0.05 mmol, 22.2% yield) as a yellow solid. This was used directly for SFC separation.

### Step 4:

84-4 (40 mg, 0.05 mmol) was subjected to supercritical fluid chromatography (SFC) separation to afford **Compound 84** and **Compound 85.**

**Compound 84** (15.15 mg, 0.02 mmol, 37.6% yield) was obtained as a yellow solid.

ESI-MS (*m*/*z*): 802.5 [M+H]⁺.

¹H NMR (400 MHz, DMSO-*d₆*) *δ* 10.96 (s, 1H), 10.50 (s, 1H), 9.36 (d, *J =* 7.05 Hz, 1H), 8.90 (d, *J =* 2.60 Hz, 1H), 8.68 (s, 1H), 8.35 (s, 1H), 7.51 (d, *J =* 8.04 Hz, 1H), 7.34 (dd, *J =* 6.80, 4.45 Hz, 1H), 7.02 - 7.11 (m, 2H), 6.74 (s, 1H), 5.05 (dd, *J =* 13.42, 4.64 Hz, 1H), 4.58 (t, *J =* 5.44 Hz, 1H), 4.16 - 4.37 (m, 2H), 3.89 (d, *J =* 11.75 Hz, 2H), 3.41 (m, 2H), 2.79 - 3.03 (m, 7H), 2.55 - 2.70 (m, 5H), 2.32 - 2.45 (m, 3H), 2.21 (m, 2H), 1.93 - 2.02 (m, 1H), 1.68 - 1.90 (m, 9H), 1.48 - 1.66 (m, 3H), 1.14 - 1.27 (m, 2H).

**Compound 85** (15.98 mg, 0.02 mmol, 39.6% yield) was obtained as a yellow solid.

ESI-MS (*m*/*z*): 802.5 [M+H]⁺.

¹H NMR (400 MHz, DMSO-*d₆*) *δ* 10.96 (s, 1H), 10.50 (s, 1H), 9.37 (d, *J =* 6.31 Hz, 1H), 8.91 (d, *J* = 3.22 Hz, 1H), 8.69 (s, 1H), 8.35 (s, 1H), 7.51 (d, J *=* 8.41 Hz, 1H), 7.35 (dd, *J =* 6.99, 4.14 Hz, 1H), 6.99 - 7.13 (m, 2H), 6.74 (s, 1H), 5.05 (dd, *J =* 13.36, 4.95 Hz, 1H), 4.57 (t, *J =* 5.32 Hz, 1H), 4.16 - 4.39 (m, 2H), 3.83 - 3.96 (m, 2H), 3.39 - 3.43 (m, 2H), 2.79 - 3.05 (m, 7H), 2.54 - 2.71 (m, 5H), 2.33 - 2.46 (m, 3H), 2.16 - 2.26 (m, 2H), 1.94 - 2.02 (m, 1H), 1.69 - 1.89 (m, 9H), 1.48 - 1.65 (m, 3H), 1.14 - 1.27 (m, 2H).
The following examples were synthesized using a method similar to Example 34:

| **Example** | **Chemical Structure and Structural Characterization Data** |
|---|---|
| **86** | 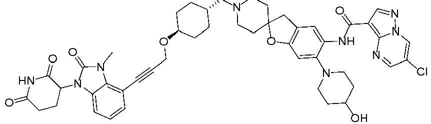 |
| | ESI-MS (*m*/*z*): 890.4 [M+H]⁺. |
| | ¹H NMR (400 MHz, DMSO-*d₆*) *δ* 11.14 (br s, 1H), 10.40 (s, 1H), 9.83 (d, *J* = 2.01 Hz, 1H), 8.91 (d, *J* = 2.13 Hz, 1H), 8.71 (s, 1H), 8.33 (s, 1H), 7.18 (br d, *J* = 8.03 Hz, 1H), 7.09 - 7.15 (m, 1H), 7.00 - 7.07 (m, 1H), 6.72 (s, 1H), 5.41 (dd, *J* = 12.61, 5.46 Hz, 1H), 4.79 (br d, *J =* 4.39 Hz, 1H), 4.47 (s, 2H), 3.65 (s, 4H), 3.38 - 3.52 (m, 2H), 2.83 - 3.02 (m, 5H), 2.63 - 2.77 (m, 4H), 2.29 - 2.46 (m, 3H), 1.99 - 2.17 (m, 5H), 1.69 - 1.94 (m, 10H), 1.41 - 1.54 (m, 1H), 1.12 - 1.25 (m, 2H), 0.83 - 0.97 (m, 2H). |
| **87** | 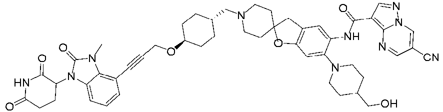 |
| | ESI-MS (*m*/*z*): 895.5 [M+H]⁺. |
| | ¹H NMR (400 MHz, DMSO-*d*₆) *δ* 11.13 (s, 1H), 10.42 (s, 1H), 10.22 (d, *J =* 1.88 Hz, 1H), 9.17 (d, *J* = 1.88 Hz, 1H), 8.89 (s, 1H), 8.33 (s, 1H), 8.19 (s, 1H), 7.18 (d, *J =* 7.75 Hz, 1H), 7.10 - 7.15 (m, 1H), 7.00 - 7.08 (m, 1H), 6.75 (s, 1H), 5.41 (dd, *J =* 12.57, 5.44 Hz, 1H), 4.65 (br d, *J =* 1.50 Hz, 1H), 4.48 (s, 2H), 3.65 (s, 3H), 3.44 (br s, 3H), 2.99 (s, 2H), 2.85 - 2.95 (m, 3H), 2.61 - 2.75 (m, 5H), 2.32 - 2.44 (m, 3H), 2.13 (br d, *J =* 6.88 Hz, 2H), 2.00 - 2.09 (m, 3H), 1.63 - 1.87 (m, 10H), 1.49 (br d, *J* = 6.13 Hz, 2H), 1.12 - 1.26 (m, 2H), 0.90 (q, *J =* 11.84 Hz, 2H). |
| **88** | 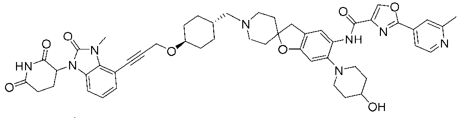 |
| | ESI-MS (*m*/*z*): 897.7[M+H]⁺. |
| | ¹H NMR (500 MHz, DMSO-*d₆*) *δ* 11.10 (s, 1H), 10.11 (s, 1H), 8.97 (s, 1H), 8.67 (d, *J =* 5.0 Hz, 1H), 8.22 (s, 1H), 7.88 (s, 1H), 7.78 (d, *J =* 4.6 Hz, 1H), 7.18 (d, *J =* 7.8 Hz, 1H), 7.12 (d, *J =* 7.7 Hz, 1H), 7.04 (t, *J =* 7.8 Hz, 1H), 6.74 (s, 1H), 5.40 (dd, *J =* 12.7, 5.2 Hz, 1H), 4.89 (s, 1H), 4.48 (s, 2H), 3.72 (s, 1H), 3.65 (s, 3H), 3.02 (s, 3H), 2.90 (dd, *J =* 28.1, 8.3 Hz, 4H), 2.77 - 2.60 (m, 8H), 2.37 (s, 2H), 2.04 (dd, *J =* 26.2, 14.4 Hz, 6H), 1.83 (d, *J* = 9.1 Hz, 7H), 1.55 (s, 1H), 1.35 - 1.10 (m, 6H). |
| **89** | 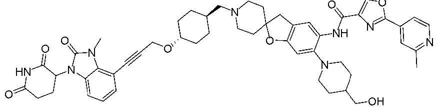 |
| | ESI-MS (*m*/*z*): 911.4 [M+H]⁺. |
| | ¹H NMR (400 MHz, DMSO-*d₆*) *δ* 11.13 (br s, 1H), 10.01 (s, 1H), 9.00 (s, 1H), 8.68 (d, *J =* 5.00 Hz, 1H), 8.23 (s, 1H), 7.76 - 7.86 (m, 2H), 7.18 (d, *J =* 7.88 Hz, 1H), 7.10 - 7.14 (m, 1H), 7.01 - 7.07 (m, 1H), 6.76 (s, 1H), 5.41 (dd, *J* = 12.82, 5.19 Hz, 1H), 4.61 (t, *J =* 5.00 Hz, 1H), 4.47 (s, 2H), 3.65 (s, 3H), 3.41 - 3.54 (m, 3H), 2.84 - 3.05 (m, 5H), 2.62 - 2.83 (m, 5H), 2.61 (s, 3H), 2.29 - 2.45 (m, 3H), 1.99 - 2.17 (m, 5H), 1.69 - 1.91 (m, 8H), 1.42 - 1.66 (m, 4H), 1.11 - 1.27 (m, 2H), 0.84 - 0.98 (m, 2H). |
| **90** | 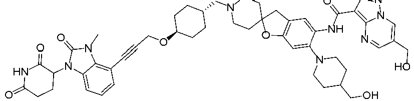 |
| | ESI-MS (*m*/*z*): 901 [M+H]⁺. |
| | ¹H NMR (400 MHz, DMSO-*d₆) δ* 11.08 (s, 1H), 10.48 (s, 1H), 9.21 (s, 1H), 8.90 (d, *J* = 1.75 Hz, 1H), 8.64 (s, 1H), 8.32 (s, 1H), 7.18 (d, *J* = 7.63 Hz, 1H), 7.10 - 7.15 (m, 1H), 7.01 - 7.07 (m, 1H), 6.73 (s, 1H), 5.61 (t, *J =* 5.63 Hz, 1H), 5.41 (br dd, *J =* 12.51, 5.25 Hz, 1H), 4.68 (d, *J* = 5.50 Hz, 2H), 4.56 (t, *J =* 5.19 Hz, 1H), 4.48 (s, 2H), 3.65 (s, 3H), 3.38 - 3.46 (m, 3H), 2.89 - 3.03 (m, 5H), 2.60 - 2.82 (m, 5H), 2.30 - 2.45 (m, 3H), 2.02 - 2.16 (m, 5H), 1.66 - 1.83 (m, 8H), 1.48 - 1.66 (m, 4H), 1.12 - 1.24 (m, 2H), 0.83 - 0.97 (m, 2H). |

### Example 91:

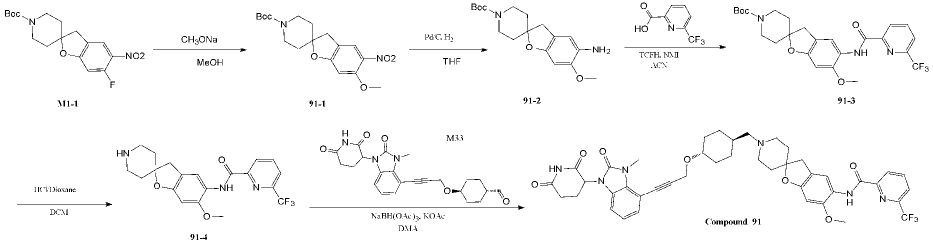

### Step 1:

M1-1 (900 mg, 2.55 mmol, 1.0 eq) was placed in a 100 mL single-necked flask. 30% Sodium methoxide in methanol (2.29 g, 12.7 mmol, 5.0 eq) was added, and the mixture was stirred at 40°C for 4 hours. After completion, water (20 mL) was added to the reaction mixture, followed by extraction with ethyl acetate (20 mL × 3). The combined organic phases were back-washed once with saturated brine (20 mL), dried over anhydrous Na₂SO₄, and concentrated under reduced pressure to afford 91-1 (900 mg, 2.47 mmol, 96.7% yield) as a yellow solid.

ESI-MS (*m*/*z*): 365.2 [M+H]⁺.

¹H NMR (400 MHz, CDCl₃) *δ* 7.85 (s, 1H), 6.47 (s, 1H), 3.93 (s, 3H), 3.75 (m, 2H), 3.34 - 3.43 (m, 2H), 2.98 (s, 2H), 1.92 (m, 2H), 1.69 - 1.79 (m, 2H), 1.48 (s, 9H).

### Step 2:

To a 75 mL hydrogenation bottle, 10% Pd/C (900 mg) and THF (7 mL) were added. A solution of 91-1 (900 mg, 2.47 mmol, 1.0 eq) in THF (10 mL) was then added to the reaction vessel. The mixture was stirred at 25°C under 50 psi hydrogen pressure for 12 hours. After completion, the reaction mixture was filtered, and the filtrate was concentrated under reduced pressure to afford 91-2 (800 mg, 2.39 mmol, yield 96.9%) as a yellow solid.

ESI-MS (*m*/*z*): 335.2 [M+H]⁺.

¹H NMR (400 MHz, CDCl₃) *δ* 6.57 (s, 1H), 6.37 (s, 1H), 3.81 (s, 3H), 3.75 (br t, *J =* 6.50 Hz, 2H), 3.35 - 3.45 (m, 2H), 2.89 (s, 2H), 1.83 - 1.93 (m, 2H), 1.63 - 1.72 (m, 2H), 1.48 (s, 9H).

### Step 3:

91-2 (700 mg, 2.09 mmol, 1.0 eq) was placed in a 100 mL single-necked flask, and acetonitrile (7 mL), 6-(trifluoromethyl)pyridine-2-carboxylic acid (400 mg, 2.09 mmol, 1.0 eq), NMI (0.8 mL, 10.4 mmol, 5.0 eq), and TCFH (704 mg, 2.51 mmol, 1.2 eq) were added to the reaction mixture. The mixture was stirred at room temperature for 12 hours. The precipitated solid was filtered off, and the filter cake was dried under reduced pressure to afford 91-3 (1.00 g, 1.97 mmol, yield 94.1%) as a white solid.

ESI-MS (*m*/*z*): 508.2 [M+H]⁺.

¹H NMR (400 MHz, CDCl₃) *δ* 10.31 (s, 1H), 8.47 (d, *J =* 7.75 Hz, 1H), 8.33 (s, 1H), 8.10 (t, *J =* 7.81 Hz, 1H), 7.84 (d, *J =* 7.75 Hz, 1H), 6.47 (s, 1H), 3.92 (s, 3H), 3.74 (m, 2H), 3.37 - 3.47 (m, 2H), 3.00 (s, 2H), 1.92 (m, 2H), 1.68 - 1.77 (m, 2H), 1.49 (s, 9H).

### Step 4:

91-3 (500 mg, 0.99 mmol, 1.0 eq) was placed in a 50 mL single-necked flask, and DCM (5 mL) was added. A solution of 4M HCl in dioxane (5 mL) was introduced into the mixture. The reaction was stirred at 15°C for 2 hours. After completion, the mixture was concentrated under reduced pressure. Saturated aqueous sodium bicarbonate solution was added to adjust the pH to 7-8. The resulting suspension was filtered, and the filter cake was washed with water. The solid was then concentrated under reduced pressure to afford 91-4 (400 mg, 0.98 mmol, yield 99.7%) as a yellow solid.

ESI-MS (*m*/*z*): 408.0 [M+H]⁺.

### Step 5:

91-4 (200 mg, 0.49 mmol, 1.0 eq) was placed in a 50 mL single-necked flask, and DMA (3.5 mL) was added. M33 (207.89 mg, 0.49 mmol, 1.0 eq), KOAc (144 mg, 1.47 mmol, 3.0 eq), and sodium triacetoxyborohydride (103 mg, 0.49 mmol, 1.0 eq) were added to the reaction mixture, which was stirred at room temperature for 1 hour. The reaction mixture was purified by preparative HPLC to afford **Compound 91** (161 mg, 0.20 mmol, yield 40.2%) as a yellow solid.

ESI-MS (*m*/*z*): 815.4 [M+H]⁺.

¹H NMR (400 MHz, DMSO-*d₆*) *δ* 11.13 (s, 1H), 10.12 (s, 1H), 8.33 - 8.45 (m, 2H), 8.11 - 8.23 (m, 2H), 7.18 (d, *J =* 7.75 Hz, 1H), 7.10 - 7.14 (m, 1H), 7.00 - 7.06 (m, 1H), 6.68 (s, 1H), 5.41 (br dd, *J =* 12.69, 5.32 Hz, 1H), 4.47 (s, 2H), 3.86 (s, 3H), 3.65 (s, 3H), 3.41 - 3.51 (m, 2H), 2.97 (s, 2H), 2.84 - 2.94 (m, 1H), 2.59 - 2.78 (m, 2H), 2.38 (m, 3H), 2.11 (br d, *J =* 7.13 Hz, 2H), 1.97 - 2.07 (m, 3H), 1.68 - 1.88 (m, 6H), , 1.38 - 1.52 (m, 1H), 1.11 - 1.23 (m, 2H), 0.82 - 0.96 (m, 2H).

### Example 92:

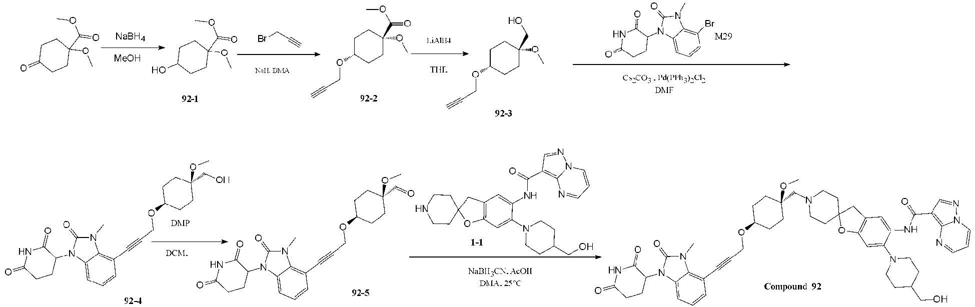

### Step 1:

Methyl 1-methoxy-4-oxocyclohexane-1-carboxylate (25.0 g, 134 mmol, 1.0 eq) was placed in a 250 mL three-necked flask, and MeOH (175 mL) was added. NaBH₄ (10.2 g, 268 mmol, 2.0 eq) was slowly added at 0°C. The reaction mixture was stirred in an ice bath for 1.5 h. Then, a saturated aqueous ammonium chloride solution (200 mL) was added to the reaction mixture, followed by water (100 mL) to dissolve the salts. The mixture was extracted with ethyl acetate (200 mL × 3). The combined organic phases were dried over anhydrous sodium sulfate and concentrated under reduced pressure. The crude product was purified by column chromatography (petroleum ether : ethyl acetate = 5:1-1:1) to afford 92-1 (14.0 g, 74.38 mmol, 55.4% yield) as a clear, colorless oil.

ESI-MS (*m*/*z*): 189.1 [M+H]⁺.

¹H NMR (400 MHz, CDCl₃) *δ* 3.72 (s, 3H), 3.59 - 3.68 (m, 1H), 3.23 (s, 3H), 1.98 - 2.07 (m, 2H), 1.78 - 1.86 (m, 2H), 1.67 - 1.75 (m, 2H), 1.51 - 1.62 (m, 2H).

### Step 2:

Compound 92-1 (5.00 g, 26.5 mmol, 1.0 eq) was placed in a 100 mL three-necked flask, and DMA (35 mL) was added. Under stirring at 0 °C, NaH (1.59 g, 39.8 mmol, 1.5 eq) was added slowly, and the mixture was stirred at 0 °C for 5 hours. 3-Bromopropyne (5.93 g, 39.8 mmol, 1.5 eq) was then added dropwise to the reaction solution at 0 °C, followed by stirring at room temperature for 12 hours. The reaction mixture was slowly poured into saturated aqueous ammonium chloride solution (100 mL) and extracted with ethyl acetate (150 mL × 3). The combined organic phases were back-washed with saturated brine (100 mL), dried over anhydrous sodium sulfate, and concentrated under reduced pressure. The crude product was purified by column chromatography (petroleum ether : ethyl acetate = 10:1 - 2:1) to afford 92-2 (220 mg, 0.97 mmol, yield 18.3%) as a yellow oil.

¹H NMR (400 MHz, CDCl₃) *δ* 4.13 (d, *J =* 2.38 Hz, 2H), 3.68 (s, 3H), 3.41 - 3.53 (m, 1H), 3.17 (s, 3H), 2.34 (t, *J* = 2.32 Hz, 1H), 1.98 (m, 2H), 1.76 - 1.84 (m, 2H), 1.63 - 1.73 (m, 2H), 1.49 - 1.59 (m, 2H).

### Step 3:

Compound 92-2 (1.50 g, 6.63 mmol, 1.0 eq) was placed in a 100 mL three-necked flask, and THF (15 mL) was added. A THF solution of LiAlH₄ (8.0 mL, 19.9 mmol, 3.0 eq, 2.5 M) was added dropwise at 0 °C. The mixture was stirred at room temperature for 2 hours. The reaction was quenched under an ice bath by sequential addition of water (1 mL), 15% aqueous NaOH solution (1 mL), and water (3 mL). Ethyl acetate (40 mL) was added, and the mixture was stirred for 10 minutes before filtration. The filtrate was concentrated under reduced pressure to afford compound 92-3 (1.13 g, 5.70 mmol, 86.0% yield) as a yellow oil.

¹H NMR (400 MHz, CDCl₃) *δ* 4.20 (s, 2H), 3.49 - 3.56 (m, 1H), 3.46 (br s, 2H), 3.20 (s, 3H), 2.41 (br s, 1H), 1.94 (m, 2H), 1.74 - 1.87 (m, 3H), 1.53 - 1.63 (m, 2H), 1.18 - 1.33 (m, 2H).

### Step 4:

M29 (800 mg, 2.37 mmol) was placed in a 100 mL single-necked flask, and DMF (10 mL), 92-3 (703 mg, 3.55 mmol, 1.5 eq), and Cs₂CO₃ (3.00 g, 9.46 mmol, 4.0 eq) were added. Bis(triphenylphosphine)palladium dichloride (184 mg, 0.24 mmol, 0.1 eq) was then introduced into the reaction mixture, which was stirred at 100°C for 2 hours. After completion, THF (50 mL) was added to the reaction solution, followed by filtration. The filtrate was collected and concentrated under reduced pressure. The crude product was purified by column chromatography using a gradient eluent of petroleum ether:THF (from 2:1 to 1:2). The resulting solid was stirred with ethyl acetate (10 mL) for 10 minutes, filtered, and the filter cake was collected to afford 92-4 (0.17 g, 0.37 mmol, yield 15.8%) as a white solid.

ESI-MS *(m*/*z*): 478.2 [M+23]⁺.

¹H NMR (400 MHz, DMSO-*d*₆) *δ* ppm 11.12 (s, 1H), 7.18 (d, *J =* 7.75 Hz, 1H), 7.10 - 7.15 (m, 1H), 7.00 - 7.07 (m, 1H), 5.40 (dd, *J =* 12.69, 5.32 Hz, 1H), 4.47 (s, 2H), 4.44 - 4.46 (m, 1H), 3.65 (s, 3H), 3.44 - 3.54 (m, 1H), 3.29 (d, *J =* 5.63 Hz, 2H), 3.11 (s, 3H), 2.83 - 2.96 (m, 1H), 2.63 - 2.79 (m, 2H), 1.99 - 2.09 (m, 1H), 1.70 - 1.82 (m, 4H), 1.32 - 1.45 (m, 2H), 1.19 - 1.31 (m, 2H).

### Step 5:

92-4 (130 mg, 0.29 mmol, 1.0 eq) was placed in a 40 mL single-necked flask, and DCM (3 mL) was added. DMP (145 mg, 0.34 mmol, 1.2 eq) was then introduced into the reaction mixture, which was stirred at room temperature for 2 hours. After completion, saturated aqueous NaHCO₃ solution (10 mL) was added to the reaction mixture. The mixture was extracted with DCM (10 mL × 2). The combined organic phases were back-washed once with saturated brine (8 mL). The organic layer was separated, dried over anhydrous Na₂SO₄, and concentrated under reduced pressure to afford 92-5 (80 mg, 0.18 mmol, yield 61.8%) as a white solid.

ESI-MS (*m*/*z*): 454.2 [M+1]⁺.

¹H NMR (400 MHz, DMSO-*d*₆) *δ* 11.15 (br s, 1H), 9.56 (s, 1H), 7.20 (d, *J* = 7.82 Hz, 1H), 7.15 (d, *J* = 7.34 Hz, 1H), 7.03 - 7.09 (m, 1H), 5.43 (dd, *J* = 12.71, 5.38 Hz, 1H), 4.51 (s, 2H), 3.67 (s, 3H), 3.54 - 3.64 (m, 1H), 3.22 (s, 3H), 2.86 - 2.98 (m, 1H), 2.65 - 2.80 (m, 2H), 2.01 - 2.10 (m, 1H), 1.78 - 1.93 (m, 4H), 1.39 - 1.61 (m, 4H).

### Step 6:

92-5 (80 mg, 0.18 mmol, 1.0 eq) was placed in a 40 mL single-necked flask, and DMA (3 mL), 1-1 (82 mg, 0.18 mmol, 1.0 eq), AcOH (21.19 mg, 0.35 mmol, 2.0 eq), and sodium cyanoborohydride (22 mg, 0.35 mmol, 1.0 eq) were added to the reaction mixture. The mixture was stirred at room temperature for 2 hours. After completion, the reaction solution was filtered and purified by preparative HPLC to afford **Compound 92** (40.79 mg, 0.05 mmol, yield 25.7%) as a yellow solid.

ESI-MS *(m*/*z*): 900.5 [M+1]⁺.

¹H NMR (400 MHz, DMSO-*d*₆) *δ* 11.13 (s, 1H), 10.49 (s, 1H), 9.36 (dd, *J =* 7.00, 1.38 Hz, 1H), 8.90 (dd, *J =* 4.13, 1.50 Hz, 1H), 8.68 (s, 1H), 8.33 (s, 1H), 7.34 (dd, *J =* 6.94, 4.19 Hz, 1H), 7.18 (d, *J =* 7.88 Hz, 1H), 7.11 - 7.16 (m, 1H), 7.01 - 7.07 (m, 1H), 6.72 (s, 1H), 5.41 (dd, *J* = 12.63, 5.25 Hz, 1H), 4.55 (t, *J =* 5.32 Hz, 1H), 4.48 (s, 2H), 3.66 (s, 3H), 3.47 - 3.56 (m, 1H), 3.40 (br t, *J* = 5.19 Hz, 2H), 3.10 (s, 3H), 2.85 - 3.00 (m, 5H), 2.60 - 2.76 (m, 6H), 2.52 - 2.58 (m, 2H), 2.33 (br s, 2H), 2.00 - 2.11 (m, 1H), 1.68 - 1.90 (m, 10H), 1.48 - 1.64 (m, 3H), 1.26 - 1.45 (m, 4H).

### Example 93:

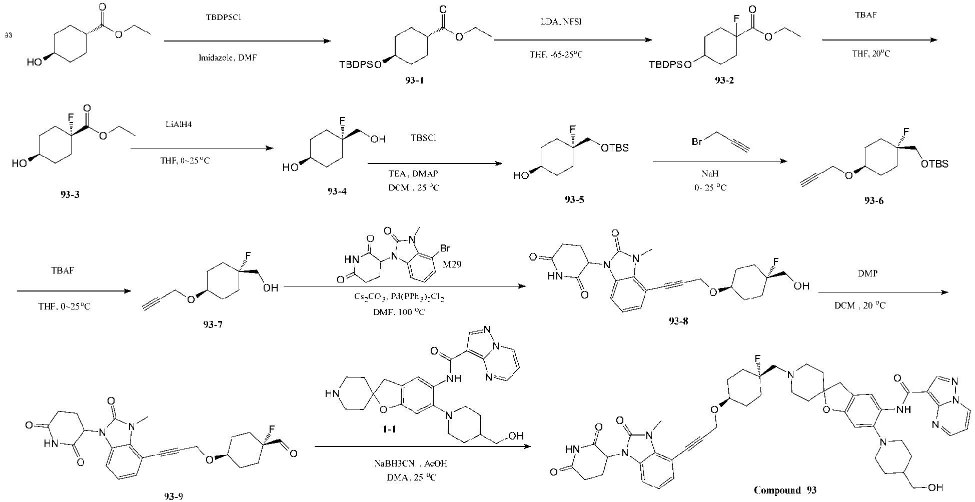

### Step 1:

(1*R*,4*R*)-Ethyl 4-hydroxycyclohexanecarboxylate (20.0 g, 116 mmol, 1.0 equiv) was charged into a 1 L three-necked round-bottom flask and dissolved in DMF (140 mL). To this solution were added imidazole (8.70 g, 127 mmol, 1.1 eq) and tert-butyldiphenylsilyl chloride (TBDPSCl) (33.5 g, 122 mmol, 1.05 eq), which was added dropwise at room temperature. The reaction mixture was stirred at room temperature overnight. After completion, the reaction was quenched with water (200 mL) and extracted with ethyl acetate (500 mL). The organic layer was washed sequentially with water (100 mL) and saturated aqueous sodium chloride (80 mL), dried over anhydrous sodium sulfate, and concentrated under reduced pressure. The crude product was purified by column chromatography (petroleum ether/ethyl acetate = 30:1 to 1:1) to afford 93-1 as a colorless oil (40.5 g, 98.63 mmol, 84.9% yield).

¹H NMR (400 MHz, CDCl₃) *δ* 7.67 (dd, *J* = 7.94, 1.44 Hz, 4H), 7.35 - 7.46 (m, 6H), 4.07 (q, *J =* 7.13 Hz, 2H), 3.55 - 3.66 (m, 1H), 2.21 (tt, *J =* 11.15, 3.42 Hz, 1H), 1.81 - 1.95 (m, 4H), 1.27 - 1.47 (m, 4H), 1.21 (t, *J =* 7.07 Hz, 3H), 1.06 (s, 9H).

### Step 2:

Compound 93-1 (35.0 g, 85.2 mmol, 1.0 eq) was placed in a 1 L three-necked round-bottom flask and dissolved in THF (150 mL) under a nitrogen atmosphere. The reaction mixture was cooled to -65 °C, and LDA (51.1 mL, 102 mmol, 1.2 eq) in THF was added dropwise. After complete addition, the mixture was stirred at -65 °C for 1 hour. A solution of NFSI (22.3 mL, 102 mmol, 1.2 equiv) in THF (150 mL) was then added dropwise at -65 °C. The resulting mixture was allowed to warm to room temperature and stirred overnight. The reaction was quenched by pouring into saturated aqueous ammonium chloride solution (300 mL) and extracted with ethyl acetate (150 mL × 2). The combined organic layers were dried over anhydrous sodium sulfate, concentrated under reduced pressure, and purified by column chromatography (petroleum ether/ethyl acetate = 100:1 -10:1) to afford 93-2 as a yellow oil (36 g, 83.99 mmol, 98.5% yield).

ESI-MS (*m*/*z*): 429.3 [M+H]⁺.

¹H NMR (400 MHz, CDCl₃) *δ* 7.65 - 7.69 (m, 4H), 7.36 - 7.45 (m, 6H), 4.27 (q, *J* = 7.13 Hz, 2H), 4.09 - 4.14 (m, 1H), 2.32 - 2.53 (m, 2H), 1.76 - 1.85 (m, 2H), 1.58 - 1.69 (m, 4H), 1.35 (t, *J =* 7.13 Hz, 3H), 1.10 (s, 8H).

### Step 3:

Compound 93-2 (36.0 g, 83.9 mmol, 1.0 eq) was placed in a 1 L single-necked round-bottom flask and dissolved in THF (300 mL). A solution of TBAF (168.0 mL, 167.98 mmol, 1.0 M in THF, 2.0 eq) was added, and the reaction mixture was stirred at room temperature overnight. The solvent was then removed under reduced pressure, and the crude product was purified by column chromatography (petroleum ether/ethyl acetate = 10:1- 1:1) to afford 93-3 as a pale yellow oil (4.20 g, 22.08 mmol, 26.3% yield).

1H NMR (400 MHz, CDCl₃) *δ* 4.25 (q, *J =* 7.13 Hz, 2H), 4.10 - 4.16 (m, 1H), 2.17 - 2.37 (m, 2H), 1.79 - 1.98 (m, 4H), 1.69 - 1.78 (m, 2H), 1.32 (t, *J =* 7.13 Hz, 3H).

NOESY: Correlation observed between H-9 and H-8.Proton Data: H-7a (δ 2.3 ppm, *J* = 36 Hz). Conclusion: H-9e, F-13a.

### Step 4:

Compound 93-3 (5.00 g, 26.3 mmol, 1.0 eq) was placed in a 100 mL three-necked round-bottom flask and dissolved in THF (50 mL). The solution was cooled to 0°C , and a solution of LiAlH₄ (15.8 mL, 39.4 mmol, 2.5 M in THF, 1.5 eq) was added dropwise. After complete addition, the reaction mixture was allowed to warm to room temperature and stirred overnight. The reaction was carefully quenched at 0°C by slow addition of solid Na₂SO₄·10H₂O,(5.0 g) with stirring for 1 hour. THF (100 mL) was then added to improve solubility, and the mixture was filtered to remove inorganic salts. The filtrate was concentrated under reduced pressure to afford 93-4 as a pale yellow oil (3.00 g, 20.3 mmol, 77.0% yield).

¹H NMR (400 MHz, CD₃OD) *δ* 3.92 (dt, *J =* 4.97, 2.45 Hz, 1H), 3.53 (s, 1H), 3.48 (s, 1H), 1.72 - 1.86 (m, 4H), 1.53 - 1.68 (m, 4H).

### Step 5:

Compound 93-4 (3.00 g, 14.8 mmol, 1.0 eq) was placed in a 100 mL three-necked flask, and DCM (15 mL), TEA, (2.25 g, 22.27 mmol, 1.1 eq), and DMAP (20 mg, 0.20 mmol, 0.01 eq) were added sequentially. The reaction mixture was cooled to 0°C, and a solution of TBSCl (3.36 g, 22.27 mmol, 1.1 eq) in DCM (15 mL) was added dropwise under stirring. After the addition was complete, the reaction was allowed to warm to room temperature and stirred overnight. To quench the reaction, water (80 mL) and DCM (100 mL) were added to the mixture. The organic phase was separated, washed with saturated brine (80 mL), dried over anhydrous sodium sulfate, and concentrated under reduced pressure to give a crude residue. The residue was purified by column chromatography on silica gel using a gradient eluent of petroleum ether/ethyl acetate (from 100:1-10:1-) to afford compound 93-5 as a colorless oil (3.30 g, 12.6 mmol, yield: 62.1%).

¹H NMR (400 MHz, CDCl₃) *δ* 4.08 (br d, *J* = 2.00 Hz, 1H), 3.61 (d, *J* = 19.76 Hz, 2H), 1.77 - 1.95 (m, 4H), 1.60 - 1.72 (m, 4H), 0.91 (s, 9H), 0.08 (s, 6H).

### Step 6:

Compound 93-5 (5.50 g, 20.9 mmol, 1.0 eq) was placed in a 100 mL three-necked flask, and THF (50 mL) was added. NaH(1.26 g, 31.44 mmol, 1.5 eq) was then added at 0°C, and the mixture was stirred for 3 hours at this temperature. Bromopropargyl (2.99 g, 25.2 mmol, 1.2 eq) was added dropwise to the reaction mixture at 0°C, and the mixture was stirred at room temperature overnight. The reaction mixture was poured into a saturated ammonium chloride solution (80 mL), and the organic layer was extracted with ethyl acetate (100 mL × 2). The organic phase was washed with saturated brine (50 mL), dried over anhydrous sodium sulfate, and concentrated under reduced pressure. The crude residue was purified by column chromatography on silica gel using a gradient eluent of petroleum ether/ethyl acetate (10:1 - 1:1) to afford compound 93-6 as a yellow oil (3.50 g, 11.6 mmol, yield: 55.6%).

¹H NMR (400 MHz, CDCl₃) *δ* 4.15 (d, *J =* 2.25 Hz, 2H), 3.80 - 3.87 (m, 1H), 3.59 (d, *J =* 19.76 Hz, 2H), 2.40 (t, *J* = 2.31 Hz, 1H), 1.75 - 1.84 (m, 5H), 1.65 - 1.73 (m, 3H), 0.91 (s, 9H), 0.07 (s, 6H).

### Step 7:

Compound 93-6 (3.50 g, 11.7 mmol, 1.0 eq) was placed in a 100 mL single-necked flask, followed by the addition of THF (35 mL). TBAF (23.3 mL, 23.3 mmol, 1 M in THF, 2.0 eq) was added to the reaction mixture, which was stirred at room temperature overnight. The reaction mixture was concentrated under reduced pressure and purified by column chromatography (petroleum ether : ethyl acetate = 10:1-1:1) to afford 93-7 (2.00 g, 10.7 mmol, 92.2% yield) as a pale yellow oil.

¹H NMR (400 MHz, CDCl₃) *δ* 4.16 (d, *J* = 2.25 Hz, 2H), 3.84 (m, 1H), 3.55 - 3.66 (m, 2H), 2.41 (t, *J =* 2.38 Hz, 1H), 1.72 - 1.83 (m, 8H).

### Step 8:

93-7 (1.00 g, 5.37 mmol, 2.0 eq) was placed in a 100 mL single-necked flask, and DMF (10 mL) was added. M29 (0.91 g, 2.68 mmol, 1.0 eq), cesium carbonate (1.75 g, 5.37 mmol, 2.0 eq), and tetrakis(triphenylphosphine)palladium (0.21 g, 0.27 mmol, 0.1 eq) were then added to the reaction mixture. The reaction solution was stirred at 100 °C for 2 h. Afterward, THF (150 mL) was added, and the mixture was stirred for 30 min before being filtered. The filtrate was collected and concentrated under reduced pressure. The crude product was purified by column chromatography (petroleum ether : THF = 2:1- 1:2). To the obtained product, ethyl acetate (8 mL) was added, and the mixture was stirred for 10 min, followed by filtration. The solid residue was collected to afford 93-8 (350 mg, 0.79 mmol, 29.4% yield) as a white solid.

ESI-MS (*m*/*z*): 444.1 [M+H]⁺.

¹H NMR (400 MHz, DMSO-*d*₆) *δ* 11.13 (s, 1H), 7.18 (d, *J* = 7.88 Hz, 1H), 7.10 - 7.14 (m, 1H), 7.00 - 7.07 (m, 1H), 5.40 (dd, *J =* 12.63, 5.38 Hz, 1H), 4.90 (t, *J =* 6.00 Hz, 1H), 4.47 (s, 2H), 3.83 (br s, 1H), 3.64 (s, 3H), 3.42 (d, *J =* 6.00 Hz, 1H), 3.35 - 3.39 (m, 1H), 2.86 (m, 1H), 2.59 - 2.73 (m, 2H), 2.01 - 2.09 (m, 1H), 1.53 - 1.80 (m, 8H).

### Step 9:

93-8 (150 mg, 0.34 mmol, 1.0 eq) was placed in a 40 mL single-necked flask, and DCM (4 mL) was added. At 0 °C, DMP (288 mg, 0.68 mmol, 2.0 eq) was slowly added. The reaction mixture was stirred at room temperature for 3 h. The reaction solution was then slowly poured into a saturated aqueous sodium bicarbonate solution (10 mL) and extracted with dichloromethane (10 mL × 2). The combined organic phases were dried over anhydrous sodium sulfate and concentrated under reduced pressure. The crude product was purified by column chromatography (petroleum ether : THF = 1 : 2) to afford 93-9 (100 mg, 0.23 mmol, 67.0% yield) as a white solid.

ESI-MS (*m*/*z*): 442.3 [M+H]⁺.

¹H NMR (400 MHz, CDCl₃) *δ* 9.72 (d, *J =* 5.38 Hz, 1H), 8.18 (br s, 1H), 7.18 (d, *J =* 7.34 Hz, 1H), 6.99 - 7.02 (m, 1H), 6.77 (d, *J =* 7.34 Hz, 1H), 5.21 (dd, *J =* 12.47, 5.26 Hz, 1H), 3.94 (br s, 1H), 3.78 (s, 3H), 2.68 - 3.01 (m, 3H), 1.67 - 2.11 (m, 9H).

### Step 10:

93-9 (70 mg, 0.16 mmol, 1.0 eq) was placed in a 40 mL single-necked flask, and DMA (2 mL) was added. 1-1 (73 mg, 0.16 mmol, 1.0 eq), sodium cyanoborohydride (20 mg, 0.32 mmol, 2.0 eq), and AcOH (10 mg, 0.16 mmol, 1.0 eq) were then added. The reaction mixture was stirred at room temperature for 1 h. After filtration, the crude product was purified by HPLC to afford **Compound 93** (39.69 mg, 0.04 mmol, 27.9% yield) as a pale yellow solid.

ESI-MS (m/z): 888.4 [M+H]⁺.

¹H NMR (400 MHz, DMSO-*d*₆) *δ* 11.16 (s, 1H), 10.51 (s, 1H), 9.33 - 9.42 (m, 1H), 8.88 - 8.97 (m, 1H), 8.70 (s, 1H), 8.36 (s, 1H), 7.36 (dd, *J* = 6.93, 4.21 Hz, 1H), 7.20 (d, *J =* 7.92 Hz, 1H), 7.13 - 7.17 (m, 1H), 7.03 - 7.09 (m, 1H), 6.76 (s, 1H), 5.43 (dd, *J =* 12.62, 5.44 Hz, 1H), 4.59 (t, *J =* 5.26 Hz, 1H), 4.50 (s, 2H), 3.85 (br s, 1H), 3.67 (s, 3H), 3.42 (br t, *J =* 5.01 Hz, 3H), 2.87 - 3.04 (m, 5H), 2.56 - 2.79 (m, 8H), 2.01 - 2.10 (m, 1H), 1.68 - 1.88 (m, 14H), 1.51 - 1.66 (m, 3H).

### Example 94:

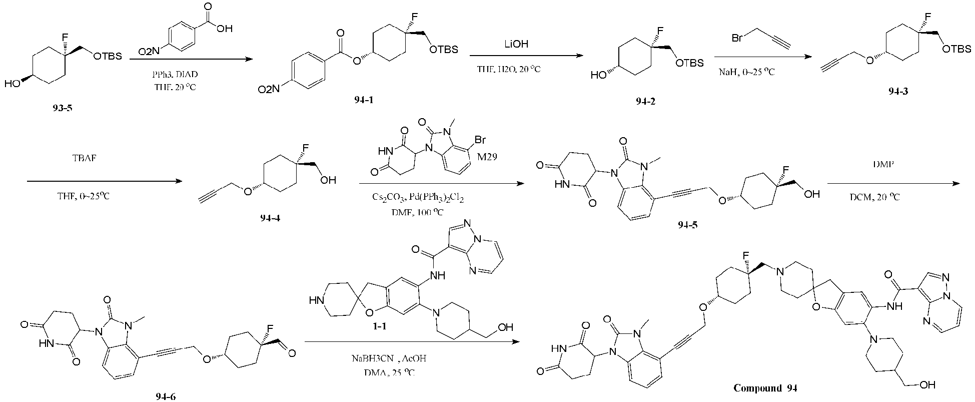

### Step 1:

Compound 93-5 (6.80 g, 25.9 mmol) was placed in a 250 mL three-necked flask and dissolved in THF (70 mL). To this solution were added 4-nitrobenzoic acid (5.20 g, 31.1 mmol, 1.2 eq.) and triphenylphosphine (8.16 g, 31.1 mmol, 1.2 equiv.). The reaction mixture was cooled to 0°C, and DIAD (6.29 g, 31.1 mmol, 1.2 equiv.) was added dropwise. After complete addition, the reaction was stirred at room temperature for 2 hours.Upon completion, water (100 mL) was added to the reaction mixture, followed by extraction with ethyl acetate (80 mL × 2). The combined organic layers were back-washed with saturated brine (50 mL), dried over anhydrous sodium sulfate, and concentrated under reduced pressure. The crude product was purified by column chromatography (petroleum ether/ethyl acetate = 30:1-10:1) to afford compound 94-1 (3.50 g, 8.50 mmol, 32.8% yield) as a white solid.

¹H NMR (400 MHz, CDCl₃) *δ* 8.18 - 8.25 (m, 2H), 8.11 - 8.17 (m, 2H), 7.18 (s, 1H), 4.89 - 4.98 (m, 1H), 3.52 (d, *J =* 16.63 Hz, 2H), 1.78 - 1.95 (m, 5H), 1.44 - 1.69 (m, 3H), 0.83 (s, 9H), 0.00 (s, 6H).

### Step 2:

Compound 94-1 (3.50 g, 8.50 mmol) was placed in a 100 mL single-necked flask and dissolved in THF, (15 mL). A solution of LiOH (710 mg, 17.0 mmol, 2.0 equiv.) in H₂O (15 mL) was added to the reaction mixture. The resulting mixture was stirred at room temperature overnight. Upon completion, water (80 mL) was added, and the aqueous layer was extracted with EtOAc (100 mL × 2). The combined organic phases were washed with saturated brine (50 mL), dried over anhydrous Na₂SO₄, and concentrated under reduced pressure. The crude product was purified by column chromatography (petroleum ether/ethyl acetate = 10:1 - 1:1) to afford compound 94-2 (1.70 g, 6.48 mmol, 76.2% yield) as a colorless oil.

¹H NMR (400 MHz, CDCl₃) *δ* 3.60 - 3.67 (m, 1H), 3.56 (d, *J =* 16.76 Hz, 2H), 1.83 - 1.97 (m, 4H), 1.58 - 1.69 (m, 2H), 1.37 - 1.51 (m, 2H), 0.91 (s, 9H), 0.07 (s, 6H).

### Step 3:

A mixture of compound 94-2 (1.70 g, 6.48 mmol, 1.0 eq) in THF (17 mL) was charged into a 100 mL three-necked flask. NaH (0.52 g, 12.9 mmol, 2.0 eq) was added at 0°C, and the reaction mixture was stirred at this temperature for 3 hours. Propargyl bromide (1.16 g, 7.77 mmol, 1.2 eq) was then added dropwise to the reaction mixture at 0°C. After completion of the addition, the reaction was stirred at room temperature overnight. The mixture was poured into saturated ammonium chloride solution (80 mL) and extracted with ethyl acetate (100 mL × 2). The combined organic layers were back-washed once with saturated brine (50 mL), dried over anhydrous sodium sulfate, and concentrated under reduced pressure. The residue was purified by column chromatography (petroleum ether/ethyl acetate = 10:1 → 1:1) to afford compound 94-3 as a pale yellow oil(1.30 g, 4.33 mmol, 66.8% yield).

¹H NMR (400 MHz, CDCl₃) *δ* 4.22 (d, *J =* 2.35 Hz, 2H), 3.56 (d, *J =* 16.70 Hz, 2H), 3.46 - 3.53 (m, 1H), 2.41 (t, *J* = 2.23 Hz, 1H), 1.87 - 1.98 (m, 4H), 1.59 - 1.70 (m, 2H), 1.39 - 1.55 (m, 2H), 0.91 (s, 9H), 0.07 (s, 6H).

### Step 4:

Compound 94-3 (1.30 g, 4.33 mmol, 1.0 equiv) was placed in a 100 mL single-necked round-bottom flask and dissolved in THF (10 mL). A solution of TBAF (8.70 mL, 8.65 mmol, 1.0 M, 2.0 equiv) was then added to the reaction mixture. The resulting solution was stirred at room temperature overnight. After completion of the reaction, the solvent was removed under reduced pressure, and the residue was purified by column chromatography using a gradient eluent of petroleum ether to ethyl acetate (10:1- 1:1) to afford Compound 94-4 (700 mg, 3.76 mmol, 86.9% yield) as a light yellow oil.

¹H NMR (400 MHz, CDCl₃) *δ* 4.22 (d, *J =* 2.35 Hz, 2H), 3.59 (d, *J =*19.67 Hz, 2H), 3.50 - 3.55 (m, 1H), 2.42 (t, *J =* 2.35 Hz, 1H), 2.04 - 2.10 (m, 2H), 1.88 - 1.96 (m, 2H), 1.59 - 1.72 (m, 2H), 1.34 - 1.52 (m, 2H).

### Step 5:

Compound 94-4 (550 mg, 2.96 mmol, 2.0 equiv) was introduced into a 100 mL single-necked round-bottom flask and dissolved in DMF (6 mL). To this solution was added M29 (500 mg, 1.48 mmol, 1.0 eq), cesium carbonate (963 mg, 2.96 mmol, 2.0 equiv), and tetrakis(triphenylphosphine)palladium (115 mg, 0.15 mmol, 0.1 equiv). The reaction mixture was stirred at 100 °C for 2 hours. Upon completion, the reaction was cooled and diluted with THF (30 mL). The resulting mixture was filtered, and the filtrate was concentrated under reduced pressure. The crude residue was purified by column chromatography (petroleum ether: tetrahydrofuran =2:1-1:2). The obtained oil was further treated with ethyl acetate (5 mL), stirred for 10 minutes, and then filtered. The solid residue was collected to afford compound 94-5 as a white solid (80 mg, 0.18 mmol, 12.2% yield).

ESI-MS (*m*/*z*): 444.2 [M+H]⁺.

¹H NMR (400 MHz, CDCl₃) *δ* 8.03 (br s, 1H), 7.18 (d, *J =* 7.75 Hz, 1H), 7.00 (t, *J =* 7.94 Hz, 1H), 6.77 (d, *J =* 7.38 Hz, 1H), 5.20 (dd, *J =* 12.57, 5.32 Hz, 1H), 4.49 (s, 2H), 3.79 (s, 3H), 3.54 - 3.68 (m, 3H), 2.70 - 3.02 (m, 3H), 2.21 - 2.29 (m, 1H), 2.04 - 2.14 (m, 2H), 1.92 - 2.01 (m, 2H), 1.65 - 1.79 (m, 3H), 1.35 - 1.54 (m, 2H).

### Step 6:

**Compound** 94-5 (80 mg, 0.18 mmol, 1.0 equiv) was placed in a 40 mL single-necked round-bottom flask and dissolved in DCM (3 mL). DMP (114 mg, 0.27 mmol, 1.5 equiv) was then added to the reaction mixture. The solution was stirred at room temperature for 2 hours. Upon completion, the reaction was quenched with saturated aqueous sodium bicarbonate solution (10 mL) and extracted with ethyl acetate (10 mL × 2). The combined organic layers were dried over anhydrous sodium sulfate, concentrated under reduced pressure, and purified by thin-layer chromatography (TLC) using a solvent system of petroleum ether : tetrahydrofuran (0:1) to afford compound 94-6 was obtained as a white solid (70 mg, 0.16 mmol, 87.9% yield).

ESI-MS (*m*/*z*): 442.2 [M+H]⁺.

¹H NMR (400 MHz, DMSO-*d*₆) *δ* 11.13 (s, 1 H), 9.67 (d, *J* = 6.50 Hz, 1H), 7.11 - 7.21 (m, 2H), 7.01 - 7.08 (m, 1H), 5.41 (dd, *J =* 12.51, 5.13 Hz, 1H), 4.52 (s, 2H), 3.63 - 3.68 (m, 4H), 2.84 - 2.97 (m, 1H), 2.59 - 2.78 (m, 2H), 2.01 (m, 2H), 1.81 - 1.94 (m, 3H), 1.66 - 1.76 (m, 2H), 1.40 - 1.53 (m, 2H).

### Step 7:

Compound 94-6 (70 mg, 0.16 mmol, 1.0 equiv) was charged into a 40 mL single-necked flask and dissolved in DMA (2 mL). To this solution were sequentially added 1-1 (73 mg, 0.16 mmol, 1.0 equiv), acetic acid (10 mg, 0.16 mmol, 1.0 equiv), and sodium cyanoborohydride (15 mg, 0.24 mmol, 1.5 equiv). The reaction mixture was stirred at room temperature for 2 hours. After completion, the mixture was filtered and purified by preparative HPLC to afford **Compound** 94 as a pale yellow solid (21.05 mg, 0.02 mmol, 14.8% yield).

ESI-MS (*m*/*z*): 888.5 [M+H]⁺.

¹H NMR (400 MHz, DMSO-*d*₆) *δ* 11.13 (s, 1H), 10.49 (s, 1H), 9.36 (d, *J =* 7.00 Hz, 1H), 8.90 (d, *J =* 3.88 Hz, 1H), 8.68 (s, 1H), 8.33 (s, 1H), 7.34 (dd, *J =* 6.94, 4.19 Hz, 1H), 7.18 (d, *J =* 7.75 Hz, 1H), 7.11 - 7.16 (m, 1H), 7.01 - 7.08 (m, 1H), 6.73 (s, 1H), 5.41 (dd, *J =* 12.63, 5.25 Hz, 1H), 4.55 (t, *J =* 5.19 Hz, 1H), 4.50 (s, 2H), 3.66 (s, 3H), 3.59 (br s, 1H), 3.40 (t, *J =* 5.07 Hz, 3H), 2.86 - 3.02 (m, 5H), 2.56 - 2.78 (m, 7H), 2.47 (br s, 1H), 2.00 - 2.07 (m, 1H), 1.69 - 1.99 (m, 10H), 1.42 - 1.63 (m, 7H).

### Example 95:

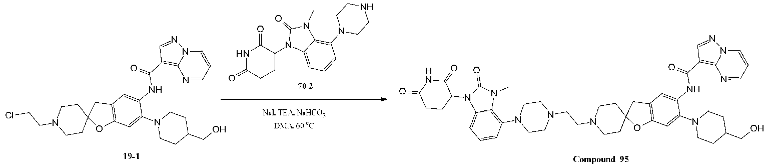

A mixture of 19-1 (40 mg, 0.08 mmol, 1.0 eq), DMF (1.5 mL), 70-2 (31 mg, 0.09 mmol, 1.2 eq), NaI (11 mg, 0.08 mmol, 1.0 eq), NaHCO₃ (27 mg, 0.32 mmol, 4.0 eq), and TEA (32 mg, 0.32 mmol, 4.0 eq) was stirred at 60°C overnight in a 40 mL single-necked flask. After completion, the reaction mixture was filtered and purified by preparative HPLC to afford Compound 95 as a pale yellow solid (15.4 mg, 0.02 mmol, 22.9% yield).

ESI-MS (*m*/*z*): 832.5 [M+H]⁺.

¹H NMR (400 MHz, DMSO-*d*₆) *δ* 11.12 (s, 1H), 10.51 (s, 1H), 9.38 (dd, *J =* 7.00, 1.50 Hz, 1H), 8.91 (dd, *J =* 4.13, 1.38 Hz, 1H), 8.69 (s, 1H), 8.35 (s, 1H), 7.35 (dd, *J =* 7.00, 4.25 Hz, 1H), 6.86 - 7.05 (m, 3H), 6.75 (s, 1H), 5.37 (dd, *J =* 12.57, 5.19 Hz, 1H), 4.59 (t, *J* = 5.25 Hz, 1H), 3.64 (s, 3H), 3.41 (m, 3H), 2.84 - 3.04 (m, 12H), 2.56 - 2.75 (m, 10H), 2.25 - 2.37 (m, 2H), 1.97 - 2.05 (m, 1H), 1.69 - 1.88 (m, 7H), 1.45 - 1.67 (m, 4H).

### Example 96:

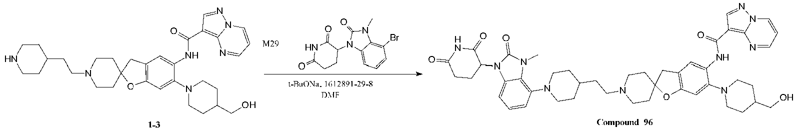

A mixture of M29 (20 mg, 0.06 mmol, 1.0 eq), 1-3 (33 mg, 0.06 mmol, 1.0 eq), DMF (1 mL), sodium tert-butoxide (17 mg, 0.18 mmol, 3.0 eq), and CAS: 1612891-29-8 (5 mg, 0.01 mmol, 0.1 eq) was stirred at 100°C for 2 hours in a 40 mL single-necked flask. The reaction mixture was purified by preparative HPLC to afford **Compound 96** as a pale yellow solid (1.44 mg, 2.9% yield).

ESI-MS (m/z): 831.4 [M+H]⁺.

¹H NMR (400 MHz, DMSO-*d*₆) *δ* 10.85 - 11.37 (s, 1H), 10.49 (s, 1H), 9.35 (d, *J =* 7.00 Hz, 1H), 8.89 (br d, *J =* 3.25 Hz, 1H), 8.67 (s, 1H), 8.33 (s, 1H), 7.33 (dd, *J* = 7.00, 4.25 Hz, 1H), 6.95 - 7.02 (m, 1H), 6.89 - 6.94 (m, 1H), 6.86 (br d, *J =* 8.00 Hz, 1H), 6.70 - 6.75 (m, 1H), 5.34 (br dd, *J =* 12.63, 5.75 Hz, 1H), 4.56 (br t, *J* = 5.13 Hz, 1H), 3.63 (s, 3H), 3.02 - 3.13 (m, 3H), 2.88 - 3.01 (m, 5H), 2.59 - 2.74 (m, 7H), 2.30 - 2.46 (m, 5H), 1.96 - 2.06 (m, 1H), 1.66 - 1.88 (m, 9H), 1.35 - 1.64 (m, 8H).

### Example 97:

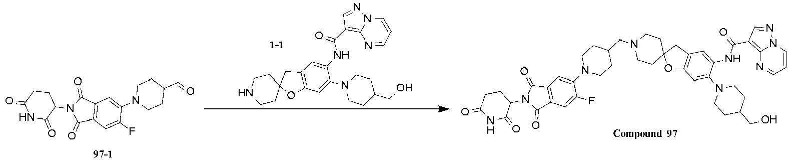

Compound 97-1 was synthesized according to the procedure described in WO2022143856A1 (pages 79-80 of the specification). A mixture of 1-1 (83 mg, 0.18 mmol), 97-1 (70 mg, 0.18 mmol), KOAc (35 mg, 0.36 mmol), sodium triacetoxyborohydride (114 mg, 0.54 mmol) and DMA (1 mL) was added to a 10 mL single-necked flask and stirred at room temperature for 1 h. After completion of the reaction, water (1 mL) was added, and the mixture was filtered and purified by column chromatography using DCM:MeOH=1:0-10:1 as eluent to afford **Compound 97** as a yellow solid (50 mg) with 95.22% purity.

ESI-MS (*m*/*z*): 834.6 [M+H]⁺.

¹H NMR (500 MHz, DMSO-*d₆*) δ11.11 (s, 1H), 10.49 (s, 1H), 9.35 (dd, J=6.96, 1.44 Hz, 1H), 8.89 (dd, J=4.14, 1.38 Hz, 1H), 8.68 (s, 1H), 8.34 (s, 1H), 7.70 (d, J=11.42 Hz, 1H), 7.43 (d, J=7.40 Hz, 1 H), 7.27 - 7.38 (m, 1H), 6.73 (s, 1H), 5.11 (dd, J=12.86, 5.33 Hz, 1H), 4.55 (t, J=5.21 Hz, 1H), 3.61 (br d, J=11.54 Hz, 2H), 3.40 (br t, J=4.77 Hz, 2H), 2.83 - 3.03 (m, 7H), 2.52 - 2.70 (m, 6H), 2.42 (br s, 2H), 2.22 (br d, J=6.65 Hz, 2H), 2.00 - 2.09 (m, 1H), 1.66 - 1.90 (m, 9H), 1.48 - 1.65 (m, 3H), 1.21 - 1.36 (m, 2H).

### Example 98:

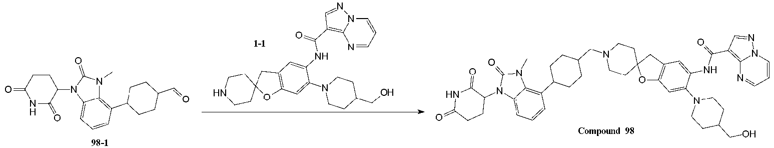

Compound 98-1 was synthesized according to the procedure described in WO2022147465A1 (page 735 of the specification).A mixture of 98-1 (88 mg, 0.19 mmol), 1-1 (70 mg, 0.19 mmol), KOAc (37 mg, 0.38 mmol), sodium triacetoxyborohydride (120 mg, 0.57 mmol), and DMA (1 mL) was stirred at room temperature for 1 hour in a 100 mL single-necked flask. After completion of the reaction, water (1 mL) was added, and the mixture was filtered. The crude product was purified by column chromatography using a gradient elution of DCM:MeOH =1:0-10:1 to afford **Compound 98** as a yellow solid (30 mg).

ESI-MS (*m*/*z*): 816.7 [M+H]⁺.

¹H NMR (500 MHz, DMSO-*d₆*) δ11.10 (s, 1H), 10.49 (s, 1H), 9.33 - 9.40 (m, 1H), 8.90 (br d, J=2.89 Hz, 1H), 8.68 (s, 1H), 8.35 (s, 1H), 7.34 (dd, J=6.84, 4.33 Hz, 1H), 6.94 - 7.09 (m, 3H), 6.74 (s, 1H), 5.38 (br dd, J=12.42, 5.40 Hz, 1H), 4.56 (t, J=5.21 Hz, 1H), 3.56 - 3.61 (m, 3H), 3.40 (br t, J=4.89 Hz, 2H), 3.18 - 3.30 (m, 1H), 2.84 - 3.04 (m, 5H), 2.54 - 2.78 (m, 6H), 2.44 (br d, J=7.28 Hz, 3H), 1.49 - 2.06 (m, 20H).

### Example 99:

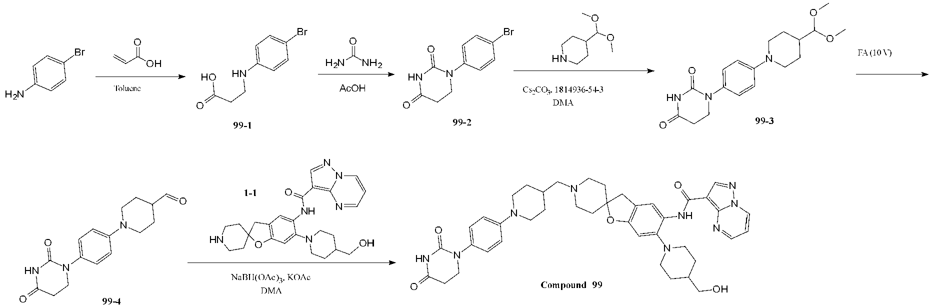

### Step 1:

A mixture of p-bromoaniline (10.0 g, 58.1 mmol, 1.0 eq), toluene (7 mL), and acrylic acid (5.03 g, 69.7 mmol, 1.2 eq) was stirred at 100°C for 8 hours in a 100 mL single-necked flask. The reaction mixture was treated with aqueous NaOH solution (1 N, 200 mL), and the aqueous phase was separated. The pH of the aqueous phase was adjusted to 3 with 2 M hydrochloric acid, followed by extraction with ethyl acetate (250 mL × 3). The combined organic layers were dried over anhydrous sodium sulfate, concentrated under reduced pressure, and purified by column chromatography (petroleum ether:ethyl acetate = 10:1- 0:1) to afford compound 99-1 as a light brown solid (10 g, 40.9 mmol, 70.5% yield).

ESI-MS (*m*/*z*): Br, 244.0[M+1]⁺, 246.0 [M+3]⁺.

¹H NMR (400 MHz, CDCl₃) *δ* 7.22 - 7.27 (m, 2H), 6.46 - 6.53 (m, 2H), 3.42 (t, *J =* 6.26 Hz, 2H), 2.65 (t, *J* = 6.26 Hz, 2H).

### Step 2:

Compound 99-1 (5.00 g, 20.4 mmol, 1.0 eq) was combined with acetic acid (35 mL) and urea (2.46 g, 40.9 mmol, 2.0 eq) in a 100 mL single-necked flask and stirred at 120°C for 12 hours. Water (100 mL) was added to the reaction mixture, resulting in the precipitation of a white solid. The solid was collected by filtration and concentrated under reduced pressure to afford compound 99-2 (2.5 g, 9.29 mmol, 45.4% yield).

ESI-MS (*m*/*z*): Br, 269.0[M+1]⁺, 271.0 [M+3]⁺.

¹H NMR (400 MHz, DMSO-*d₆*) *δ* 10.43 (s, 1H), 7.52 - 7.62 (m, 2H), 7.26 - 7.34 (m, 2H), 3.78 (t, *J =* 6.62 Hz, 2H), 2.70 (t, *J* = 6.68 Hz, 2H).

### Step 3:

A mixture of compound 99-2 (300 mg, 1.11 mmol, 1.0 eq), DMA (3 mL), 4-(dimethoxymethyl)piperidine (532 mg, 3.34 mmol, 3.0 eq), Cs₂CO₃ (182 mg, 0.56 mmol, 3.0 eq), and catalyst (CAS No.: 1814936-54-3, 108 mg, 0.11 mmol, 0.1 eq) was stirred at 100°C under nitrogen atmosphere for 2 hours in a 50 mL single-necked flask. The reaction mixture was concentrated under reduced pressure, diluted with dichloromethane, and filtered. The filtrate was concentrated and purified by column chromatography (petroleum ether:ethyl acetate = 5:1-0:1) to afford compound 99-3 as a pale yellow solid (120 mg, 0.35 mmol, 31.0% yield).

ESI-MS (*m*/*z*): 348.1 [M+H]⁺.

¹H NMR (400 MHz, CDCl₃) *δ* 7.53 (br s, 1H), 7.15 (d, *J* = 9.01 Hz, 2H), 6.94 (d, *J* = 9.01 Hz, 2H), 4.09 (d, *J =* 7.13 Hz, 1H), 3.81 (t, *J =* 6.69 Hz, 2H), 3.71 (br d, *J =* 12.26 Hz, 2H), 3.38 (s, 6H), 2.81 (t, *J =* 6.69 Hz, 2H), 2.70 (td, *J =* 12.26, 2.25 Hz, 2H), 1.85 (m, 2H), 1.77 (m, 1H), 1.37 - 1.54 (m, 2H).

### Step 4:

Compound 99-3 (72 mg, 0.21 mmol, 1.0 eq) was dissolved in formic acid (3 mL) in a 40 mL single-necked flask and stirred at 25°C for 2 hours. The reaction mixture was concentrated under reduced pressure to afford compound 99-4 as a brown oil (62 mg, 0.21 mmol, 100.0% yield).

ESI-MS (*m*/*z*): 302.1 [M+H]⁺.

### Step 5:

A mixture of compound 99-4 (62 mg, 0.21 mmol, 1.0 eq), DMA (2 mL), compound 1-1 (95 mg, 0.21 mmol, 1.0 eq), KOAc (60 mg, 0.62 mmol, 3.0 eq), and sodium triacetoxyborohydride (43 mg, 0.21 mmol, 1.0 eq) was stirred at room temperature for 1 hour in a 50 mL single-necked flask. The reaction mixture was filtered and purified by preparative high-performance liquid chromatography (HPLC) to afford **Compound 99** as a yellow solid (65.0 mg, 0.09 mmol, 42.2% yield).

ESI-MS (*m*/*z*): 748.6 [M+H]⁺.

¹H NMR (400 MHz, DMSO-*d*₆) *δ* 10.49 (s, 1H), 10.25 (s, 1H), 9.36 (dd, *J* = 6.88, 1.50 Hz, 1H), 8.90 (dd, *J =* 4.13, 1.50 Hz, 1H), 8.68 (s, 1H), 8.35 (s, 1H), 7.34 (dd, *J =* 7.00, 4.25 Hz, 1H), 7.14 (d, *J =* 9.01 Hz, 2H), 6.93 (d, *J* = 9.01 Hz, 2H), 6.74 (s, 1H), 4.55 (t, *J* = 5.32 Hz, 1H), 3.65 - 3.73 (m, 4H), 3.40 (br t, *J =* 5.25 Hz, 2H), 3.00 (s, 2H), 2.93 (d, *J* = 10.51 Hz, 2H), 2.62 - 2.72 (m, 8H), 2.22 (d, *J* = 6.75 Hz, 2H), 1.67 - 1.90 (m, 10H), 1.45 - 1.65 (m, 4H), 1.16 - 1.30 (m, 2H).

### Example 100:

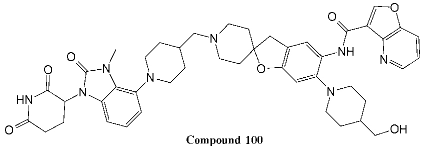

**Compound 100** was obtained as a white solid according to a synthetic method analogous to that described for Example 78.

ESI-MS (*m*/*z*): 817.5 [M+H]⁺.

1H NMR (400 MHz, DMSO-*d₆*) *δ* 11.09 (br s, 1H), 10.93 (s, 1H), 8.97 (s, 1H), 8.75 (br d, *J* = 4.41 Hz, 1H), 8.33 (s, 1H), 8.25 (br d, *J* = 8.58 Hz, 1H), 7.57 (dd, *J* = 8.34, 4.77 Hz, 1H), 6.94 - 7.01 (m, 1H), 6.85 - 6.92 (m, 2H), 6.75 (s, 1H), 5.36 (dd, *J* = 12.40, 5.01 Hz, 1H), 4.54 (t, *J* = 5.13 Hz, 1H), 3.63 (s, 3H), 3.38 (t, *J* = 5.13 Hz, 2H), 3.11 (m, 2H), 2.84 - 3.04 (m, 5H), 2.56 - 2.76 (m, 7H), 2.42 (m, 2H), 2.25 (m, 2H), 1.96 - 2.05 (m, 1H), 1.67 - 1.91 (m, 9H), 1.45 - 1.67 (m, 4H), 1.32 (m, 2H).

### Example 101:

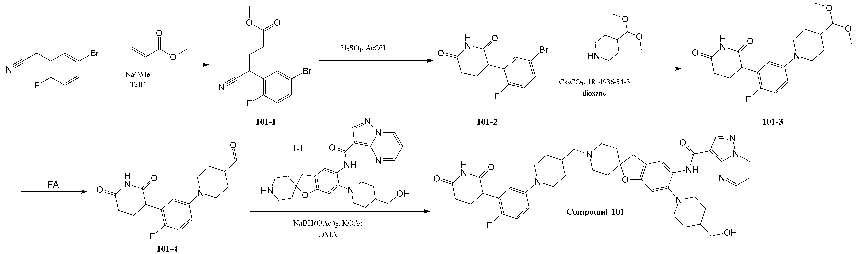

### Step 1:

(5-Bromo-2-fluorophenyl)acetonitrile (5.00 g, 23.36 mmol, 1.0 equiv.) was charged into a 100 mL single-necked flask and dissolved in THF (35 mL). Methyl acrylate (2.21 g, 25.70 mmol, 1.1 equiv.) was added, followed by dropwise addition of sodium methoxide (0.13 g, 2.34 mmol, 0.1 equiv.) at 0°C. The reaction mixture was stirred at 25°C for 2 hours. After completion, the reaction solution was concentrated under reduced pressure and purified by column chromatography (petroleum ether/ethyl acetate = 1:1) to afford compound 101-1 (670 mg, 1.43 mmol, 8.1% yield) as a white solid.

ESI-MS (*m*/*z*): Br, 300.3[M+1]⁺, 302.0 [M+3]⁺.

¹HNMR (400 MHz, CDCl₃) *δ* 7.59 (dd, *J =* 6.54, 2.38 Hz, 1H), 7.46 (m, 1H), 7.01 (t, *J =* 9.17 Hz, 1H), 4.24 (t, *J* = 7.46 Hz, 1H), 3.70 (s, 3H), 2.45 - 2.61 (m, 2H), 2.16 - 2.29 (m, 2H).

### Step 2:

Compound 101-1 (670 mg, 2.23 mmol, 1.0 equiv.) was placed in a 40 mL single-necked flask, followed by addition of AcOH,(6.7 mL) and H₂SO₄ (0.67 mL). The reaction mixture was stirred at 90°C for 2 hours. Upon completion, water was added to the reaction mixture, resulting in precipitation of a white solid. The solid was collected by filtration, and the filter cake was concentrated under reduced pressure to afford compound 101-2 (500 mg, 1.75 mmol, 78.3% yield) as a white solid.

ESI-MS (*m*/*z*): Br, 285.9 [M+1]⁺, 287.9 [M+3]⁺.

¹H NMR (400 MHz, DMSO-*d₆*) *δ* 10.92 (s, 1H), 7.50 - 7.61 (m, 2H), 7.20 (t, *J* = 9.36 Hz, 1H), 4.08 (dd, *J* = 12.87, 4.89 Hz, 1H), 2.67 - 2.79 (m, 1H), 2.55 - 2.59 (m, 1H), 2.24 (m, 1H), 1.96 - 2.04 (m, 1H).

### Step 3:

Compound 101-2 (500 mg, 1.86 mmol, 1.0 eq) was placed in a 50 mL single-necked flask, and 1,4-dioxane (5 mL) was added. Then, 4-(dimethoxymethyl)piperidine (834 mg, 5.24 mmol, 3.0 eq), Cs₂CO₃ (1708 mg, 5.24 mmol, 3.0 eq), and a catalyst (CAS No.: 1814936-54-3, 170 mg, 0.17 mmol, 0.1 eq) were added to the reaction mixture. The system was purged with nitrogen, and the mixture was stirred at 100 °C for 2 hours. After completion, the reaction was cooled to room temperature and diluted with dichloromethane (20 mL). The mixture was filtered, and the filtrate was concentrated under reduced pressure. The residue was purified by column chromatography on silica gel using a gradient eluent of petroleum ether and ethyl acetate (5:1 to 0:1) to afford compound 101-3 as a yellow solid (175 mg, 0.48 mmol, 27.5% yield), purity: 88.4%.

ESI-MS (*m*/*z*): 365.1 [M+H]⁺.

¹H NMR (400 MHz, CDCl₃) *δ* 7.89 (br s, 1H), 6.86 - 6.96 (m, 1H), 6.73 - 6.83 (m, 1H), 6.59 - 6.68 (m, 1H), 4.02 (d, *J =* 7.25 Hz, 1H), 3.77 (dd, *J =* 11.26, 5.25 Hz, 1H), 3.45 - 3.52 (m, 2H), 3.30 (s, 6H), 2.67 - 2.76 (m, 1H), 2.49 - 2.66 (m, 3H), 2.11 - 2.30 (m, 2H), 1.74 - 1.82 (m, 2H), 1.65 (m, 1H), 1.32 - 1.45 (m, 2H).

### Step 4:

To a 40 mL single-necked flask, compound 101-3 (160 mg, 0.44 mmol, 1.0 eq) was added, followed by formic acid (5 mL). The mixture was stirred at 25 °C for 3 hours. The reaction was concentrated under reduced pressure to afford compound 101-4 as a yellow oil (139 mg, 0.44 mmol).
ESI-MS (*m*/*z*): 319.1 [M+H]⁺.

### Step 5:

Compound 101-4 (139 mg, 0.44 mmol, 1.0 eq) was placed in a 50 mL single-necked flask, and DMA (4 mL) was added. Subsequently, compound 1-1 (201 mg, 0.44 mmol, 1.0 eq), potassium acetate (KOOAc, 128 mg, 1.31 mmol, 3.0 eq), and sodium triacetoxyborohydride (92 mg, 0.44 mmol, 1.0 eq) were added. The reaction mixture was stirred at 20 °C for 1 hour. After completion, the reaction mixture was filtered, and the filtrate was purified by preparative HPLC to afford **compound 101** as a yellow solid (127 mg, 0.17 mmol, 38.3% yield), purity: 98.2%.

ESI-MS (*m*/*z*): 765.4 [M+H]⁺.

¹H NMR (400 MHz, DMSO-*d*₆) *δ* 10.84 (br s, 1H), 10.49 (s, 1H), 9.36 (dd, *J* = 7.03, 1.43 Hz, 1H), 8.90 (dd, *J =* 4.05, 1.43 Hz, 1H), 8.68 (s, 1H), 8.35 (s, 1H), 7.33 (dd, *J =* 6.97, 4.23 Hz, 1H), 7.01 (t, *J =* 9.30 Hz, 1H), 6.80 - 6.91 (m, 2H), 6.73 (s, 1H), 4.56 (t, *J* = 5.25 Hz, 1H), 3.95 (dd, *J* = 12.58, 4.95 Hz, 1H), 3.58 (br d, *J =* 10.97 Hz, 2H), 3.40 (br t, *J =* 5.01 Hz, 2H), 2.99 (s, 2H), 2.93 (br d, *J =* 10.97 Hz, 2H), 2.53 - 2.81 (m, 7H), 2.33 - 2.50 (m, 3H), 2.17 - 2.30 (m, 3H), 1.95 - 2.05 (m, 1 H), 1.69 - 1.90 (m, 8H), 1.47 - 1.68 (m, 4H), 1.14 - 1.28 (m, 2H).

### Example 102:

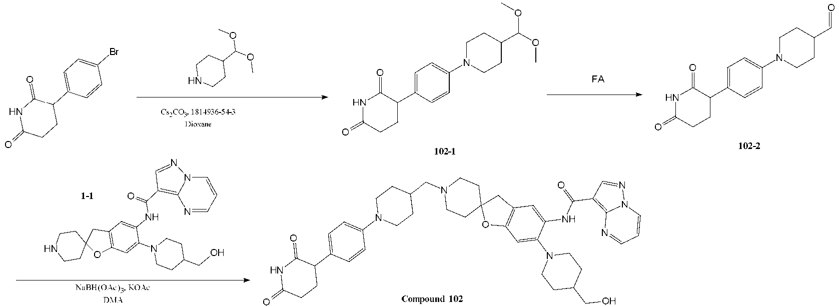

### Step 1:

A mixture of 3-(4-bromophenyl)piperidine-2,6-dione (500 mg, 1.86 mmol, 1.0 eq) was placed into a 50 mL single-necked flask, and 1,4-dioxane (5 mL) was added. To this reaction mixture was added 4-(dimethoxymethyl)piperidine (890 mg, 5.59 mmol, 3.0eq), Cs₂CO₃ (1.82g, 5.59mmol, 3.0eq), and the compound with CAS number 1814936-54-3 (181 mg, 0.19 mmol, 0.1 eq). The reaction was stirred at 100 °C under a nitrogen atmosphere for 2 hours. Upon completion, the reaction mixture was diluted with DCM (80 mL), filtered, and the filtrate was concentrated under reduced pressure. And purified by column chromatography (PE:EA= 3:1-0:1) to afford compound 102-1 (100 mg, 0.29 mmol, yield 15.5%) as a yellow solid.

ESI-MS (*m*/*z*): 347.2 [M+H]⁺.

¹H NMR (400 MHz, CDCl₃) *δ* 7.92 (br s, 1H), 7.08 (d, *J* = 8.63 Hz, 2H), 6.93 (d, *J* = 8.63 Hz, 2H), 4.09 (d, *J =* 7.25 Hz, 1H), 3.67 - 3.76 (m, 3H), 3.38 (s, 6H), 2.60 - 2.76 (m, 4H), 2.15 - 2.33 (m, 2H), 1.85 (br d, *J =* 13.26 Hz, 2H), 1.70 - 1.78 (m, 1H), 1.45 (m, 2H).

### Step 2:

Compound 102-1 (90 mg, 0.26 mmol, 1.0 eq) was placed into a 40 mL single-necked flask, and formic acid (4 mL) was added. The reaction mixture was stirred at 25 °C for 1 hour. After the reaction was complete, the solvent was removed under reduced pressure to afford compound 102-2 (78.03 mg, 0.26 mmol) as a yellow oil.
ESI-MS *(m*/*z*): 300.3 [M+H]⁺.

### Step 3:

To a 50 mL single-necked flask was added compound 102-2 (78 mg, 0.26 mmol, 1.0 eq). Dimethylacetamide (DMA, 3 mL), compound 1-1 (120 mg, 0.26 mmol, 1.0 eq), potassium acetate (KOAc, 76 mg, 0.78 mmol, 3.0 eq), and sodium triacetoxyborohydride (55 mg, 0.26 mmol, 1.0 eq) were added to the reaction mixture. The solution was stirred at room temperature for 1 hour. After completion, the reaction mixture was filtered and purified by preparative HPLC to afford **Compound 102** (67.41 mg, 0.09 mmol, yield 34.8%) as a yellow solid.

ESI-MS (*m*/*z*): 747.4 [M+H]⁺.

¹H NMR (400 MHz, DMSO-*d*₆) *δ* 10.78 (s, 1H), 10.49 (s, 1H), 9.36 (dd, *J* = 7.03, 1.43 Hz, 1H), 8.90 (dd, *J* = 4.17, 1.55 Hz, 1H), 8.68 (s, 1H), 8.34 (s, 1H), 7.30 - 7.37 (m, 1H), 7.04 (d, *J =* 8.70 Hz, 2H), 6.89 (d, *J* = 8.70 Hz, 2H), 6.74 (s, 1H), 4.56 (t, *J =* 5.25 Hz, 1H), 3.63 - 3.77 (m, 3H), 3.40 (br t, *J =* 5.13 Hz, 2H), 2.99 (s, 2H), 2.93 (br d, *J* = 11.09 Hz, 2H), 2.59 - 2.70 (m, 5H), 2.35 - 2.49 (m, 4H), 2.21 (br d, *J* = 7.03 Hz, 2H), 1.98 - 2.17 (m, 2H), 1.45 - 1.91 (m, 13H), 1.15 - 1.28 (m, 2H).

### Example 103:

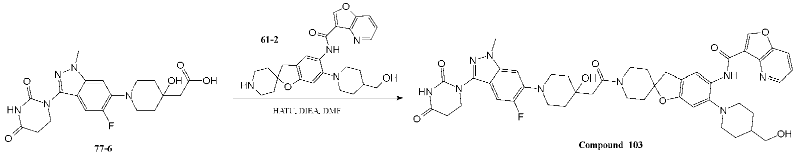

Compound 77-6 (300 mg, 0.72 mmol, 1.0 eq) was charged into a 40 mL single-necked flask containing DMF (5 mL). To this solution were sequentially added compound 61-2 (330 mg, 0.72 mmol, 1.0 eq), DIEA (,185 mg, 1.43 mmol, 2.0 eq), and HATU (299 mg, 0.79 mmol, 1.1 eq). The reaction mixture was stirred at room temperature for 2 hours. After filtration, the filtrate was purified by HPLC to afford **Compound 103** (357.08 mg, 0.40 mmol, yield 55.6%) as a white solid.

ESI-MS (*m*/*z*): 864.4 [M+H]⁺.

¹H NMR (400 MHz, DMSO-*d*₆) *δ* 10.94 (s, 1H), 10.53 (s, 1H), 8.98 (s, 1H), 8.75 (d, *J =* 4.17 Hz, 1H), 8.36 (s, 1H), 8.22 - 8.30 (m, 1H), 7.51 - 7.66 (m, 1H), 7.34 (d, *J =* 12.87 Hz, 1H), 7.13 (d, *J =* 7.15 Hz, 1H), 6.78 (s, 1H), 5.02 (s, 1H), 4.54 (t, *J* = 5.13 Hz, 1H), 3.95 (s, 3H), 3.84 - 3.93 (m, 3H), 3.75 (m, 1H), 3.61 (m, 1H), 3.36 - 3.50 (m, 3H), 3.15 - 3.23 (m, 2H), 3.03 - 3.13 (m, 4H), 2.96 (m, 2H), 2.75 (t, *J* = 6.68 Hz, 2H), 2.59 - 2.70 (m, 4H), 1.66 - 1.95 (m, 10H), 1.44 - 1.65 (m, 3H).

### Experimental Method 1: IRAK4 Degradation Assay in THP-1 Cells

In a 6-well cell culture plate, an appropriate number of THP-1 cells were seeded. The plate was placed in a 5% CO₂ incubator at 37°C for overnight culture. Then, the test compound's dimethyl sulfoxide solution was added, with the final concentration ranging from 0.0128 to 1000 nM. After continuous culture for 24 hours, the culture medium was removed, and the cells were collected in a 1.5 mL centrifuge tube. After adding lysis buffer and grinding thoroughly, the mixture was placed on ice for 30 minutes. It was then centrifuged at 15,000 xg and 4°C for 20 minutes. The supernatant was taken, and the level of IRAK4 protein was detected by Western Blot. The experimental results are shown in Table 1:

**Table 1: Degradative Activity of the Invented Compound on IRAK4 Protein**

| **Compound** | **DC₅₀(nM)** | **Dₘₐₓ%** |
|---|---|---|
| **1** | 1.09 | 91.9 |
| **3** | 4.14 | 93.5 |
| **6** | 9.65 | 85.1 |
| **14** | 3.40 | 89.2 |
| **15** | 2.22 | 86.0 |
| **19** | 2.18 | 87.9 |
| **21** | 3.58 | 91.6 |
| **23** | 3.08 | 88.3 |
| **28** | 3.38 | 86.5 |
| **29** | 7.12 | 92.8 |
| **32** | 1.53 | 95.4 |
| **33** | 1.66 | 94.8 |
| **34** | 2.20 | 90.6 |
| **35** | 4.69 | 93.4 |
| **36** | 2.27 | 86.5 |
| **37** | 3.32 | 90.5 |
| **38** | 2.81 | 88.8 |
| **41** | 3.86 | 90.5 |
| **44** | 6.38 | 84.9 |
| **45** | 1.52 | 87.4 |
| **46** | 1.37 | 87.0 |
| **51** | 2.00 | 83.9 |
| **52** | 5.25 | 89.2 |
| **53** | 2.77 | 77.5 |
| **54** | 3.50 | 91.1 |
| **55** | 3.44 | 89.3 |
| **56** | 7.79 | 90.4 |
| **57** | 3.30 | 88.1 |
| **58** | 3.69 | 85.6 |
| **59** | 1.17 | 88.6 |
| **62** | 7.34 | 82.8 |
| **63** | 5.57 | 86.2 |
| **64** | 1.30 | 89.3 |
| **69** | 2.76 | 90.6 |
| **70** | 1.10 | 91.2 |
| **72** | 7.70 | 92.6 |
| **74** | 2.78 | 86.2 |
| **75** | 2.18 | 86.4 |
| **76** | 1.92 | 87.5 |
| **77** | 1.28 | 85.7 |
| **78** | 0.86 | 94.0 |
| **79** | 1.55 | 89.5 |
| **80** | 0.64 | 83.4 |
| **81** | 1.02 | 90.2 |
| **82** | 3.44 | 85.6 |
| **84** | 1.13 | 88.3 |
| **85** | 0.66 | 89.8 |
| **86** | 2.98 | 86.2 |
| **87** | 4.06 | 90.0 |
| **88** | 5.40 | 89.0 |
| **89** | 6.02 | 84.6 |
| **90** | 2.24 | 87.1 |
| **92** | 3.68 | 88.5 |
| **94** | 5.94 | 87.7 |
| **95** | 0.89 | 95.0 |
| **96** | 1.65 | 92.0 |
| **97** | 7.85 | 87.9 |
| **98** | 1.10 | 88.6 |

The experimental results show that the compounds of the present invention exhibit excellent degradation effects on IRAK4 protein.

### Experimental example 2: Pharmacokinetic study in mice

### 1. Test Methods:

Six male mice weighing 20-30 g were randomly divided into two groups. One group received an intravenous (IV) administration of the test compound at a dose of 2 mg/kg, while the other group received an oral (gavage) administration at a dose of 10 mg/kg. Blood samples were collected at 0.083, 0.25, 0.5, 1, 2, 4, 6, 8, and 24 hours post-administration. Plasma samples were processed and analyzed using LC/MS/MS in MRM)mode. A validated standard curve was established for the quantitative determination of the target compound in plasma, enabling the generation of plasma drug concentration-time profiles. Pharmacokinetic parameters were calculated using WinNonlin software with a non-compartmental model.

### 2. Test Results:

The compound of the present invention was subjected to the above-described pharmacokinetic study, and the results are presented in Table 2.

**Table 2. Experimental results of the pharmacokinetic parameters of the compounds provided in some examples of the present invention**

| **Compound** | **p.o. (10 mg/kg)** | | | | **i.v. (2 mg/kg)** | |
|---|---|---|---|---|---|---|
| | **T_{1/2}(h)** | **Tₘₐₓ(h)** | **Cₘₐₓ(ng/mL )** | **AUC₀₋ₜ(h*ng/mL)** | **T_{1/2}(h)** | **AUC₀₋ₜ(h*ng/mL)** |
| **15** | 23.6 | 6.00 | 1323 | 23205 | 22.2 | 14394 |
| **34** | 3.70 | 2.00 | 1540 | 14793 | 2.69 | 7155 |
| **35** | 4.34 | 2.00 | 2073 | 23388 | 3.78 | 10556 |
| **36** | 3.22 | 2.00 | 1743 | 17969 | 2.84 | 8726 |
| **37** | 3.94 | 4.00 | 2150 | 29312 | 3.36 | 12121 |
| **38** | 4.27 | 4.00 | 2277 | 26962 | 4.07 | 11671 |
| **41** | 2.62 | 2.00 | 2987 | 21210 | 2.13 | 5122 |
| **45** | 4.40 | 2.00 | 1190 | 7391 | 2.02 | 3192 |
| **46** | 3.82 | 4.00 | 3493 | 42143 | 2.76 | 14148 |
| **55** | 3.36 | 1.00 | 1120 | 10007 | 3.00 | 6192 |
| **70** | 2.28 | 2.00 | 870 | 6678 | 3.10 | 7877 |
| **77** | 3.85 | 1.00 | 4830 | 38237 | 4.68 | 12939 |
| **78** | 6.58 | 1.00 | 2603 | 44945 | 33.0 | 32728 |
| **95** | 2.78 | 2.00 | 1210 | 11149 | 2.16 | 4567 |

The experimental results demonstrate that the compound of the present invention exhibits favorable pharmacokinetic properties in mice.

## Claims

1. A compound of formula (I), or an isomer, an N-oxide, a hydrate, a solvate, a metabolite, a pharmaceutically acceptable salt or a prodrug thereof, 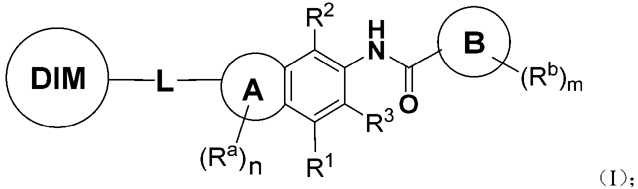 wherein:
ring A is an 8-13 membered spirocyclic ring having 0-3 heteroatoms independently selected from nitrogen, oxygen, and sulfur;
ring B is phenyl, naphthyl, a 5-6 membered monocyclic heteroaryl, or a 9-10 membered bicyclic heteroaryl;
R¹, R², and R³ are each independently hydrogen, deuterium, halogen, CN, OH, NO₂, NH₂, C₁₋₆ alkyl, C₂₋₆ alkenyl, C₁₋₆ alkoxy, -(C₀₋₃ alkylene)-C₃₋₆ cycloalkyl, or -(C₀₋₃ alkylene)-3-8 membered heterocyclyl,wherein C₁₋₆ alkyl, C₂₋₆ alkenyl, C₁₋₆ alkoxy, -(C₀₋₃ alkylene)-C₃₋₆ cycloalkyl, and -(C₀₋₃ alkylene)-3-8 membered heterocyclyl are each independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, C₁₋₃ hydroxyalkyl, and C₁₋₃ haloalkyl;
each R^{a} and R^{b} is independently hydrogen, deuterium, halogen, oxo, CN, NO₂, -R⁴, -OR^{c}, -SR^{c}, -N(R^{c})₂, -C(R^{c})₃, -S(=O)₂R^{c}, -S(=O)₂N(R^{c})₂, -S(=O)R^{c}, -S(=O)(NR^{c})R^{c}, -P(=O)(OR^{c})₂, -P(=O)(N(R^{c})₂)₂, -CF(R^{c})₂, -CF₂(R^{c}), -CF₃, -C(R^{c})₂-OR^{c}, -C(R^{c})₂-N(R^{c})₂, -C(=O)R^{c}, -C(=O)OR^{c}, or -C(=O)N(R^{c})₂; or two R^{a} groups attached to the same atom optionally join to form a C₃₋₄ cycloalkyl or 3-4 membered heterocyclyl with the atom to which they are attached;
each R⁴ is independently C₁₋₆ alkyl, C₂₋₆ alkenyl, phenyl, C₃₋₇ cycloalkyl, 3-7 membered heterocyclyl, or 5-6 membered heteroaryl, wherein R⁴ is independently optionally substituted with 1, 2, or 3 substituents selected from deuterium, halogen, CN, OH, NO₂, NH₂, oxo, C₁₋₃ alkyl, C₁₋₃ alkoxy, C₁₋₃ haloalkoxy, and C₁₋₃ haloalkyl;
each R^{c} is independently hydrogen, deuterium, C₁₋₆ alkyl, phenyl, C₄₋₇ cycloalkyl, 4-7 membered heterocyclyl, or 5-6 membered heteroaryl, wherein C₁₋₆ alkyl, phenyl, C₄₋₇ cycloalkyl, 4-7 membered heterocyclyl, and 5-6 membered heteroaryl are each independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, and C₁₋₃ haloalkyl;
or two R^{c} groups attached to the same atom optionally join to form a C₄₋₇ cycloalkyl, a 4-11 membered bridged bicyclic ring, or a 4-11 membered spirocyclic ring having 0-3 heteroatoms independently selected from nitrogen, oxygen, and sulfur, wherein C₄₋₇ cycloalkyl, 4-11 membered bridged bicyclic ring, and 4-11 membered spirocyclic ring are each independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, and C₁₋₃ haloalkyl;
m and n are each independently 0, 1, 2, 3, 4, 5, 6, 7, or 8;
L is C₁₋₂₀ alkylene, wherein 1, 2, 3, 4, or 5 methylene units are each independently optionally replaced by a unit selected from -CR^{d}=CR^{d}-, -C≡C-, -C(R^{d})₂-, -Cy-, -O-, -C(=O)-, and -N(R^{d})-;wherein each -Cy- is independently C₄₋₇ cycloalkyl, 4-11 membered heterocyclyl, 5-11 membered spirocyclic, 5-11 membered bridged bicyclic, phenyl, or 5-6 membered heteroaryl,and each -Cy- is independently optionally substituted with 1, 2, or 3 substituents selected from deuterium, halogen, CN, OH, NO₂, NH₂, oxo, C₁₋₃ alkyl, C₁₋₃ alkoxy,
C₁₋₃ haloalkoxy, and C₁₋₃ haloalkyl;
each R^{d} is independently hydrogen, deuterium, halogen, CN, OH, NO₂, NH₂, or C₁₋₃ alkyl;
DIM is an E3 ubiquitin ligase binding moiety.

2. The compound according to claim 1, wherein ring A is: 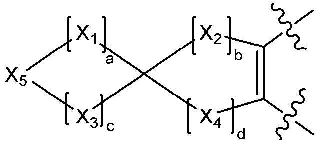 wherein:
X₁, X₂, X₃, X₄, and X₅ are each independently CH₂, -C(=O)-, NH, O, or S;
a and c are each independently 1 or 2;
b and d are each independently 0, 1, or 2, wherein b and d are not both 0, and the sum of b and d is 2, 3, or 4.

3. The compound according to either of claim 1 or claim 2, wherein each R^{a} is independently hydrogen, deuterium, halogen, oxo, CN, OH, NO₂, NH₂, CF₃, or C₁₋₆ alkyl; or two R^{a} groups on the same atom together form a C₃₋₄ cycloalkyl group.

4. The compound according to any one of claims 1-3, wherein each R^{a} is independently hydrogen, deuterium, halogen, oxo, CN, OH, NO₂, NH₂, CF₃, methyl, ethyl, n-propyl, or iso-propyl; or two R^{a} groups attached to the same atom form cyclopropyl or cyclobutyl.

5. The compound according to any one of claims 1-4, wherein ring B is:

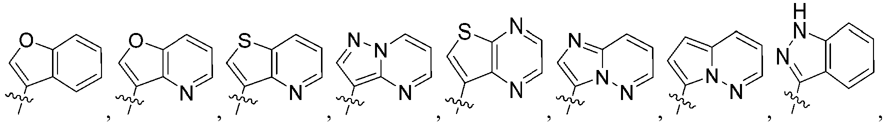

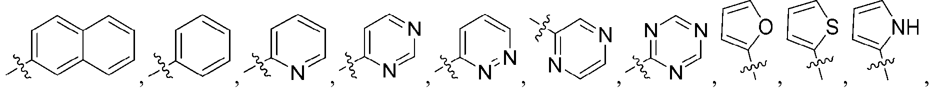

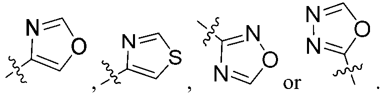

6. The compound according to any one of claims 1-5, wherein each R^{b} is independently hydrogen, deuterium, halogen, oxo, CN, OH, NO₂, NH₂, -COOH, C₁₋₆ alkyl, C₃₋₆ cycloalkyl, 4-6 membered heterocyclyl, or 7-8 membered bridged bicyclyl;wherein C₁₋₆ alkyl, C₃₋₆ cycloalkyl, 4-6 membered heterocyclyl, and 7-8 membered bridged bicyclyl are each independently optionally substituted with 1, 2, or 3 substituents independently selected from deuterium, halogen, OH, CN, oxo, C₁₋₃ alkyl, C₁₋₃ alkoxy, C₁₋₃ haloalkoxy, and C₁₋₃ haloalkyl.

7. The compound according to any one of claims 1-6, wherein each R^{b} is independently hydrogen, deuterium, halogen, CN, OH, NO₂, NH₂, -COOH, methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, or 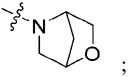 wherein methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, and 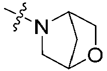 are each independently optionally substituted with 1, 2, or 3 substituents selected from hydrogen, deuterium, halogen, oxo, CN, OH, NO₂, NH₂, methyl, ethyl, methoxy, ethoxy, -CHF₂, -CF₃, -OCHF₂, and -OCF₃.

8. The compound according to any one of claims 1-7, wherein 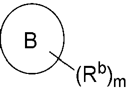 is: 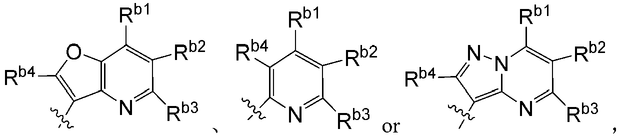 wherein:
R^{b1} and ^{Rb4} are each independently hydrogen, deuterium, halogen, CN,OH,NO₂,NH₂,CH₃,CH₂CH₃,-CF₂ or -CF₃;
R^{b2} and R^{b3} are each independently hydrogen, deuterium, halogen, CN, OH, NO₂, NH₂, -COOH, methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, or 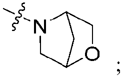 wherein methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, and 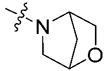 are each independently optionally substituted with 1, 2, or t3 substituents selected from hydrogen, deuterium, halogen, oxo, CN, OH, NO₂, NH₂, methyl, ethyl, methoxy, ethoxy, -CHF₂, -CF₃, -OCHF₂, and -OCF₃.

9. The compound according to any one of claims 1-5, wherein 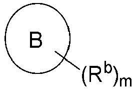 is: 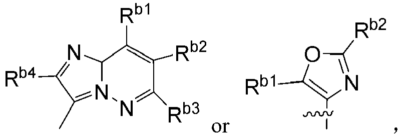 wherein:
R^{b1} and ^{Rb4} are each independently hydrogen, deuterium, halogen, CN,OH,NO₂,NH₂,CH₃,CH₂CH₃,-CF₂ or -CF₃;
R^{b2} and R^{b3} are each independently hydrogen, deuterium, halogen, CN, OH, NO₂, NH₂, -COOH, methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, or 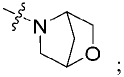 wherein methyl, ethyl, n-propyl, isopropyl, cyclopropyl, cyclobutyl, cyclopentyl, cyclohexyl, tetrahydrofuran-2-yl, dihydrofuran-2-yl, tetrahydropyran-2-yl, piperidin-1-yl, piperazin-1-yl, morpholin-4-yl, and 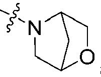 are each independently optionally substituted with 1, 2, or 3 substituents selected from hydrogen, deuterium, halogen, oxo, CN, OH, NO₂, NH₂, methyl, ethyl, methoxy, ethoxy, -CHF₂, -CF₃, -OCHF₂, and -OCF₃.

10. The compound according to any one of claims 1-9, wherein R¹ is hydrogen, deuterium, halogen, CN,OH,NO₂,NH₂, C₁₋₆ alkyl, C₁₋₆ alkoxy, or C₃₋₆ cycloalkyl; R² is hydrogen, deuterium, halogen, CN,O H,NO₂,NH₂, C₁₋₆ alkyl, C₁₋₆ alkoxy, or C₃₋₆ cycloalkyl.

11. The compound according to any one of claims 1-10, wherein R¹ is hydrogen, deuterium, halogen, CN,OH ,NO₂ NH₂, methyl, ethyl, n-propyl, isopropyl, methoxy, cyclopropyl, cyclobutyl, cyclopentyl, or cyclohexyl; R² is hydrogen, deuterium, halogen, CN ,OH, NO₂ ,NH₂, methyl, ethyl, n-propyl, isopro pyl, methoxy, cyclopropyl, cyclobutyl, cyclopentyl, or cyclohexyl.

12. The compound according to any one of claims 1-11, wherein R³ is hydrogen, deuterium, halogen, CN, OH, NO₂, NH₂, C₁₋₆ alkyl, C₁₋₄ alkoxy, C₃₋₆ cycloalkyl, or a 5-6 membered heterocyclyl group, and wherein the C₁₋₆ alkyl, C₁₋₄ alkoxy, C₃₋₆ cycloalkyl, and 5-6 membered heterocyclyl groups are ea ch independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, C₁₋₃ hydroxyalkyl, and C₁₋₃ haloalky 1.

13. The compound according to any one of claims 1-12, wherein R³ is hydrogen, deuterium, halogen, CN, OH, NO₂, NH₂, methyl, ethyl, isopropyl, methoxy, ethoxy, cyclopropyl, cyclobutyl, cyclopentyl, cyclohexyl, piperidinyl, piperazinyl, or morpholinyl, and wherein the methyl, ethyl, isopropyl, methoxy, ethoxy, cyclopropyl, cyclobutyl, cyclopentyl, cyclohexyl, piperidinyl, piperazinyl, and morpholinyl groups are each independently optionally substituted with 1, 2, or 3 substituents selected from deuterium, halogen, oxo, CN, OH, NO₂, NH₂, methyl, methoxy, -CH₂OH, -CHF₂, -CF₃, -CHFCH₂F, -CF₂CHF₂, -CH₂CF₃, -OCHF₂, and -OCF₃.

14. The compound according to any one of claims 1-12, wherein R³ is hydrogen, deuterium, halogen, CN, OH, NO₂, NH₂, methyl, ethyl, isopropyl, methoxy, ethoxy, cyclopropyl, cyclobutyl, cyclopentyl, cyclohexyl, piperidinyl, piperazinyl, or morpholinyl, and wherein the methyl, ethyl, isopropyl, methoxy, ethoxy, cyclopropyl, cyclobutyl, cyclopentyl, cyclohexyl, piperidinyl, piperazinyl, and morpholinyl groups are each independently optionally substituted with 1, 2, or 3 substituents selected from -CH₂CHF₂ and -C(CH₃)₂OH.

15. The compound according to any one of claims 1-14, wherein DIM moiety is: 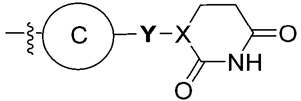 wherein:
X is CH or N;
Y is bond, -CH₂-, -NH-, -O-, -C(=O)-, or -C(=O)NH-;
ring C is phenyl, a 5- 6 membered monocyclic heteroaryl, or a 9- 10 membered bicyclic heteroaryl;
said Ring C is independently optionally substituted with 1, 2, or 3 substituents selected from deuterium, halogen, oxo, CN, OH, NO₂, NH₂, C₁₋₃ alkyl, C₁₋₃ alkoxy, C₁₋₃ haloalkoxy, and C₁₋₃ haloalkyl.

16. The compound according to any one of claims 1-15, wherein DIM moiety is:

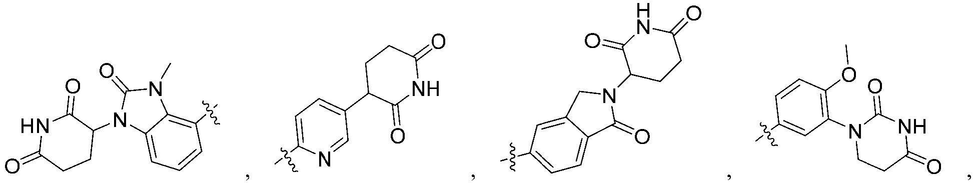

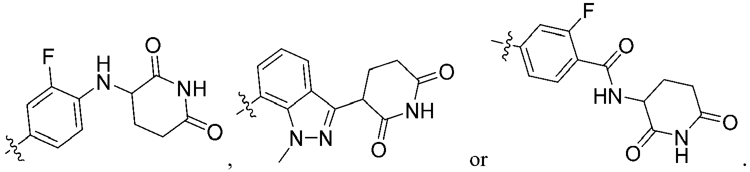

17. The compound according to any one of claims 1-15, wherein DIM moiety is:

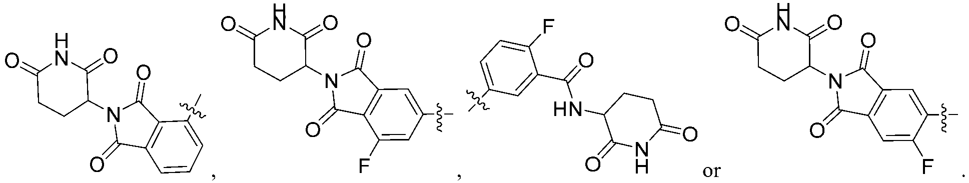

18. The compound according to any one of claims 1-15, wherein DIM moiety is: 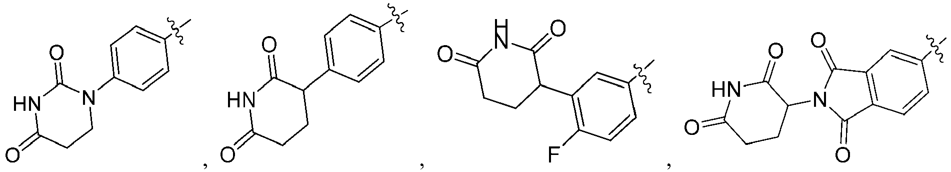 or

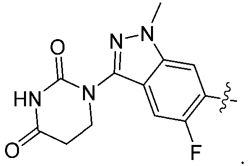

19. The compound according to any one of claims 1-15, wherein DIM moiety is

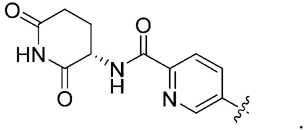

20. The compound according to any one of claims 1-15, wherein DIM moiety is

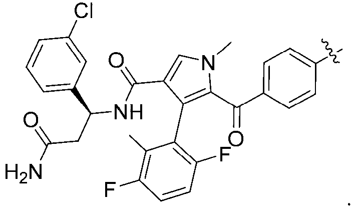

21. The compound according to any one of claims 1-20, wherein L is C₃₋₁₂ alkylene, wherein 1, 2, 3, 4 or 5 methylene units are each independently optionally replaced by a unit selected from -CR^{d}=CR^{d}-,-C≡C-,-C(R^{d})₂-,-Cy-,-O-,-C(=O)- and -N(R^{d})-.

22. The compound according to any one of claims 1-20, wherein L is C₃₋₉ alkylene, wherein 1, 2 or 3 methylene units are each independently optionally replaced by a unit selected from -CR^{d}=CR^{d}-,,-C(R^{d})₂-,-O-,-C(=O)-,-NH-, 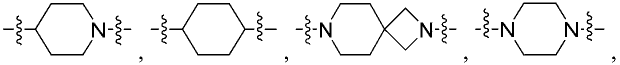 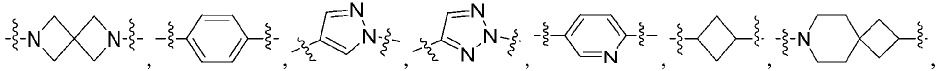 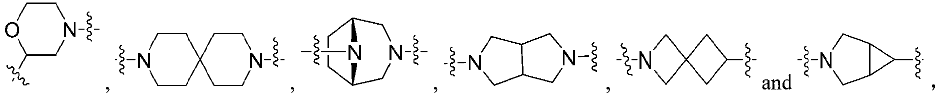 wherein 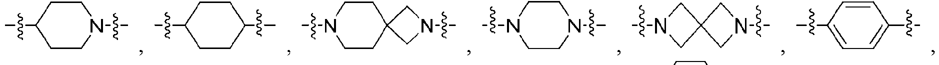 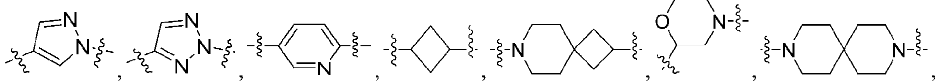 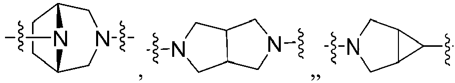 are independently optionally substituted with 1, 2, or 3 substituents selected from deuterium, halogen, CN, OH, NO₂, NH₂, oxo, methyl, ethyl, isopropyl, methoxy, isopropoxy, -CHF₂, -CF ₃, -CHFCH₂F, -CF₂CHF₂, -CH₂CF₃, -OCHF₂, and -OCF₃;
Each R^{d} is independently hydrogen, deuterium, halogen, CN, OH, NO₂, NH₂, methyl, ethyl, n-propyl, or isopropyl.

23. The compound according to any one of claims 1-22, wherein L is: 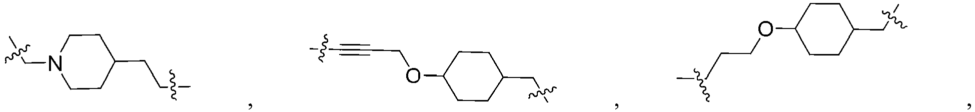 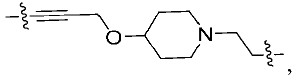 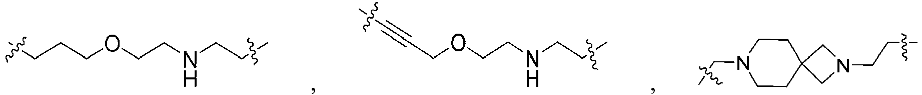 or

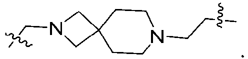

24. The compound according to any one of claims 1-20, wherein L is C₁₋₉ alkylene, wherein 1, 2, 3 or 4 methylene units are each independently optionally replaced by a unit selected from -CR^{d}=CR^{d}-,-C≡C-,-C(R^{d})₂-,-O-,-C(=O)-,-NH-, 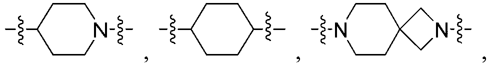 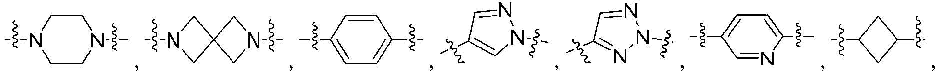 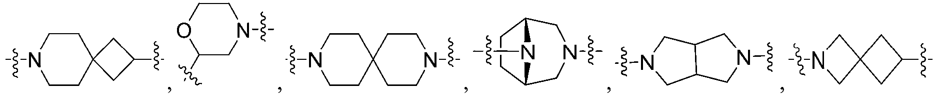 and 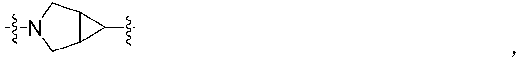 wherein 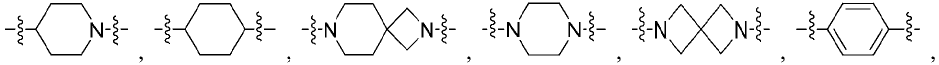 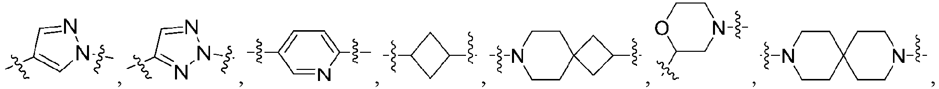 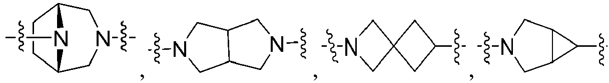 are independently optionally substituted with 1, 2, or 3 substituents selected from deuterium, halogen, CN, OH, NO₂, NH₂, oxo, methyl, ethyl, isopropyl, methoxy, isopropoxy, -CHF₂, -CF ₃, -CHFCH₂F, -CF₂CHF₂, -CH₂CF₃, -OCHF₂, and -OCF₃;
each R^{d} is independently hydrogen, deuterium, halogen, CN, OH, NO₂, NH₂, methyl, ethyl, n-propyl, or isopropyl.

25. The compound according to any one of claims 1-20, 24, wherein L is:

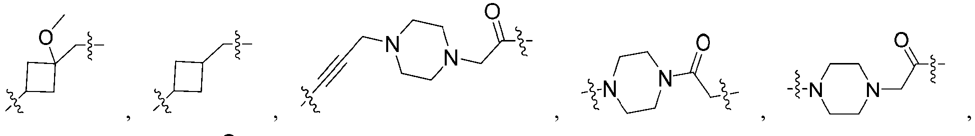

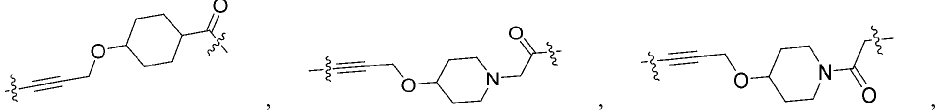

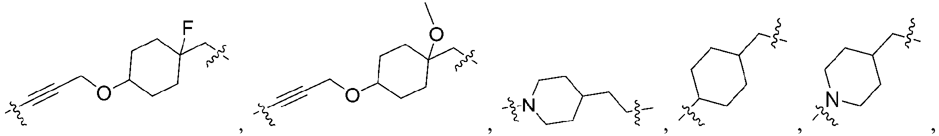

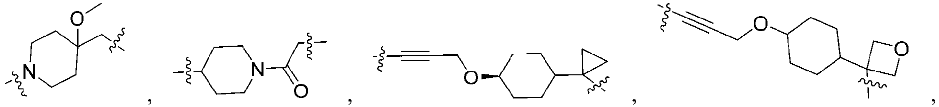

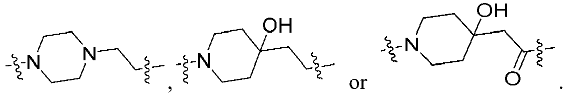

26. The compound according to any one of claims 1-25, wherein a structure represented by Formula (II), (III), (IV), (V), or (VI): 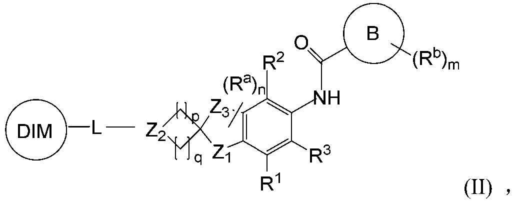 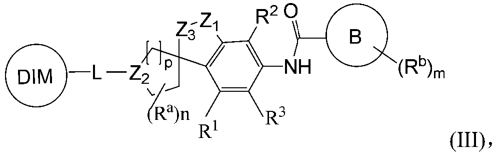 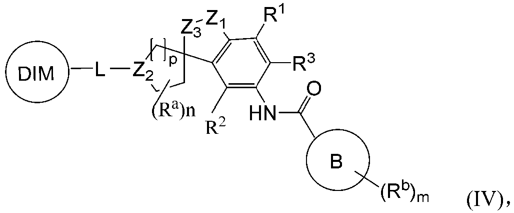 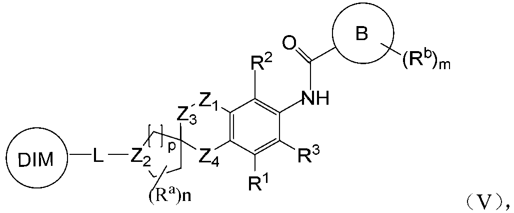 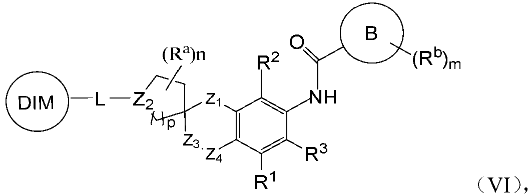 wherein:
Z₁, Z₃, and Z₄ are each independently CH₂, -C(=O)-, NH, O, or S;
Z₂ is CH or N;
p and q are each independently 1 or 2.

27. A compound which is having one of the following structures, and an isomer, an N-oxide, a hydrate, a solvate, a metabolite, a pharmaceutically acceptable salt or a prodrug thereof: 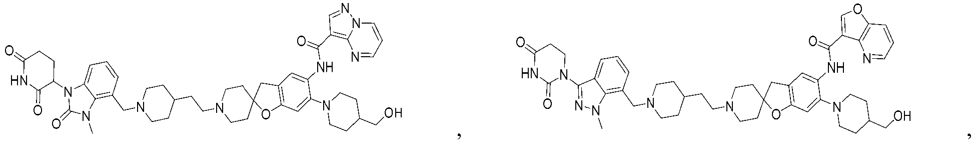 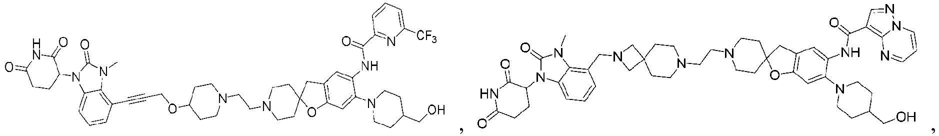 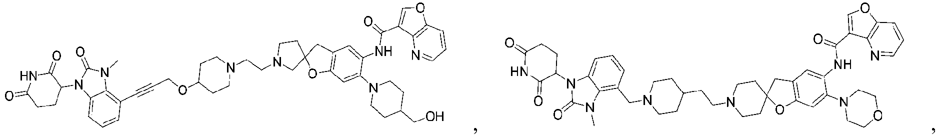 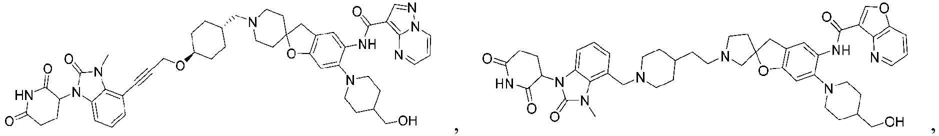 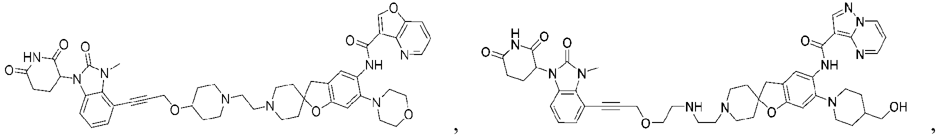 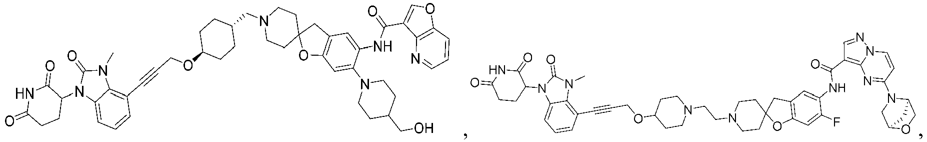 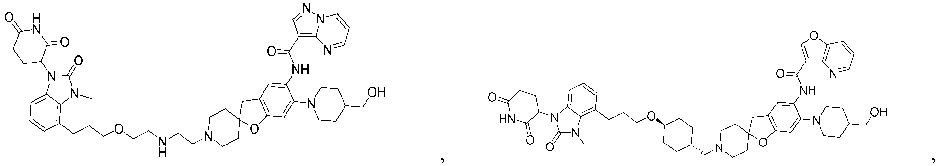 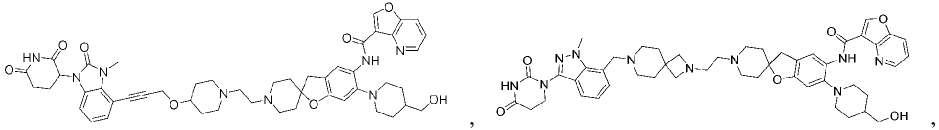 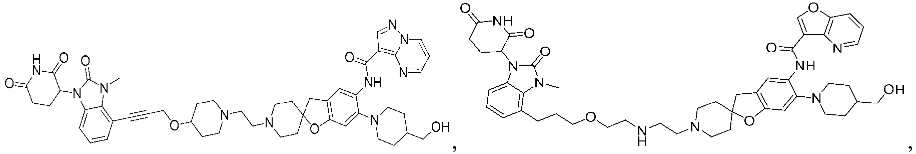 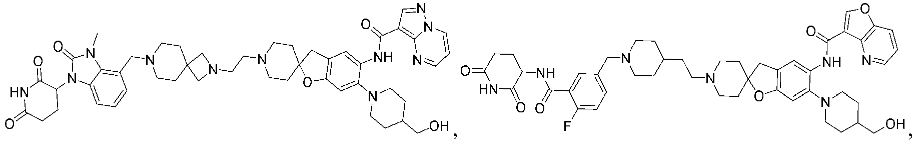 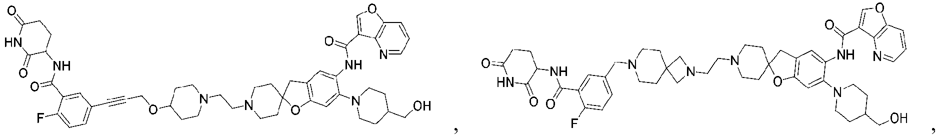 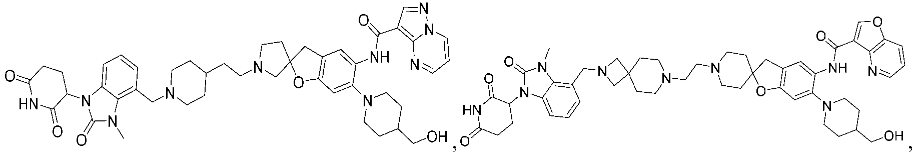 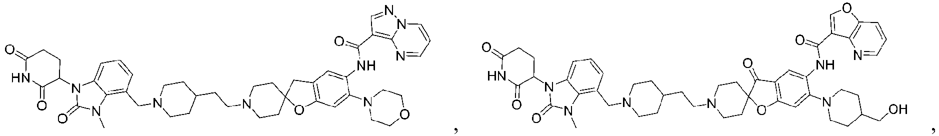 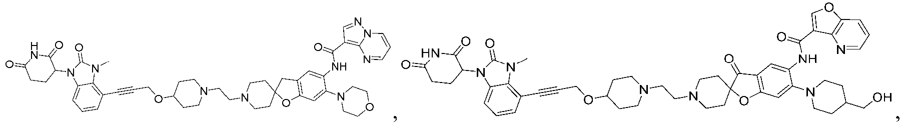 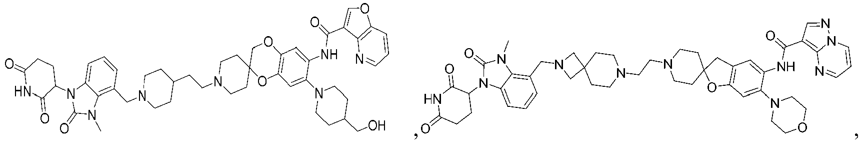 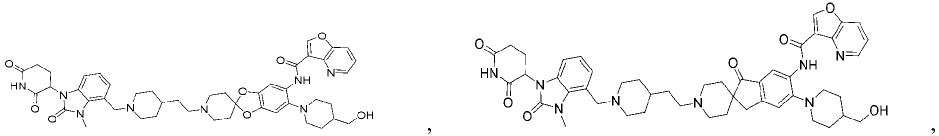 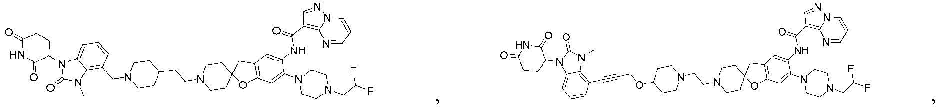 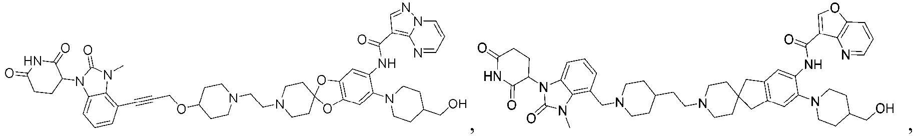 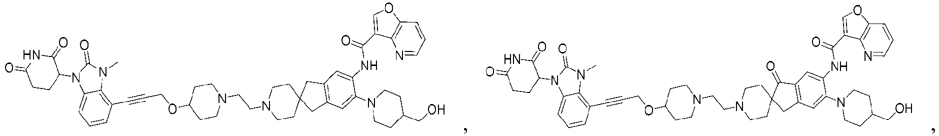 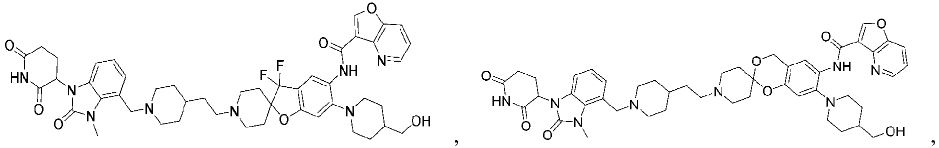 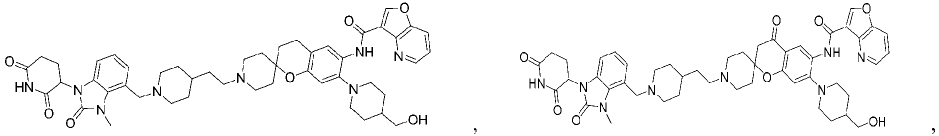 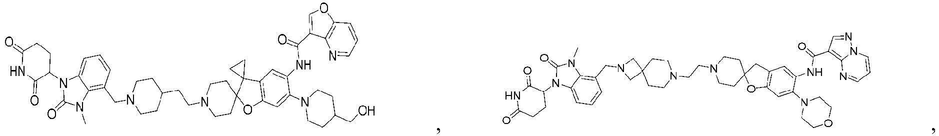 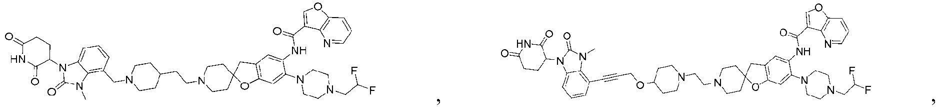 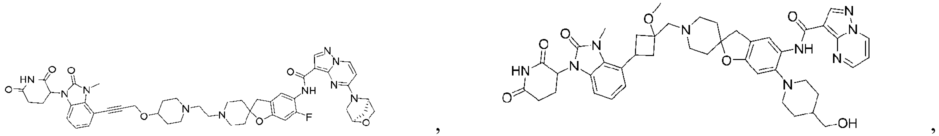 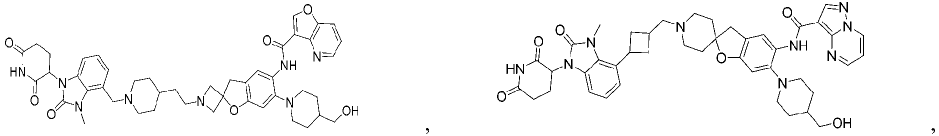 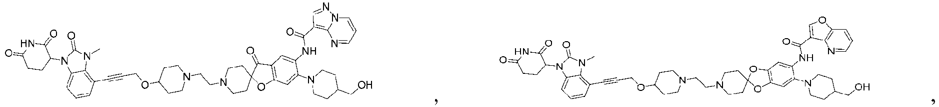 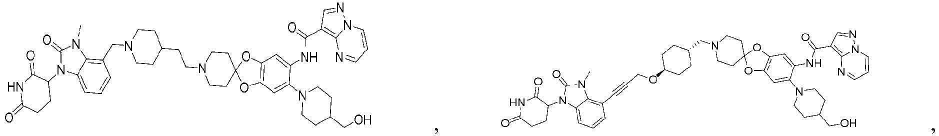 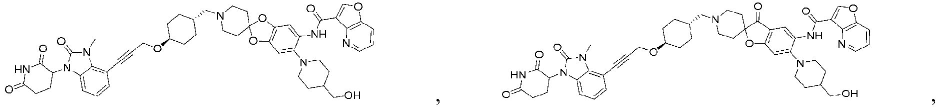 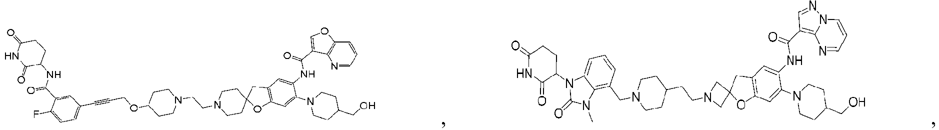 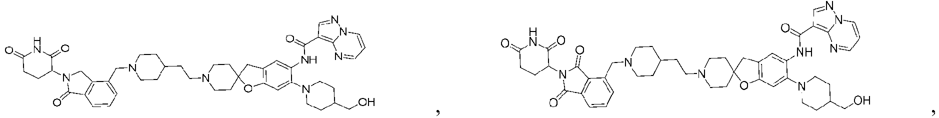 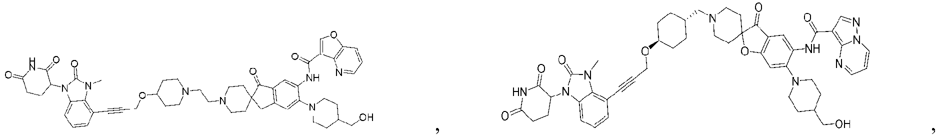 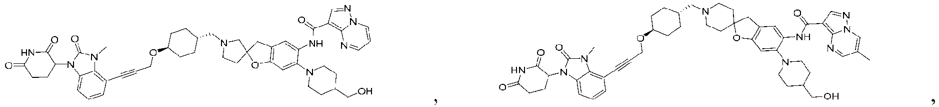 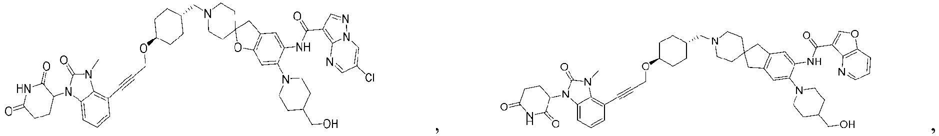 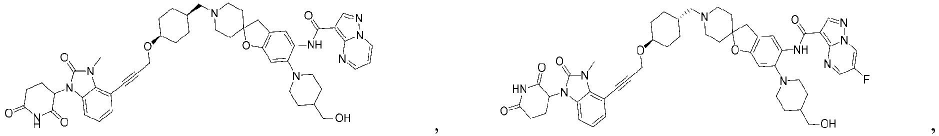 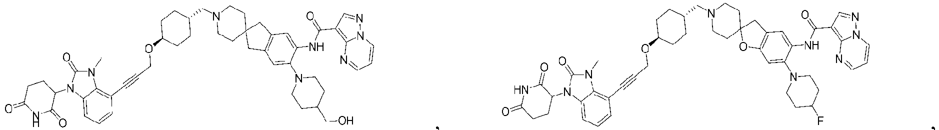 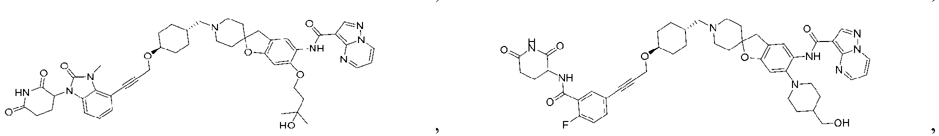 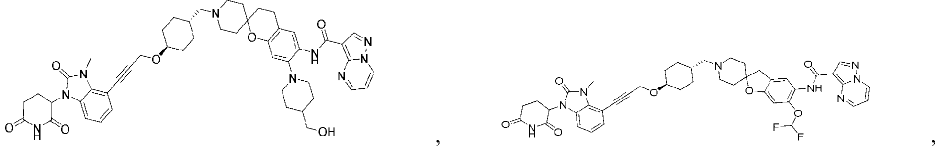 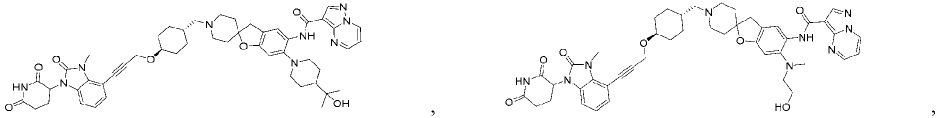 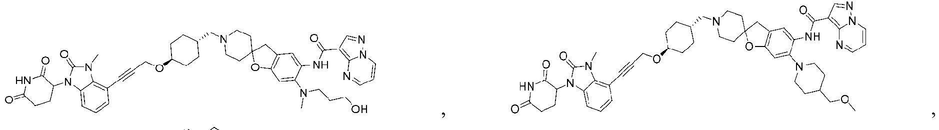 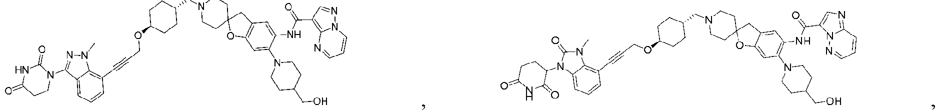 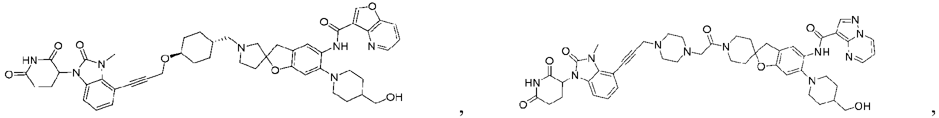 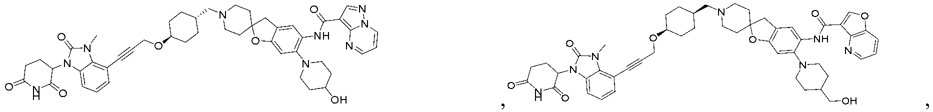 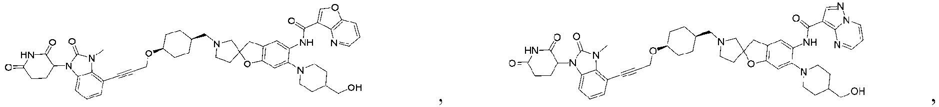 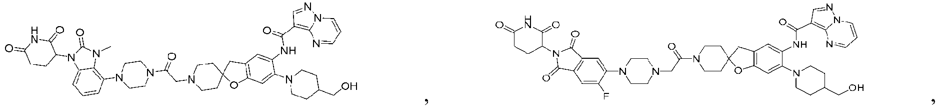 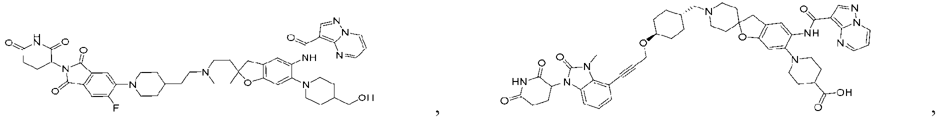 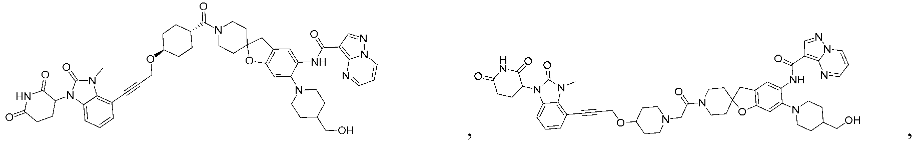 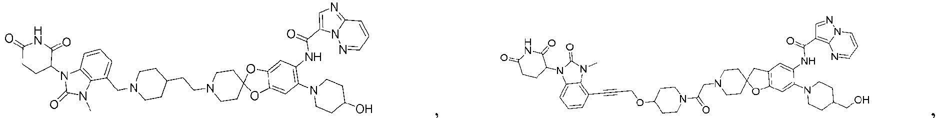 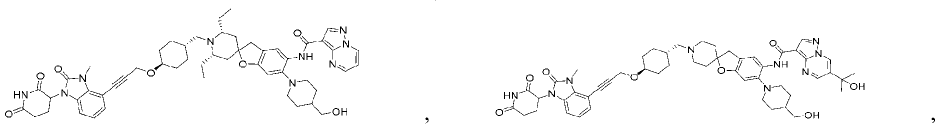 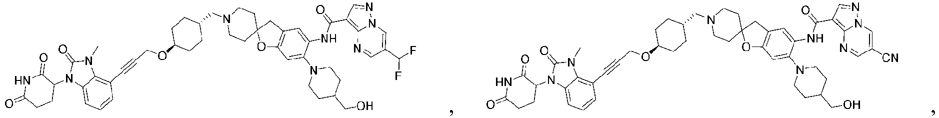 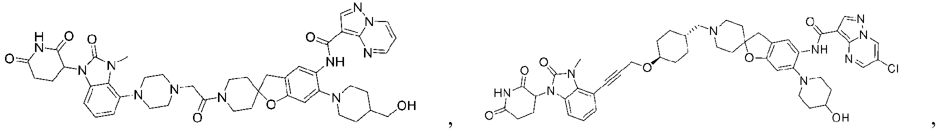 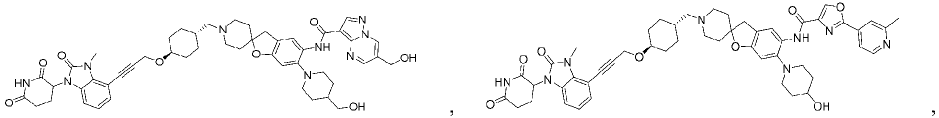 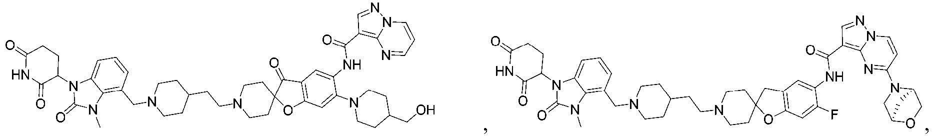 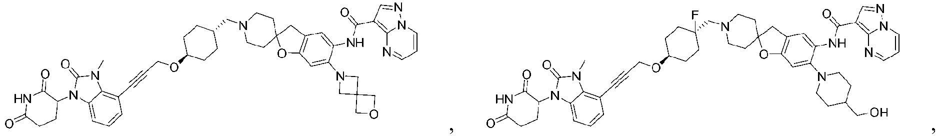 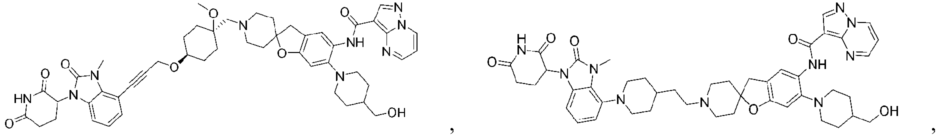 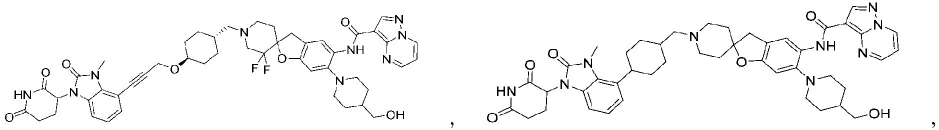 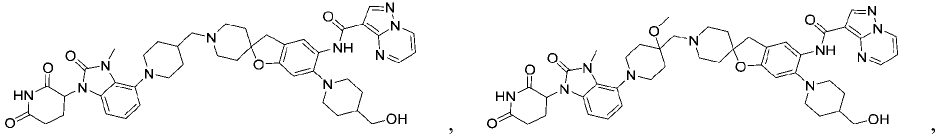 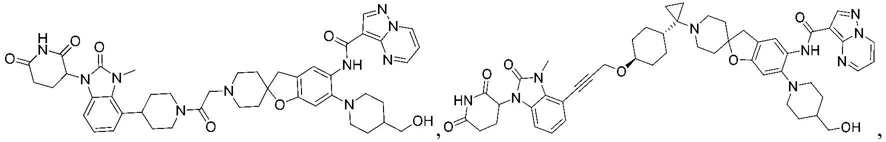 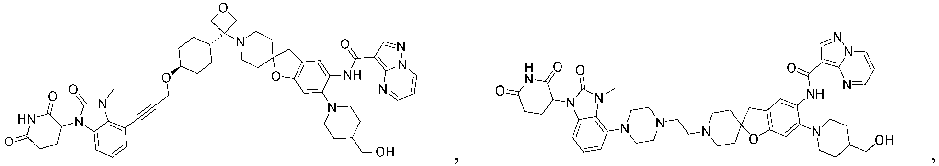 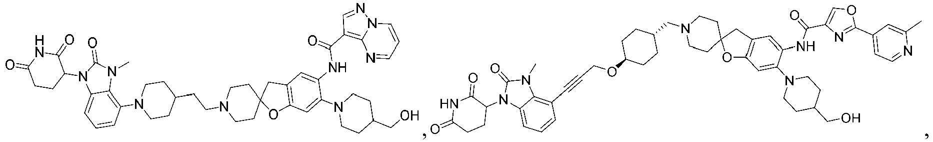 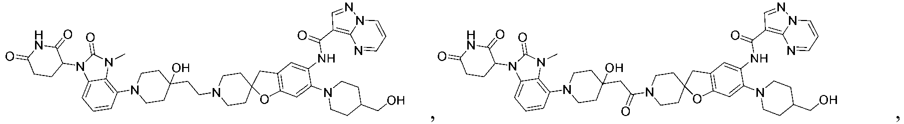 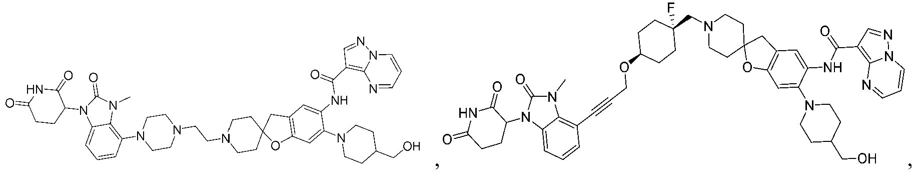 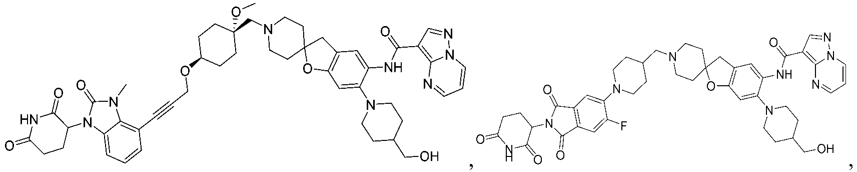 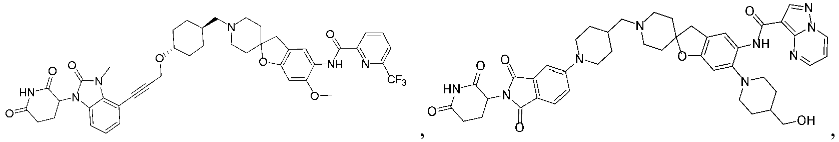 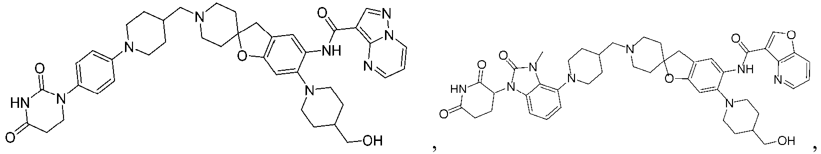 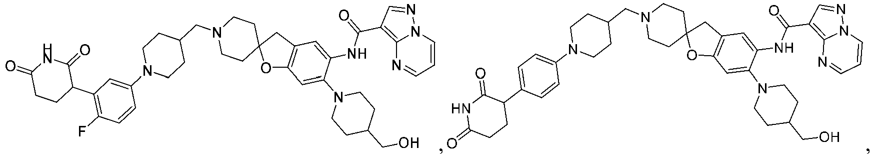 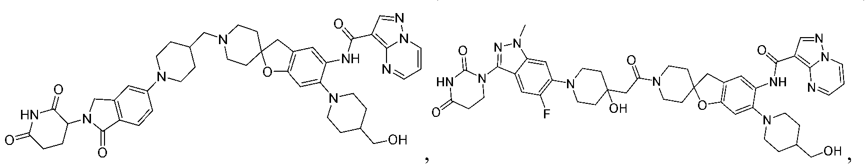 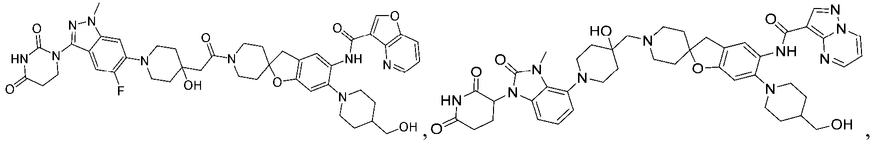 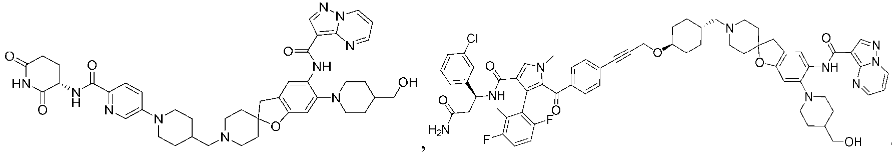 or

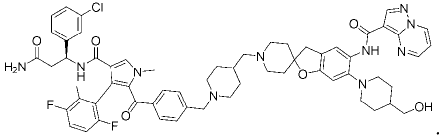

28. A pharmaceutical composition comprising a compound according to any one of claims 1-27, wherein pharmaceutical composition optionally further comprises a pharmaceutically acceptable excipient, carrier, adjuvant, or any combination thereof.

29. Use of the compound according to any one of claims 1-27 or the pharmaceutical composition according to claim 28 in the preparation of a medicament for preventing, treating, or alleviating IRAK4-related diseases in patients.

30. The use according to claim 29, wherein the disease is selected from inflammatory diseases, infections such as viruses, bacteria, fungi, and parasitic infections HIV-1 infection, sepsis, autoimmune disorders or diseases such as rheumatoid arthritis and multiple sclerosis, gout, juvenile idiopathic arthritis, Muckle Wells disease, familial Mediterranean fever, Behcet's disease, adult Still's disease, proliferative diseases such as cancer, hyperplasia, restenosis, cardiac hypertrophy, leukemia, intravascular coagulation, bone disease, metabolic diseases, neurological and neurodegenerative diseases, cardiovascular diseases, fibrosis and allergic diseases, asthma, atopic dermatitis, suppurative sweat gland inflammation, Alzheimer's disease, hormone related diseases, external injuries, hemodialysis, ischemic diseases, non infectious hepatitis, ultraviolet radiation, closed head injury, pancreatitis, periodontitis, graft-versus-host disease and/or transplant rejection.
